(19) Europäisches Patentamt
European Patent Office
Office européen des brevets

(11) EP 4 410 308 A1

(12) EUROPEAN PATENT APPLICATION
published in accordance with Art. 153(4) EPC

(43) Date of publication:
**07.08.2024 Bulletin 2024/32**

(21) Application number: **22876287.8**

(22) Date of filing: **28.09.2022**

(51) International Patent Classification (IPC):
***A61K 39/395*** *(2006.01)* ***A61P 35/00*** *(2006.01)*
***A61P 43/00*** *(2006.01)*

(52) Cooperative Patent Classification (CPC):
**A61K 39/00; A61K 39/395; A61K 39/461; A61K 39/464402; A61P 35/00; A61P 35/04; A61P 43/00; C07K 16/28**

(86) International application number:
**PCT/JP2022/036060**

(87) International publication number:
**WO 2023/054421 (06.04.2023 Gazette 2023/14)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **29.09.2021 PCT/JP2021/035917**

(71) Applicant: **CHUGAI SEIYAKU KABUSHIKI KAISHA**
**Tokyo 115-8543 (JP)**

(72) Inventors:
- **ISHII, Shinya**
**Kamakura-shi, Kanagawa 247-8530 (JP)**
- **KAMIKAWA, Takayuki**
**Kamakura-shi, Kanagawa 247-8530 (JP)**
- **KIMURA, Naoki**
**Kamakura-shi, Kanagawa 247-8530 (JP)**
- **KODAMA, Tatsushi**
**Tokyo 103-8324 (JP)**

(74) Representative: **Vossius & Partner Patentanwälte Rechtsanwälte mbB**
**Siebertstrasse 3**
**81675 München (DE)**

Remarks:
The complete document including Reference Table(s) and the Sequence Listing(s) can be downloaded from the EPO website

# (54) CYTOTOXICITY-INDUCING THERAPEUTIC AGENT FOR USE IN TREATMENT OF CANCER

(57) The present disclosure provides anticancer agents containing a multispecific antigen-binding molecule that can efficiently and specifically recruit T cells to the target cancer cells, particularly CLDN6-expressing cancer cells and such, and can treat cancer through the cytotoxic activity of T cells against target cancer tissues containing CLDN6-expressing cells; combination therapies using the anticancer agent and at least one other anticancer agent; and pharmaceutical compositions for use in the combination therapies.



Processed by Luminess, 75001 PARIS (FR)

(Cont. next page)

[Fig.1]

CLDN6 expression
TPM (log10)
10000
1000
100
10
1
0.1
Cholangiocarcinoma
Adrenocortical carcinoma
Acute myeloid leukemia
Cervical cancer
Breast cancer
Glioma
Glioblastoma
Sarcoma
Colon cancer
Liver cancer
Kidney cancer
Esophageal cancer
Mesothelioma
Pancreas cancer
Ovary cancer
DLBCL
NSCLC (Ad)
NSCLC (Sq)
Paraganglioma
Prostate cancer
Rectum cancer
Skin cancer
Germ cell tumor
Gastric cancer
Thyroid cancer
Thymic carcinoma
Upper aerodigestive tract cancer
Urinary bladder cancer
Uterine cancer
Uveal melanoma

## Description

[Technical Field]

The present disclosure relates to anticancer agents comprising a multispecific antigen-binding molecule targeting claudin 6, and combination therapies with at least one other anticancer agent.

[Background Art]

Claudin family is the family of cell membrane proteins of approximately 23 kD in molecular weight which have four transmembrane domains and constitute tight junctions. The Claudin family includes 24 members in humans and mice, and each member of the Claudin family is known to exhibit a very unique expression pattern depending on each epithelial cell type (NPL 1 to NPL 4). In the sheet of epithelial cells, a mechanism works to prevent substances from leaking (diffusing) in the intercellular spaces, and cell-cell adhesion systems called tight junctions have been shown to really play a central role as a "barrier" in the mechanism to prevent leakage.
A tight junction molecule Claudin 6 (CLDN6), a member of Claudin family proteins, shows transcriptionally silent expression in normal adult tissues (NPL 5 and NPL 6), while showing up-regulation in several kind of cancers such as ovarian cancer, NSCLC, and gastric cancers (NPL 7 to NPL 9).
Regarding anti-CLDN6 antibodies, monospecific antibodies against CLDN6 have been reported to have ADCC activity or internalization activity against CLDN6 positive cancer lines (PTL 1 to PTL 5). So far, CLDN6 targeting T cell-redirecting bispecific antibodies, named as 6PHU3, has been engineered using bispecific $sc(Fv)_2$ format with anti-CD3/anti-CLDN6 specificities (PTL 6 to PTL7). In preclinical evaluation, 6PHU3 has been reported to show a potent killing of cancer cells in vitro and in vivo (NPL 10).

[Citation List]

[Patent Literature]

[PTL 1] WO2009/087978
[PTL 2] WO2011/057788
[PTL 3] WO2012/003956
[PTL 4] WO2012/156018
[PTL 5] WO2015/069794
[PTL 6] WO2014/075697
[PTL 7] WO2014/075788

[Non Patent Literature]

[NPL 1] Furuse and Tsukita, TRENDS in Cell Biology 2006, 16: 181
[NPL 2] Wilcox, et al., Cell 2001, 104: 165
[NPL 3] Rahner, et al., GASTROENTEROLOGY 2001, 120: 411
[NPL 4] Morita, et al., Proc. Natl. Acad. Sci. USA 1999, 96: 511
[NPL 5] Dev Dyn. 2004 Oct;231(2):425-31.
[NPL 6] Am J Physiol Renal Physiol. 2006 Dec;291(6):F1132-41.
[NPL 7] Int J Cancer. 2014 Nov 1;135(9):2206-14.
[NPL 8] Histopathology. 2012 Dec;61(6):1043-56.
[NPL 9] J Gastrointest Cancer. 2010 Mar;41(1):52-9.
[NPL 10] Oncoimmunology. 2015 Oct 29;5(3):e1091555.

[Summary of Invention]

[Technical Problem]

An objective of the present disclosure is to provide anticancer agents comprising as an active ingredient a multispecific antigen-binding molecule that can efficiently and specifically recruit T cells to the target cancer cells, particularly CLDN6-expressing cancer cells and such, and can treat cancer through the cytotoxic activity of T cells against target cancer tissues containing CLDN6-expressing cells. The present invention further aims at providing combination therapies that use the multispecific antigen-binding molecule with another pharmaceutical agent.

[Solution to Problem]

The present inventors found multispecific antigen-binding molecules that comprise a first antigen-binding moiety that is capable of binding to CD3 and CD137 (4-1BB) and that binds to either CD3 or CD137 (i.e., that has dual affinity to CD3 and CD137 but do not bind to the both simultaneously), and a second antigen-binding moiety that is capable of binding to a molecule expressed specifically in cancer tissues, particularly claudin 6 (CLDN6). The present inventors revealed that the multispecific antigen-binding molecules of the present invention damage cancer cells including CLDN6-expressing cancer cells. The present invention provides anticancer agents comprising the multispecific antigen-binding molecule as an active ingredient, combination therapies that use the multispecific antigen-binding molecule with at least one other anticancer agent, and pharmaceutical compositions comprising a combination of the multispecific antigen-binding molecule and an anticancer agent.

More specifically, the present disclosure provides the following:

(A-1) An anticancer agent comprising as an active ingredient a multispecific antigen-binding molecule, the multispecific antigen-binding molecule comprising (i) a first antigen-binding moiety that is capable of binding to CD3 and CD137 and that binds to either CD3 or CD137, and (ii) a second antigen-binding moiety that is capable of binding to claudin 6 (CLDN6);

(A-2) An anticancer agent comprising as an active ingredient a multispecific antigen-binding molecule of any of (1) to (6) below:

(1) a multispecific antigen-binding molecule that comprises a first antibody variable region comprising the complementarity determining region (CDR) 1 of SEQ ID NO: 11, the CDR2 of SEQ ID NO: 17, and the CDR3 of SEQ ID NO: 23; a second antibody variable region comprising the CDR1 of SEQ ID NO: 32, the CDR2 of SEQ ID NO: 36, and the CDR3 of SEQ ID NO: 40; a third antibody variable region comprising the complementarity determining region (CDR) 1 of SEQ ID NO: 7, the CDR2 of SEQ ID NO: 13, and the CDR3 of SEQ ID NO: 19; and a fourth antibody variable region comprising the CDR1 of SEQ ID NO: 29, the CDR2 of SEQ ID NO: 33, and the CDR3 of SEQ ID NO: 37;

(2) a multispecific antigen-binding molecule that comprises a first antibody variable region comprising the complementarity determining region (CDR) 1 of SEQ ID NO: 9, the CDR2 of SEQ ID NO: 15, and the CDR3 of SEQ ID NO: 21; a second antibody variable region comprising the CDR1 of SEQ ID NO: 31, the CDR2 of SEQ ID NO: 35, and the CDR3 of SEQ ID NO: 39; a third antibody variable region comprising the complementarity determining region (CDR) 1 of SEQ ID NO: 8, the CDR2 of SEQ ID NO: 14, and the CDR3 of SEQ ID NO: 20; and a fourth antibody variable region comprising the CDR1 of SEQ ID NO: 30, the CDR2 of SEQ ID NO: 34, and the CDR3 of SEQ ID NO: 38;

(3) a multispecific antigen-binding molecule that comprises a first antibody variable region comprising the complementarity determining region (CDR) 1 of SEQ ID NO: 10, the CDR2 of SEQ ID NO: 16, and the CDR3 of SEQ ID NO: 22; a second antibody variable region comprising the CDR1 of SEQ ID NO: 31, the CDR2 of SEQ ID NO: 35, and the CDR3 of SEQ ID NO: 39; a third antibody variable region comprising the complementarity determining region (CDR) 1 of SEQ ID NO: 8, the CDR2 of SEQ ID NO: 14, and the CDR3 of SEQ ID NO: 20; and a fourth antibody variable region comprising the CDR1 of SEQ ID NO: 30, the CDR2 of SEQ ID NO: 34, and the CDR3 of SEQ ID NO: 38;

(4) a multispecific antigen-binding molecule that comprises a first antibody variable region comprising the complementarity determining region (CDR) 1 of SEQ ID NO: 12, the CDR2 of SEQ ID NO: 18, and the CDR3 of SEQ ID NO: 24; a second antibody variable region comprising the CDR1 of SEQ ID NO: 32, the CDR2 of SEQ ID NO: 36, and the CDR3 of SEQ ID NO: 40; a third antibody variable region comprising the complementarity determining region (CDR) 1 of SEQ ID NO: 7, the CDR2 of SEQ ID NO: 13, and the CDR3 of SEQ ID NO: 19; and a fourth antibody variable region comprising the CDR1 of SEQ ID NO: 29, the CDR2 of SEQ ID NO: 33, and the CDR3 of SEQ ID NO: 37;

(5) a multispecific antigen-binding molecule that comprises a first antibody variable region comprising the complementarity determining region (CDR) 1 of SEQ ID NO: 11, the CDR2 of SEQ ID NO: 17, and the CDR3 of SEQ ID NO: 23; a second antibody variable region comprising the CDR1 of SEQ ID NO: 32, the CDR2 of SEQ ID NO: 36, and the CDR3 of SEQ ID NO: 40; a third antibody variable region comprising the complementarity determining region (CDR) 1 of SEQ ID NO: 29, the CDR2 of SEQ ID NO: 33, and the CDR3 of SEQ ID NO: 37;

and a fourth antibody variable region comprising the CDR1 of SEQ ID NO: 7, the CDR2 of SEQ ID NO: 13, and the CDR3 of SEQ ID NO: 19; and

(6) a multispecific antigen-binding molecule that comprises a first antibody variable region comprising the complementarity determining region (CDR) 1 of SEQ ID NO: 12, the CDR2 of SEQ ID NO: 18, and the CDR3 of SEQ ID NO: 24; a second antibody variable region comprising the CDR1 of SEQ ID NO: 32, the CDR2 of SEQ ID NO: 36, and the CDR3 of SEQ ID NO: 40; a third antibody variable region comprising the complementarity determining region (CDR) 1 of SEQ ID NO: 29, the CDR2 of SEQ ID NO: 33, and the CDR3 of SEQ ID NO: 37; and a fourth antibody variable region comprising the CDR1 of SEQ ID NO: 7, the CDR2 of SEQ ID NO: 13, and the CDR3 of SEQ ID NO: 19;

(A-3) The anticancer agent of (A-2), wherein at least one selected from the group consisting of the first antibody variable region, the second antibody variable region, the third antibody variable region, and the fourth antibody variable region, comprises a human antibody framework or a humanized antibody framework;

(A-4) An anticancer agent comprising as an active ingredient a multispecific antigen-binding molecule of any of (I) to (VI) below:

(I) a multispecific antigen-binding molecule that comprises a first antibody variable region comprising the amino acid sequence of SEQ ID NO: 5; a second antibody variable region comprising the amino acid sequence of SEQ ID NO: 28; a third antibody variable region comprising the amino acid sequence of SEQ ID NO: 1; and a fourth antibody variable region comprising the amino acid sequence of SEQ ID NO: 25;

(II) a multispecific antigen-binding molecule that comprises a first antibody variable region comprising the amino acid sequence of SEQ ID NO: 3; a second antibody variable region comprising the amino acid sequence of SEQ ID NO: 27; a third antibody variable region comprising the amino acid sequence of SEQ ID NO: 2; and a fourth antibody variable region comprising the amino acid sequence of SEQ ID NO: 26;

(III) a multispecific antigen-binding molecule that comprises a first antibody variable region comprising the amino acid sequence of SEQ ID NO: 4; a second antibody variable region comprising the amino acid sequence of SEQ ID NO: 27; a third antibody variable region comprising the amino acid sequence of SEQ ID NO: 2; and a fourth antibody variable region comprising the amino acid sequence of SEQ ID NO: 26;

(IV) a multispecific antigen-binding molecule that comprises a first antibody variable region comprising the amino acid sequence of SEQ ID NO: 6; a second antibody variable region comprising the amino acid sequence of SEQ ID NO: 28; a third antibody variable region comprising the amino acid sequence of SEQ ID NO: 1; and a fourth antibody variable region comprising the amino acid sequence of SEQ ID NO: 25;

(V) a multispecific antigen-binding molecule that comprises a first antibody variable region comprising the amino acid sequence of SEQ ID NO: 5; a second antibody variable region comprising the amino acid sequence of SEQ ID NO: 28; a third antibody variable region comprising the amino acid sequence of SEQ ID NO: 25; and a fourth antibody variable region comprising the amino acid sequence of SEQ ID NO: 1; and

(VI) a multispecific antigen-binding molecule that comprises a first antibody variable region comprising the amino acid sequence of SEQ ID NO: 6; a second antibody variable region comprising the amino acid sequence of SEQ ID NO: 28; a third antibody variable region comprising the amino acid sequence of SEQ ID NO: 25; and a fourth antibody variable region comprising the amino acid sequence of SEQ ID NO: 1;

(A-5) The anticancer agent of any one of (A-2) to (A-4), wherein the first antibody variable region and the second antibody variable constitute a first antigen-binding moiety that is capable of binding to CD3 and CD137 and that binds to either CD3 or CD137, and the third antibody variable region and the fourth antibody variable region constitute a second antigen-binding moiety that is capable of binding to CLDN6;

(A-6) The anticancer agent of any one of (A-2) to (A-4), wherein the first antibody variable region and the second antibody variable constitute a first antigen-binding moiety that binds to CD3, and the third antibody variable region and the fourth antibody variable region constitute a second antigen-binding moiety that is capable of binding to CLDN6;

(A-7) The anticancer agent of any one of (A-2) to (A-4), wherein the first antibody variable region and the second antibody variable region constitute a first antigen-binding moiety that binds to CD137, and the third antibody variable region and the fourth antibody variable region constitute a second antigen-binding moiety that is capable of binding to CLDN6;

(A-8) An anticancer agent comprising as an active ingredient a multispecific antigen-binding molecule that comprises (i) a first antigen-binding moiety that binds to CD3 and (ii) a second antigen-binding moiety that binds to claudin 6 (CLDN6), wherein the first antigen-binding moiety comprises any one of (a1) to (a4) below:

(a1) a first antibody variable region comprising the complementarity-determining region (CDR) 1 of SEQ ID NO:

9, the CDR2 of SEQ ID NO: 15, and the CDR3 of SEQ ID NO: 21, and a second antibody variable region comprising the CDR1 of SEQ ID NO: 31, the CDR2 of SEQ ID NO: 35, and the CDR3 of SEQ ID NO: 39;
(a2) a first antibody variable region comprising the complementarity-determining region (CDR) 1 of SEQ ID NO: 10, the CDR2 of SEQ ID NO: 16, and the CDR3 of SEQ ID NO: 22, and a second antibody variable region comprising the CDR1 of SEQ ID NO: 31, the CDR2 of SEQ ID NO: 35, and the CDR3 of SEQ ID NO: 39;
(a3) a first antibody variable region comprising the complementarity-determining region (CDR) 1 of SEQ ID NO: 11, the CDR2 of SEQ ID NO: 17, and the CDR3 of SEQ ID NO: 23, and a second antibody variable region comprising the CDR1 of SEQ ID NO: 32, the CDR2 of SEQ ID NO: 36, and the CDR3 of SEQ ID NO: 40; and
(a4) a first antibody variable region comprising the complementarity-determining region (CDR) 1 of SEQ ID NO: 12, the CDR2 of SEQ ID NO: 18, and the CDR3 of SEQ ID NO: 24, and a second antibody variable region comprising the CDR1 of SEQ ID NO: 32, the CDR2 of SEQ ID NO: 36, and the CDR3 of SEQ ID NO: 40;

(A-9) The anticancer agent of (A-8), wherein the second antigen-binding moiety comprises any one of (b1) to (b3) below:

(b1) a third antibody variable region comprising the complementarity-determining region (CDR) 1 of SEQ ID NO: 8, the CDR2 of SEQ ID NO: 14, and the CDR3 of SEQ ID NO: 20, and a fourth antibody variable region comprising the CDR1 of SEQ ID NO: 30, the CDR2 of SEQ ID NO: 34, and the CDR3 of SEQ ID NO: 38;
(b2) a third antibody variable region comprising the complementarity-determining region (CDR) 1 of SEQ ID NO: 7, the CDR2 of SEQ ID NO: 13, and the CDR3 of SEQ ID NO: 19, and a fourth antibody variable region comprising the CDR1 of SEQ ID NO: 29, the CDR2 of SEQ ID NO: 33, and the CDR3 of SEQ ID NO: 37; and
(b3) a third antibody variable region comprising the complementarity-determining region (CDR) 1 of SEQ ID NO: 29, the CDR2 of SEQ ID NO: 33, and the CDR3 of SEQ ID NO: 37, and a fourth antibody variable region comprising the CDR1 of SEQ ID NO: 7, the CDR2 of SEQ ID NO: 13, and the CDR3 of SEQ ID NO: 19;

(A-10) An anticancer agent comprising as an active ingredient a multispecific antigen-binding molecule that comprises (i) a first antigen-binding moiety that binds to CD3 and (ii) a second antigen-binding moiety that binds to claudin 6 (CLDN6), wherein the second antigen-binding moiety comprises any one of (b1) to (b3) below:

(b1) a third antibody variable region comprising the complementarity-determining region (CDR) 1 of SEQ ID NO: 8, the CDR2 of SEQ ID NO: 14, and the CDR3 of SEQ ID NO: 20, and a fourth antibody variable region comprising the CDR1 of SEQ ID NO: 30, the CDR2 of SEQ ID NO: 34, and the CDR3 of SEQ ID NO: 38;
(b2) a third antibody variable region comprising the complementarity-determining region (CDR) 1 of SEQ ID NO: 7, the CDR2 of SEQ ID NO: 13, and the CDR3 of SEQ ID NO: 19, and a fourth antibody variable region comprising the CDR1 of SEQ ID NO: 29, the CDR2 of SEQ ID NO: 33, and the CDR3 of SEQ ID NO: 37; and
(b3) a third antibody variable region comprising the complementarity-determining region (CDR) 1 of SEQ ID NO: 29, the CDR2 of SEQ ID NO: 33, and the CDR3 of SEQ ID NO: 37, and a fourth antibody variable region comprising the CDR1 of SEQ ID NO: 7, the CDR2 of SEQ ID NO: 13, and the CDR3 of SEQ ID NO: 19;

(A-11) The anticancer agent of any one of (A-8) to (A-10), wherein at least one selected from the group consisting of the first antibody variable region, the second antibody variable region, the third antibody variable region, and the fourth antibody variable region, comprises a human antibody framework or a humanized antibody framework;
(A-12) An anticancer agent comprising as an active ingredient a multispecific antigen-binding molecule that comprises (i) a first antigen-binding moiety that binds to CD3 and (ii) a second antigen-binding moiety that binds to claudin 6 (CLDN6), wherein the first antigen-binding moiety comprises any one of (c1) to (c4) below:

(c1) a first antibody variable region comprising the amino acid sequence of SEQ ID NO: 3, and a second antibody variable region comprising the amino acid sequence of SEQ ID NO: 27;
(c2) a first antibody variable region comprising the amino acid sequence of SEQ ID NO: 4, and a second antibody variable region comprising the amino acid sequence of SEQ ID NO: 27;
(c3) a first antibody variable region comprising the amino acid sequence of SEQ ID NO: 5, and a second antibody variable region comprising the amino acid sequence of SEQ ID NO: 28; and
(c4) a first antibody variable region comprising the amino acid sequence of SEQ ID NO: 6, and a second antibody variable region comprising the amino acid sequence of SEQ ID NO: 28;

(A-13) The anticancer agent of (A-12), wherein the second antigen-binding moiety comprises any one of (d1) to (d3) below:

(d1) a third antibody variable region comprising the amino acid sequence of SEQ ID NO: 2, and a fourth antibody variable region comprising the amino acid sequence of SEQ ID NO: 26;
(d2) a third antibody variable region comprising the amino acid sequence of SEQ ID NO: 1, and a fourth antibody variable region comprising the amino acid sequence of SEQ ID NO: 25; and
(d3) a third antibody variable region comprising the amino acid sequence of SEQ ID NO: 25, and a fourth antibody variable region comprising the amino acid sequence of SEQ ID NO: 1;

(A-14) An anticancer agent comprising as an active ingredient a multispecific antigen-binding molecule that comprises (i) a first antigen-binding moiety that binds to CD3 and (ii) a second antigen-binding moiety that binds to claudin 6 (CLDN6), wherein the second antigen-binding moiety comprises any one of (d1) to (d3) below:

(d1) a third antibody variable region comprising the amino acid sequence of SEQ ID NO: 2, and a fourth antibody variable region comprising the amino acid sequence of SEQ ID NO: 26;
(d2) a third antibody variable region comprising the amino acid sequence of SEQ ID NO: 1, and a fourth antibody variable region comprising the amino acid sequence of SEQ ID NO: 25; and
(d3) a third antibody variable region comprising the amino acid sequence of SEQ ID NO: 25, and a fourth antibody variable region comprising the amino acid sequence of SEQ ID NO: 1;

(A-15) An anticancer agent comprising as an active ingredient a multispecific antigen-binding molecule that comprises (i) a first antigen-binding moiety that binds to CD137, and (ii) a second antigen-binding moiety that binds to claudin 6 (CLDN6), wherein the first antigen-binding moiety comprises any one of (a1) to (a4) below:

(a1) a first antibody variable region comprising the complementarity-determining region (CDR) 1 of SEQ ID NO: 9, the CDR2 of SEQ ID NO: 15, and the CDR3 of SEQ ID NO: 21, and a second antibody variable region comprising the CDR1 of SEQ ID NO: 31, the CDR2 of SEQ ID NO: 35, and the CDR3 of SEQ ID NO: 39;
(a2) a first antibody variable region comprising the complementarity-determining region (CDR) 1 of SEQ ID NO: 10, the CDR2 of SEQ ID NO: 16, and the CDR3 of SEQ ID NO: 22, and a second antibody variable region comprising the CDR1 of SEQ ID NO: 31, the CDR2 of SEQ ID NO: 35, and the CDR3 of SEQ ID NO: 39;
(a3) a first antibody variable region comprising the complementarity-determining region (CDR) 1 of SEQ ID NO: 11, the CDR2 of SEQ ID NO: 17, and the CDR3 of SEQ ID NO: 23, and a second antibody variable region comprising the CDR1 of SEQ ID NO: 32, the CDR2 of SEQ ID NO: 36, and the CDR3 of SEQ ID NO: 40; and
(a4) a first antibody variable region comprising the complementarity-determining region (CDR) 1 of SEQ ID NO: 12, the CDR2 of SEQ ID NO: 18, and the CDR3 of SEQ ID NO: 24, and a second antibody variable region comprising the CDR1 of SEQ ID NO: 32, the CDR2 of SEQ ID NO: 36, and the CDR3 of SEQ ID NO: 40;

(A-16) The anticancer agent of (A-15), wherein the second antigen-binding moiety comprises any one of (b1) to (b3) below:

(b1) a third antibody variable region comprising the complementarity-determining region (CDR) 1 of SEQ ID NO: 8, the CDR2 of SEQ ID NO: 14, and the CDR3 of SEQ ID NO: 20, and a fourth antibody variable region comprising the CDR1 of SEQ ID NO: 30, the CDR2 of SEQ ID NO: 34, and the CDR3 of SEQ ID NO: 38;
(b2) a third antibody variable region comprising the complementarity-determining region (CDR) 1 of SEQ ID NO: 7, the CDR2 of SEQ ID NO: 13, and the CDR3 of SEQ ID NO: 19, and a fourth antibody variable region comprising the CDR1 of SEQ ID NO: 29, the CDR2 of SEQ ID NO: 33, and the CDR3 of SEQ ID NO: 37; and
(b3) a third antibody variable region comprising the complementarity-determining region (CDR) 1 of SEQ ID NO: 29, the CDR2 of SEQ ID NO: 33, and the CDR3 of SEQ ID NO: 37, and a fourth antibody variable region comprising the CDR1 of SEQ ID NO: 7, the CDR2 of SEQ ID NO: 13, and the CDR3 of SEQ ID NO: 19;

(A-17) An anticancer agent comprising as an active ingredient a multispecific antigen-binding molecule that comprises (i) a first antigen-binding moiety that binds to CD137, and (ii) a second antigen-binding moiety that binds to claudin 6 (CLDN6), wherein the second antigen-binding moiety comprises any one of (b1) to (b3) below:

(b 1) a third antibody variable region comprising the complementarity-determining region (CDR) 1 of SEQ ID NO: 8, the CDR2 of SEQ ID NO: 14, and the CDR3 of SEQ ID NO: 20, and a fourth antibody variable region comprising the CDR1 of SEQ ID NO: 30, the CDR2 of SEQ ID NO: 34, and the CDR3 of SEQ ID NO: 38;
(b2) a third antibody variable region comprising the complementarity-determining region (CDR) 1 of SEQ ID NO: 7, the CDR2 of SEQ ID NO: 13, and the CDR3 of SEQ ID NO: 19, and a fourth antibody variable region comprising the CDR1 of SEQ ID NO: 29, the CDR2 of SEQ ID NO: 33, and the CDR3 of SEQ ID NO: 37; and

(b3) a third antibody variable region comprising the complementarity-determining region (CDR) 1 of SEQ ID NO: 29, the CDR2 of SEQ ID NO: 33, and the CDR3 of SEQ ID NO: 37, and a fourth antibody variable region comprising the CDR1 of SEQ ID NO: 7, the CDR2 of SEQ ID NO: 13, and the CDR3 of SEQ ID NO: 19;

(A-18) The anticancer agent of any one of (A-15) to (A-17), wherein at least one selected from the group consisting of the first antibody variable region, the second antibody variable region, the third antibody variable region, and the fourth antibody variable region, comprises a human antibody framework or a humanized antibody framework;
(A-19) An anticancer agent comprising as an active ingredient a multispecific antigen-binding molecule that comprises (i) a first antigen-binding moiety that binds to CD137, and (ii) a second antigen-binding moiety that binds to claudin 6 (CLDN6), wherein the first antigen-binding moiety comprises any one of (c1) to (c4) below:

(c1) a first antibody variable region comprising the amino acid sequence of SEQ ID NO: 3, and a second antibody variable region comprising the amino acid sequence of SEQ ID NO: 27;
(c2) a first antibody variable region comprising the amino acid sequence of SEQ ID NO: 4, and a second antibody variable region comprising the amino acid sequence of SEQ ID NO: 27;
(c3) a first antibody variable region comprising the amino acid sequence of SEQ ID NO: 5, and a second antibody variable region comprising the amino acid sequence of SEQ ID NO: 28; and
(c4) a first antibody variable region comprising the amino acid sequence of SEQ ID NO: 6, and a second antibody variable region comprising the amino acid sequence of SEQ ID NO: 28;

(A-20) The anticancer agent of (A-19), wherein the second antigen-binding moiety comprises any one of (d1) to (d3) below:

(d1) a third antibody variable region comprising the amino acid sequence of SEQ ID NO: 2, and a fourth antibody variable region comprising the amino acid sequence of SEQ ID NO: 26;
(d2) a third antibody variable region comprising the amino acid sequence of SEQ ID NO: 1, and a fourth antibody variable region comprising the amino acid sequence of SEQ ID NO: 25; and
(d3) a third antibody variable region comprising the amino acid sequence of SEQ ID NO: 25, and a fourth antibody variable region comprising the amino acid sequence of SEQ ID NO: 1;

(A-21) An anticancer agent comprising as an active ingredient a multispecific antigen-binding molecule that comprises (i) a first antigen-binding moiety that binds to CD137, and (ii) a second antigen-binding moiety that binds to claudin 6 (CLDN6), wherein the second antigen-binding moiety comprises any one of (d1) to (d3) below:

(d1) a third antibody variable region comprising the amino acid sequence of SEQ ID NO: 2, and a fourth antibody variable region comprising the amino acid sequence of SEQ ID NO: 26;
(d2) a third antibody variable region comprising the amino acid sequence of SEQ ID NO: 1, and a fourth antibody variable region comprising the amino acid sequence of SEQ ID NO: 25; and
(d3) a third antibody variable region comprising the amino acid sequence of SEQ ID NO: 25, and a fourth antibody variable region comprising the amino acid sequence of SEQ ID NO: 1;

(A-22) An anticancer agent comprising as an active ingredient a multispecific antigen-binding molecule that comprises any one of (c1) to (c4) below:

(c1) a first antibody variable region comprising the amino acid sequence of SEQ ID NO: 3, and a second antibody variable region comprising the amino acid sequence of SEQ ID NO: 27;
(c2) a first antibody variable region comprising the amino acid sequence of SEQ ID NO: 4, and a second antibody variable region comprising the amino acid sequence of SEQ ID NO: 27;
(c3) a first antibody variable region comprising the amino acid sequence of SEQ ID NO: 5, and a second antibody variable region comprising the amino acid sequence of SEQ ID NO: 28; and
(c4) a first antibody variable region comprising the amino acid sequence of SEQ ID NO: 6, and a second antibody variable region comprising the amino acid sequence of SEQ ID NO: 28;

(A-23) The anticancer agent of (A-22), which further comprises any one of (d1) to (d3) below

(d1) a third antibody variable region comprising the amino acid sequence of SEQ ID NO: 2, and a fourth antibody variable region comprising the amino acid sequence of SEQ ID NO: 26;
(d2) a third antibody variable region comprising the amino acid sequence of SEQ ID NO: 1, and a fourth antibody variable region comprising the amino acid sequence of SEQ ID NO: 25; and
(d3) a third antibody variable region comprising the amino acid sequence of SEQ ID NO: 25, and a fourth antibody variable region comprising the amino acid sequence of SEQ ID NO: 1;

(A-24) An anticancer agent comprising as an active ingredient a multispecific antigen-binding molecule that comprises any one of (d1) to (d3) below:

(d1) a third antibody variable region comprising the amino acid sequence of SEQ ID NO: 2, and a fourth antibody variable region comprising the amino acid sequence of SEQ ID NO: 26;
(d2) a third antibody variable region comprising the amino acid sequence of SEQ ID NO: 1, and a fourth antibody variable region comprising the amino acid sequence of SEQ ID NO: 25; and
(d3) a third antibody variable region comprising the amino acid sequence of SEQ ID NO: 25, and a fourth antibody variable region comprising the amino acid sequence of SEQ ID NO: 1;

(A-25) An anticancer agent comprising as an active ingredient a multispecific antigen-binding molecule that comprises any one of (a1) to (a4) below:

(a1) a first antibody variable region comprising the complementarity-determining region (CDR) 1 of SEQ ID NO: 9, the CDR2 of SEQ ID NO: 15, and the CDR3 of SEQ ID NO: 21, and a second antibody variable region comprising the CDR1 of SEQ ID NO: 31, the CDR2 of SEQ ID NO: 35, and the CDR3 of SEQ ID NO: 39;
(a2) a first antibody variable region comprising the complementarity-determining region (CDR) 1 of SEQ ID NO: 10, the CDR2 of SEQ ID NO: 16, and the CDR3 of SEQ ID NO: 22, and a second antibody variable region comprising the CDR1 of SEQ ID NO: 31, the CDR2 of SEQ ID NO: 35, and the CDR3 of SEQ ID NO: 39;
(a3) a first antibody variable region comprising the complementarity-determining region (CDR) 1 of SEQ ID NO: 11, the CDR2 of SEQ ID NO: 17, and the CDR3 of SEQ ID NO: 23, and a second antibody variable region comprising the CDR1 of SEQ ID NO: 32, the CDR2 of SEQ ID NO: 36, and the CDR3 of SEQ ID NO: 40; and
(a4) a first antibody variable region comprising the complementarity-determining region (CDR) 1 of SEQ ID NO: 12, the CDR2 of SEQ ID NO: 18, and the CDR3 of SEQ ID NO: 24, and a second antibody variable region comprising the CDR1 of SEQ ID NO: 32, the CDR2 of SEQ ID NO: 36, and the CDR3 of SEQ ID NO: 40;

(A-26) The anticancer agent of (A-25) further comprising any one of (b1) to (b3) below:

(b1) a third antibody variable region comprising the complementarity-determining region (CDR) 1 of SEQ ID NO: 8, the CDR2 of SEQ ID NO: 14, and the CDR3 of SEQ ID NO: 20, and a fourth antibody variable region comprising the CDR1 of SEQ ID NO: 30, the CDR2 of SEQ ID NO: 34, and the CDR3 of SEQ ID NO: 38;
(b2) a third antibody variable region comprising the complementarity-determining region (CDR) 1 of SEQ ID NO: 7, the CDR2 of SEQ ID NO: 13, and the CDR3 of SEQ ID NO: 19, and a fourth antibody variable region comprising the CDR1 of SEQ ID NO: 29, the CDR2 of SEQ ID NO: 33, and the CDR3 of SEQ ID NO: 37; and
(b3) a third antibody variable region comprising the complementarity-determining region (CDR) 1 of SEQ ID NO: 29, the CDR2 of SEQ ID NO: 33, and the CDR3 of SEQ ID NO: 37, and a fourth antibody variable region comprising the CDR1 of SEQ ID NO: 7, the CDR2 of SEQ ID NO: 13, and the CDR3 of SEQ ID NO: 19;

(A-27) An anticancer agent comprising as an active ingredient a multispecific antigen-binding molecule that comprises any one of (b1) to (b3) below:

(b1) a third antibody variable region comprising the complementarity-determining region (CDR) 1 of SEQ ID NO: 8, the CDR2 of SEQ ID NO: 14, and the CDR3 of SEQ ID NO: 20, and a fourth antibody variable region comprising the CDR1 of SEQ ID NO: 30, the CDR2 of SEQ ID NO: 34, and the CDR3 of SEQ ID NO: 38;
(b2) a third antibody variable region comprising the complementarity-determining region (CDR) 1 of SEQ ID NO: 7, the CDR2 of SEQ ID NO: 13, and the CDR3 of SEQ ID NO: 19, and a fourth antibody variable region comprising the CDR1 of SEQ ID NO: 29, the CDR2 of SEQ ID NO: 33, and the CDR3 of SEQ ID NO: 37; and
(b3) a third antibody variable region comprising the complementarity-determining region (CDR) 1 of SEQ ID NO: 29, the CDR2 of SEQ ID NO: 33, and the CDR3 of SEQ ID NO: 37, and a fourth antibody variable region comprising the CDR1 of SEQ ID NO: 7, the CDR2 of SEQ ID NO: 13, and the CDR3 of SEQ ID NO: 19;

(A-28) The anticancer agent of any one of (A-1) to (A-27), wherein the multispecific antigen-binding molecule further comprises (iii) an Fc domain exhibiting decreased binding affinity for human Fcγ receptor compared to a natural human IgG1 Fc domain;

(A-29) The anticancer agent of (A-28), wherein the Fc domain is composed of a first Fc region subunit and a second Fc region subunit;
(A-30) The anticancer agent of (A-29), wherein the Fc domain comprises (e1) or (e2) below, and the amino acid positions are numbered according to the EU index:

(e1) a first Fc region subunit comprising Cys at position 349, Ser at position 366, Ala at position 368, and Val at position 407, and a second Fc region subunit comprising Cys at position 354 and Trp at position 366;
(e2) a first Fc region subunit comprising Glu at position 439 and a second Fc region subunit comprising Lys at position 356;

(A-31) The anticancer agent of (A-29) or (A-30), wherein the first and/or second Fc region subunits comprise (f1) or (f2) below, and the amino acid positions are numbered according to the EU index:

(f1) Ala at position 234 and Ala at position 235;
(f2) Ala at position 234, Ala at position 235, and Ala at position 297;

(A-32) The anticancer agent of any one of (A-29) to (A-31) wherein the Fc domain exhibits stronger FcRn binding affinity to human FcRn compared to a natural human IgG1 Fc domain;
(A-33) The anticancer agent of (A-32), wherein the first and/or second Fc region subunit comprises Leu at position 428, Ala at position 434, Arg at position 438, and Glu at position 440, and the amino acid positions are numbered according to the EU index;
(A-34) The anticancer agent of any one of (A-1) to (A-27), wherein the first antibody variable region of the first antigen-binding moiety is fused to a first heavy chain constant region, the second antibody variable region of the first antigen-binding moiety is fused to a first light chain constant region, the third antibody variable region of the second antigen-binding moiety is fused to a second heavy chain constant region, and the fourth antibody variable region of the second antigen-binding moiety is fused to a second light chain constant region, and
the constant region is any one of (g1) to (g7) below:

(g1) a first heavy chain constant region comprising the amino acid sequence of SEQ ID NO: 74, a first light chain constant region comprising the amino acid sequence of SEQ ID NO: 87, a second heavy chain constant region comprising the amino acid sequence of SEQ ID NO: 73, and a second light chain constant region comprising the amino acid sequence of SEQ ID NO: 88;
(g2) a first heavy chain constant region comprising the amino acid sequence of SEQ ID NO: 74, a first light chain constant region comprising the amino acid sequence of SEQ ID NO: 85, a second heavy chain constant region comprising the amino acid sequence of SEQ ID NO: 81, and a second light chain constant region comprising the amino acid sequence of SEQ ID NO: 86;
(g3) a first heavy chain constant region comprising the amino acid sequence of SEQ ID NO: 79, a first light chain constant region comprising the amino acid sequence of SEQ ID NO: 72, a second heavy chain constant region comprising the amino acid sequence of SEQ ID NO: 80, and a second light chain constant region comprising the amino acid sequence of SEQ ID NO: 89;
(g4) a first heavy chain constant region comprising the amino acid sequence of SEQ ID NO: 83, a first light chain constant region comprising the amino acid sequence of SEQ ID NO: 87, a second heavy chain constant region comprising the amino acid sequence of SEQ ID NO: 82, and a second light chain constant region comprising the amino acid sequence of SEQ ID NO: 88;
(g5) a first heavy chain constant region comprising the amino acid sequence of SEQ ID NO: 83, a first light chain constant region comprising the amino acid sequence of SEQ ID NO: 85, a second heavy chain constant region comprising the amino acid sequence of SEQ ID NO: 84, and a second light chain constant region comprising the amino acid sequence of SEQ ID NO: 86;
(g6) a first heavy chain constant region comprising the amino acid sequence of SEQ ID NO: 77, a first light chain constant region comprising the amino acid sequence of SEQ ID NO: 72, a second heavy chain constant region comprising the amino acid sequence of SEQ ID NO: 78, and a second light chain constant region comprising the amino acid sequence of SEQ ID NO: 89; and
(g7) a first heavy chain constant region comprising the amino acid sequence of SEQ ID NO: 75, a first light chain constant region comprising the amino acid sequence of SEQ ID NO: 72, a second heavy chain constant region comprising the amino acid sequence of SEQ ID NO: 76, and a second light chain constant region comprising the amino acid sequence of SEQ ID NO: 89;

(A-35) An anticancer agent comprising as an active ingredient a multispecific antigen-binding molecule that comprises any one of the combinations of four peptide chains of (h01) to (h18) below:

(h01) a heavy chain comprising the amino acid sequence of SEQ ID NO: 42 (chain 1) and a light chain comprising the amino acid sequence of SEQ ID NO: 51 (chain 2), and a heavy chain comprising the amino acid sequence of SEQ ID NO: 56 (chain 3) and a light chain comprising the amino acid sequence of SEQ ID NO: 69 (chain 4);
(h02) a heavy chain comprising the amino acid sequence of SEQ ID NO: 41 (chain 1) and a light chain comprising the amino acid sequence of SEQ ID NO: 50 (chain 2), and a heavy chain comprising the amino acid sequence of SEQ ID NO: 54 (chain 3) and a light chain comprising the amino acid sequence of SEQ ID NO: 68 (chain 4);
(h03) a heavy chain comprising the amino acid sequence of SEQ ID NO: 41 (chain 1) and a light chain comprising the amino acid sequence of SEQ ID NO: 50 (chain 2), and a heavy chain comprising the amino acid sequence of SEQ ID NO: 55 (chain 3) and a light chain comprising the amino acid sequence of SEQ ID NO: 68 (chain 4);
(h04) a heavy chain comprising the amino acid sequence of SEQ ID NO: 42 (chain 1) and a light chain comprising the amino acid sequence of SEQ ID NO: 51 (chain 2), and a heavy chain comprising the amino acid sequence of SEQ ID NO: 57 (chain 3) and a light chain comprising the amino acid sequence of SEQ ID NO: 69 (chain 4);
(h05) a heavy chain comprising the amino acid sequence of SEQ ID NO: 44 (chain 1) and a light chain comprising the amino acid sequence of SEQ ID NO: 52 (chain 2), and a heavy chain comprising the amino acid sequence of SEQ ID NO: 60 (chain 3) and a light chain comprising the amino acid sequence of SEQ ID NO: 70 (chain 4);
(h06) a heavy chain comprising the amino acid sequence of SEQ ID NO: 44 (chain 1) and a light chain comprising the amino acid sequence of SEQ ID NO: 52 (chain 2), and a heavy chain comprising the amino acid sequence of SEQ ID NO: 61 (chain 3) and a light chain comprising the amino acid sequence of SEQ ID NO: 70 (chain 4);
(h07) a heavy chain comprising the amino acid sequence of SEQ ID NO: 45 (chain 1) and a light chain comprising the amino acid sequence of SEQ ID NO: 50 (chain 2), and a heavy chain comprising the amino acid sequence of SEQ ID NO: 62 (chain 3) and a light chain comprising the amino acid sequence of SEQ ID NO: 68 (chain 4);
(h08) a heavy chain comprising the amino acid sequence of SEQ ID NO: 45 (chain 1) and a light chain comprising the amino acid sequence of SEQ ID NO: 50 (chain 2), and a heavy chain comprising the amino acid sequence of SEQ ID NO: 63 (chain 3) and a light chain comprising the amino acid sequence of SEQ ID NO: 68 (chain 4);
(h09) a heavy chain comprising the amino acid sequence of SEQ ID NO: 46 (chain 1) and a light chain comprising the amino acid sequence of SEQ ID NO: 51 (chain 2), and a heavy chain comprising the amino acid sequence of SEQ ID NO: 64 (chain 3) and a light chain comprising the amino acid sequence of SEQ ID NO: 69 (chain 4);
(h10) a heavy chain comprising the amino acid sequence of SEQ ID NO: 46 (chain 1) and a light chain comprising the amino acid sequence of SEQ ID NO: 51 (chain 2), and a heavy chain comprising the amino acid sequence of SEQ ID NO: 65 (chain 3) and a light chain comprising the amino acid sequence of SEQ ID NO: 69 (chain 4);
(h11) a heavy chain comprising the amino acid sequence of SEQ ID NO: 47 (chain 1) and a light chain comprising the amino acid sequence of SEQ ID NO: 52 (chain 2), and a heavy chain comprising the amino acid sequence of SEQ ID NO: 66 (chain 3) and a light chain comprising the amino acid sequence of SEQ ID NO: 70 (chain 4);
(h12) a heavy chain comprising the amino acid sequence of SEQ ID NO: 47 (chain 1) and a light chain comprising the amino acid sequence of SEQ ID NO: 52 (chain 2), and a heavy chain comprising the amino acid sequence of SEQ ID NO: 67 (chain 3) and a light chain comprising the amino acid sequence of SEQ ID NO: 70 (chain 4);
(h13) a heavy chain comprising the amino acid sequence of SEQ ID NO: 48 (chain 1) and a light chain comprising the amino acid sequence of SEQ ID NO: 53 (chain 2), and a heavy chain comprising the amino acid sequence of SEQ ID NO: 56 (chain 3) and a light chain comprising the amino acid sequence of SEQ ID NO: 71 (chain 4);
(h14) a heavy chain comprising the amino acid sequence of SEQ ID NO: 48 (chain 1) and a light chain comprising the amino acid sequence of SEQ ID NO: 53 (chain 2), and a heavy chain comprising the amino acid sequence of SEQ ID NO: 57 (chain 3) and a light chain comprising the amino acid sequence of SEQ ID NO: 71 (chain 4);
(h15) a heavy chain comprising the amino acid sequence of SEQ ID NO: 49 (chain 1) and a light chain comprising the amino acid sequence of SEQ ID NO: 53 (chain 2), and a heavy chain comprising the amino acid sequence of SEQ ID NO: 64 (chain 3) and a light chain comprising the amino acid sequence of SEQ ID NO: 71 (chain 4);
(h16) a heavy chain comprising the amino acid sequence of SEQ ID NO: 49 (chain 1) and a light chain comprising the amino acid sequence of SEQ ID NO: 53 (chain 2), and a heavy chain comprising the amino acid sequence of SEQ ID NO: 65 (chain 3) and a light chain comprising the amino acid sequence of SEQ ID NO: 71 (chain 4);
(h17) a heavy chain comprising the amino acid sequence of SEQ ID NO: 43 (chain 1) and a light chain comprising the amino acid sequence of SEQ ID NO: 52 (chain 2), and a heavy chain comprising the amino acid sequence of SEQ ID NO: 58 (chain 3) and a light chain comprising the amino acid sequence of SEQ ID NO: 70 (chain 4); and
(h18) a heavy chain comprising the amino acid sequence of SEQ ID NO: 43 (chain 1) and a light chain comprising the amino acid sequence of SEQ ID NO: 52 (chain 2), and a heavy chain comprising the amino acid sequence of SEQ ID NO: 59 (chain 3) and a light chain comprising the amino acid sequence of SEQ ID NO: 70 (chain 4);

(A-36) The anticancer agent of (A-35), wherein

(i) the antibody variable region included in chain 3 and the antibody variable region included in chain 4 form a first antigen-binding moiety that is capable of binding to CD3 and CD137 and that binds to either CD3 or CD137;
(ii) the antibody variable region included in chain 1 and the antibody variable region included in chain 2 form a second antigen-binding moiety that is capable of binding to claudin 6 (CLDN6); and
(iii) the antibody Fc region subunit included in chain 1 and the antibody Fc region subunit included in chain 3 form an Fc domain;

(A-37) The anticancer agent of (A-35), wherein

(i) the antibody variable region included in chain 3 and the antibody variable region included in chain 4 form a first antigen-binding moiety that binds to CD3;
(ii) the antibody variable region included in chain 1 and the antibody variable region included in chain 2 form a second antigen-binding moiety that binds to claudin 6 (CLDN6); and
(iii) the antibody Fc region subunit included in chain 1 and the antibody Fc region subunit included in chain 3 form an Fc domain;

(A-38) The anticancer agent of (A-35), wherein

(i) the antibody variable region included in chain 3 and the antibody variable region included in chain 4 form a first antigen-binding moiety that binds to CD137;
(ii) the antibody variable region included in chain 1 and the antibody variable region included in chain 2 form a second antigen-binding moiety that binds to claudin 6 (CLDN6); and
(iii) the antibody Fc region subunit included in chain 1 and the antibody Fc region subunit included in chain 3 form an Fc domain;

(A-39) An anticancer agent comprising as an active ingredient a multispecific antigen-binding molecule that binds to epitopes overlapping and/or competing with epitopes on each of CLDN6 and CD3/CD137 bound by the multispecific antigen-binding molecule of any of (A-5) to (A-7);
(A-40) The anticancer agent of any one of (A-1) to (A-39), wherein the cancer of interest is CLDN6-positive cancer;
(A-41) The anticancer agent of any one of (A-1) to (A-40), wherein the cancer of interest is at least one cancer selected from the group consisting of ovary cancer, non-small cell lung cancer, gastric cancer, liver cancer, endometrial cancer, germ cell tumor, large bowel cancer, urinary bladder cancer, and atypical teratoid rhabdoid tumor;
(A-42) The anticancer agent of any one of (A-1) to (A-41), wherein the cancer of interest is cancer metastasized to the peritoneum;
(A-43) The anticancer agent of any one of (A-1) to (A-42), wherein the cancer of interest is peritoneally disseminated cancer;
(A-44) The anticancer agent of any one of (A-1) to (A-43), which is for treating patients with cancer unresponsive to treatment with the at least one other anticancer agent or an anticancer agent which is different from the at least one other anticancer agent;
(A-45) The anticancer agent of any one of (A-40) to (A-44), wherein the CLDN6-positive cancer is cancer previously treated with other anticancer agents;
(A-46) The anticancer agent of any one of (A-40) to (A-45), wherein the CLDN6-positive cancer is cancer for which desired effects could not be obtained by treatment with administration of another anticancer agent alone;
(A-47) The anticancer agent of any one of (A-1) to (A-46), which further comprises a pharmaceutically acceptable carrier;
(A-48) The anticancer agent of any one of (A-1) to (A-47), wherein the multispecific antigen-binding molecule induces cytotoxicity; and
(A-49) The anticancer agent of (A-48), wherein the cytotoxicity is T cell-dependent cytotoxicity.

Furthermore, the present disclosure provides the following:

(B-1) A pharmaceutical composition for use in combination with at least one other anticancer agent, wherein the pharmaceutical composition comprises as an active ingredient a multispecific antigen-binding molecule,
the multispecific antigen-binding molecule comprising (i) a first antigen-binding moiety that is capable of binding to CD3 and CD137 and that binds to either CD3 or CD137, and (ii) a second antigen-binding moiety that is capable of binding to claudin 6 (CLDN6);
(B-2) A pharmaceutical composition for use in combination with at least one other anticancer agent, wherein the pharmaceutical composition comprises as an active ingredient the multispecific antigen-binding molecule of any of (A-2) to (A-39);

(B-3) The pharmaceutical composition of (B-1) or (B-2), wherein the cancer of interest is CLDN6-positive cancer;

(B-4) The pharmaceutical composition of any one of (B-1) to (B-3), wherein the cancer of interest is any cancer selected from the group consisting of ovary cancer, non-small cell lung cancer, gastric cancer, liver cancer, endometrial cancer, germ cell tumor, large bowel cancer, urinary bladder cancer, and atypical teratoid rhabdoid tumor;

(B-5) The pharmaceutical composition of any one of (B-1) to (B-4), wherein the cancer of interest is cancer metastasized to the peritoneum;

(B-6) The pharmaceutical composition of any one of (B-1) to (B-5), wherein the cancer of interest is peritoneally disseminated cancer;

(B-7) The pharmaceutical composition of any one of (B-1) to (B-6), which is for treating patients with cancer unresponsive to treatment with the at least one other anticancer agent or an anticancer agent which is different from the at least one other anticancer agent;

(B-8) The pharmaceutical composition of any one of (B-1) to (B-7), wherein the cancer of interest is cancer previously treated with the at least one other anticancer agent or an anticancer agent which is different from the at least one other anticancer agent;

(B-9) The pharmaceutical composition of any one of (B-1) to (B-8), wherein the cancer of interest is cancer for which desired effects could not be obtained by treatment with administration of the at least one other anticancer agent alone;

(B-10) The pharmaceutical composition of any one of (B-1) to (B-9), wherein the multispecific antigen-binding molecule and the at least one other anticancer agent are administered separately or sequentially;

(B-11) The pharmaceutical composition of any one of (B 1) to (B-10), wherein the multispecific antigen-binding molecule is administered before, simultaneously with, and/or after the administration of the at least one other anticancer agent;

(B-12) The pharmaceutical composition of any one of (B 1) to (B-11), wherein the multispecific antigen-binding molecule is administered to a patient with cancer in which expression of CLDN6 has been increased by the administration of the at least one other anticancer agent;

(B-13) The pharmaceutical composition of any one of (B-1) to (B-12), characterized in that the multispecific antigen-binding molecule is administered to a subject with cancer in which expression of TGFβ has been increased by the administration of the at least one other anticancer agent;

(B-14) The pharmaceutical composition of any one of (B-1) to (B-13), characterized in that the multispecific antigen-binding molecule is administered to a subject with cancer in which expression of TGFβ1 has been increased by the administration of the at least one other anticancer agent;

(B-15) The pharmaceutical composition of any one of (B-1) to (B-14), wherein administration of the at least one other anticancer agent enhances the antitumor effect of the multispecific antigen-binding molecule;

(B-16) The pharmaceutical composition of any one of (B-1) to (B-15), wherein the at least one other anticancer agent is at least one selected from the group consisting of a chemotherapeutic agent, an immune checkpoint inhibitor, and a PARP inhibitor; (B-17) The pharmaceutical composition of any one of (B-1) to (B-16), wherein the at least one other anticancer agent is an agent that enhances expression of CLDN6 in a cell;

(B-18) The pharmaceutical composition of any one of (B 1) to (B-17), wherein the at least one other anticancer agent is a pharmaceutical agent having an effect of inducing TGF-β;

(B-19) The pharmaceutical composition of any one of (B 16) to (B-18), wherein the at least one other anticancer agent is a platinum preparation, an alkaloid, or an antimetabolite;

(B-20) The pharmaceutical composition of any one of (B 16) to (B-19), wherein the at least one other anticancer agent is a platinum preparation;

(B-21) The pharmaceutical composition of any one of (B 16) to (B-20), wherein the at least one other anticancer agent is an alkaloid;

(B-22) The pharmaceutical composition of any one of (B 16) to (B-19), wherein the at least one other anticancer agent is a topoisomerase inhibitor;

(B-23) The pharmaceutical composition of any one of (B 16) to (B-19), wherein the at least one other anticancer agent is an antimetabolite;

(B-24) The pharmaceutical composition of any one of (B 16) to (B-19), wherein the at least one other anticancer agent is carboplatin or cisplatin;

(B-25) The pharmaceutical composition of any one of (B 16) to (B-19), wherein the at least one other anticancer agent is irinotecan;

(B-26) The pharmaceutical composition of any one of (B 16) to (B-19), wherein the at least one other anticancer agent is gemcitabine;

(B-27) The pharmaceutical composition of (B 16), wherein the at least one other anticancer agent is an anti-PD-L1 antibody;

(B-28) The pharmaceutical composition of (B 16), wherein the at least one other anticancer agent is olaparib;

(B-29) A cytotoxicity-inducing agent, a cell growth suppressor, a cell growth inhibitor, an immune response activator, a cancer therapeutic agent, or a cancer preventive agent, comprising the pharmaceutical composition of any one of (B-1) to (B-28);

(B2-1) A pharmaceutical composition for use in combination with at least one therapeutic method that induces TGF-β, wherein the pharmaceutical composition comprises as an active ingredient a multispecific antigen-binding molecule, the multispecific antigen-binding molecule comprising (i) a first antigen-binding moiety that is capable of binding to CD3 and CD137 and that binds to either CD3 or CD137, and (ii) a second antigen-binding moiety that is capable of binding to claudin 6 (CLDN6);

(B2-2) The pharmaceutical composition of (B2-1), wherein the therapeutic method that induces TGF-β is administration of an agent that induces TGF-β;

(B2-3) The pharmaceutical composition of (B2-1) or (B2-2), wherein the therapeutic method that induces TGF-β is administration of an agent that induces TGF-β1;

(B2-4) The pharmaceutical composition of any one of (B2-1) to (B2-3), wherein the cancer of interest is CLDN6-positive cancer;

(B2-5) The pharmaceutical composition of any one of (B2-1) to (B2-4), wherein the cancer of interest is any cancer selected from the group consisting of ovary cancer, non-small-cell lung cancer, gastric cancer, liver cancer, endometrial cancer, germ cell tumor, large bowel cancer, urinary bladder cancer, and atypical teratoid rhabdoid tumor;

(B2-6) The pharmaceutical composition of any one of (B2-1) to (B2-5), wherein the cancer of interest is cancer metastasized to the peritoneum;

(B2-7) The pharmaceutical composition of any one of (B2-1) to (B2-6), wherein the cancer of interest is peritoneally disseminated cancer;

(B2-8) The pharmaceutical composition of any one of (B2-1) to (B2-7), which is for treating patients with cancer unresponsive to treatment with the therapeutic method that induces TGF-β;

(B2-9) The pharmaceutical composition of any one of (B2-1) to (B2-8), wherein the cancer of interest is cancer previously treated with the therapeutic method that induces TGF-β, or cancer for which desired effects could not be obtained by treatment with the therapeutic method that induces TGF-β;

(B2-10) The pharmaceutical composition of any one of (B2-1) to (B2-9), wherein the pharmaceutical composition comprising the multispecific antigen-binding molecule is administered separately or sequentially with the treatment that induces TGF-β;

(B2-11) The pharmaceutical composition of any one of (B2-2) to (B-10), wherein the multispecific antigen-binding molecule is administered before, simultaneously with, and/or after the treatment that induces TGF-β;

(B2-12) The pharmaceutical composition of any one of (B2-1) to (B2-11), wherein the multispecific antigen-binding molecule is administered to patients having cancer in which expression of CLDN6 has been increased by the treatment that induces TGF-β;

(B2-13) The pharmaceutical composition of any one of (B2-1) to (B2-12), wherein the multispecific antigen-binding molecule is administered to patients having cancer in which expression of TGF-β has been increased by the treatment that induces TGF-β;

(B2-14) The pharmaceutical composition of any one of (B2-1) to (B2-13), wherein the multispecific antigen-binding molecule is administered to patients having cancer in which expression of TGF-β1 has been increased by the treatment that induces TGF-β;

(B2-15) The pharmaceutical composition of any one of (B2-1) to (B2-14), which enhances the antitumor effect of the multispecific antigen-binding molecule by the treatment that induces TGF-β; and

(B2-16) A cytotoxicity-inducing agent, a cell growth suppressor, a cell growth inhibitor, an immune response activator, a cancer therapeutic agent, or a cancer preventive agent, comprising the pharmaceutical composition of any one of (B2-1) to (B2-15).

(B3-1) A pharmaceutical composition for use in combination with at least one CLDN6 expression-inducing agent, which comprises as an active ingredient a multispecific antigen-binding molecule,

the multispecific antigen-binding molecule comprising (i) a first antigen-binding moiety that is capable of binding to CD3 and CD137 and that binds to either CD3 or CD137, and (ii) a second antigen-binding moiety that is capable of binding to claudin 6 (CLDN6);

(B3-2) The pharmaceutical composition of (B3-1), wherein the cancer of interest is CLDN6-positive cancer;

(B3-3) The pharmaceutical composition of (B3-1) or (B3-2), wherein the cancer of interest is any cancer selected from the group consisting of ovary cancer, non-small-cell lung cancer, gastric cancer, liver cancer, endometrial cancer, germ cell tumor, large bowel cancer, urinary bladder cancer, and atypical teratoid rhabdoid tumor;

(B3-4) The pharmaceutical composition of any one of (B3-1) to (B3-3), wherein the cancer of interest is cancer metastasized to the peritoneum;

(B3-5) The pharmaceutical composition of any one of (B3-1) to (B3-4), wherein the cancer of interest is peritoneally disseminated cancer;

(B3-6) The pharmaceutical composition of any one of (B3-1) to (B3-5), which is for treating patients with cancer unresponsive to treatment with the CLDN6 expression-inducing agent;
(B3-7) The pharmaceutical composition of any one of (B3-1) to (B3-6), wherein the cancer of interest is cancer previously treated with the CLDN6 expression-inducing agent, or cancer for which desired effects could not be obtained by treatment with the CLDN6 expression-inducing agent;
(B3-8) The pharmaceutical composition of any one of (B3-1) to (B3-7), wherein the pharmaceutical composition comprising the multispecific antigen-binding molecule is administered separately or sequentially with the CLDN6 expression-inducing agent;
(B3-9) The pharmaceutical composition of any one of (B3-1) to (B3-8), wherein the multispecific antigen-binding molecule is administered before, simultaneously with, and/or after the administration of the CLDN6 expression-inducing agent;
(B3-10) The pharmaceutical composition of any one of (B3-1) to (B3-9), wherein the multispecific antigen-binding molecule is administered to patients having cancer in which expression of CLDN6 has been increased by the administration of the CLDN6 expression-inducing agent;
(B3-11) The pharmaceutical composition of any one of (B3-1) to (B3-10), wherein the multispecific antigen-binding molecule is administered to patients having cancer in which expression of TGF-β has been increased by the administration of the CLDN6 expression-inducing agent;
(B3-12) The pharmaceutical composition of any one of (B3-1) to (B3-11), wherein the multispecific antigen-binding molecule is administered to patients having cancer in which expression of TGF-β1 has been increased by the administration of the CLDN6 expression-inducing agent;
(B3-13) The pharmaceutical composition of any one of (B3-1) to (B3-12), which enhances the antitumor effect of the multispecific antigen-binding molecule by administration of the CLDN6 expression-inducing agent; and
(B3-14) A cytotoxicity-inducing agent, a cell growth suppressor, a cell growth inhibitor, an immune response activator, a cancer therapeutic agent, or a cancer preventive agent, comprising the pharmaceutical composition of any one of (B3-1) to (B3-13).

Furthermore, the present disclosure provides the following:

(C-1) An anticancer agent, comprising as an active ingredient a multispecific antigen-binding molecule that comprises (i) a first antigen-binding moiety that is capable of binding to CD3 and CD137 and that binds to either CD3 or CD137, and (ii) a second antigen-binding moiety that is capable of binding to claudin 6 (CLDN6) and that has higher cytotoxic activity than when the first antigen-binding moiety is an antigen-binding moiety that can only bind to CD3;
(C-2) An anticancer agent, comprising as an active ingredient a multispecific antigen-binding molecule that comprises (i) a first antigen-binding moiety that is capable of binding to CD3 and CD137 and that binds to either CD3 or CD137, and (ii) a second antigen-binding moiety that is capable of binding to claudin 6 (CLDN6) and that has lower toxicity than when the first antigen-binding moiety is an antigen-binding moiety that can only bind to CD3;
(C-3) An anticancer agent, comprising as an active ingredient a multispecific antigen-binding molecule that comprises (1) a first antigen-binding domain that is capable of binding to a T cell receptor complex, and (2) a second antigen-binding moiety that is capable of binding to CLDN6 and that has T cell cytotoxic activity which is equivalent to or higher than that of a bispecific antibody (CS3348) comprising an antigen-binding moiety that is capable of binding to a T cell receptor complex, which is a moiety comprising a heavy chain that comprises the amino acid sequence of SEQ ID NO: 194 and a light chain that comprises the amino acid sequence of SEQ ID NO: 192, and an antigen-binding moiety that is capable of binding to CLDN6, which is a moiety comprising a heavy chain that comprises the amino acid sequence of SEQ ID NO: 193 and a light chain that comprises the amino acid sequence of SEQ ID NO: 195.
(C-4) The anticancer agent of any one of (C-1) to (C-3), wherein the multispecific antigen-binding molecule further comprises (3) an Fc domain exhibiting decreased binding affinity to a human Fcγ receptor compared to a natural human IgG1 Fc domain;
(C-5) The anticancer agent of (C-1), (C-3), or (C-4), wherein the multispecific antigen-binding molecule further comprises lower toxicity compared to the multispecific antibody (CS3348);
(C-6) The anticancer agent of any one of (C-1) to (C-5), wherein the first antigen-binding moiety comprises the combination of antibody variable regions selected from (a1) and (a2) below, or a combination of antibody variable regions functionally equivalent thereto:

(a1) a first antibody variable region comprising the complementarity-determining region (CDR) 1 of SEQ ID NO: 11, the CDR2 of SEQ ID NO: 17, and the CDR3 of SEQ ID NO: 23, and a second antibody variable region comprising the CDR1 of SEQ ID NO: 32, the CDR2 of SEQ ID NO: 36, and the CDR3 of SEQ ID NO: 40;
(a2) a first antibody variable region comprising the complementarity-determining region (CDR) 1 of SEQ ID NO: 10, the CDR2 of SEQ ID NO: 16, and the CDR3 of SEQ ID NO: 22, and a second antibody variable region comprising the CDR1 of SEQ ID NO: 31, the CDR2 of SEQ ID NO: 35, and the CDR3 of SEQ ID NO: 39;

(C-7) The anticancer agent of any one of (C-1) to (C-6), wherein the second antigen-binding moiety comprises the combination of antibody variable regions selected from (b1) and (b2) below, or a combination of antibody variable regions functionally equivalent thereto:

(b1) a third antibody variable region comprising the complementarity-determining region (CDR) 1 of SEQ ID NO: 7, the CDR2 of SEQ ID NO: 13, and the CDR3 of SEQ ID NO: 19, and a fourth antibody variable region comprising the CDR1 of SEQ ID NO: 29, the CDR2 of SEQ ID NO: 33, and the CDR3 of SEQ ID NO: 37; and
(b2) a third antibody variable region comprising the complementarity-determining region (CDR) 1 of SEQ ID NO: 8, the CDR2 of SEQ ID NO: 14, and the CDR3 of SEQ ID NO: 20, and a fourth antibody variable region comprising the CDR1 of SEQ ID NO: 30, the CDR2 of SEQ ID NO: 34, and the CDR3 of SEQ ID NO: 38;

(C-8) The anticancer agent of any one of (C-1) to (C-7), wherein the first antigen-binding moiety comprises the combination of antibody variable regions selected from (c1) and (c2) below, or a combination of antibody variable regions functionally equivalent thereto:

(c1) a first antibody variable region comprising the amino acid sequence of SEQ ID NO: 5, and a second antibody variable region comprising the amino acid sequence of SEQ ID NO: 28; and
(c2) a first antibody variable region comprising the amino acid sequence of SEQ ID NO: 4, and a second antibody variable region comprising the amino acid sequence of SEQ ID NO: 27;

(C-9) The anticancer agent of any one of (C-1) to (C-8), wherein the second antigen-binding moiety comprises the combination of antibody variable regions selected from (d1) and (d2) below, or a combination of antibody variable regions functionally equivalent thereto:

(d1) a third antibody variable region comprising the amino acid sequence of SEQ ID NO: 1, and a fourth antibody variable region comprising the amino acid sequence of SEQ ID NO: 25; and
(d2) a third antibody variable region comprising the amino acid sequence of SEQ ID NO: 2, and a fourth antibody variable region comprising the amino acid sequence of SEQ ID NO: 26;

(C-10) The anticancer agent of any one of (C-4) to (C-9), wherein the Fc domain is composed of a first Fc region subunit and a second Fc region subunit;
(C-11) The anticancer agent of any one of (C-4) to (C-10), wherein the Fc domain comprises (e1) or (e2) below, and the amino acid positions are numbered according to the EU index:

(e1) a first Fc region subunit comprising Cys at position 349, Ser at position 366, Ala at position 368, and Val at position 407, and a second Fc region subunit comprising Cys at position 354 and Trp at position 366;
(e2) a first Fc region subunit comprising Glu at position 439 and a second Fc region subunit comprising Lys at position 356;

(C-12) The anticancer agent of (C-9) or (C-11), wherein the first and/or second Fc region subunits comprise (f1) or (f2) below, and the amino acid positions are numbered according to the EU index:

(f1) Ala at position 234 and Ala at position 235;
(f2) Ala at position 234, Ala at position 235, and Ala at position 297;

(C-13) The anticancer agent of any one of (C-4) to (C-12), wherein the Fc domain exhibits stronger FcRn binding affinity to human FcRn compared to a natural human IgG1 Fc domain; and
(C-14) The anticancer agent of any one of (C-1) to (C-13), which further comprises a pharmaceutically acceptable carrier.

Furthermore, the present disclosure provides the following:

(D-1) A pharmaceutical composition which comprises as an active ingredient at least one other anticancer agent, and is for use in combination with a multispecific antigen-binding molecule comprising (i) a first antigen-binding moiety that is capable of binding to CD3 and CD137 and that binds to either CD3 or CD137, and (ii) a second antigen-binding moiety that is capable of binding to claudin 6 (CLDN6);

(D-2) The pharmaceutical composition of (D-1), wherein the multispecific antigen-binding molecule is any one of the multispecific antigen-binding molecules of (A-2) to (A-39);

(D-3) The pharmaceutical composition of either (D-1) or (D-2), wherein the cancer of interest is CLDN6-positive cancer;

(D-4) The pharmaceutical composition of any one of (D-1) to (D-3), wherein the cancer of interest is any cancer selected from the group consisting of ovary cancer, non-small cell lung cancer, gastric cancer, liver cancer, endometrial cancer, germ cell tumor, large bowel cancer, urinary bladder cancer, and atypical teratoid rhabdoid tumor;

(D-5) The pharmaceutical composition of any one of (D-1) to (D-4), wherein the cancer of interest is cancer metastasized to the peritoneum;

(D-6) The pharmaceutical composition of any one of (D-1) to (D-5), wherein the cancer of interest is peritoneally disseminated cancer;

(D-7) The pharmaceutical composition of any one of (D-1) to (D-6), which is for treating patients with cancer unresponsive to treatment with the at least one other anticancer agent or an anticancer agent which is different from the at least one other anticancer agent;

(D-8) The pharmaceutical composition of any one of (D-1) to (D-7), wherein the cancer of interest is cancer previously treated with the at least one other anticancer agent or an anticancer agent which is different from the at least one other anticancer agent;

(D-9) The pharmaceutical composition of any one of (D-1) to (D-8), wherein the cancer of interest is cancer for which desired effects could not be obtained by treatment with administration of the at least one other anticancer agent alone;

(D-10) The pharmaceutical composition of any one of (D-1) to (D-9), wherein the at least one other anticancer agent and the multispecific antigen-binding molecule are administered separately or sequentially;

(D-11) The pharmaceutical composition of any one of (D-1) to (D-10), wherein the at least one other anticancer agent is administered before, simultaneously with, and/or after the administration of the multispecific antigen-binding molecule;

(D-12) The pharmaceutical composition of any one of (D-1) to (D-11), wherein administration of the multispecific antigen-binding molecule enhances the antitumor effect of the at least one other anticancer agent;

(D-13) The pharmaceutical composition of any one of (D-1) to (D-12), wherein the at least one other anticancer agent is at least one selected from the group consisting of a chemotherapeutic agent, an immune checkpoint inhibitor, and a PARP inhibitor;

(D-14) The pharmaceutical composition of any one of (D-1) to (D-13), wherein the at least one other anticancer agent is an agent that enhances expression of CLDN6 in a cell;

(D-15) The pharmaceutical composition of any one of (D-1) to (D-14), wherein the at least one other anticancer agent is an agent that induces expression of TGFβ in a cell;

(D-16) The pharmaceutical composition of any one of (D-1) to (D-15), wherein the at least one other anticancer agent is an agent that induces expression of TGFβ1 in a cell;

(D-17) The pharmaceutical composition of any one of (D-13) to (D-16),wherein the at least one other anticancer agent is a platinum preparation, an alkaloid, or an antimetabolite;

(D-18) The pharmaceutical composition of any one of (D-13) to (D-17),wherein the at least one other anticancer agent is a platinum preparation;

(D-19) The pharmaceutical composition of any one of (D-13) to (D-17),wherein the at least one other anticancer agent is a plant alkaloid;

(D-20) The pharmaceutical composition of any one of (D-3) to (D-17),wherein the at least one other anticancer agent is a topoisomerase inhibitor;

(D-22) The pharmaceutical composition of any one of (D-13) to (D-17),wherein the at least one other anticancer agent is an antimetabolite;

(D-23) The pharmaceutical composition of any one of (D-13) to (D-17), wherein the at least one other anticancer agent is carboplatin or cisplatin;

(D-24) The pharmaceutical composition of any one of (D-13) to (D-17),wherein the at least one other anticancer agent is irinotecan;

(D-25) The pharmaceutical composition of any one of (D-13) to (D-17),wherein the at least one other anticancer agent is gemcitabine;

(D-26) The pharmaceutical composition of (D-13), wherein the at least one other anticancer agent is an anti-PD-L1 antibody;

(D-27) The pharmaceutical composition of (D-13), wherein the at least one other anticancer agent is olaparib; and

(D-28) A cytotoxicity-inducing agent, a cell growth suppressor, a cell growth inhibitor, an immune response activator, a cancer therapeutic agent, or a cancer preventive agent, comprising the pharmaceutical composition of any one of (D-1) to (D-27).

Furthermore, the present disclosure provides the following:

(E-1) A pharmaceutical composition for treating or preventing cancer formed by combining a multispecific antigen-binding molecule and at least one other anticancer agent,
the multispecific antigen-binding molecule comprising (i) a first antigen-binding moiety that is capable of binding to CD3 and CD137 and that binds to either CD3 or CD137, and (ii) a second antigen-binding moiety that is capable of binding to claudin 6 (CLDN6);
(E-2) The pharmaceutical composition of (E-1), wherein the multispecific antigen-binding molecule is any of the multispecific antigen-binding molecules of (A-2) to (A-39);
(E-3) The pharmaceutical composition of either (E-1) or (E-2), wherein the cancer is CLDN6-positive cancer;
(E-4) The pharmaceutical composition of any one of (E-1) to (E-3), wherein the cancer is any cancer selected from the group consisting of ovary cancer, non-small cell lung cancer, gastric cancer, liver cancer, endometrial cancer, germ cell tumor, large bowel cancer, urinary bladder cancer, and atypical teratoid rhabdoid tumor;
(E-5) The pharmaceutical composition of any one of (E-1) to (E-4), wherein the cancer is cancer metastasized to the peritoneum;
(E-6) The pharmaceutical composition of any one of (E-1) to (E-5), wherein the cancer is peritoneally disseminated cancer;
(E-7) The pharmaceutical composition of any one of (E-1) to (E-6), which is for treating patients with cancer unresponsive to treatment with the at least one other anticancer agent or an anticancer agent which is different from the at least one other anticancer agent;
(E-8) The pharmaceutical composition of any one of (E-1) to (E-7), wherein the cancer is cancer previously treated with the at least one other anticancer agent or an anticancer agent which is different from the at least one other anticancer agent;
(E-9) The pharmaceutical composition of any one of (E-1) to (E-8), wherein the cancer is cancer for which desired effects could not be obtained by treatment with administration of the at least one other anticancer agent alone;
(E-10) The pharmaceutical composition of any one of (E-1) to (E-9), wherein administration of the at least one other anticancer agent enhances the antitumor effect of the multispecific antigen-binding molecule;
(E-11) The pharmaceutical composition of any one of (E-1) to (E-9), wherein administration of the multispecific antigen-binding molecule enhances the antitumor effect of the at least one other anticancer agent;
(E-12) The pharmaceutical composition of any one of (E-1) to (E-11), wherein the pharmaceutical composition is a combination preparation;
(E-13) the pharmaceutical composition of any one of (E-1) to (E-12), wherein the multispecific antigen-binding molecule and the at least one other anticancer agent are administered separately or sequentially;
(E-14) The pharmaceutical composition of (E-13), wherein the multispecific antigen-binding molecule is administered before, simultaneously with, and/or after the administration of the at least one other anticancer agent;
(E-15) The pharmaceutical composition of any one of (E-1) to (E-14), wherein the at least one other anticancer agent is at least one selected from the group consisting of a chemotherapeutic agent, an immune checkpoint inhibitor, and a PARP inhibitor;
(E-16) The pharmaceutical composition of any one of (E-1) to (E-15), wherein the at least one other anticancer agent is an agent that enhances expression of CLDN6 in a cancer cell;
(E-17) The pharmaceutical composition of any one of (E-1) to (E-16), wherein the at least one other anticancer agent is an agent that induces expression of TGF$\beta$ in a cell;
(E-18) The pharmaceutical composition of any one of (E-1) to (E-17), wherein the at least one other anticancer agent is an agent that induces expression of TGF$\beta$1 in a cell;
(E-19) The pharmaceutical composition of any one of (E-15) to (E-18),wherein the at least one other anticancer agent is a platinum preparation, an alkaloid, or an antimetabolite;
(E-20) The pharmaceutical composition of any one of (E-15) to (E-19),wherein the at least one other anticancer agent is a platinum preparation;
(E-21) The pharmaceutical composition of any one of (E-15) to (E-19),wherein the at least one other anticancer agent is an alkaloid;
(E-22) The pharmaceutical composition of any one of (E-15) to (E-19),wherein the at least one other anticancer agent is a topoisomerase inhibitor;
(E-23) The pharmaceutical composition of any one of (E-15) to (E-19),wherein the at least one other anticancer agent is an antimetabolite;
(E-24) The pharmaceutical composition of any one of (E-15) to (E-19), wherein the at least one other anticancer agent is carboplatin or cisplatin;
(E-25) The pharmaceutical composition of any one of (E-15) to (E-19),wherein the at least one other anticancer agent is irinotecan;

(E-26) The pharmaceutical composition of any one of (E-15) to (E-19),wherein the at least one other anticancer agent is gemcitabine;

(E-27) The pharmaceutical composition of (E-15), wherein the at least one other anticancer agent is an anti-PD-L1 antibody;

(E-28) The pharmaceutical composition of (E-15), wherein the at least one other anticancer agent is olaparib;

(E-29) A cytotoxicity-inducing agent, a cell growth suppressor, a cell growth inhibitor, an immune response activator, a cancer therapeutic agent, or a cancer preventive agent, comprising the pharmaceutical composition of any one of (E-1) to (E-28);

(E2-1) A pharmaceutical composition for treating or preventing cancer formed by combining a multispecific antigen-binding molecule and at least one TGFβ inducing agent,

wherein the multispecific antigen-binding molecule comprising (i) a first antigen-binding moiety that is capable binding to CD3 and CD137 and that binds to either CD3 or CD137, and (ii) a second antigen-binding moiety that is capable of binding to claudin 6 (CLDN6);

(E2-2) The pharmaceutical composition of (E2-1), wherein the multispecific antigen-binding molecule is any of the multispecific antigen-binding molecules of (A-2) to (A-3 9);

(E2-3) The pharmaceutical composition of either (E2-1) or (E2-2), wherein the cancer is CLDN6-positive cancer;

(E2-4) The pharmaceutical composition of any one of (E2-1) to (E2-3), wherein the cancer is any cancer selected from the group consisting of ovary cancer, non-small-cell lung cancer, gastric cancer, liver cancer, endometrial cancer, germ cell tumor, large bowel cancer, urinary bladder cancer, and atypical teratoid rhabdoid tumor;

(E2-5) The pharmaceutical composition of any one of (E2-1) to (E2-4), wherein the cancer is cancer metastasized to the peritoneum;

(E2-6) The pharmaceutical composition of any one of (E2-1) to (E2-5), wherein the cancer is peritoneally disseminated cancer;

(E2-7) The pharmaceutical composition of any one of (E2-1) to (E2-6), which is for treating patients with cancer unresponsive to treatment with the at least one TGFβ inducing agent or a TGFβ inducing agent which is different from the at least one TGFβ inducing agent;

(E2-8) The pharmaceutical composition of any one of (E2-1) to (E2-7), wherein the cancer is cancer previously treated with the at least one TGFβ inducing agent or a TGFβ inducing agent which is different from the at least one TGFβ inducing agent;

(E2-9) The pharmaceutical composition of any one of (E2-1) to (E2-8), wherein the cancer is cancer for which desired effects could not be obtained by treatment with administration of the at least one TGFβ inducing agent alone;

(E2-10) The pharmaceutical composition of any one of (E2-1) to (E2-9), wherein administration of the at least one TGFβ inducing agent enhances the antitumor effect of the multispecific antigen-binding molecule;

(E2-11) The pharmaceutical composition of any one of (E2-1) to (E2-10), wherein the pharmaceutical composition is a combination preparation;

(E2-12) The pharmaceutical composition of any one of (E2-1) to (E2-11), wherein the multispecific antigen-binding molecule and the at least one TGFβ inducing agent are administered separately or sequentially;

(E2-13) The pharmaceutical composition of (E2-12), wherein the multispecific antigen-binding molecule is administered before, simultaneously with, and/or after the administration of the at least one TGFβ inducing agent;

(E2-14) A cytotoxicity-inducing agent, a cell growth suppressor, a cell growth inhibitor, an immune response activator, a cancer therapeutic agent, or a cancer preventive agent, comprising the pharmaceutical composition of any one of (E2-1) to (E2-13);

(E3-1) A pharmaceutical composition for treating or preventing cancer formed by combining a multispecific antigen-binding molecule and at least one CLDN6 expression inducing agent,

wherein the multispecific antigen-binding molecule comprising (i) a first antigen-binding moiety that is capable of binding to CD3 and CD137 and that binds to either CD3 or CD137, and (ii) a second antigen-binding moiety that is capable of binding to claudin 6 (CLDN6);

(E3-2) The pharmaceutical composition of (E3-1), wherein the multispecific antigen-binding molecule is any of the multispecific antigen-binding molecules of (A-2) to (A-3 9);

(E3-3) The pharmaceutical composition of either (E3-1) or (E3-2), wherein the cancer is CLDN6-positive cancer;

(E3-4) The pharmaceutical composition of any one of (E3-1) to (E3-3), wherein the cancer is any cancer selected from the group consisting of ovary cancer, non-small-cell lung cancer, gastric cancer, liver cancer, endometrial cancer, germ cell tumor, large bowel cancer, urinary bladder cancer, and atypical teratoid rhabdoid tumor;

(E3-5) The pharmaceutical composition of any one of (E3-1) to (E3-4), wherein the cancer is cancer metastasized to the peritoneum;

(E3-6) The pharmaceutical composition of any one of (E3-1) to (E3-5), wherein the cancer is peritoneally disseminated cancer;

(E3-7) The pharmaceutical composition of any one of (E3-1) to (E3-6), which is for treating patients with cancer unresponsive to treatment with the at least one CLDN6 expression inducing agent or a CLDN6 expression inducing agent which is different from the at least one CLDN6 expression inducing agent;
(E3-8) The pharmaceutical composition of any one of (E3-1) to (E3-7), wherein the cancer is cancer previously treated with the at least one CLDN6 expression inducing agent or a CLDN6 expression inducing agent which is different from the at least one CLDN6 expression inducing agent;
(E3-9) The pharmaceutical composition of any one of (E3-1) to (E3-8), wherein the cancer is cancer for which desired effects could not be obtained by treatment with administration of the at least one CLDN6 expression inducing agent alone;
(E3-10) The pharmaceutical composition of any one of (E3-1) to (E3-9), wherein administration of the at least one CLDN6 expression inducing agent enhances the antitumor effect of the multispecific antigen-binding molecule;
(E3-11) The pharmaceutical composition of any one of (E3-1) to (E3-10), wherein the pharmaceutical composition is a combination preparation;
(E3-12) The pharmaceutical composition of any one of (E3-1) to (E3-11), wherein the multispecific antigen-binding molecule and the at least one TGFβ inducing agent are administered separately or sequentially;
(E3-13) The pharmaceutical composition of (E3-12), wherein the multispecific antigen-binding molecule is administered before, simultaneously with, and/or after the administration of the at least one TGFβ inducing agent; and
(E3-14) A cytotoxicity-inducing agent, a cell growth suppressor, a cell growth inhibitor, an immune response activator, a cancer therapeutic agent, or a cancer preventive agent, comprising the pharmaceutical composition of any one of (E3-1) to (E3-13).
(F-1) A combination of a multispecific antigen-binding molecule and at least one other anticancer agent,
the multispecific antigen-binding molecule comprising (i) a first antigen-binding moiety that is capable of binding to CD3 and CD137 and that binds to either CD3 or CD137, and (ii) a second antigen-binding moiety that is capable of binding to claudin 6 (CLDN6);
(F-2) The combination of (F-1), wherein the multispecific antigen-binding molecule is the multispecific antigen-binding molecule of any of (A-2) to (A-39);
(F-3) The combination of (F-1) or (F-2), wherein the cancer of interest is CLDN6-positive cancer;
(F-4) The combination of any one of (F-1) to (F-3), wherein the cancer of interest is any cancer selected from the group consisting of ovary cancer, non-small cell lung cancer, gastric cancer, liver cancer, endometrial cancer, germ cell tumor, large bowel cancer, urinary bladder cancer, and atypical teratoid rhabdoid tumor;
(F-5) The combination of any one of (F-1) to (F-4), wherein the cancer of interest is cancer metastasized to the peritoneum;
(F-6) The combination of any one of (F-1) to (F-5), wherein the cancer of interest is peritoneally disseminated cancer;
(F-7) The combination of any one of (F-1) to (F-6), which is for treating patients with cancer unresponsive to treatment with the at least one other anticancer agent or an anticancer agent which is different from the at least one other anticancer agent;
(F-8) The combination of any one of (F-1) to (F-7), wherein the cancer of interest is cancer previously treated with the at least one other anticancer agent or an anticancer agent which is different from the at least one other anticancer agent;
(F-9) The combination of any one of (F-1) to (F-8), wherein the cancer of interest is cancer for which desired effects could not be obtained by treatment with administration of the at least one other anticancer agent alone;
(F-10) The combination of any one of (F-1) to (F-9), wherein the at least one other anticancer agent enhances the antitumor effect of the multispecific antigen-binding molecule;
(F-1 1) The combination of any one of (F-1) to (F-9), wherein the multispecific antigen-binding molecule enhances the antitumor effect of the at least one other anticancer agent;
(F-12) The combination of any one of (F-1) to (F-11), wherein the multispecific antigen-binding molecule and the at least one other anticancer agent are administered separately or sequentially;
(F-13) The combination of any one of (F-1) to (F-12), wherein the multispecific antigen-binding molecule is administered before, simultaneously with, and/or after the administration of the at least one other anticancer agent;
(F-14) The combination of any one of (F-1) to (F-13), wherein the at least one other anticancer agent is at least one selected from the group consisting of a chemotherapeutic agent, an immune checkpoint inhibitor, and a PARP inhibitor;
(F-15) The combination of any one of (F-1) to (F-14), wherein the at least one other anticancer agent is an agent that enhances expression of CLDN6 in a cell;
(F-16) The combination of any one of (F-1) to (F-15), wherein the at least one other anticancer agent is an agent that induces expression of TGFβ in a cell;
(F-17) The combination of any one of (F-1) to (F-16), wherein the at least one other anticancer agent is an agent that induces expression of TGFβ1 in a cell;
(F-18) The combination of any one of (F-14) to (F-17),wherein the at least one other anticancer agent is a platinum preparation, an alkaloid, or an antimetabolite;

(F-19) The combination of any one of (F-14) to (F-18),wherein the at least one other anticancer agent is a platinum preparation;

(F-20) The combination of any one of (F-14) to (F-18),wherein the at least one other anticancer agent is an alkaloid;

(F-21) The combination of any one of (F-14) to (F-18),wherein the at least one other anticancer agent is a topoisomerase inhibitor;

(F-22) The combination of any one of (F-14) to (F-18),wherein the at least one other anticancer agent is an antimetabolite;

(F-23) The combination of any one of (F-14) to (F-17), wherein the at least one other anticancer agent is carboplatin or cisplatin;

(F-24) The combination of any one of (F-14) to (F-17),wherein the at least one other anticancer agent is irinotecan;

(F-25) The combination of any one of (F-14) to (F-17),wherein the at least one other anticancer agent is gemcitabine;

(F-26) The combination of (F-14), wherein the at least one other anticancer agent is an anti-PD-L1 antibody;

(F-26) The combination of (F-14), wherein the at least one other anticancer agent is olaparib;

(F-27) A cytotoxicity-inducing agent, a cell growth suppressor, a cell growth inhibitor, an immune response activator, a cancer therapeutic agent, or a cancer preventive agent, comprising the combination of any one of (F-1) to (F-26);

(F2-1) A combination of a multispecific antigen-binding molecule and at least one TGFβ inducing agent,

the multispecific antigen-binding molecule comprising (i) a first antigen-binding moiety that is capable of binding to CD3 and CD137 and that binds to either CD3 or CD137, and (ii) a second antigen-binding moiety that is capable of binding to claudin 6 (CLDN6);

(F2-2) The combination of (F2-1), wherein the multispecific antigen-binding molecule is any of the multispecific antigen-binding molecules of (A-2) to (A-39);

(F2-3) The combination of either (F2-1) or (F2-2), wherein the cancer of interest is CLDN6-positive cancer;

(F2-4) The combination of any one of (F2-1) to (F2-3), wherein the cancer is any cancer selected from the group consisting of ovary cancer, non-small-cell lung cancer, gastric cancer, liver cancer, endometrial cancer, germ cell tumor, large bowel cancer, urinary bladder cancer, and atypical teratoid rhabdoid tumor;

(F2-5) The combination of any one of (F2-1) to (F2-4), wherein the cancer is cancer metastasized to the peritoneum;

(F2-6) The pharmaceutical composition of any one of (F2-1) to (F2-5), wherein the cancer is peritoneally disseminated cancer;

(F2-7) The combination of any one of (F2-1) to (F2-6), which is for treating patients with cancer unresponsive to treatment with the at least one TGFβ inducing agent or a TGFβ inducing agent which is different from the at least one TGFβ inducing agent;

(F2-8) The combination of any one of (F2-1) to (F2-7), wherein the cancer is cancer previously treated with the at least one TGFβ inducing agent or a TGFβ inducing agent which is different from the at least one TGFβ inducing agent;

(F2-9) The combination of any one of (F2-1) to (F2-8), wherein the cancer is cancer for which desired effects could not be obtained by treatment with administration of the at least one TGFβ inducing agent alone;

(F2-10) The combination of any one of (F2-1) to (F2-9), wherein administration of the at least one TGFβ inducing agent enhances the antitumor effect of the multispecific antigen-binding molecule;

(F2-11) The combination of any one of (F2-1) to (F2-10), wherein the multi specific antigen-binding molecule and the at least one TGFβ inducing agent are administered separately or sequentially;

(F2-12) The combination of any one of (F2-1) to (F2-11), wherein the multispecific antigen-binding molecule is administered before, simultaneously with, and/or after the administration of the at least one TGFβ inducing agent;

(F2-13) The combination of any one of (F2-1) to (F2-12), wherein the at least one TGFβ inducing agent is an agent that induces expression of CLDN6 in a cell;

(F2-14) The combination of any one of (F2-1) to (F2-13), wherein the at least one TGFβ inducing agent is an agent that induces expression of TGFβ1 in a cell;

(F2-15) A cytotoxicity-inducing agent, a cell growth suppressor, a cell growth inhibitor, an immune response activator, a cancer therapeutic agent, or a cancer preventive agent, comprising the pharmaceutical composition of any one of (F2-1) to (F2-14);

(F3-1) A combination of a multispecific antigen-binding molecule and at least one CLDN6 expression inducing agent, the multispecific antigen-binding molecule comprising (i) a first antigen-binding moiety that is capable of binding to CD3 and CD137 and that binds to either CD3 or CD137, and (ii) a second antigen-binding moiety that is capable of binding to claudin 6 (CLDN6);

(F3-2) The combination of (F3-1), wherein the multispecific antigen-binding molecule is any of the multispecific antigen-binding molecules of (A-2) to (A-39);

(F3-3) The combination of either (F3-1) or (F3-2), wherein the cancer of interest is CLDN6-positive cancer;

(F3-4) The combination of any one of (F3-1) to (F3-3), wherein the cancer is any cancer selected from the group consisting of ovary cancer, non-small-cell lung cancer, gastric cancer, liver cancer, endometrial cancer, germ cell tumor, large bowel cancer, urinary bladder cancer, and atypical teratoid rhabdoid tumor;
(F3-5) The combination of any one of (F3-1) to (F3-4), wherein the cancer is cancer metastasized to the peritoneum;
(F3-6) The pharmaceutical composition of any one of (F3-1) to (F3-5), wherein the cancer is peritoneally disseminated cancer;
(F3-7) The combination of any one of (F3-1) to (F3-6), which is for treating patients with cancer unresponsive to treatment with the at least one CLDN6 expression inducing agent or a CLDN6 expression inducing agent which is different from the at least one CLDN6 expression inducing agent;
(F3-8) The combination of any one of (F3-1) to (F3-7), wherein the cancer is cancer previously treated with the at least one CLDN6 expression inducing agent or a CLDN6 expression inducing agent which is different from the at least one CLDN6 expression inducing agent;
(F3-9) The combination of any one of (F3-1) to (F3-8), wherein the cancer is cancer for which desired effects could not be obtained by treatment with administration of the at least one CLDN6 expression inducing agent alone;
(F3-10) The combination of any one of (F3-1) to (F3-9), wherein administration of the at least one CLDN6 expression inducing agent enhances the antitumor effect of the multispecific antigen-binding molecule;
(F3-11) The combination of any one of (F3-1) to (F3-10), wherein the multispecific antigen-binding molecule and the at least one CLDN6 expression inducing agent are administered separately or sequentially;
(F3-12) The combination of any one of (F3-1) to (F3-11), wherein the multispecific antigen-binding molecule is administered before, simultaneously with, and/or after the administration of the at least one CLDN6 expression inducing agent;
(F3-13) The combination of any one of (F3-1) to (F3-12), wherein the at least one CLDN6 expression inducing agent is an agent that induces expression of TGFβ in a cell;
(F3-14) The combination of any one of (F3-1) to (F3-13), wherein the at least one CLDN6 expression inducing agent is an agent that induces expression of TGFβ1 in a cell; and
(F3-15) A cytotoxicity-inducing agent, a cell growth suppressor, a cell growth inhibitor, an immune response activator, a cancer therapeutic agent, or a cancer preventive agent, comprising the pharmaceutical composition of any one of (F3-1) to (F3-14).
(G-1) A method for inducing cytotoxicity, suppressing cell proliferation, inhibiting cell proliferation, activating immune response, treating cancer, or preventing cancer in an individual, comprising administering an effective amount of a multispecific antigen-binding molecule and an effective amount of at least one other anticancer agent,
wherein the multispecific antigen-binding molecule comprises (i) a first antigen-binding moiety that is capable of binding to CD3 and CD137 and that binds to either CD3 or CD137, and (ii) a second antigen-binding moiety that is capable of binding to claudin 6 (CLDN6);
(G-2) A method for inducing cytotoxicity, suppressing cell proliferation, inhibiting cell proliferation, activating immune response, treating cancer, or preventing cancer in an individual by combined use of a multispecific antigen-binding molecule, comprising administering to the individual an effective amount of at least one other anticancer agent,
wherein the multispecific antigen-binding molecule comprises (i) a first antigen-binding moiety that is capable of binding to CD3 and CD137 and that binds to either CD3 or CD137, and (ii) a second antigen-binding moiety that is capable of binding to claudin 6 (CLDN6);
(G-3) A method for enhancing effects of inducing cytotoxicity, suppressing cell proliferation, inhibiting cell proliferation, activating immune response, treating cancer, or preventing cancer in an individual by a multispecific antigen-binding molecule, comprising administering to the individual an effective amount of at least one other anticancer agent,
wherein the multispecific antigen-binding molecule comprises (i) a first antigen-binding moiety that is capable of binding to CD3 and CD137 and that binds to either CD3 or CD137, and (ii) a second antigen-binding moiety that is capable of binding to claudin 6 (CLDN6);
(G-4) A method for enhancing effects of inducing cytotoxicity, suppressing cell proliferation, inhibiting cell proliferation, activating immune response, treating cancer, or preventing cancer in an individual by at least one other anticancer agent, comprising administering to the individual an effective amount of a multispecific antigen-binding molecule,
wherein the multispecific antigen-binding molecule comprises (i) a first antigen-binding moiety that is capable of binding to CD3 and CD137 and that binds to either CD3 or CD137, and (ii) a second antigen-binding moiety that is capable of binding to claudin 6 (CLDN6);
(G-5) The method of any one of (G-1) to (G-4), wherein the multispecific antigen-binding molecule is the multispecific antigen-binding molecule of any of (A-2) to (A-39);
(G-6) The method of any one of (G-1) to (G-5), wherein the cancer is CLDN6-positive cancer;
(G-7) The method of any one of (G-1) to (G-6), wherein the cancer is any cancer selected from the group consisting of ovary cancer, non-small cell lung cancer, gastric cancer, liver cancer, endometrial cancer, germ cell tumor, large bowel cancer, urinary bladder cancer, and atypical teratoid rhabdoid tumor;
(G-8) The method of any one of (G-1) to (G-7), wherein the cancer is cancer metastasized to the peritoneum;
(G-9) The method of any one of (G-1) to (G-8), wherein the cancer is peritoneally disseminated cancer;

(G-10) The method of any one of (G-1) to (G-9), wherein the multispecific antigen-binding molecule and the at least one other anticancer agent are administered separately or sequentially;
(G-11) The method of any one of (G-1) to (G-10), wherein the multispecific antigen-binding molecule is administered before, simultaneously with, and/or after the administration of the at least one other anticancer agent;
(G-12) The method of any one of (G-1) to (G-11), wherein the at least one other anticancer agent is at least one selected from the group consisting of a chemotherapeutic agent, an immune checkpoint inhibitor, and a PARP inhibitor;
(G-13) The method of any one of (G-1) to (G-12), wherein the at least one other anticancer agent is an agent that enhances expression of CLDN6 in a cell;
(G-14) The method of any one of (G-1) to (G-13), wherein the at least one other anticancer agent is an agent that induces expression of TGFβ in a cell;
(G-15) The method of any one of (G-1) to (G-14), wherein the at least one other anticancer agent is an agent that induces expression of TGFβ1 in a cell;
(G-16) The method of any one of (G-12) to (G-15),wherein the at least one other anticancer agent is a platinum preparation, an alkaloid, or an antimetabolite;
(G-17) The method of any one of (G-12) to (G-16),wherein the at least one other anticancer agent is a platinum preparation;
(G-18) The method of any one of (G-12) to (G-16),wherein the at least one other anticancer agent is an alkaloid;
(G-19) The method of any one of (G-12) to (G-16),wherein the at least one other anticancer agent is a topoisomerase inhibitor;
(G-20) The method of any one of (G-12) to (G-16),wherein the at least one other anticancer agent is an antimetabolite;
(G-21) The method of any one of (G-12) to (G-16), wherein the at least one other anticancer agent is carboplatin or cisplatin;
(G-22) The method of any one of (G-12) to (G-16),wherein the at least one other anticancer agent is irinotecan;
(G-23) The method of any one of (G-12) to (G-16),wherein the at least one other anticancer agent is gemcitabine;
(G-24) The method of (G-12), wherein the at least one other anticancer agent is an anti-PD-L1 antibody;
(G-25) The method of (G-12), wherein the at least one other anticancer agent is olaparib;
(G2-1) A method for inducing cytotoxicity, suppressing cell proliferation, inhibiting cell proliferation, activating immune response, treating cancer, or preventing cancer in an individual, comprising administering an effective amount of a multispecific antigen-binding molecule and an effective amount of at least one TGFβ inducing agent,
wherein the multispecific antigen-binding molecule comprises (i) a first antigen-binding moiety that is capable of binding to CD3 and CD137 and that binds to either CD3 or CD137, and (ii) a second antigen-binding moiety that is capable of binding to claudin 6 (CLDN6);
(G2-2) A method for inducing cytotoxicity, suppressing cell proliferation, inhibiting cell proliferation, activating immune response, treating cancer, or preventing cancer in an individual by combined use of a multispecific antigen-binding molecule, comprising administering to the individual an effective amount of at least one TGFβ inducing agent,
wherein the multispecific antigen-binding molecule comprises (i) a first antigen-binding moiety that is capable of binding to CD3 and CD137 and that binds to either CD3 or CD137, and (ii) a second antigen-binding moiety that is capable of binding to claudin 6 (CLDN6);
(G2-3) A method for inducing cytotoxicity, suppressing cell proliferation, inhibiting cell proliferation, activating immune response, treating cancer, or preventing cancer in an individual by a multispecific antigen-binding molecule, comprising administering to the individual an effective amount of at least one TGFβ inducing agent,
wherein the multispecific antigen-binding molecule comprises (i) a first antigen-binding moiety that is capable of binding to CD3 and CD137 and that binds to either CD3 or CD137, and (ii) a second antigen-binding moiety that is capable of binding to claudin 6 (CLDN6);
(G2-4) The method of any one of (G2-1) to (G2-3), wherein the multispecific antigen-binding molecule is the multispecific antigen-binding molecule of any of (A-2) to (A-39);
(G2-5) The method of either (G2-1) or (G2-4), wherein the cancer is CLDN6-positive cancer;
(G2-6) The method of any one of (G2-1) to (G2-5), wherein the cancer is any cancer selected from the group consisting of ovary cancer, non-small-cell lung cancer, gastric cancer, liver cancer, endometrial cancer, germ cell tumor, large bowel cancer, urinary bladder cancer, and atypical teratoid rhabdoid tumor;
(G2-7) The method of any one of (G2-1) to (G2-6), wherein the cancer is cancer metastasized to the peritoneum;
(G2-8) The method of any one of (G2-1) to (G2-7), wherein the cancer is peritoneally disseminated cancer;
(G2-9) The method of any one of (G2-1) to (G2-8), wherein the multispecific antigen-binding molecule and the at least one TGFβ inducing agent are administered separately or sequentially;
(G2-10) The method of any one of (G2-1) to (G2-9), wherein the multispecific antigen-binding molecule is administered before, simultaneously with, and/or after the administration of the at least one TGFβ inducing agent;
(G2-11) The method of any one of (G2-1) to (G2-10), wherein the at least one TGFβ inducing agent is an agent that enhances expression of CLDN6 in a cell;

(G2-12) The method of any one of (G2-1) to (G2-11), wherein the at least one TGFβ inducing agent is an agent that induces expression of TGFβ1 in a cancer cell;

(G3-1) A method for inducing cytotoxicity, suppressing cell proliferation, inhibiting cell proliferation, activating immune response, treating cancer, or preventing cancer in an individual, comprising administering an effective amount of a multispecific antigen-binding molecule and an effective amount of at least one CLDN6 expression inducing agent, wherein the multispecific antigen-binding molecule comprises (i) a first antigen-binding moiety that is capable of binding to CD3 and CD137 and that binds to either CD3 or CD137, and (ii) a second antigen-binding moiety that is capable of binding to claudin 6 (CLDN6);

(G3-2) A method for inducing cytotoxicity, suppressing cell proliferation, inhibiting cell proliferation, activating immune response, treating cancer, or preventing cancer in an individual by combined use of a multispecific antigen-binding molecule, comprising administering to the individual an effective amount of at least one CLDN6 expression inducing agent,

wherein the multispecific antigen-binding molecule comprises (i) a first antigen-binding moiety that is capable of binding to CD3 and CD137 and that binds to either CD3 or CD137, and (ii) a second antigen-binding moiety that is capable of binding to claudin 6 (CLDN6);

(G3-3) A method for inducing cytotoxicity, suppressing cell proliferation, inhibiting cell proliferation, activating immune response, treating cancer, or preventing cancer in an individual by a multispecific antigen-binding molecule, comprising administering to the individual an effective amount of at least one CLDN6 expression inducing agent,

wherein the multispecific antigen-binding molecule comprises (i) a first antigen-binding moiety that is capable of binding to CD3 and CD137 and that binds to either CD3 or CD137, and (ii) a second antigen-binding moiety that is capable of binding to claudin 6 (CLDN6);

(G3-4) The method of any one of (G3-1) to (G3-3), wherein the multispecific antigen-binding molecule is the multispecific antigen-binding molecule of any of (A-2) to (A-39);

(G3-5) The method of either (G3-1) or (G3-4), wherein the cancer is CLDN6-positive cancer;

(G3-6) The method of any one of (G3-1) to (G3-5), wherein the cancer is any cancer selected from the group consisting of ovary cancer, non-small-cell lung cancer, gastric cancer, liver cancer, endometrial cancer, germ cell tumor, large bowel cancer, urinary bladder cancer, and atypical teratoid rhabdoid tumor;

(G3-7) The method of any one of (G3-1) to (G3-6), wherein the cancer is cancer metastasized to the peritoneum;

(G3-8) The method of any one of (G3-1) to (G3-7), wherein the cancer is peritoneally disseminated cancer;

(G3-9) The method of any one of (G3-1) to (G3-8), wherein the multispecific antigen-binding molecule and the at least one TGFβ inducing agent are administered separately or sequentially;

(G3-10) The method of any one of (G3-1) to (G3-9), wherein the multispecific antigen-binding molecule is administered before, simultaneously with, and/or after the administration of the at least one TGFβ inducing agent;

(G3-11) The method of any one of (G3-1) to (G3-10), wherein the at least one TGFβ inducing agent is an agent that enhances expression of CLDN6 in a cell; and (G3-12) The method of any one of (G3-1) to (G3-11), wherein the at least one TGFβ inducing agent is an agent that induces expression of TGFβ1 in a cancer cell.

(H-1) A method for inducing damage to a cancer cell or a cancer cell-comprising tumor tissue, or a method for suppressing proliferation of a cancer cell or growth of a cancer cell-comprising tumor tissue, by contacting a cancer cell with a multispecific antigen-binding molecule and at least one other anticancer agent,

wherein the multispecific antigen-binding molecule comprises (i) a first antigen-binding moiety that is capable of binding to CD3 and CD137 and that binds to either CD3 or CD137, and (ii) a second antigen-binding moiety that is capable of binding to claudin 6 (CLDN6);

(H-2) A method for assessing whether a multispecific antigen-binding molecule and at least one other anticancer agent induce damage to a cancer cell or a cancer cell-comprising tumor tissue, or suppress proliferation of a cancer cell or growth of a cancer cell-comprising tumor tissue, by contacting a cancer cell with the multispecific antigen-binding molecule and the at least one other anticancer agent,

wherein the multispecific antigen-binding molecule comprises (i) a first antigen-binding moiety that is capable of binding to CD3 and CD137 and that binds to either CD3 or CD137, and (ii) a second antigen-binding moiety that is capable of binding to claudin 6 (CLDN6);

(H-3) The method of (H-1) or (H-2), wherein the multispecific antigen-binding molecule is the multispecific antigen-binding molecule of any of (A-2) to (A-39);

(H-4) The method of any one of (H-1) to (H-3), wherein the cancer cell is a CLDN6-positive cancer cell;

(H-5) The method of any one of (H-1) to (H-4), wherein the cancer cell is from at least one cancer selected from the group consisting of ovary cancer, non-small cell lung cancer, gastric cancer, liver cancer, endometrial cancer, germ cell tumor, large bowel cancer, urinary bladder cancer, and atypical teratoid rhabdoid tumor;

(H-6) The method of any one of (H-1) to (H-5), wherein the cancer cell is from peritoneally disseminated cancer

(H-7) The method of any one of (H-1) to (H-6), wherein the at least one other anticancer agent is at least one selected from the group consisting of a chemotherapeutic agent, an immune checkpoint inhibitor, and a PARP inhibitor;
(H-8) The method of any one of (H-1) to (H-7), wherein the at least one other anticancer agent is an agent that enhances expression of CLDN6 in a cell;
(H-9) The method of any one of (H-1) to (H-8), wherein the at least one other anticancer agent is an agent that induces expression of TGFβ in a cancer cell;
(H-10) The method of any one of (H-1) to (H-9), wherein the at least one other anticancer agent is an agent that induces expression of TGFβ1 in a cancer cell;
(H-11) The method of any one of (H-7) to (H-10),wherein the at least one other anticancer agent is a platinum preparation, an alkaloid, or an antimetabolite;
(H-12) The method of any one of (H-7) to (H-1 1),wherein the at least one other anticancer agent is a platinum preparation;
(H-13) The method of any one of (H-7) to (H-1 1),wherein the at least one other anticancer agent is an alkaloid;
(H-14) The method of any one of (H-7) to (H-1 1),wherein the at least one other anticancer agent is a topoisomerase inhibitor;
(H-15) The method of any one of (H-7) to (H-1 1),wherein the at least one other anticancer agent is an antimetabolite;
(H-16) The method of any one of (H-7) to (H-1 1), wherein the at least one other anticancer agent is carboplatin or cisplatin;
(H-16) The method of any one of (H-7) to (H-1 1),wherein the at least one other anticancer agent is irinotecan;
(H-17) The method of any one of (H-7) to (H-1 1),wherein the at least one other anticancer agent is gemcitabine;
(H-18) The method of (H-11), wherein the at least one other anticancer agent is an anti-PD-L1 antibody; and
(H-19) The method of (H-11), wherein the at least one other anticancer agent is olaparib.
(I-1) A kit comprising:

(A) a pharmaceutical composition comprising a multispecific antigen-binding molecule that comprises a first antigen-binding moiety that is capable of binding to CD3 and CD137 and that binds to either CD3 or CD137, and a second antigen-binding moiety that has binding activity to claudin 6 (CLDN6);
(B) a container; and
(C) an instruction or a label indicating that the multispecific antigen-binding molecule and at least one type of at least one other anticancer agent is administered in combination to an individual for treating or preventing cancer in the individual;

(I-2) A kit comprising:

(A) at least one other anticancer agent;
(B) a container; and
(C) an instruction or a label indicating that the at least one other anticancer agent and a pharmaceutical composition comprising a multispecific antigen-binding molecule that comprises a first antigen-binding moiety that is capable of binding to CD3 and CD137 and that binds to either CD3 or CD137, and a second antigen-binding moiety that has binding activity to claudin 6 (CLDN6) are administered in combination to an individual for treating or preventing cancer in the individual;

(I-3) The kit of (I-1) or (I-2), wherein the multispecific antigen-binding molecule or at least one other anticancer agent is loaded into the container;
(I-4) A kit comprising:

(A) a pharmaceutical composition comprising a multispecific antigen-binding molecule comprising a first antigen-binding moiety that is capable of binding to CD3 and CD137 and that binds to either CD3 or CD137, and a second antigen-binding moiety that has binding activity to claudin 6 (CLDN6);
(B) a container; and
(C) at least one other anticancer agent;

(I-5) The kit of (I-4), wherein the multispecific antigen-binding molecule and the at least one other anticancer agent are loaded into a single container, or loaded into separate containers, respectively;
(I-6) The kit of any one of (I-1) to (I-5), wherein the multispecific antigen-binding molecule is the multispecific antigen-binding molecule of any of (A-2) to (A-39);
(I-7) The kit of any one of (I-1) to (I-6), wherein the cancer of interest is CLDN6-positive cancer;
(I-8) The kit of any one of (I-1) to (I-7), wherein the cancer of interest is any cancer selected from the group consisting of ovary cancer, non-small cell lung cancer, gastric cancer, liver cancer, endometrial cancer, germ cell tumor, large bowel cancer, urinary bladder cancer, and atypical teratoid rhabdoid tumor;
(I-9) The kit of any one of (I-1) to (I-8), wherein the cancer is cancer metastasized to the peritoneum;
(I-10) The kit of any one of (I-1) to (I-9), wherein the cancer of interest is peritoneally disseminated cancer;
(I-11) The kit of any one of (I-1) to (I-10), wherein the multi specific antigen-binding molecule and the at least one other anticancer agent are administered separately or sequentially;
(I-12) The kit of any one of (I-1) to (I-11), wherein the multi specific antigen-binding molecule is administered before, simultaneously with, and/or after the administration of the at least one other anticancer agent;
(I-13) The kit of any one of (I-1) to (I-12), wherein the at least one other anticancer agent is at least one selected from the group consisting of a chemotherapeutic agent, an immune checkpoint inhibitor, and a PARP inhibitor;
(I-14) The kit of any one of (I-1) to (I-13), wherein the at least one other anticancer agent is an agent that enhances expression of CLDN6 in a cell;
(I-15) The kit of any one of (I-1) to (I-14), wherein the at least one other anticancer agent is an agent that induces expression of TGFβ in a cancer cell;
(I-16) The kit of any one of (I-1) to (I-15), wherein the at least one other anticancer agent is an agent that induces expression of TGFβ1 in a cancer cell;
(I-17) The kit of any one of (I-1) to (I-16),wherein the at least one other anticancer agent is a platinum preparation, an alkaloid, or an antimetabolite;
(I-18) The kit of any one of (I-1) to (I-17),wherein the at least one other anticancer agent is a platinum preparation;
(I-19) The kit of any one of (I-1) to (I-17),wherein the at least one other anticancer agent is an alkaloid;
(I-20) The kit of any one of (I-1) to (I-17),wherein the at least one other anticancer agent is a topoisomerase inhibitor;
(I-21) The kit of any one of (I-1) to (I-17),wherein the at least one other anticancer agent is an antimetabolite;
(I-22) The kit of any one of (I-1) to (I-17), wherein the at least one other anticancer agent is carboplatin or cisplatin;
(I-22) The kit of any one of (I-1) to (I-17),wherein the at least one other anticancer agent is irinotecan;
(I-23) The kit of any one of (I-1) to (I-17),wherein the at least one other anticancer agent is gemcitabine;
(I-24) The kit of any one of (I-1) to (I-17), wherein the at least one other anticancer agent is an anti-PD-L1 antibody; and
(I-25) The kit of any one of (I-1) to (I-17), wherein the at least one other anticancer agent is olaparib.
(I2-1) A kit comprising:

(A) at least one TGFβ inducing agent;
(B) a container; and
(C) an instruction or a label indicating that the at least one other anticancer agent and a pharmaceutical composition comprising a multispecific antigen-binding molecule that comprises a first antigen-binding moiety that is capable of binding to CD3 and CD137 and that binds to either CD3 or CD137, and a second antigen-binding moiety that has binding activity to claudin 6 (CLDN6) are administered in combination to an individual for treating or preventing cancer in the individual;

(I2-2) The kit of (I2-1), wherein the multispecific antigen-binding molecule and the at least one TGFβ inducing agent are loaded into the container;
(I2-3) A kit comprising:

(A) a pharmaceutical composition comprising a multispecific antigen-binding molecule that comprises a first antigen-binding moiety that is capable of binding to CD3 and CD137 and that binds to either CD3 or CD137, and a second antigen-binding moiety that has binding activity to claudin 6 (CLDN6);
(B) a container; and
(C) at least one TGFβ inducing agent;

(I2-4) The kit of (I2-3), wherein the multispecific antigen-binding molecule and the at least one TGFβ inducing agent are loaded into a single container, or loaded into separate containers, respectively;
(I2-5) The kit of any one of (12-1) to (I2-4), wherein the multi specific antigen-binding molecule is the multispecific antigen-binding molecule of any of (A-2) to (A-39);
(I2-6) The method of any one of (12-1) to (I2-5), wherein cancer of interest is CLDN6-positive cancer;
(I2-7) The kit of any one of (12-1) to (I2-6), wherein cancer of interest is any cancer selected from the group consisting of ovary cancer, non-small-cell lung cancer, gastric cancer, liver cancer, endometrial cancer, germ cell tumor, large bowel cancer, urinary bladder cancer, and atypical teratoid rhabdoid tumor;
(I2-8) The kit of any one of (I2-1) to (I2-7), wherein the cancer is cancer metastasized to the peritoneum;
(I2-9) The kit of any one of (I2-1) to (I2-8), wherein the cancer is peritoneally disseminated cancer;
(I2-10) The method of any one of (I2-1) to (I2-9), wherein the multispecific antigen-binding molecule and the at least one TGFβ inducing agent are administered separately or sequentially;

(I2-11) The method of any one of (I2-1) to (I2-10), wherein the multispecific antigen-binding molecule is administered before, simultaneously with, and/or after the administration of the at least one TGFβ inducing agent;
(I2-12) The method of any one of (I2-1) to (I2-11), wherein the at least one TGFβ inducing agent is an agent that enhances expression of CLDN6 in a cell;
(I2-13) The method of any one of (I2-1) to (I2-12), wherein the at least one TGFβ inducing agent is an agent that induces expression of TGFβ in a cell;
(I2-14) The method of any one of (I2-1) to (I2-13), wherein the at least one TGFβ inducing agent is an agent that induces expression of TGFβ1 in a cell;
(I3-1) A kit comprising:

(A) at least one CLDN6 expression inducing agent;
(B) a container; and
(C) an instruction or a label indicating that the at least one other anticancer agent and a pharmaceutical composition comprising a multispecific antigen-binding molecule that comprises a first antigen-binding moiety that is capable of binding to CD3 and CD137 and that binds to either CD3 or CD137, and a second antigen-binding moiety that has binding activity to claudin 6 (CLDN6) are administered in combination to an individual for treating or preventing cancer in the individual;

(I3-2) The kit of (I3-1), wherein the multispecific antigen-binding molecule and the at least one CLDN6 expression inducing agent are loaded into the container;
(I3-3) A kit comprising:

(A) a pharmaceutical composition comprising a multispecific antigen-binding molecule that comprises a first antigen-binding moiety that is capable of binding to CD3 and CD137 and that binds to either CD3 or CD137, and a second antigen-binding moiety that has binding activity to claudin 6 (CLDN6);
(B) a container; and
(C) at least one CLDN6 expression inducing agent;

(I3-4) The kit of (I3-3), wherein the multispecific antigen-binding molecule and the at least one CLDN6 expression inducing agent are loaded into a single container, or loaded into separate containers, respectively;
(I3-5) The kit of any one of (13-1) to (13-4), wherein the multi specific antigen-binding molecule is the multispecific antigen-binding molecule of any of (A-2) to (A-39);
(I3-6) The method of any one of (13-1) to (13-5), wherein cancer of interest is CLDN6-positive cancer;
(I3-7) The kit of any one of (13-1) to (13-6), wherein cancer of interest is any cancer selected from the group consisting of ovary cancer, non-small-cell lung cancer, gastric cancer, liver cancer, endometrial cancer, germ cell tumor, large bowel cancer, urinary bladder cancer, and atypical teratoid rhabdoid tumor;
(I3-8) The kit of any one of (13-1) to (13-7), wherein the cancer is cancer metastasized to the peritoneum;
(I3-9) The kit of any one of (13-1) to (13-8), wherein the cancer is peritoneally disseminated cancer;
(I3-10) The method of any one of (I3-1) to (I3-9), wherein the multispecific antigen-binding molecule and the at least one CLDN6 expression inducing agent are administered separately or sequentially;
(I3-11) The method of any one of (I3-1) to (I3-10), wherein the multispecific antigen-binding molecule is administered before, simultaneously with, and/or after the administration of the at least one CLDN6 expression inducing agent;
(I3-12) The method of any one of (I3-1) to (I3-11), wherein the at least one CLDN6 expression inducing agent is an agent that enhances CLDN6 expression in a cell;
(I3-13) The method of any one of (I3-1) to (I3-12), wherein the at least one CLDN6 expression inducing agent is an agent that induces expression of TGFβ in a cell; and
(I3-14) The method of any one of (I3-1) to (I3-13), wherein the at least one CLDN6 expression inducing agent is an agent that induces expression of TGFβ1 in a cell.
(J-1) A multispecific antigen-binding molecule for use in cancer therapy, wherein the multispecific antigen-binding molecule comprises (i) a first antigen-binding moiety that is capable of binding to CD3 and CD137 and that binds to either CD3 or CD137, and (ii) a second antigen-binding moiety that has binding activity to claudin 6 (CLDN6); (J-2) The multispecific antigen-binding molecule of (J-1), which is the multispecific antigen-binding molecule of any of (A-2) to (A-39);
(J-3) The multispecific antigen-binding molecule of any one of (J-1) or (J-2), which is used for cancer therapy in combination with at least one agent selected from a group consisting of another anticancer agent, a TGFβ inducing agent, and CLDN6 expression inducing agent;
(J-4) A combination of a multispecific antigen-binding molecule and at least one agent selected from a group consisting of another anticancer agent, a TGFβ inducing agent, and CLDN6 expression inducing agent,

the multispecific antigen-binding molecule comprising (i) a first antigen-binding moiety that is capable of binding to CD3 and CD137 and that binds to either CD3 or CD137, and (ii) a second antigen-binding moiety that has binding activity to claudin 6 (CLDN6);

(J-5) The combination of (J-4), wherein the multispecific antigen-binding molecule is the multispecific antigen-binding molecule of any of (A-2) to (A-39);

(J-6) The multispecific antigen-binding molecule or the combination of any one of (J-3) to (J-5), wherein the multispecific antigen-binding molecule and the at least one agent are administered separately or sequentially;

(J-7) The multispecific antigen-binding molecule or the combination of (J-6), wherein the multispecific antigen-binding molecule is administered before, simultaneously with, and/or after the administration of the at least one agent;

(J-8) The multispecific antigen-binding molecule or the combination of any one of (J-3) to (J-7), wherein the at least one agent is at least one selected from the group consisting of a chemotherapeutic agent, an immune checkpoint inhibitor, and a PARP inhibitor;

(J-9) The multispecific antigen-binding molecule or the combination of any one of (J-3) to (J-8), wherein the at least one agent is an agent that induces expression of CLDN6 in a cell;

(J-10) The multispecific antigen-binding molecule or the combination of any one of (J-3) to (J-9), wherein the at least one agent is an agent that induces expression of TGFβ in a cancer cell;

(1-11) The multispecific antigen-binding molecule or the combination of any one of (J-3) to (J-10), wherein the at least one agent is an agent that induces expression of TGFβ1 in a cancer cell;

(J-12) The multispecific antigen-binding molecule or the combination of any one of (J-8) to (J-1 1),wherein the at least one agent is a platinum preparation, an alkaloid, or an antimetabolite;

(J-13) The multispecific antigen-binding molecule or the combination of any one of (J-8) to (J-12),wherein the at least one agent is a platinum preparation;

(J-14) The multispecific antigen-binding molecule or the combination of any one of (J-8) to (J-12),wherein the at least one agent is an alkaloid;

(J-15) The multispecific antigen-binding molecule or the combination of any one of (J-8) to (J-12),wherein the at least one agent is a topoisomerase inhibitor;

(J-16) The multispecific antigen-binding molecule or the combination of any one of (J-8) to (J-12),wherein the at least one agent is an antimetabolite;

(J-17) The multispecific antigen-binding molecule or the combination of any one of (J-8) to (J-12), wherein the at least one agent is carboplatin or cisplatin;

(J-18) The multispecific antigen-binding molecule or the combination of any one of (J-8) to (J-12),wherein the at least one agent is irinotecan;

(J-19) The multispecific antigen-binding molecule or the combination of any one of (J-8) to (J-12),wherein the at least one agent is gemcitabine;

(J-20) The multispecific antigen-binding molecule or the combination of (J-8), wherein the immune checkpoint inhibitor is an anti-PD-L1 antibody;

(J-21) The multispecific antigen-binding molecule or the combination of (J-8), wherein the at least one agent is olaparib;

(J-22) The multispecific antigen-binding molecule or the combination of any one of (J-1) to (J-21), wherein the cancer is CLDN6-positive cancer;

(J-23) The multispecific antigen-binding molecule or the combination of any one of (J-1) to (J-22), wherein the cancer is any cancer selected from the group consisting of ovary cancer, non-small cell lung cancer, gastric cancer, liver cancer, endometrial cancer, germ cell tumor, large bowel cancer, urinary bladder cancer, and atypical teratoid rhabdoid tumor;

(J-24) The multispecific antigen-binding molecule or the combination of any one of (J-1) to (J-23), wherein the cancer is cancer metastasized to the peritoneum;

(J-25) The multispecific antigen-binding molecule or the combination of any one of (J-1) to (J-24), wherein the cancer is peritoneally disseminated cancer;

(J-26) The multispecific antigen-binding molecule or the combination of any one of (J-1) to (J-25), which is for treating patients with cancer unresponsive to treatment with an immune checkpoint inhibitor;

(J-27) The multispecific antigen-binding molecule or the combination of any one of (J-1) to (J-26), wherein the cancer is cancer previously treated with the other anticancer agent or an anticancer agent which is different from the other anticancer agent; and

(J-28) The multispecific antigen-binding molecule or the combination of any one of (J-1) to (J-27), wherein the cancer is cancer for which desired effects could not be obtained by treatment with administration of the other anticancer agent alone.

(K-1) Use of a multispecific antigen-binding molecule in the manufacture of a medicament for treating cancer,
the multispecific antigen-binding molecule comprising (i) a first antigen-binding moiety that is capable of binding to CD3 and CD137 and that binds to either CD3 or CD137, and (ii) a second antigen-binding moiety that has binding activity to claudin 6 (CLDN6);

(K-2) The use of (K-1), wherein the multispecific antigen-binding molecule is the multispecific antigen-binding molecule of any one of (A-2) to (A-39);

(K-3) The use of any one of (K-1) or (K-2), wherein the multispecific antigen-binding molecule is used in combination with at least one agent selected from a group consisting of another anticancer agent, a TGFβ inducing agent, and CLDN6 expression inducing agent;

(K-4) Use of a combination of a multispecific antigen-binding molecule and at least one agent selected from a group consisting of another anticancer agent, a TGFβ inducing agent, and CLDN6 expression inducing agent, in the manufacture of a medicament for treating cancer,

the multispecific antigen-binding molecule comprising (i) a first antigen-binding moiety that is capable of binding to CD3 and CD137 and that binds to either CD3 or CD137, and (ii) a second antigen-binding moiety that has binding activity to claudin 6 (CLDN6);

(K-5) The use of (K-4), wherein the multispecific antigen-binding molecule is the multispecific antigen-binding molecule of any of (A-2) to (A-39);

(K-6) The use of (K-4) or (K-5), wherein the medicament is a combination preparation containing a multispecific antigen-binding molecule and the at least one agent;

(K-7) The use of any one of (K-3) to (K-5), wherein the multispecific antigen-binding molecule and the at least one agent are administered separately or sequentially;

(K-8) The use of (K-7), wherein the multispecific antigen-binding molecule is administered before, simultaneously with, and/or after the administration of the at least one agent;

(K-9) The use of any one of (K-3) to (K-8), wherein the at least one agent is at least one selected from the group consisting of a chemotherapeutic agent, an immune checkpoint inhibitor, and a PARP inhibitor;

(K-10) The use of any one of (K-3) to (K-9), wherein the at least one agent is an agent that enhances expression of CLDN6 in a cell;

(K-11) The use of any one of (K-3) to (K-10), wherein the at least one agent is an agent that induces expression of TGFβ in a cancer cell;

(K-12) The use of any one of (K-3) to (K-11), wherein the at least one agent is an agent that induces expression of TGFβ1 in a cancer cell;

(K-13) The use of any one of (K-9) to (K-12),wherein the at least one agent is a platinum preparation, an alkaloid, or an antimetabolite;

(K-14) The use of any one of (K-9) to (K-13),wherein the at least one agent is a platinum preparation;

(K-15) The use of any one of (K-9) to (K-13),wherein the at least one agent is an alkaloid;

(K-16) The use of any one of (K-9) to (K-13),wherein the at least one agent is a topoisomerase inhibitor;

(K-17) The use of any one of (K-9) to (K-13),wherein the at least one agent is an antimetabolite;

(K-18) The use of any one of (K-9) to (K-13), wherein the at least one agent is carboplatin or cisplatin;

(K-19) The use of any one of (K-9) to (K-13), wherein the at least one agent is irinotecan;

(K-20) The use of any one of (K-9) to (K-13), wherein the at least one agent is gemcitabine;

(K-21) The use of (K-9), wherein the at least one agent is an anti-PD-L1 antibody; (K-22) The use of (K-9), wherein the at least one agent is olaparib;

(K-23) The use of any one of (K-1) to (K-22), wherein the cancer is CLDN6-positive cancer;

(K-24) The use of any one of (K-1) to (K-23), wherein the cancer is any cancer selected from the group consisting of ovary cancer, non-small cell lung cancer, gastric cancer, liver cancer, endometrial cancer, germ cell tumor, large bowel cancer, urinary bladder cancer, and atypical teratoid rhabdoid tumor;

(K-25) The use of any one of (K-1) to (K-24), wherein the cancer is cancer metastasized to the peritoneum;

(K-26) The use of any one of (K-1) to (K-25), wherein the cancer is peritoneally disseminated cancer;

(K-27) The use of any one of (K-1) to (K-26), which is for treating patients with cancer unresponsive to treatment with an immune checkpoint inhibitor;

(K-28) The use of any one of (K-1) to (K-27), wherein the cancer is cancer previously treated with the at least one other anticancer agent or an anticancer agent which is different from the at least one other anticancer agent; and

(K-29) The use of any one of (K-1) to (K-28), wherein the cancer is cancer for which desired effects could not be obtained by treatment with administration of the at least one other anticancer agent alone.

[Brief Description of Drawings]

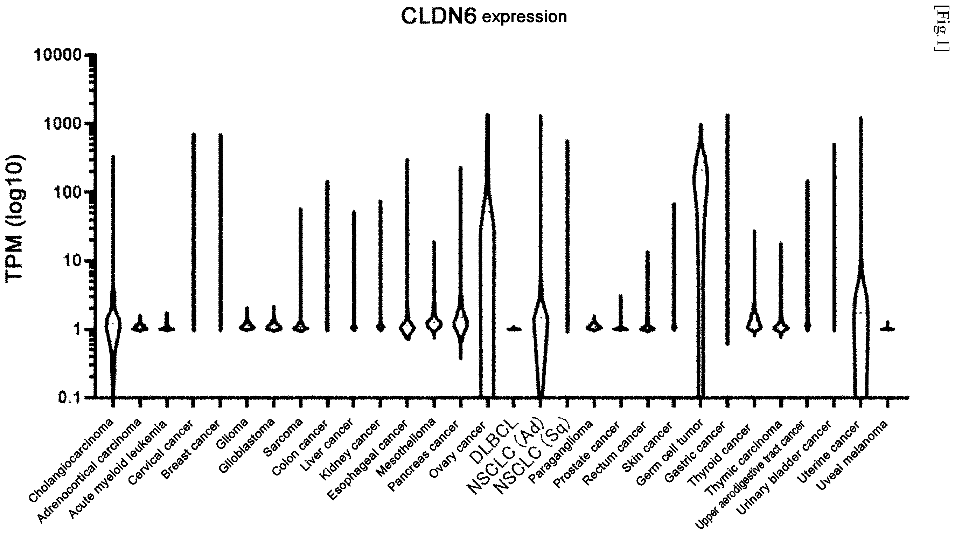

[Fig. 1] Fig. 1 is a graph comparing CLDN6 expression in various cancer tissues, based on the TCGA (The Cancer Genome Atlas) data.

[Fig. 2]

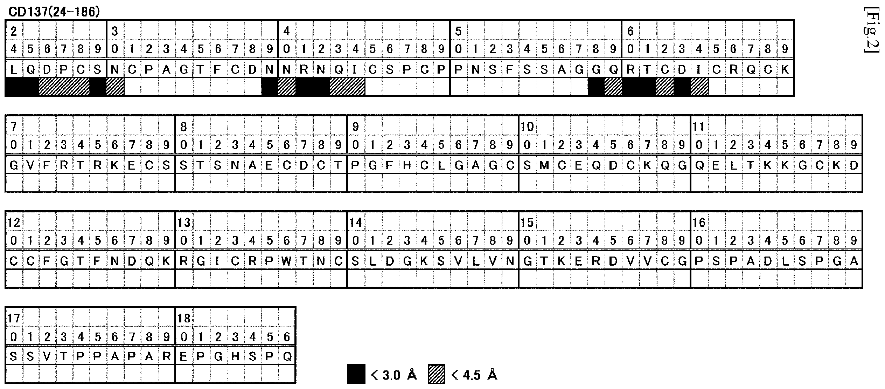

Figure 2 shows the epitope of the H0868L0581 Fab contact region on the CD137. Epitope mapping in the CD137 amino acid sequence (black: closer than 3.0 angstrom, stripes: closer than 4.5 angstrom from H0868L0581).

[Fig. 3]

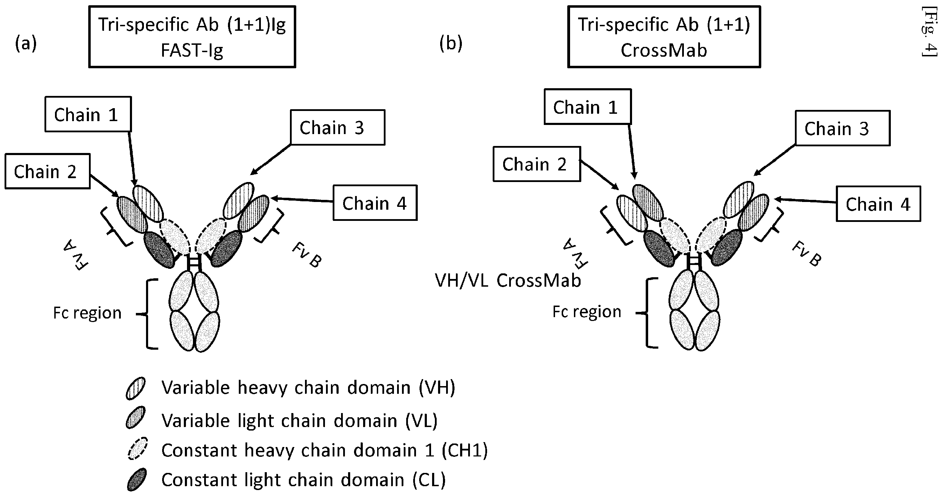

Figure 4 shows the epitope of the H0868L0581 Fab contact region on the CD137. Epitope mapping in the crystal structure (dark gray spheres: closer than 3.0 angstrom, light gray sticks: closer than 4.5 angstrom from H0868L0581).

[Fig. 4]

Figure 4 illustrates various antibody formats. Annotation of each Fv region for Table 4 and naming rule for Table 4, Table 5 and Table 6. Diagram depicting (a) 1+1 Bispecific antibodies by utilizing FAST-Ig; (b) 1+1 Bispecific antibodies by utilizing CrossMab technology.

[Fig. 5]

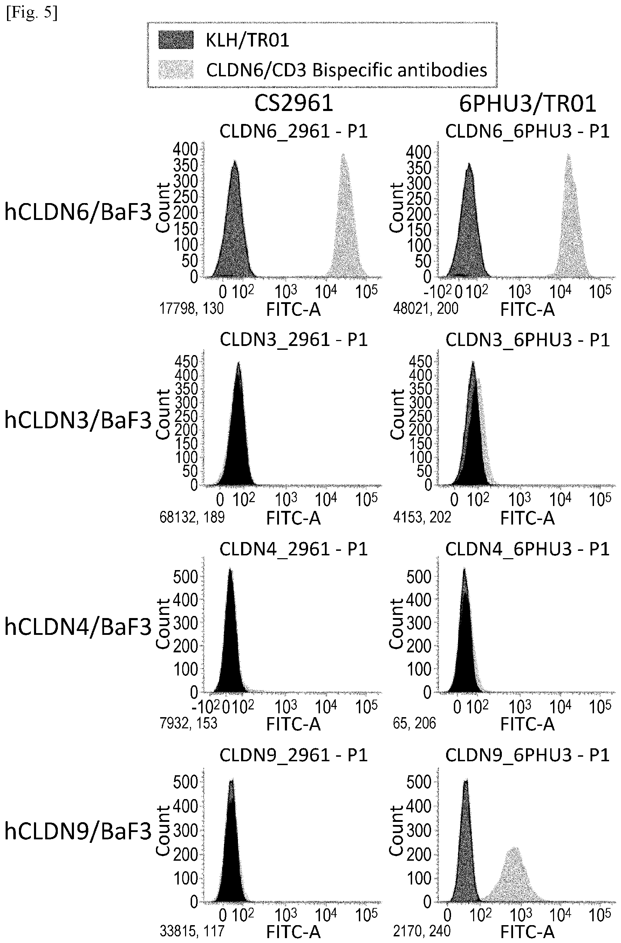

Figure 5 shows binding activity of anti-CLDN6/CD3 bispecific antibodies (CS2961, and 6PHU3/TR01) to human CLDN family proteins (CLDN3, CLDN4, CLDN6, and CLDN9). Binding activity of anti-CLDN6/CD3 bispecific antibodies to BaF3 transfectants (hCLDN6/BaF, hCLDN3/BaF, hCLDN4/BaF, and hCLDN9BaF) was examined by flow cytometer in a concentration of 15 micro g/ml, and plotted as histogram. KLH/TR01 was used as a negative control.

[Fig. 6]

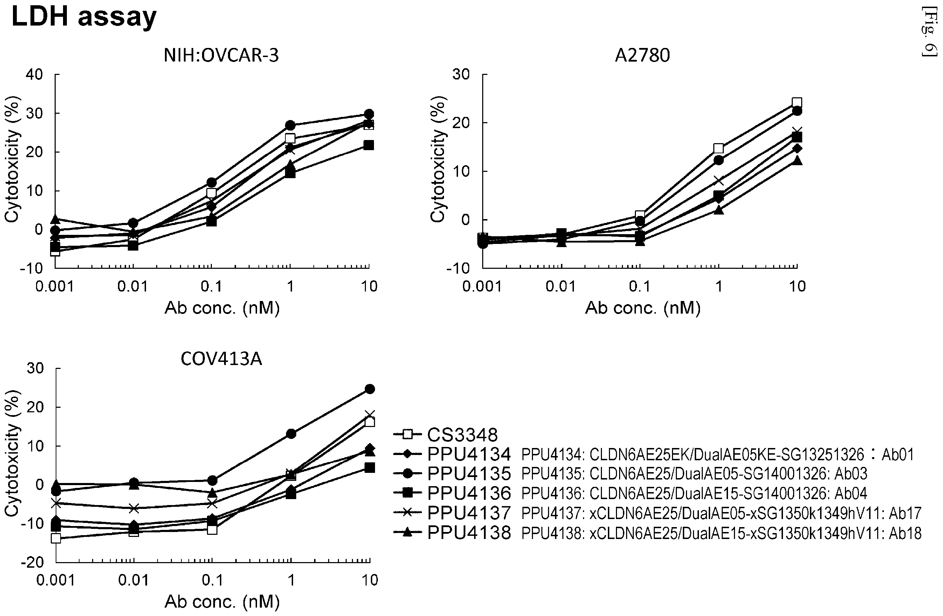

Figure 6 shows T-cell-dependent cell cytotoxicity evaluation by LDH assay.

[Fig. 7]

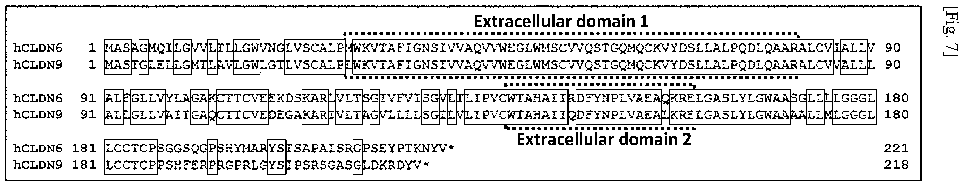

Figure 7 shows amino acid sequence alignment of human CLDN9 and human CLDN6. Human CLDN9 and human CLDN6 comprise almost the same sequence in extracellular domain 1, except the N-terminus residue (Met/Leu at position 29). Two amino acids in extracellular domain 2 are different between human CLDN9 and human CLDN6 (Arg/Leu at position 145 and Gln/Leu at position 156).

[Fig. 8]

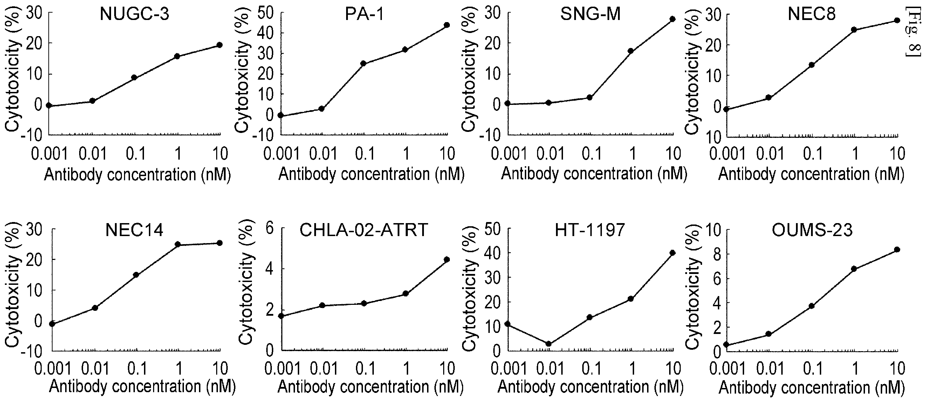

Figure 8 shows T-cell-dependent cell cytotoxicity evaluation results of the antibody (PPU4135) towards various cancer cell lines (NUGC-3, PA-1, SNG-M, NEC8, NEC14, CHLA-02-ATRT, HT-1197, and OUMS-23) by LDH assay.

[Fig. 9]

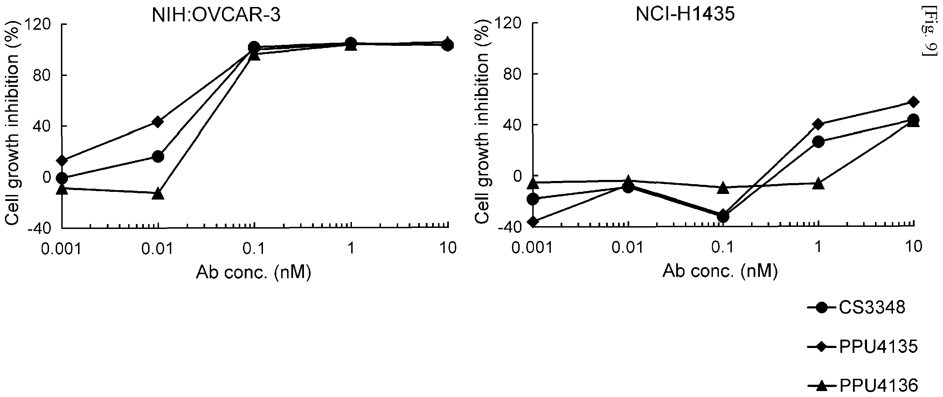

Figure 9 shows real-time cell growth inhibition assay results of the antibodies (CS3348, PPU4135 and PPU4136) towards various cancer cell lines by xCELLigence assay.

[Fig. 10]

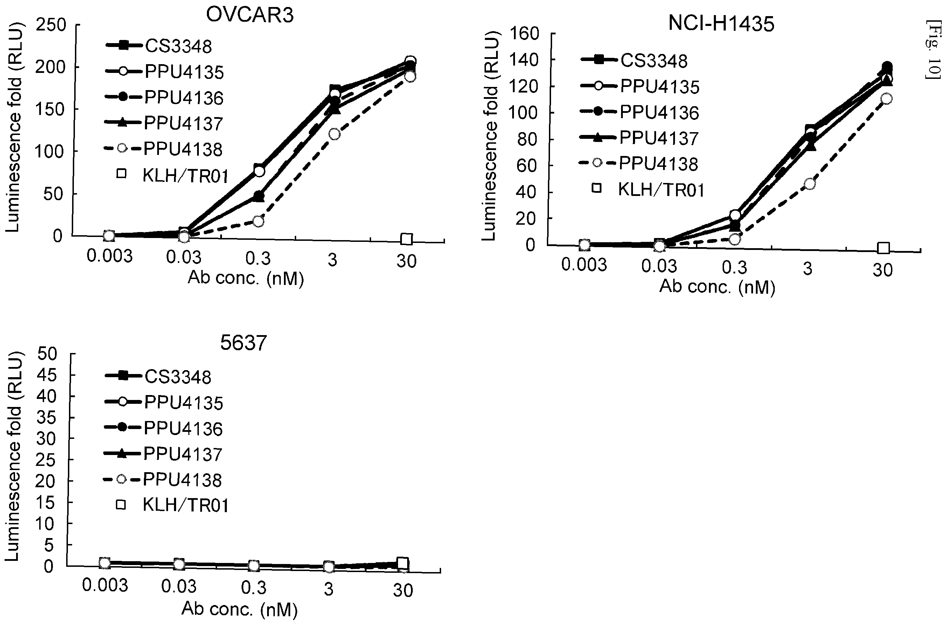

Figure 10 shows T cell activation through CD3 binding by the antibodies (CS3348, PPU4135, PPU4136, PPU4137 and PPU4138), under co-culture with CLDN6 expressing human cell lines (OVCAR3 and NCI-H1435) and CLDN6 negative cell line (5637). KLH/TR01 was used as a negative control.

[Fig. 11]

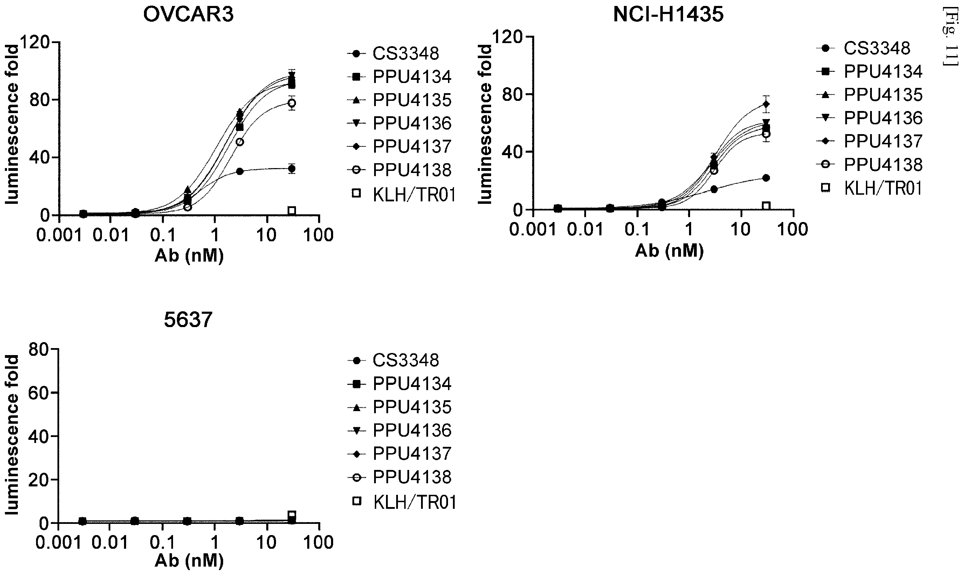

Figure 11 shows NF kappa B activation through CD137 binding by the antibodies (CS3348, PPU4134, PPU4135, PPU4136, PPU4137 and PPU4138), under co-culture with CLDN6 expressing human cell lines (OVCAR3 and NCI-H1435) and CLDN6 negative cell line (5637). KLH/TR01 was used as a negative control.

[Fig. 12]

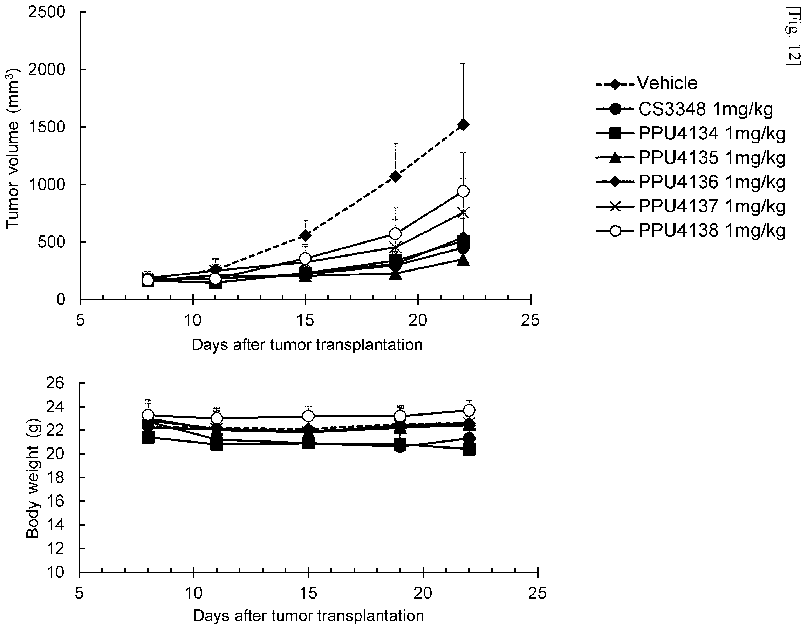

Figure 12 shows in vivo anti-tumor efficacy of the antibodies (CS3348, PPU4134, PPU4135, PPU4136, PPU4137 and PPU4138) at a dose of 1 mg/kg using NCI-H1435/HuNOG mice model.

[Fig. 13]

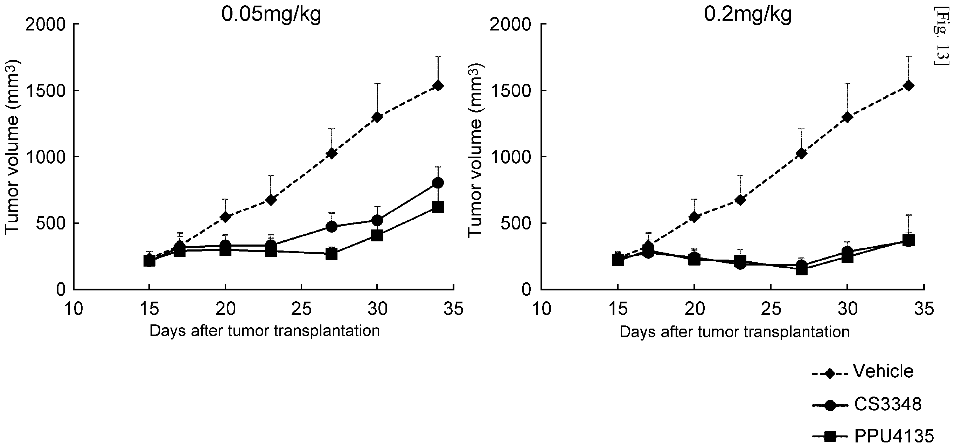

Figure 13 shows in vivo anti-tumor efficacy of the antibodies (CS3348 and PPU4135) at doses of 0.05 mg/kg and 0.2 mg/kg using OV-90/HuNOG mice model.

[Fig. 14]

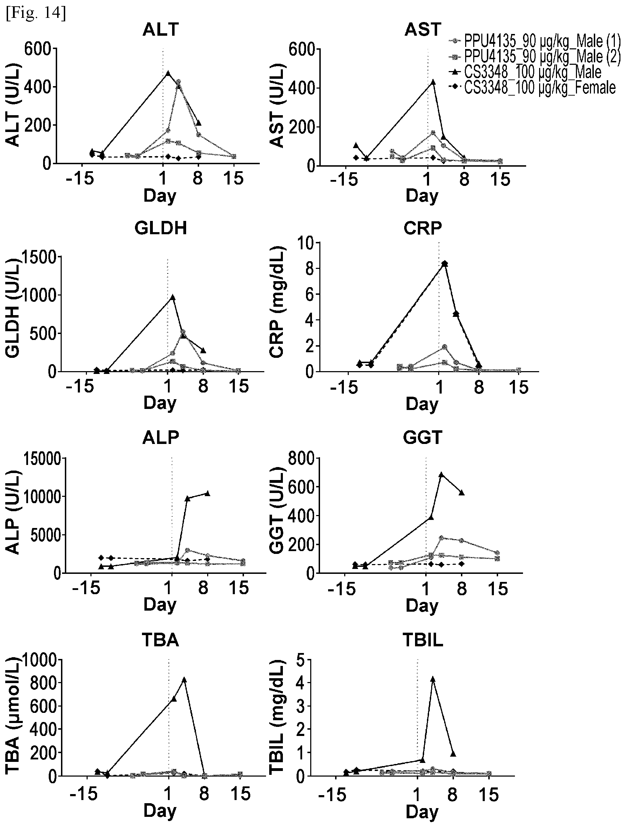

Figure 14 shows AST, ALT, GLDH (hepatic enzymes), ALP, TBIL, GGT, TBA (hepatobiliary damage parameters), and CRP (inflammatory marker) level changes mediated by CS3348 or PPU4135 administration.

[Fig. 15]

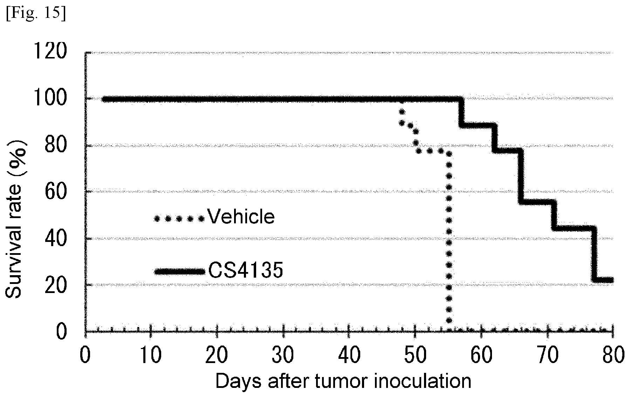

Figure 15 shows the survival rate of mouse peritoneal dissemination models after anti-CLDN6/Dual-Fab antibody administration.

[Fig. 16]

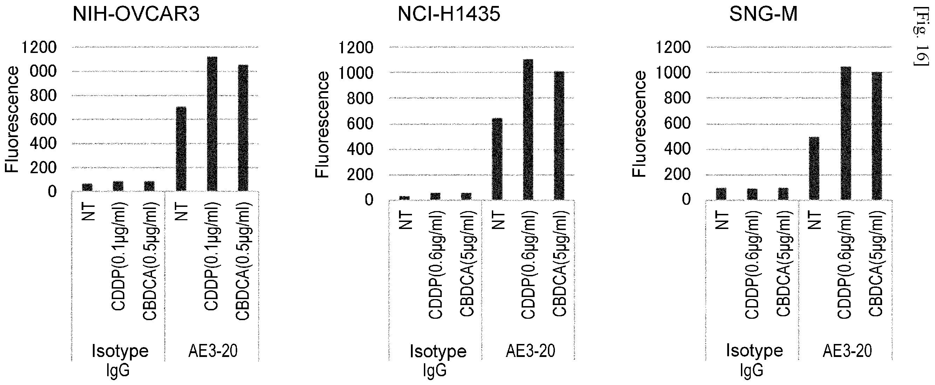

Figure 16 shows the change in the amount of CLDN6 expression after treating various cancer cells (human ovarian cancer cell line (NIH-OVCAR3), human lung cancer cell line (NCI-H1435), and human endometrial cancer cell line (SNG-M)) with a chemotherapeutic agent (cisplatin (CDDP) or carboplatin (CBDCA)).

[Fig. 17]

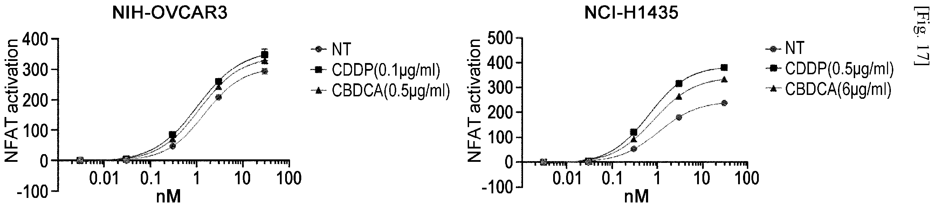

Figure 17 presents evaluation of change in T cell activation ability of the CS4135 antibody via CD3 binding after treatment with a chemotherapeutic agent (cisplatin (CDDP) or carboplatin (CBDCA)), by a luciferase assay system that uses GloResponse NFAT-luc2 Jurkat cells.

[Fig. 18]

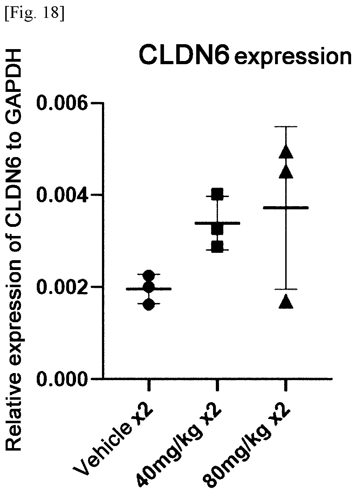

Figure 18 presents examination of the relative amount of CLDN6 expression after the administration of carboplatin in SNG-M tumor-transplanted mice, which was examined by real-time PCR using GAPDH as the internal control.

[Fig. 19]

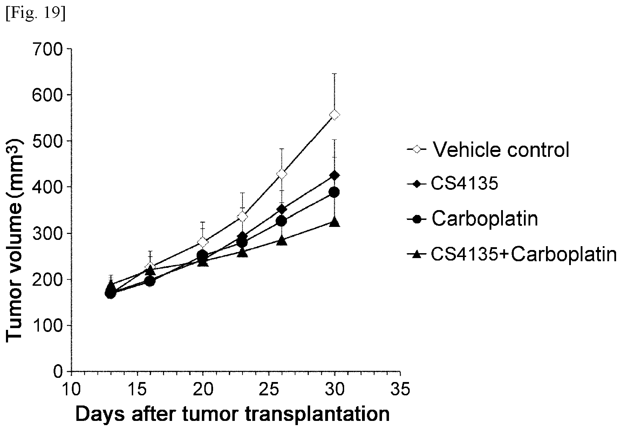

Figure 19 shows the change in tumor volume by single-agent administration of the CS4135 antibody, single-agent administration of carboplatin, and combined administration of the CS4135 antibody and carboplatin, in a xenograft transplantation model of NOG(huNOG) mice transplanted with human uterus cancer cell line SNG-M and human CD34-positive cells.

[Fig. 20]

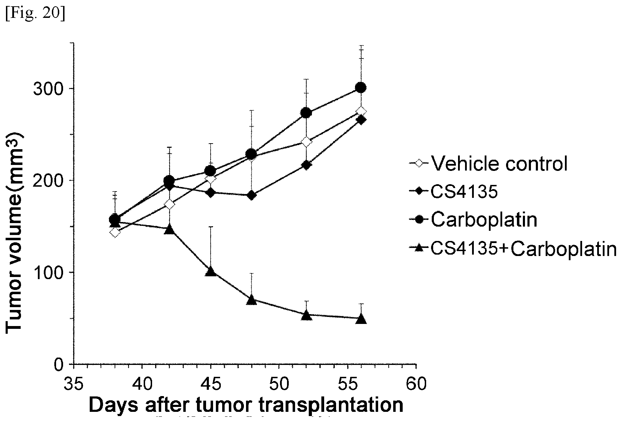

Figure 20 shows the change in tumor volume by single-agent administration of the CS4135 antibody, single-agent administration of carboplatin, and combined (simultaneous) administration of the CS4135 antibody and carboplatin, in a xenograft transplantation model of NOG(huNOG) mice transplanted with ovarian cancer cell line OVCAR3 and human CD34-positive cells.

[Fig. 21]

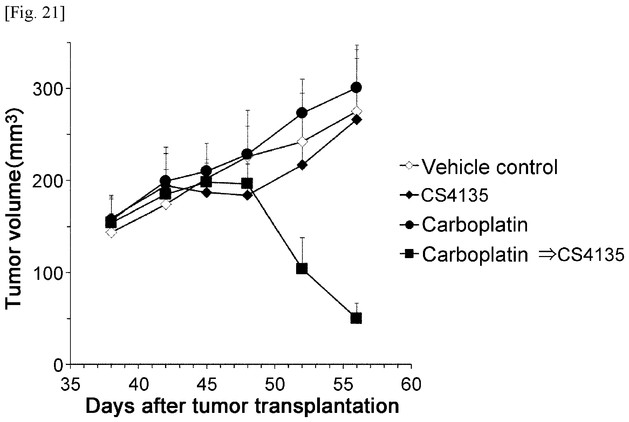

Figure 21 shows the change in tumor volume by single-agent administration of the CS4135 antibody, single-agent administration of carboplatin, and administration of carboplatin followed by sequential administration of the CS4135 antibody, in a xenograft transplantation model of NOG(huNOG) mice transplanted with ovarian cancer cell line OVCAR3 and human CD34-positive cells.

[Fig. 22]

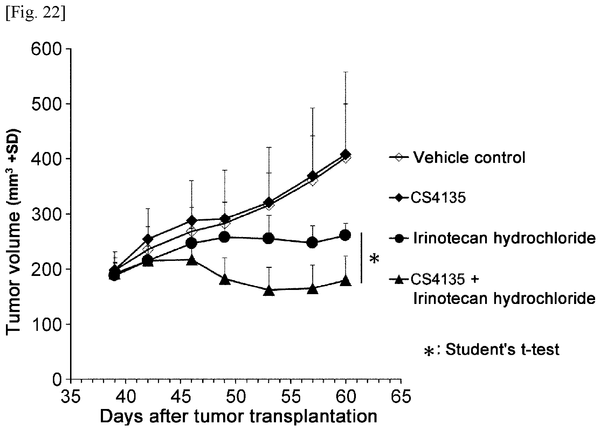

Figure 22 shows the change in tumor volume by single-agent administration of the CS4135 antibody, single-agent administration of irinotecan hydrochloride, and combined administration of the CS4135 antibody and irinotecan hydrochloride, in a xenograft transplantation model of NOG(huNOG) mice transplanted with ovarian cancer cell line OVCAR3 and human CD34-positive cells.

[Fig. 23]

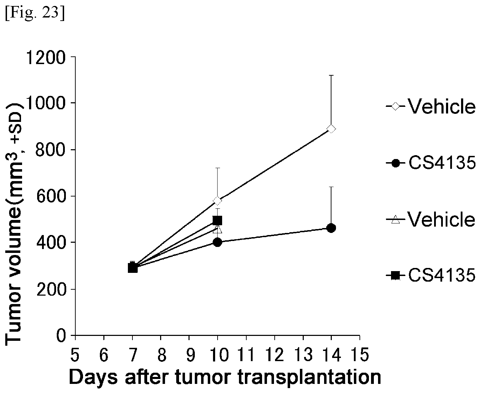

Figure 23 shows the change in tumor volume by single-agent administration of the CS4135 antibody in hCD137 KI/hCD3Tg mice transplanted with lung cancer cell line LLC1 forced to express claudin 6 (CLDN6).

[Fig. 24]

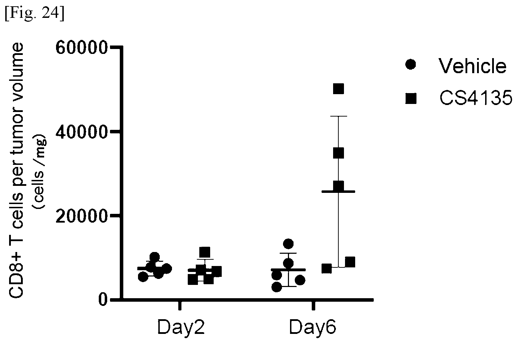

Figure 24 shows the result of administering the CS4135 antibody to hCD137 KI/hCD3Tg mice transplanted with lung cancer cell line LLC1 forced to express claudin 6 (CLDN6), and then analyzing the number of CD8-positive T cells in the tumor tissue by flow cytometry (FCM).

[Fig. 25]

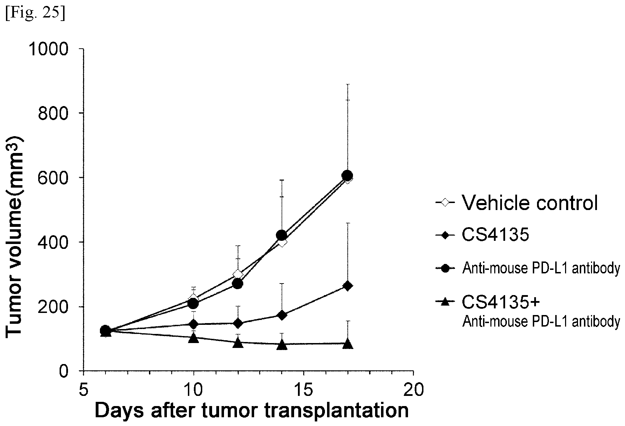

Figure 25 shows the change in tumor volume by single-agent administration of the CS4135 antibody, single-agent administration of the anti-mouse PD-L1 antibody, and combined administration of the CS4135 antibody and the anti-mouse PD-L1 antibody, in hCD137 KI/hCD3Tg mice transplanted with lung cancer cell line LLC1 forced to express claudin 6 (CLDN6).

[Fig. 26]

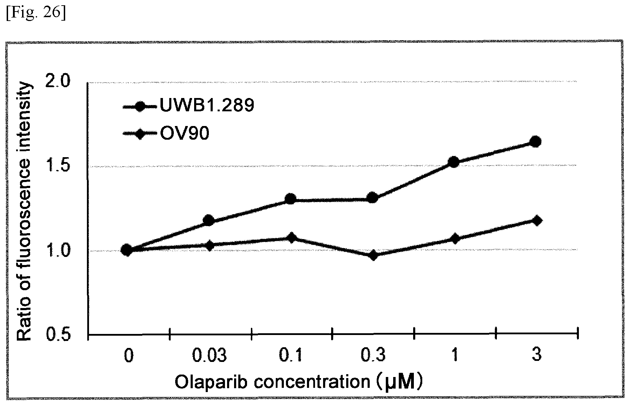

Figure 26 shows the change in the amount of CLDN6 expression in human CLDN6-expressing BRCA1-deficient ovarian cancer cell line UWB1.289 or BRAC1 wildtype ovarian cancer cell line OV-90 (ATCC) after treating with a PARP inhibitor (olaparib), which was investigated by FACS analysis.

[Fig. 27]

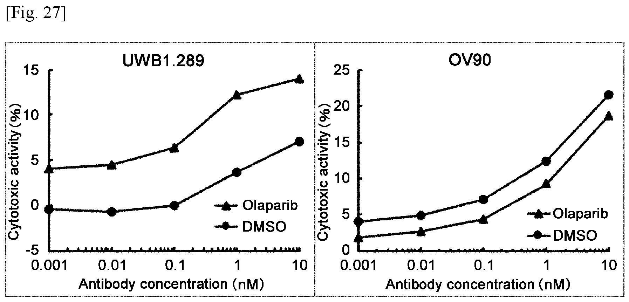

Figure 27 shows the cytotoxic activity of the CS4135 antibody in human CLDN6-expressing BRCA1-deficient ovarian cancer cell line UWB1.289 or BRAC1 wildtype ovarian cancer cell line OV-90 to which a PARP inhibitor (olaparib) was added, which was evaluated by lactase dehydrogenase (LDH) release assay using human PBMC as effector cell.

[Fig. 28]

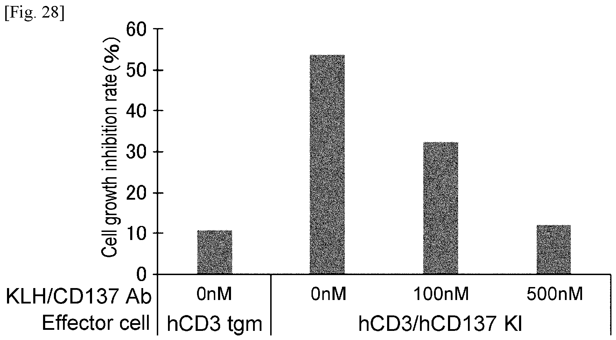

Figure 28 shows the analysis of cytotoxic activity by the CS4135 antibody in the presence or absence of the KLH/CD137 bispecific antibody, using real-time cell growth inhibition assay (xCELLigence assay). Mouse colon cancer cell line MC38/CLDN6 expressing human CLDN6 was used as target cells, and T cells derived from hCD3 transgenic mice or hCD3/hCD137 knock-in mice were used as effector cells.

[Fig. 29]

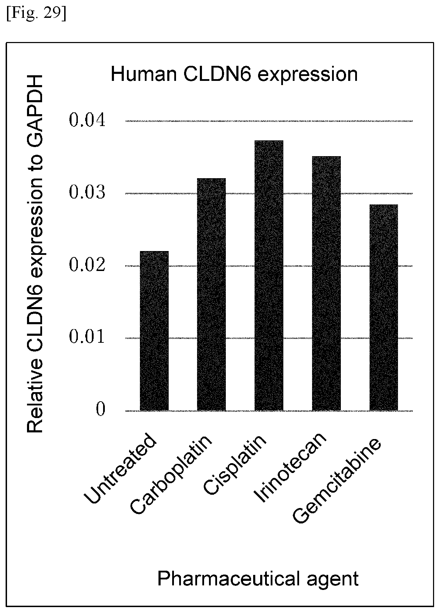

Fig. 29 shows the results of measuring the amounts of CLDN6 expression by quantitative PCR in the human ovarian cancer cell line (NIH:OVCAR-3) treated with carboplatin, cisplatin, irinotecan, or gemcitabine, and comparing them with the amount of CLDN6 expression in the untreated cells.

[Fig. 30]

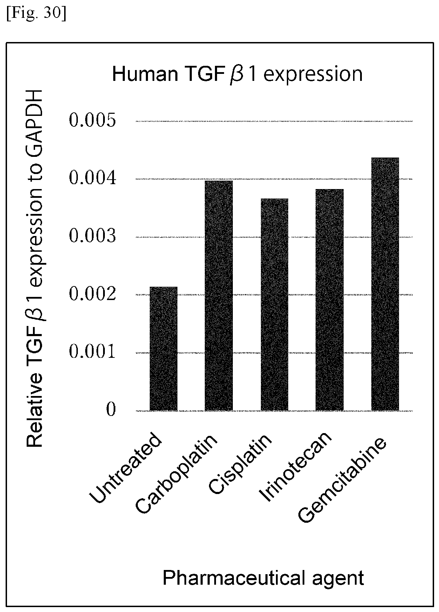

Fig. 30 shows the results of measuring the amounts of TGF-β1 expression by quantitative PCR in the human ovarian cancer cell line (NIH:OVCAR-3) treated with carboplatin, cisplatin, irinotecan, or gemcitabine, and comparing them with the amount of TGF-β1 expression in the untreated cells.

[Fig. 31]

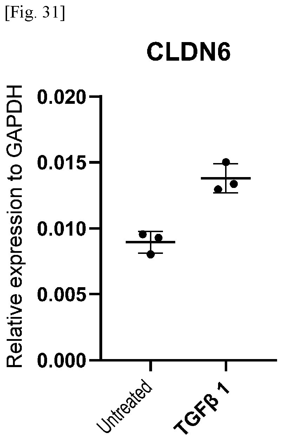

Fig. 31 shows the result of measuring the amount of CLDN6 expression by quantitative PCR in the human ovarian cancer cell line (NIH:OVCAR-3) stimulated with TGF-β1, and comparing this with the amount of CLDN6 expression in the untreated cells.

[Fig. 32]

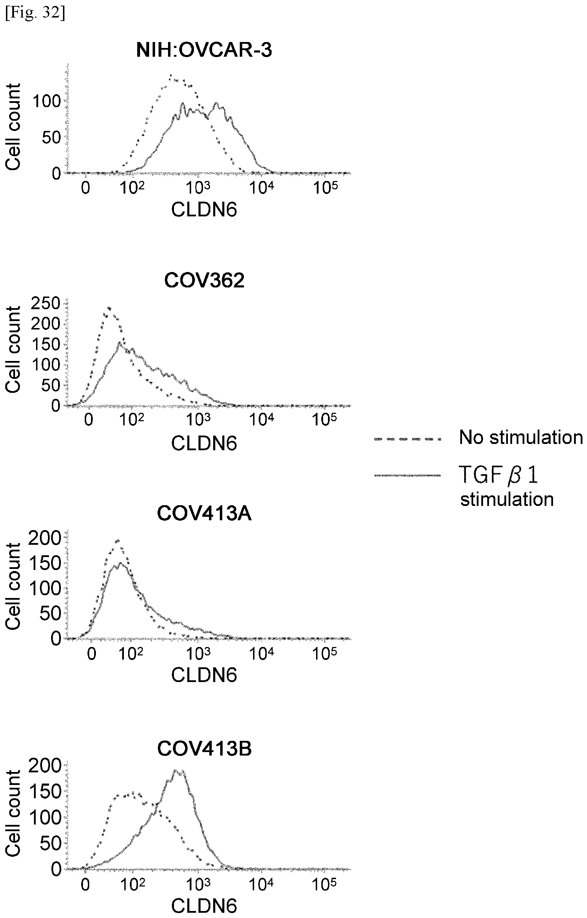

Fig. 32 shows the results of analyzing the amount of CLDN6 expression by FACS in various types of ovarian cancer cell lines (NIH:OVCAR-3, COV362, COV413A, and COV413B) stimulated with TGF-β1, and comparing them to the amount of CLDN6 expression in the unstimulated cells.

[Description of Embodiments]

The techniques and procedures described or referenced herein are generally well understood and commonly employed using conventional methodology by those skilled in the art, such as, for example, the widely utilized methodologies described in Sambrook et al., Molecular Cloning: A Laboratory Manual 3d edition (2001) Cold Spring Harbor Laboratory Press, Cold Spring Harbor, N.Y.; Current Protocols in Molecular Biology (F.M. Ausubel, et al. eds., (2003)); the series Methods in Enzymology (Academic Press, Inc.): PCR 2: A Practical Approach (M.J. MacPherson, B.D. Hames and G.R. Taylor eds. (1995)), Harlow and Lane, eds. (1988) Antibodies, A Laboratory Manual, and Animal Cell Culture (R.I. Freshney, ed. (1987)); Oligonucleotide Synthesis (M.J. Gait, ed., 1984); Methods in Molecular Biology, Humana Press; Cell Biology: A Laboratory Notebook (J.E. Cellis, ed., 1998) Academic Press; Animal Cell Culture (R.I. Freshney), ed., 1987); Introduction to Cell and Tissue Culture (J. P. Mather and P.E. Roberts, 1998) Plenum Press; Cell and Tissue Culture: Laboratory Procedures (A. Doyle, J.B. Griffiths, and D.G. Newell, eds., 1993-8) J. Wiley and Sons; Handbook of Experimental Immunology (D.M. Weir and C.C. Blackwell, eds.); Gene Transfer Vectors for Mammalian Cells (J.M. Miller and M.P. Calos, eds., 1987); PCR: The Polymerase Chain Reaction, (Mullis et al., eds., 1994); Current Protocols in Immunology (J.E. Coligan et al., eds., 1991); Short Protocols in Molecular Biology (Wiley and Sons, 1999); Immunobiology (C.A. Janeway and P. Travers, 1997); Antibodies (P. Finch, 1997); Antibodies: A Practical Approach (D. Catty., ed., IRL Press, 1988-1989); Monoclonal Antibodies: A Practical Approach (P. Shepherd and C. Dean, eds., Oxford University Press, 2000); Using Antibodies: A Laboratory Manual (E. Harlow and D. Lane (Cold Spring Harbor Laboratory Press, 1999); The Antibodies (M. Zanetti and J. D. Capra, eds., Harwood Academic Publishers, 1995); and Cancer: Principles and Practice of Oncology (V.T. DeVita et al., eds., J.B. Lippincott Company, 1993).

The definitions and detailed description below are provided to facilitate understanding of the present disclosure illustrated herein.

Definitions

Amino acids

Herein, amino acids are described by one- or three-letter codes or both, for example, Ala/A, Leu/L, Arg/R, Lys/K, Asn/N, Met/M, Asp/D, Phe/F, Cys/C, Pro/P, Gln/Q, Ser/S, Glu/E, Thr/T, Gly/G, Trp/W, His/H, Tyr/Y, Ile/I, or Val/V.

Alteration of Amino Acids

For amino acid alteration (also described as "amino acid substitution" or "amino acid mutation" within this description) in the amino acid sequence of an antigen-binding molecule, known methods such as site-directed mutagenesis methods (Kunkel et al. (Proc. Natl. Acad. Sci. USA (1985) 82, 488-492)) and overlap extension PCR may be appropriately employed. Furthermore, several known methods may also be employed as amino acid alteration methods for substitution to non-natural amino acids (Annu Rev. Biophys. Biomol. Struct. (2006) 35, 225-249; and Proc. Natl. Acad. Sci. U.S.A. (2003) 100 (11), 6353-6357). For example, it is suitable to use a cell-free translation system (Clover Direct (Protein Express)) containing a tRNA which has a non-natural amino acid bound to a complementary amber suppressor tRNA of one of the stop codons, the UAG codon (amber codon).

In the present specification, the meaning of the term "and/or" when describing the site of amino acid alteration includes every combination where "and" and "or" are suitably combined. Specifically, for example, "the amino acids at positions 33, 55, and/or 96 are substituted" includes the following variation of amino acid alterations: amino acid(s) at (a) position 33, (b) position 55, (c) position 96, (d) positions 33 and 55, (e) positions 33 and 96, (f) positions 55 and 96, and (g) positions 33, 55, and 96.

Furthermore, herein, as an expression showing alteration of amino acids, an expression that shows before and after a number indicating a specific position, one-letter or three-letter codes for amino acids before and after alteration, respectively, may be used appropriately. For example, the alteration N100bL or Asn100bLeu used when substituting an amino acid contained in an antibody variable region indicates substitution of Asn at position 100b (according to Kabat numbering) with Leu. That is, the number shows the amino acid position according to Kabat numbering, the one-letter or three-letter amino-acid code written before the number shows the amino acid before substitution, and the one-letter or three-letter amino-acid code written after the number shows the amino acid after substitution. Similarly the alteration P238D or Pro238Asp used when substituting an amino acid of the Fc region contained in an antibody constant region indicates substitution of Pro at position 238 (according to EU numbering) with Asp. That is, the number shows the amino acid position according to EU numbering, the one-letter or three-letter amino-acid code written before the number shows the amino acid before substitution, and the one-letter or three-letter amino-acid code written after the number shows the amino acid after substitution.

Polypeptides

As used herein, term "polypeptide" refers to a molecule composed of monomers (amino acids) linearly linked by amide bonds (also known as peptide bonds). The term "polypeptide" refers to any chain of two or more amino acids, and does not refer to a specific length of the product. Thus, peptides, dipeptides, tripeptides, oligopeptides, "protein," "amino acid chain," or any other term used to refer to a chain of two or more amino acids, are included within the definition of "polypeptide," and the term "polypeptide" may be used instead of, or interchangeably with any of these terms. The term "polypeptide" is also intended to refer to the products of post-expression modifications of the polypeptide, including without limitation glycosylation, acetylation, phosphorylation, amidation, derivatization by known protecting/blocking groups, proteolytic cleavage, or modification by non-naturally occurring amino acids. A polypeptide may be derived from a natural biological source or produced by recombinant technology, but is not necessarily translated from a designated nucleic acid sequence. It may be generated in any manner, including by chemical synthesis. A polypeptide as described herein may be of a size of about 3 or more, 5 or more, 10 or more, 20 or more, 25 or more, 50 or more, 75 or more, 100 or more, 200 or more, 500 or more, 1,000 or more, or 2,000 or more amino acids. Polypeptides may have a defined three-dimensional structure, although they do not necessarily have such structure. Polypeptides with a defined three-dimensional structure are referred to as folded, and polypeptides which do not possess a defined three-dimensional structure, but rather can adopt a large number of different conformations, and are referred to as unfolded.

Percent (%) amino acid sequence identity

"Percent (%) amino acid sequence identity" with respect to a reference polypeptide sequence is defined as the percentage of amino acid residues in a candidate sequence that are identical with the amino acid residues in the reference polypeptide sequence, after aligning the sequences and introducing gaps, if necessary, to achieve the maximum percent sequence identity, and not considering any conservative substitutions as part of the sequence identity. Alignment for purposes of determining percent amino acid sequence identity can be achieved in various ways that are within the skill in the art, for instance, using publicly available computer software such as BLAST, BLAST-2, ALIGN or Megalign (DNASTAR) software. Those skilled in the art can determine appropriate parameters for aligning sequences, including any algorithms needed to achieve maximal alignment over the full length of the sequences being compared. For purposes herein, however, % amino acid sequence identity values are generated using the sequence comparison computer program ALIGN-2. The ALIGN-2 sequence comparison computer program was authored by Genentech, Inc., and the source code has been filed with user documentation in the U.S. Copyright Office, Washington D.C., 20559, where it is registered under U.S. Copyright Registration No. TXU510087. The ALIGN-2 program is publicly available from Genentech, Inc., South San Francisco, California, or may be compiled from the source code. The ALIGN-2 program should be compiled for use on a UNIX operating system, including digital UNIX V4.0D. All sequence comparison parameters are set by the ALIGN-2 program and do not vary. In situations where ALIGN-2 is employed for amino acid sequence comparisons, the % amino acid sequence identity of a given amino acid sequence A to, with, or against a given amino acid sequence B (which can alternatively be phrased as a given amino acid sequence A that has or comprises a certain % amino acid sequence identity to, with, or against a given amino acid sequence B) is calculated as follows:

$$100 \text{ times the fraction } X/Y$$

where X is the number of amino acid residues scored as identical matches by the sequence alignment program ALIGN-2 in that program's alignment of A and B, and where Y is the total number of amino acid residues in B. It will be appreciated that where the length of amino acid sequence A is not equal to the length of amino acid sequence B, the % amino acid sequence identity of A to B will not equal the % amino acid sequence identity of B to A. Unless specifically stated otherwise, all % amino acid sequence identity values used herein are obtained as described in the immediately preceding paragraph using the ALIGN-2 computer program.

Recombinant Methods and Compositions

Antibodies and antigen-binding molecules may be produced using recombinant methods and compositions, e.g., as described in U.S. Patent No. 4,816,567. In one embodiment, isolated nucleic acid encoding an antibody as described herein is provided. Such nucleic acid may encode an amino acid sequence comprising the VL and/or an amino acid sequence comprising the VH of the antibody (e.g., the light and/or heavy chains of the antibody). In a further embodiment, one or more vectors (e.g., expression vectors) comprising such nucleic acid are provided. In a further embodiment, a host cell comprising such nucleic acid is provided. In one such embodiment, a host cell comprises (e.g., has been transformed with): (1) a vector comprising a nucleic acid that encodes an amino acid sequence comprising the VL of the antibody and an amino acid sequence comprising the VH of the antibody, or (2) a first vector comprising a nucleic acid that encodes an amino acid sequence comprising the VL of the antibody and a second vector comprising a nucleic acid that encodes an amino acid sequence comprising the VH of the antibody. In one embodiment, the host cell is eukaryotic, e.g. a Chinese Hamster Ovary (CHO) cell or lymphoid cell (e.g., Y0, NS0, Sp2/0 cell). In one embodiment, a method of making the multispecific antigen-binding molecule of the present disclosure is provided, wherein the method comprises culturing a host cell comprising a nucleic acid encoding the antibody, as provided above, under conditions suitable for expression of the antibody, and optionally recovering the antibody from the host cell (or host cell culture medium).

For recombinant production of an antibody described herein, nucleic acid encoding an antibody, e.g., as described above, is isolated and inserted into one or more vectors for further cloning and/or expression in a host cell. Such nucleic acid may be readily isolated and sequenced using conventional procedures (e.g., by using oligonucleotide probes that are capable of binding specifically to genes encoding the heavy and light chains of the antibody).

Suitable host cells for cloning or expression of antibody-encoding vectors include prokaryotic or eukaryotic cells described herein. For example, antibodies may be produced in bacteria, in particular when glycosylation and Fc effector function are not needed. For expression of antibody fragments and polypeptides in bacteria, see, e.g., U.S. Patent Nos. 5,648,237, 5,789,199, and 5,840,523. (See also Charlton, Methods in Molecular Biology, Vol. 248 (B.K.C. Lo, ed., Humana Press, Totowa, NJ, 2003), pp. 245-254, describing expression of antibody fragments in E. coli.) After expression, the antibody may be isolated from the bacterial cell paste in a soluble fraction and can be further purified.

In addition to prokaryotes, eukaryotic microbes such as filamentous fungi or yeast are suitable cloning or expression hosts for antibody-encoding vectors, including fungi and yeast strains whose glycosylation pathways have been "humanized," resulting in the production of an antibody with a partially or fully human glycosylation pattern. See Gerngross, Nat. Biotech. 22:1409-1414 (2004), and Li et al., Nat. Biotech. 24:210-215 (2006).

Suitable host cells for the expression of glycosylated antibody are also derived from multicellular organisms (invertebrates and vertebrates). Examples of invertebrate cells include plant and insect cells. Numerous baculoviral strains have been identified which may be used in conjunction with insect cells, particularly for transfection of Spodoptera frugiperda cells.

Plant cell cultures can also be utilized as hosts. See, e.g., US Patent Nos. 5,959,177, 6,040,498, 6,420,548, 7,125,978, and 6,417,429 (describing PLANTIBODIES™ technology for producing antibodies in transgenic plants).

Vertebrate cells may also be used as hosts. For example, mammalian cell lines that are adapted to grow in suspension may be useful. Other examples of useful mammalian host cell lines are monkey kidney CV1 line transformed by SV40 (COS-7); human embryonic kidney line (293 or 293 cells as described, e.g., in Graham et al., J. Gen Virol. 36:59 (1977)); baby hamster kidney cells (BHK); mouse sertoli cells (TM4 cells as described, e.g., in Mather, Biol. Reprod. 23:243-251 (1980)); monkey kidney cells (CV1); African green monkey kidney cells (VERO-76); human cervical carcinoma cells (HELA); canine kidney cells (MDCK); buffalo rat liver cells (BRL 3A); human lung cells (W138); human liver cells (Hep G2); mouse mammary tumor (MMT 060562); TRI cells, as described, e.g., in Mather et al., Annals N.Y. Acad. Sci. 383:44-68 (1982); MRC 5 cells; and FS4 cells. Other useful mammalian host cell lines include Chinese hamster ovary (CHO) cells, including $DHFR^-$ CHO cells (Urlaub et al., Proc. Natl. Acad. Sci. USA 77:4216 (1980)); and myeloma cell lines such as Y0, NS0 and Sp2/0. For a review of certain mammalian host cell lines suitable for antibody production, see, e.g., Yazaki and Wu, Methods in Molecular Biology, Vol. 248 (B.K.C. Lo, ed., Humana Press, Totowa, NJ), pp. 255-268 (2003).

Recombinant production of an antigen-binding molecule described herein could be done with methods similar to those described above, by using a host cell comprises (e.g., has been transformed with) one or plural vectors comprising nucleic acid that encodes an amino acid sequence comprising the whole antigen-binding molecule or part of the antigen-binding molecule.

<u>Antigen-binding molecules and multispecific antigen-binding molecules</u>

The term "antigen-binding molecule", as used herein, refers to any molecule that comprises an antigen-binding site or any molecule that has binding activity to an antigen, and may further refer to molecules such as a peptide or protein having a length of about five amino acids or more. The peptide and protein are not limited to those derived from a living organism, and for example, they may be a polypeptide produced from an artificially designed sequence. They may also be any of a naturally-occurring polypeptide, synthetic polypeptide, recombinant polypeptide, and such. Scaffold molecules comprising known stable conformational structure such as alpha/beta barrel as scaffold, and in which part of the molecule is made into antigen-binding site, is also one embodiment of the antigen binding molecule described herein.

"Multispecific antigen-binding molecules" refers to antigen-binding molecules that bind specifically to two or more antigens. "Multispecific antigen-binding molecules" include antibodies and antibody fragments that specifically bind to two or more antigens. The term "bispecific" means that the antigen-binding molecule is able to specifically bind to at least two different types of antigenic determinants. The term "tri-specific" means that the antigen binding molecule is able to specifically bind to at least three different types of antigenic determinants.

In certain embodiments, the multispecific antigen-binding molecule of the present disclosure is a tri-specific antigen-binding molecule capable of specifically binding to three different types of antigens, namely, a tri-specific antigen-binding molecule capable of binding to CD3 and CD137 but does not bind to both antigens simultaneously, and capable of specifically binding to CLDN6.

In one aspect, the present disclosure provides an anticancer agent comprising as an active ingredient a multispecific antigen-binding molecule that comprises:

(i) a first antigen-binding moiety that is capable of binding to CD3 and CD137 and that binds to either CD3 or CD137; and
(ii) a second antigen-binding moiety that is capable of binding to claudin 6 (CLDN6), preferably human CLDN6.

In one aspect, the present disclosure provides a pharmaceutical composition for use in combination with at least one other anticancer agent, wherein the pharmaceutical composition comprises as an active ingredient a multispecific antigen-binding molecule that comprises:

(i) a first antigen-binding moiety that is capable of binding to CD3 and CD137 and that binds to either CD3 or CD137; and
(ii) a second antigen-binding moiety that is capable of binding to claudin 6 (CLDN6), preferably human CLDN6.

Furthermore, in one aspect, the present disclosure provides a pharmaceutical composition for use in combination with at least one TGFβ inducing agent, wherein the pharmaceutical composition comprises as an active ingredient a multispecific antigen-binding molecule that comprises:

(i) a first antigen-binding moiety that is capable of binding to CD3 and CD137 and that binds to either CD3 or CD137; and
(ii) a second antigen-binding moiety that is capable of binding to claudin 6 (CLDN6), preferably human CLDN6.

Furthermore, in one aspect, the present disclosure provides a pharmaceutical composition for use in combination with at least one CLDN6 expression inducing agent, wherein the pharmaceutical composition comprises as an active ingredient a multispecific antigen-binding molecule that comprises:

(i) a first antigen-binding moiety that is capable of binding to CD3 and CD137 and that binds to either CD3 or CD137; and
(ii) a second antigen-binding moiety that is capable of binding to claudin 6 (CLDN6), preferably human CLDN6.

In one aspect, the present disclosure provides an anticancer agent, comprising as an active ingredient a multispecific antigen-binding molecule that comprises (i) a first antigen-binding moiety that is capable of binding to CD3 and CD137 and that binds to either CD3 or CD137, and (ii) a second antigen-binding moiety that is capable of binding to claudin 6 (CLDN6) and that has higher cytotoxic activity compared to when the first antigen-binding moiety is an antigen-binding moiety that can only bind to CD3.

Furthermore, in one aspect, the present disclosure provides an anticancer agent, comprising as an active ingredient a multispecific antigen-binding molecule that comprises (i) a first antigen-binding moiety that is capable of binding to CD3 and CD137 and that binds to either CD3 or CD137, and (ii) a second antigen-binding moiety that is capable of binding to claudin 6 (CLDN6) and that has lower toxicity compared to when the first antigen-binding moiety is an antigen-binding moiety that can only bind to CD3 .

Furthermore, in one aspect, the present disclosure provides an anticancer agent, comprising as an active ingredient a multispecific antigen-binding molecule that comprises (1) a first antigen-binding domain that is capable of binding to a T cell receptor complex, and (2) a second antigen-binding moiety that is capable of binding to CLDN6 and that has T cell cytotoxic activity which is equivalent to or higher than that of a multispecific antibody (CS3348) comprising an antigen-binding moiety that can bind to a T cell receptor complex, which is a moiety comprising a heavy chain that comprises the amino acid sequence of SEQ ID NO: 194 and a light chain that comprises the amino acid sequence of SEQ ID NO: 192, and an antigen-binding moiety that can bind to CLDN6, which is a moiety comprising a heavy chain that comprises the amino acid sequence of SEQ ID NO: 193 and a light chain that comprises the amino acid sequence of SEQ ID NO: 195.

In one aspect, the present disclosure provides a pharmaceutical composition which comprises as an active ingredient at least one other anticancer agent, and is for use in combination with a multispecific antigen-binding molecule comprising (i) a first antigen-binding moiety that is capable of binding to CD3 and CD137 and that binds to either CD3 or CD137, and (ii) a second antigen-binding moiety that is capable of binding to claudin 6 (CLDN6).

In one aspect, the present disclosure provides a pharmaceutical composition for treating or preventing cancer, formed by combining the following multispecific antigen-binding molecule and at least one other anticancer agent:

a multispecific antigen-binding molecule comprising (i) a first antigen-binding moiety that is capable of binding to CD3 and CD137 and that binds to either CD3 or CD137,
and (ii) a second antigen-binding moiety that is capable of binding to claudin 6 (CLDN6).

Furthermore, in one aspect, the present disclosure provides a pharmaceutical composition for treating or preventing cancer, formed by combining the following multispecific antigen-binding molecule and at least one TGFβ inducing agent:

a multispecific antigen-binding molecule comprising (i) a first antigen-binding moiety that is capable of binding to CD3 and CD137 and that binds to either CD3 or CD137,
and (ii) a second antigen-binding moiety that is capable of binding to claudin 6 (CLDN6).

Furthermore, in one aspect, the present disclosure provides a pharmaceutical composition for treating or preventing cancer, formed by combining the following multispecific antigen-binding molecule and at least one CLDN6 expression inducing agent:

a multispecific antigen-binding molecule comprising (i) a first antigen-binding moiety that is capable of binding to CD3 and CD137 and that binds to either CD3 or CD137,
and (ii) a second antigen-binding moiety that is capable of binding to claudin 6 (CLDN6).

In one aspect, the present disclosure provides a combination of a multispecific antigen-binding molecule comprising (i) a first antigen-binding moiety that is capable of binding to CD3 and CD 137 and that binds to either CD3 or CD137, and (ii) a second antigen-binding moiety that is capable of binding to claudin 6 (CLDN6), with at least one other anticancer agent.

Furthermore, in one aspect, the present disclosure provides a combination of a multispecific antigen-binding molecule comprising (i) a first antigen-binding moiety that is capable of binding to CD3 and CD137 and that binds to either CD3 or CD137, and (ii) a second antigen-binding moiety that is capable of binding to claudin 6 (CLDN6), with at least one TGFβ inducing agent.

Furthermore, in one aspect, the present disclosure provides a combination of a multispecific antigen-binding molecule comprising (i) a first antigen-binding moiety that is capable of binding to CD3 and CD137 and that binds to either CD3 or CD137, and (ii) a second antigen-binding moiety that is capable of binding to claudin 6 (CLDN6), with at least one CLDN6 expression inducing agent.

In one aspect, the present disclosure provides a method for inducing cytotoxicity, suppressing cell proliferation, inhibiting cell proliferation, activating immune response, treating cancer, or preventing cancer in an individual, comprising administering an effective amount of a multispecific antigen-binding molecule comprising (i) a first antigen-binding moiety that is capable of binding to CD3 and CD137 and that binds to either CD3 or CD137, and (ii) a second antigen-binding moiety that is capable of binding to claudin 6 (CLDN6), with an effective amount of at least one other anticancer agent: Furthermore, in one aspect, the present disclosure provides a method for inducing cytotoxicity, suppressing cell proliferation, inhibiting cell proliferation, activating immune response, treating cancer, or preventing cancer in an individual, by combined use of a multispecific antigen-binding molecule comprising (i) a first antigen-binding moiety that is capable of binding to CD3 and CD137 and that binds to either CD3 or CD137, and (ii) a second antigen-binding moiety that is capable of binding to claudin 6 (CLDN6), the method comprising administering to the individual an effective amount of at least one other anticancer agent.

Furthermore, in one aspect, the present disclosure provides a method for enhancing effects of inducing cytotoxicity, suppressing cell proliferation, inhibiting cell proliferation, activating immune response, treating cancer, or preventing cancer in an individual by a multispecific antigen-binding molecule comprising (i) a first antigen-binding moiety that is capable of binding to CD3 and CD137 and that binds to either CD3 or CD137, and (ii) a second antigen-binding moiety that is capable of binding to claudin 6 (CLDN6), the method comprising administering to the individual an effective amount of at least one other anticancer agent.

Furthermore, in one aspect, the present disclosure provides a method for enhancing effects of inducing cytotoxicity, suppressing cell proliferation, inhibiting cell proliferation, activating immune response, treating cancer, or preventing cancer in an individual by at least one other anticancer agent, the method comprising administering to the individual an effective amount of a multispecific antigen-binding molecule comprising (i) a first antigen-binding moiety that is capable of binding to CD3 and CD137 and that binds to either CD3 or CD137, and (ii) a second antigen-binding moiety that is capable of binding to claudin 6 (CLDN6).

In one aspect, the present disclosure provides a method for inducing cytotoxicity, suppressing cell proliferation, inhibiting cell proliferation, activating immune response, treating cancer, or preventing cancer in an individual, comprising administering an effective amount of a multispecific antigen-binding molecule comprising (i) a first antigen-binding moiety that is capable of binding to CD3 and CD137 and that binds to either CD3 or CD137, and (ii) a second antigen-binding moiety that is capable of binding to claudin 6 (CLDN6), with an effective amount of at least one TGFβ inducing agent:

Furthermore, in one aspect, the present disclosure provides a method for inducing cytotoxicity, suppressing cell proliferation, inhibiting cell proliferation, activating immune response, treating cancer, or preventing cancer in an individual, by combined use of a multispecific antigen-binding molecule comprising (i) a first antigen-binding moiety that is capable of binding to CD3 and CD137 and that binds to either CD3 or CD137, and (ii) a second antigen-binding moiety that is capable of binding to claudin 6 (CLDN6), the method comprising administering to the individual an effective amount of at least one TGFβ inducing agent.

Furthermore, in one aspect, the present disclosure provides a method for enhancing effects of inducing cytotoxicity, suppressing cell proliferation, inhibiting cell proliferation, activating immune response, treating cancer, or preventing cancer in an individual by a multispecific antigen-binding molecule comprising (i) a first antigen-binding moiety that is capable of binding to CD3 and CD137 and that binds to either CD3 or CD137, and (ii) a second antigen-binding moiety that is capable of binding to claudin 6 (CLDN6), the method comprising administering to the individual an effective amount of at least one TGFβ inducing agent.

In one aspect, the present disclosure provides a method for inducing cytotoxicity, suppressing cell proliferation, inhibiting cell proliferation, activating immune response, treating cancer, or preventing cancer in an individual, comprising administering an effective amount of a multispecific antigen-binding molecule comprising (i) a first antigen-binding moiety that is capable of binding to CD3 and CD137 and that binds to either CD3 or CD137, and (ii) a second antigen-binding moiety that is capable of binding to claudin 6 (CLDN6), with an effective amount of at least one CLDN6 expression inducing agent:

Furthermore, in one aspect, the present disclosure provides a method for inducing cytotoxicity, suppressing cell proliferation, inhibiting cell proliferation, activating immune response, treating cancer, or preventing cancer in an individual, by combined use of a multispecific antigen-binding molecule comprising (i) a first antigen-binding moiety that is capable of binding to CD3 and CD137 and that binds to either CD3 or CD137, and (ii) a second antigen-binding moiety that is capable of binding to claudin 6 (CLDN6), the method comprising administering to the individual an effective amount of at least one CLDN6 expression inducing agent.

Furthermore, in one aspect, the present disclosure provides a method for enhancing effects of inducing cytotoxicity, suppressing cell proliferation, inhibiting cell proliferation, activating immune response, treating cancer, or preventing cancer in an individual by a multispecific antigen-binding molecule comprising (i) a first antigen-binding moiety that is capable of binding to CD3 and CD137 and that binds to either CD3 or CD137, and (ii) a second antigen-binding moiety that is capable of binding to claudin 6 (CLDN6), the method comprising administering to the individual an effective amount of at least one CLDN6 expression inducing agent.

In one aspect, the present disclosure provides a method for inducing damage to a cancer cell or a cancer cell-comprising tumor tissue, or a method for suppressing growth of a cancer cell or a cancer cell-comprising tumor tissue, by contacting the cancer cell with a multispecific antigen-binding molecule comprising (i) a first antigen-binding moiety that is capable of binding to CD3 and CD137 and that binds to either CD3 or CD137, and (ii) a second antigen-binding moiety that is capable of binding to claudin 6 (CLDN6), and at least one other anticancer agent.

In one aspect, the present disclosure provides a method for assessing whether a multispecific antigen-binding molecule comprising (i) a first antigen-binding moiety that is capable of binding to CD3 and CD137 and that binds to either CD3 or CD137, and (ii) a second antigen-binding moiety that is capable of binding to claudin 6 (CLDN6), and at least one other anticancer agent will induce damage to a cancer cell or a cancer cell-comprising tumor tissue, or suppress growth of a cancer cell or a cancer cell-comprising tumor tissue, by contacting the cancer cell with the multispecific antigen-binding molecule and the at least one other anticancer agent.

In one aspect, the present disclosure provides a kit comprising:

(A) a pharmaceutical composition comprising a multispecific antigen-binding molecule that comprises a first antigen-binding moiety that is capable of binding to CD3 and CD137 and that binds to either CD3 or CD137, and that a second antigen-binding moiety that has binding activity to claudin 6 (CLDN6);

(B) a container; and
(C) an instruction or a label indicating that the multispecific antigen-binding molecule and at least one type of at least one other anticancer agent is administered in combination to an individual for treating or preventing cancer in the individual.

In one aspect, the present disclosure provides a kit comprising:

(A) at least one other anticancer agent;
(B) a container; and
(C) an instruction or a label indicating that the at least one other anticancer agent and a pharmaceutical composition comprising a multispecific antigen-binding molecule that comprises a first antigen-binding moiety that is capable of binding to CD3 and CD137 and that binds to either CD3 or CD137, and a second antigen-binding moiety that has binding activity to claudin 6 (CLDN6) are administered in combination to an individual for treating or preventing cancer in the individual.

In one aspect, the present disclosure provides a kit comprising:

(A) a pharmaceutical composition comprising a multispecific antigen-binding molecule comprising a first antigen-binding moiety that is capable of binding to CD3 and CD137 and that binds to either CD3 or CD137, and a second antigen-binding moiety that has binding activity to claudin 6 (CLDN6);
(B) a container; and
(C) at least one other anticancer agent.

In one aspect, the present disclosure provides a kit comprising:

(A) at least one TGFβ inducing agent;
(B) a container; and
(C) an instruction or a label indicating that the multispecific antigen-binding molecule and at least one type of at least one other anticancer agent is administered in combination to an individual for treating or preventing cancer in the individual.

In one aspect, the present disclosure provides a kit comprising:

(A) a pharmaceutical composition comprising a multispecific antigen-binding molecule comprising a first antigen-binding moiety that is capable of binding to CD3 and CD137 and that binds to either CD3 or CD137, and a second antigen-binding moiety that has binding activity to claudin 6 (CLDN6);
(B) a container; and
(C) at least one TGFβ inducing agent.

In one aspect, the present disclosure provides a kit comprising:

(A) at least one CLDN6 expression inducing agent;
(B) a container; and
(C) an instruction or a label indicating that the multispecific antigen-binding molecule and at least one type of at least one other anticancer agent is administered in combination to an individual for treating or preventing cancer in the individual.

In one aspect, the present disclosure provides a kit comprising:

(A) a pharmaceutical composition comprising a multispecific antigen-binding molecule comprising a first antigen-binding moiety that is capable of binding to CD3 and CD137 and that binds to either CD3 or CD137, and a second antigen-binding moiety that has binding activity to claudin 6 (CLDN6);
(B) a container; and
(C) at least one CLDN6 expression inducing agent.

In one aspect, the present disclosure provides a multispecific antigen-binding molecule for use in cancer therapy, which comprises (i) a first antigen-binding moiety that is capable of binding to CD3 and CD137 and that binds to either CD3 or CD137, and (ii) a second antigen-binding moiety that has binding activity to claudin 6 (CLDN6).

In one aspect, the present disclosure provides a combination of a multispecific antigen-binding molecule comprising (i) a first antigen-binding moiety that is capable of binding to CD3 and CD 137 and that binds to either CD3 or CD137, and (ii) a second antigen-binding moiety that has binding activity to claudin 6 (CLDN6), and at least one agent selected from a group consisting of another anticancer agent, a TGFβ inducing agent, and CLDN6 expression inducing agent, for use in cancer therapy.

In one aspect, the present disclosure provides use of a multispecific antigen-binding molecule comprising (i) a first antigen-binding moiety that is capable of binding to CD3 and CD 137 and that binds to either CD3 or CD137, and (ii) a second antigen-binding moiety that has binding activity to claudin 6 (CLDN6), in the manufacture of a medicament for treating cancer.

In one aspect, the present disclosure provides use of a combination of a multispecific antigen-binding molecule comprising (i) a first antigen-binding moiety that is capable of binding to CD3 and CD137 and that binds to either CD3 or CD137, and (ii) a second antigen-binding moiety that has binding activity to claudin 6 (CLDN6), with at least one agent selected from a group consisting of another anticancer agent, a TGFβ inducing agent, and CLDN6 expression inducing agent, in the manufacture of a medicament for treating cancer.

In one aspect, a multispecific antigen-binding molecule contained in the anticancer agent, the pharmaceutical composition, the combination, or the kit of the present disclosure, or used in the method or use of the present disclosure, may further comprise (iii) an Fc domain exhibiting decreased binding affinity for human Fcγ receptor compared to a natural human IgG1 Fc domain.

Components of the multispecific antigen-binding molecules contained in the anticancer agent, the pharmaceutical composition, the combination, or the kit of the present disclosure, or used in the method or use of the present disclosure can be fused to each other in various formats. Exemplary formats are depicted in Fig. 4. In particular embodiments, the multispecific antigen-binding molecules comprise an Fc domain composed of a first Fc-region subunit and a second Fc-region subunit that are capable of stable association.

According to any of the above embodiments, components of the multispecific antigen-binding molecules (for example, antigen binding moiety and Fc domain) may be fused directly or via various linkers, particularly peptide linkers comprising one or more amino acids, typically about 2-20 amino acids, that are described herein or are known in the art. Suitable, non-immunogenic peptide linkers are, for example, (G4S)n, (SG4)n, (G4S)n or G4(SG4)n peptide linkers, wherein n is generally a number between 1 and 10, typically between 2 and 4.

In one aspect, a multispecific antigen-binding molecule contained in the anticancer agent, the pharmaceutical composition, the combination, or the kit of the present disclosure, or used in the method or use of the present disclosure, comprises:

(i) a first antigen-binding moiety that is capable of binding to CD3 and CD137 and that binds to either CD3 or CD137; and

(ii) a second antigen-binding moiety that is capable of binding to claudin 6 (CLDN6), wherein the first antibody variable region of the first antigen-binding moiety is fused to a first heavy chain constant region, the second antibody variable region of the first antigen-binding moiety is fused to a first light chain constant region, the third antibody variable region of the second antigen-binding moiety is fused to a second heavy chain constant region, and the fourth antibody variable region of the second antigen-binding moiety is fused to a second light chain constant region.

In one aspect, a multispecific antigen-binding molecule contained in the anticancer agent, the pharmaceutical composition, the combination, or the kit of the present disclosure, or used in the method or use of the present disclosure, comprises:

(i) a first antigen-binding moiety that is capable of binding to CD3 and CD137 and that binds to either CD3 or CD137; and

(ii) a second antigen-binding moiety that is capable of binding to claudin 6 (CLDN6), wherein the first antibody variable region of the first antigen-binding moiety is fused to a first heavy chain constant region, the second antibody variable region of the first antigen-binding moiety is fused to a first light chain constant region, the third antibody variable region of the second antigen-binding moiety is fused to a second heavy chain constant region, and the fourth antibody variable region of the second antigen-binding moiety is fused to a second light chain constant region, wherein the constant regions are any one of (g1) to (g7) below:

(g1) a first heavy chain constant region comprising the amino acid sequence of SEQ ID NO: 74, a first light chain constant region comprising the amino acid sequence of SEQ ID NO: 87, a second heavy chain constant region comprising the amino acid sequence of SEQ ID NO: 73, and a second light chain constant region comprising the amino acid sequence of SEQ ID NO: 88;

(g2) a first heavy chain constant region comprising the amino acid sequence of SEQ ID NO: 74, a first light chain constant region comprising the amino acid sequence of SEQ ID NO: 85, a second heavy chain constant region comprising the amino acid sequence of SEQ ID NO: 81, and a second light chain constant region comprising the amino acid sequence of SEQ ID NO: 86;
(g3) a first heavy chain constant region comprising the amino acid sequence of SEQ ID NO: 79, a first light chain constant region comprising the amino acid sequence of SEQ ID NO: 72, a second heavy chain constant region comprising the amino acid sequence of SEQ ID NO: 80, and a second light chain constant region comprising the amino acid sequence of SEQ ID NO: 89;
(g4) a first heavy chain constant region comprising the amino acid sequence of SEQ ID NO: 83, a first light chain constant region comprising the amino acid sequence of SEQ ID NO: 87, a second heavy chain constant region comprising the amino acid sequence of SEQ ID NO: 82, and a second light chain constant region comprising the amino acid sequence of SEQ ID NO: 88;
(g5) a first heavy chain constant region comprising the amino acid sequence of SEQ ID NO: 83, a first light chain constant region comprising the amino acid sequence of SEQ ID NO: 85, a second heavy chain constant region comprising the amino acid sequence of SEQ ID NO: 84, and a second light chain constant region comprising the amino acid sequence of SEQ ID NO: 86;
(g6) a first heavy chain constant region comprising the amino acid sequence of SEQ ID NO: 77, a first light chain constant region comprising the amino acid sequence of SEQ ID NO: 72, a second heavy chain constant region comprising the amino acid sequence of SEQ ID NO: 78, and a second light chain constant region comprising the amino acid sequence of SEQ ID NO: 89; and
(g7) a first heavy chain constant region comprising the amino acid sequence of SEQ ID NO: 75, a first light chain constant region comprising the amino acid sequence of SEQ ID NO: 72, a second heavy chain constant region comprising the amino acid sequence of SEQ ID NO: 76, and a second light chain constant region comprising the amino acid sequence of SEQ ID NO: 89.

In one aspect, the multispecific antigen-binding molecule contained in the anticancer agent, the pharmaceutical composition, the combination, or the kit of the present disclosure, or used in the method or use of the present disclosure may be a multispecific antigen-binding molecule comprising four polypeptide chains, wherein the four polypeptide chains are any one of (h01) to (h18) below:

(h01) a heavy chain comprising the amino acid sequence of SEQ ID NO: 42 (chain 1) and a light chain comprising the amino acid sequence of SEQ ID NO: 51 (chain 2), and a heavy chain comprising the amino acid sequence of SEQ ID NO: 56 (chain 3) and a light chain comprising the amino acid sequence of SEQ ID NO: 69 (chain 4);
(h02) a heavy chain comprising the amino acid sequence of SEQ ID NO: 41 (chain 1) and a light chain comprising the amino acid sequence of SEQ ID NO: 50 (chain 2), and a heavy chain comprising the amino acid sequence of SEQ ID NO: 54 (chain 3) and a light chain comprising the amino acid sequence of SEQ ID NO: 68 (chain 4);
(h03) a heavy chain comprising the amino acid sequence of SEQ ID NO: 41 (chain 1) and a light chain comprising the amino acid sequence of SEQ ID NO: 50 (chain 2), and a heavy chain comprising the amino acid sequence of SEQ ID NO: 55 (chain 3) and a light chain comprising the amino acid sequence of SEQ ID NO: 68 (chain 4);
(h04) a heavy chain comprising the amino acid sequence of SEQ ID NO: 42 (chain 1) and a light chain comprising the amino acid sequence of SEQ ID NO: 51 (chain 2), and a heavy chain comprising the amino acid sequence of SEQ ID NO: 57 (chain 3) and a light chain comprising the amino acid sequence of SEQ ID NO: 69 (chain 4);
(h05) a heavy chain comprising the amino acid sequence of SEQ ID NO: 44 (chain 1) and a light chain comprising the amino acid sequence of SEQ ID NO: 52 (chain 2), and a heavy chain comprising the amino acid sequence of SEQ ID NO: 60 (chain 3) and a light chain comprising the amino acid sequence of SEQ ID NO: 70 (chain 4);
(h06) a heavy chain comprising the amino acid sequence of SEQ ID NO: 44 (chain 1) and a light chain comprising the amino acid sequence of SEQ ID NO: 52 (chain 2), and a heavy chain comprising the amino acid sequence of SEQ ID NO: 61 (chain 3) and a light chain comprising the amino acid sequence of SEQ ID NO: 70 (chain 4);
(h07) a heavy chain comprising the amino acid sequence of SEQ ID NO: 45 (chain 1) and a light chain comprising the amino acid sequence of SEQ ID NO: 50 (chain 2), and a heavy chain comprising the amino acid sequence of SEQ ID NO: 62 (chain 3) and a light chain comprising the amino acid sequence of SEQ ID NO: 68 (chain 4);
(h08) a heavy chain comprising the amino acid sequence of SEQ ID NO: 45 (chain 1) and a light chain comprising the amino acid sequence of SEQ ID NO: 50 (chain 2), and a heavy chain comprising the amino acid sequence of SEQ ID NO: 63 (chain 3) and a light chain comprising the amino acid sequence of SEQ ID NO: 68 (chain 4);
(h09) a heavy chain comprising the amino acid sequence of SEQ ID NO: 46 (chain 1) and a light chain comprising the amino acid sequence of SEQ ID NO: 51 (chain 2), and a heavy chain comprising the amino acid sequence of SEQ ID NO: 64 (chain 3) and a light chain comprising the amino acid sequence of SEQ ID NO: 69 (chain 4);
(h10) a heavy chain comprising the amino acid sequence of SEQ ID NO: 46 (chain 1) and a light chain comprising the amino acid sequence of SEQ ID NO: 51 (chain 2), and a heavy chain comprising the amino acid sequence of SEQ ID NO: 65 (chain 3) and a light chain comprising the amino acid sequence of SEQ ID NO: 69 (chain 4);
(h11) a heavy chain comprising the amino acid sequence of SEQ ID NO: 47 (chain 1) and a light chain comprising the amino acid sequence of SEQ ID NO: 52 (chain 2), and a heavy chain comprising the amino acid sequence of SEQ ID NO: 66 (chain 3) and a light chain comprising the amino acid sequence of SEQ ID NO: 70 (chain 4);
(h12) a heavy chain comprising the amino acid sequence of SEQ ID NO: 47 (chain 1) and a light chain comprising the amino acid sequence of SEQ ID NO: 52 (chain 2), and a heavy chain comprising the amino acid sequence of SEQ ID NO: 67 (chain 3) and a light chain comprising the amino acid sequence of SEQ ID NO: 70 (chain 4);
(h13) a heavy chain comprising the amino acid sequence of SEQ ID NO: 48 (chain 1) and a light chain comprising the amino acid sequence of SEQ ID NO: 53 (chain 2), and a heavy chain comprising the amino acid sequence of SEQ ID NO: 56 (chain 3) and a light chain comprising the amino acid sequence of SEQ ID NO: 71 (chain 4);
(h14) a heavy chain comprising the amino acid sequence of SEQ ID NO: 48 (chain 1) and a light chain comprising the amino acid sequence of SEQ ID NO: 53 (chain 2), and a heavy chain comprising the amino acid sequence of SEQ ID NO: 57 (chain 3) and a light chain comprising the amino acid sequence of SEQ ID NO: 71 (chain 4);
(h15) a heavy chain comprising the amino acid sequence of SEQ ID NO: 49 (chain 1) and a light chain comprising the amino acid sequence of SEQ ID NO: 53 (chain 2), and a heavy chain comprising the amino acid sequence of SEQ ID NO: 64 (chain 3) and a light chain comprising the amino acid sequence of SEQ ID NO: 71 (chain 4);
(h16) a heavy chain comprising the amino acid sequence of SEQ ID NO: 49 (chain 1) and a light chain comprising the amino acid sequence of SEQ ID NO: 53 (chain 2), and a heavy chain comprising the amino acid sequence of SEQ ID NO: 65 (chain 3) and a light chain comprising the amino acid sequence of SEQ ID NO: 71 (chain 4);
(h17) a heavy chain comprising the amino acid sequence of SEQ ID NO: 43 (chain 1) and a light chain comprising the amino acid sequence of SEQ ID NO: 52 (chain 2), and a heavy chain comprising the amino acid sequence of SEQ ID NO: 58 (chain 3) and a light chain comprising the amino acid sequence of SEQ ID NO: 70 (chain 4);
(h18) a heavy chain comprising the amino acid sequence of SEQ ID NO: 43 (chain 1) and a light chain comprising the amino acid sequence of SEQ ID NO: 52 (chain 2), and a heavy chain comprising the amino acid sequence of SEQ ID NO: 59 (chain 3) and a light chain comprising the amino acid sequence of SEQ ID NO: 70 (chain 4).

## Pyroglutamylation

It is known that when an antibody is expressed in cells, the antibody is modified after translation. Examples of the posttranslational modification include cleavage of lysine at the C terminal of the heavy chain by a carboxypeptidase; modification of glutamine or glutamic acid at the N terminal of the heavy chain and the light chain to pyroglutamic acid by pyroglutamylation; glycosylation; oxidation; deamidation; and glycation, and it is known that such posttranslational modifications occur in various antibodies (Journal of Pharmaceutical Sciences, 2008, Vol. 97, p. 2426-2447).

The multispecific antigen-binding molecules contained in the anticancer agent, the pharmaceutical composition, the combination, or the kit of the present disclosure, or used in the method or use of the present disclosure also includes a multispecific antibody which has undergone posttranslational modification. Examples of the multispecific antigen-binding molecules thereof of the present disclosure, which undergoes posttranslational modification, include multispecific antibodies which have undergone pyroglutamylation at the N terminal of the heavy chain variable region and/or deletion of lysine at the C terminal of the heavy chain. It is known in the field that such posttranslational modification due to pyroglutamylation at the N terminal and deletion of lysine at the C terminal does not have any influence on the activity of the antibody (Analytical Biochemistry, 2006, Vol. 348, p. 24-39).

## Antigen binding moiety

As used herein, the term "antigen binding moiety" refers to a polypeptide molecule that specifically binds to an antigen. In one embodiment, an antigen binding moiety is able to direct the entity to which it is attached (e.g. a second antigen binding moiety) to a target site, for example to a specific type of tumor cell expressing the cancer antigen (CLDN6). In another embodiment an antigen binding moiety is able to activate signaling through its target antigen, for example a T cell receptor complex antigen (CD3) or co-stimulatory molecule CD137. Antigen binding moieties include antibodies and fragments thereof as further defined herein. Particular antigen binding moieties include an antigen binding domain or an antibody variable region of an antibody, comprising an antibody heavy chain variable region and an antibody light chain variable region. In certain embodiments, the antigen binding moieties may comprise antibody constant regions as further defined herein and known in the art. Useful heavy chain constant regions include any of the five isotypes: alpha, delta, epsilon, gamma, or mu. Useful light chain constant regions include any of the two isotypes: kappa and lambda.

As used herein, the terms "first", "second", "third", and "fourth" with respect to antigen binding moieties etc., are used for convenience of distinguishing when there is more than one of each type of moiety and such. Use of these terms is not intended to confer a specific order or orientation of the multispecific antigen-binding molecule unless explicitly so stated.

Antigen-binding moiety capable of binding to CD3 and CD137

The multispecific antigen-binding molecule described herein comprises at least one antigen-binding moiety capable of binding to CD3 and CD137 (also referred to herein as "dual antigen-binding moiety" or "first antigen-binding moiety" or "Dual-Ig" or "Dual-Fab"). The first antigen-binding moiety described herein is capable of binding to CD3 and CD137, and binds to either CD3 or CD137. More specifically, the first antigen-binding moiety described herein binds to CD3; alternatively, the first antigen-binding moiety described herein binds to CD137. In a particular embodiment, the multispecific antigen-binding molecule comprises not more than two antigen-binding moieties capable of specifically binding to CD3 and CD137 but binds to either CD3 or CD137. In one embodiment, the multispecific antigen-binding molecule is capable of binding to CD3 and CD137, and provides monovalent binding to either CD3 or CD137. In one embodiment, the first antigen-binding moiety has binding activity to CD3 and binding activity to CD137, but when the moiety binds to an antigen, the moiety binds to one of the two, CD3 or CD137. In one embodiment, the first antigen-binding moiety is an antigen-binding moiety that is capable of binding to CD3 and CD137, but does not bind to CD3 and CD137 simultaneously.

Antigen-binding moiety capable of binding to a T-cell receptor complex

The multispecific antigen-binding molecule described herein comprises at least one antigen-binding moiety capable of binding to a T-cell receptor complex with binding activity (also referred to herein as "first antigen-binding moiety"). The first antigen-binding moiety described herein is capable of binding to a T-cell receptor complex. An antigen-binding moiety capable of binding to a T-cell receptor complex refers to a portion of the anti-T-cell receptor complex antibody comprising a region that specifically binds to all or a portion of the T-cell receptor complex and is also complementary thereto. The T-cell receptor complex may be a T-cell receptor itself, or an adaptor molecule constituting a T-cell receptor complex with a T-cell receptor. CD3 is suitable as the adaptor.

In certain embodiments, the Dual antigen-binding moiety ("first antigen-binding moiety") is generally a Fab molecule, particularly a conventional Fab molecule. In certain embodiments, the Dual antigen binding moiety ("first antigen-binding moiety") is a domain comprising antibody light-chain and heavy-chain variable regions (VL and VH). Suitable examples of such domains comprising antibody light-chain and heavy-chain variable regions include "single chain Fv (scFv)", "single chain antibody", "Fv", "single chain Fv 2 (scFv2)", "Fab", "F(ab')2", etc.

In certain embodiments, the Dual antigen-binding moiety ("first antigen-binding moiety") specifically binds to the whole or a portion of a partial peptide of CD3. In a particular embodiment, CD3 is human CD3 or cynomolgus CD3, most particularly human CD3. In a particular embodiment, the first antigen-binding moiety is cross-reactive for (i.e. specifically binds to) human and cynomolgus CD3. In some embodiments, the first antigen-binding moiety is capable of specific binding to the epsilon subunit of CD3, in particular the human CD3 epsilon subunit of CD3 which is shown in SEQ ID NO: 170 (NP_000724.1) (RefSeq registration numbers are shown within the parentheses). In some embodiments, the first antigen-binding moiety is capable of specific binding to the CD3 epsilon chain expressed on the surface of eukaryotic cells. In some embodiments, the first antigen-binding moiety binds to the CD3 epsilon chain expressed on the surface of T cells.

In certain embodiments, the CD137 is human CD137. In some embodiments, favorable examples of an antigen-binding molecule of the present disclosure include antigen-binding molecules that bind to the same epitope as the human CD137 epitope bound by the antibody selected from the group consisting of:

antibody that recognize a region comprising the SPCPPNSFSSAGGQRTCD ICRQCKGVFRTRKECSSTSNAECDCTPGFHCLGAGCSMCEQDCKQGQELTKKG C sequence (SEQ ID NO: 182),
antibody that recognize a region comprising the DCTPGFHCLGAGCSMCEQDC KQGQELTKKGC sequence (SEQ ID NO: 181),
antibody that recognize a region comprising the LQDPCSNC PAGTFCDNNRNQIC SPCPPNSF S SAGGQRTCDICRQCKGVFRTRKECS STSNAEC sequence (SEQ ID NO: 183), and
antibody that recognize a region comprising the LQDPCSNCPAGTFCDNNRN QIC sequence (SEQ ID NO: 180) in the human CD137 protein.

In specific embodiments, the Dual antigen-binding moiety ("first antigen-binding moiety") comprises any one of the antibody variable regions of (a1) to (a4) below:

(a1) a heavy chain variable region comprising the complementarity determining region (CDR) 1 of SEQ ID NO: 9, the CDR 2 of SEQ ID NO: 15, and the CDR 3 of SEQ ID NO: 21 (the first antigen-binding moiety), and a light chain variable region comprising the CDR 1 of SEQ ID NO: 31, the CDR 2 of SEQ ID NO: 35, and the CDR 3 of SEQ ID NO: 39 (the second antigen-binding moiety);

(a2) a heavy chain variable region comprising the complementarity determining region (CDR) 1 of SEQ ID NO: 10, the CDR 2 of SEQ ID NO: 16, and the CDR 3 of SEQ ID NO: 22 (the first antigen-binding moiety), and a light chain variable region comprising the CDR 1 of SEQ ID NO: 31, the CDR 2 of SEQ ID NO: 35, and the CDR 3 of SEQ ID NO: 39(the second antigen-binding moiety);
(a3) a heavy chain variable region comprising the complementarity determining region (CDR) 1 of SEQ ID NO: 11, the CDR 2 of SEQ ID NO: 17, and the CDR 3 of SEQ ID NO: 23 (the first antigen-binding moiety), and a light chain variable region comprising the CDR 1 of SEQ ID NO: 32, the CDR 2 of SEQ ID NO: 36, and the CDR 3 of SEQ ID NO: 40 (the second antigen-binding moiety);
(a4) a heavy chain variable region comprising the complementarity determining region (CDR) 1 of SEQ ID NO: 12, the CDR 2 of SEQ ID NO: 18, and the CDR 3 of SEQ ID NO: 24 (the first antigen-binding moiety), and a light chain variable region comprising the CDR 1 of SEQ ID NO: 32, the CDR 2 of SEQ ID NO: 36, and the CDR 3 of SEQ ID NO: 40 (the second antigen-binding moiety).

In specific embodiments, the Dual antigen-binding moiety ("first antigen-binding moiety") comprises the antibody variable regions that comprise human antibody frameworks or humanized antibody frameworks.

In specific embodiments, the Dual antigen-binding moiety ("first antigen-binding moiety") comprises any one of (c1) to (c4) below:

(c1) a heavy chain variable region comprising the amino acid sequence of SEQ ID NO: 3 (the first antigen-binding moiety), and a light chain variable region comprising the amino acid sequence of SEQ ID NO: 27 (the second antigen-binding moiety);
(c2) a heavy chain variable region comprising the amino acid sequence of SEQ ID NO: 4 (the first antigen-binding moiety), and a light chain variable region comprising the amino acid sequence of SEQ ID NO: 27 (the second antigen-binding moiety);
(c3) a heavy chain variable region comprising the amino acid sequence of SEQ ID NO: 5 (the first antigen-binding moiety), and a light chain variable region comprising the amino acid sequence of SEQ ID NO: 28 (the second antigen-binding moiety);
(c4) a heavy chain variable region comprising the amino acid sequence of SEQ ID NO: 6 (the first antigen-binding moiety), and a light chain variable region comprising the amino acid sequence of SEQ ID NO: 28 (the second antigen-binding moiety).

In one embodiment, the Dual antigen-binding moiety ("first antigen-binding moiety") comprises a heavy chain variable region sequence that is at least about 95%, 96%, 97%, 98%, 99% or 100% identical to SEQ ID NO: 3 (the first antigen-binding moiety) and a light chain variable region sequence that is at least about 95%, 96%, 97%, 98%, 99% or 100% identical to SEQ ID NO: 27 (the second antigen-binding moiety).

In one embodiment, the Dual antigen-binding moiety ("first antigen-binding moiety") comprises a heavy chain variable region sequence that is at least about 95%, 96%, 97%, 98%, 99% or 100% identical to SEQ ID NO: 4 (the first antigen-binding moiety) and a light chain variable region sequence that is at least about 95%, 96%, 97%, 98%, 99% or 100% identical to SEQ ID NO: 27 (the second antigen-binding moiety).

In one embodiment, the Dual antigen-binding moiety ("first antigen-binding moiety") comprises a heavy chain variable region sequence that is at least about 95%, 96%, 97%, 98%, 99% or 100% identical to SEQ ID NO: 5 (the first antigen-binding moiety) and a light chain variable region sequence that is at least about 95%, 96%, 97%, 98%, 99% or 100% identical to SEQ ID NO: 28 (the second antigen-binding moiety).

In one embodiment the Dual antigen-binding moiety ("first antigen-binding moiety") comprises a heavy chain variable region sequence that is at least about 95%, 96%, 97%, 98%, 99% or 100% identical to SEQ ID NO: 6 (the first antigen-binding moiety) and a light chain variable region sequence that is at least about 95%, 96%, 97%, 98%, 99% or 100% identical to SEQ ID NO: 28 (the second antigen-binding moiety).

In specific embodiments, the Dual antigen-binding moiety ("first antigen-binding moiety") comprises any one of (j01) to (j 18) below:

(j01) a heavy chain comprising the amino acid sequence of SEQ ID NO: 54 (chain 3) and a light chain comprising the amino acid sequence of SEQ ID NO: 68 (chain 4);
(j02) a heavy chain comprising the amino acid sequence of SEQ ID NO: 55 (chain 3) and a light chain comprising the amino acid sequence of SEQ ID NO: 68 (chain 4);
(j03) a heavy chain comprising the amino acid sequence of SEQ ID NO: 56 (chain 3) and a light chain comprising the amino acid sequence of SEQ ID NO: 69 (chain 4);
(j04) a heavy chain comprising the amino acid sequence of SEQ ID NO: 57 (chain 3) and a light chain comprising the amino acid sequence of SEQ ID NO: 69 (chain 4);

(j05) a heavy chain comprising the amino acid sequence of SEQ ID NO: 60 (chain 3) and a light chain comprising the amino acid sequence of SEQ ID NO: 70 (chain 4);
(j06) a heavy chain comprising the amino acid sequence of SEQ ID NO: 61 (chain 3) and a light chain comprising the amino acid sequence of SEQ ID NO: 70 (chain 4);
(j07) a heavy chain comprising the amino acid sequence of SEQ ID NO: 62 (chain 3) and a light chain comprising the amino acid sequence of SEQ ID NO: 68 (chain 4);
(j08) a heavy chain comprising the amino acid sequence of SEQ ID NO: 63 (chain 3) and a light chain comprising the amino acid sequence of SEQ ID NO: 68 (chain 4);
(j09) a heavy chain comprising the amino acid sequence of SEQ ID NO: 64 (chain 3) and a light chain comprising the amino acid sequence of SEQ ID NO: 69 (chain 4);
(j 10) a heavy chain comprising the amino acid sequence of SEQ ID NO: 65 (chain 3) and a light chain comprising the amino acid sequence of SEQ ID NO: 69 (chain 4);
(j 11) a heavy chain comprising the amino acid sequence of SEQ ID NO: 66 (chain 3) and a light chain comprising the amino acid sequence of SEQ ID NO: 70 (chain 4);
(j 12) a heavy chain comprising the amino acid sequence of SEQ ID NO: 67 (chain 3) and a light chain comprising the amino acid sequence of SEQ ID NO: 70 (chain 4);
(j 13) a heavy chain comprising the amino acid sequence of SEQ ID NO: 56 (chain 3) and a light chain comprising the amino acid sequence of SEQ ID NO: 71 (chain 4);
(j 14) a heavy chain comprising the amino acid sequence of SEQ ID NO: 57 (chain 3) and a light chain comprising the amino acid sequence of SEQ ID NO: 71 (chain 4);
(j 15) a heavy chain comprising the amino acid sequence of SEQ ID NO: 64 (chain 3) and a light chain comprising the amino acid sequence of SEQ ID NO: 71 (chain 4);
(j 16) a heavy chain comprising the amino acid sequence of SEQ ID NO: 65 (chain 3) and a light chain comprising the amino acid sequence of SEQ ID NO: 71 (chain 4);
(j 17) a heavy chain comprising the amino acid sequence of SEQ ID NO: 58 (chain 3) and a light chain comprising the amino acid sequence of SEQ ID NO: 70 (chain 4);
(j 18) a heavy chain comprising the amino acid sequence of SEQ ID NO: 59 (chain 3) and a light chain comprising the amino acid sequence of SEQ ID NO: 70 (chain 4).

In certain embodiments, the dual antigen-binding moiety ("first antigen-binding moiety") comprises antibody variable regions. In certain embodiments, the dual antigen-binding moiety ("first antigen-binding moiety") comprises the first antibody variable region and the second antibody variable region described above.

The multispecific antigen-binding molecules of the present disclosure also include a multispecific antibody which has undergone posttranslational modification. Examples of the multispecific antigen-binding molecules thereof of the present disclosure, which undergoes posttranslational modification, include multispecific antigen-binding molecules which have undergone pyroglutamylation at the N terminal of the heavy chain variable region and/or deletion of lysine at the C terminal of the heavy chain. It is known in the field that such posttranslational modification due to pyroglutamylation at the N terminal and deletion of lysine at the C terminal does not have any influence on the activity of the antibody (Analytical Biochemistry, 2006, Vol. 348, p. 24-39).

Antigen-binding moiety capable of binding to CLDN6

The multispecific antigen-binding molecule described herein comprises at least one antigen-binding moiety capable of binding to CLDN6 (also referred to herein as a "CLDN6 antigen-binding moiety" or "second antigen-binding moiety"). In certain embodiments, the multispecific antigen-binding molecule comprises one antigen-binding moiety capable of binding to CLDN6.

In certain embodiments, the CLDN6 antigen-binding moiety ("second antigen-binding moiety") is generally a Fab molecule, particularly a conventional Fab molecule. In certain embodiments, the CLDN6 antigen-binding moiety ("second antigen-binding moiety") is a domain comprising antibody light-chain and heavy-chain variable regions (VL and VH). Suitable examples of such domains comprising antibody light-chain and heavy-chain variable regions include "single chain Fv (scFv)", "single chain antibody", "Fv", "single chain Fv 2 (scFv2)", "Fab", "F(ab')2", etc.

In certain embodiments, the CLDN6 antigen-binding moiety ("second antigen-binding moiety") specifically binds to the whole or a portion of a partial peptide of CLDN6. In a particular embodiment CLDN6 is human CLDN6 or cynomolgus CLDN6 or mouse CLDN6, most particularly human CLDN6. In a particular embodiment, the CLDN6 antigen-binding moiety ("second antigen-binding moiety") is cross-reactive for (i.e. specifically binds to) human and cynomolgus CLDN6.

In certain embodiments, the CLDN6 antigen-binding moiety ("second antigen-binding moiety") specifically binds to the first extracellular domain of CLDN6 (amino acids 29-81 of SEQ ID NO: 196 or 197) or the second extracellular domain of CLDN6 (amino acids 138-159 of SEQ ID NO: 196 or 197). In certain embodiments, the CLDN6 antigen-binding moiety ("second antigen-binding moiety") specifically binds to human CLDN6 expressed on the surface of eukaryotic cells. In certain embodiments, binding activity towards CLDN6 is the binding activity towards the CLDN6 protein expressed on the surface of cancer cells.

In certain embodiments, the CLDN6 antigen-binding moiety ("second antigen-binding moiety") does not substantially bind to human CLDN9.

In certain embodiments, the CLDN6 antigen-binding moiety ("second antigen-binding moiety") does not substantially bind to human CLDN4.

In certain embodiments, the CLDN6 antigen-binding moiety ("second antigen-binding moiety") does not substantially bind to human CLDN3.

In certain embodiments, the CLDN6 antigen-binding moiety ("second antigen-binding moiety") does not substantially bind to a CLDN6 mutant as defined in SEQ ID NO:205.

In certain embodiments, the CLDN6 antigen-binding moiety ("second antigen-binding moiety") is a crossover Fab molecule, i.e. a Fab molecule wherein either the variable or the constant regions of the Fab heavy and light chains are exchanged.

In specific embodiments, the CLDN6 antigen-binding moiety ("second antigen-binding moiety") comprises the antibody variable regions of (b1) or (b2) below:

(b1) a heavy chain variable region comprising the complementarity determining region (CDR) 1 of SEQ ID NO: 8, the CDR 2 of SEQ ID NO: 14, and the CDR 3 of SEQ ID NO: 20 (the third antibody variable region), and a light chain variable region comprising the CDR 1 of SEQ ID NO: 30, the CDR 2 of SEQ ID NO: 34, and the CDR 3 of SEQ ID NO: 38 (the fourth antibody variable region);
(b2) a heavy chain variable region comprising the complementarity determining region (CDR) 1 of SEQ ID NO: 7, the CDR 2 of SEQ ID NO: 13, and the CDR 3 of SEQ ID NO: 19 (the third antibody variable region), and a light chain variable region comprising the CDR 1 of SEQ ID NO: 29, the CDR 2 of SEQ ID NO: 33, and the CDR 3 of SEQ ID NO: 37 (the fourth antibody variable region); and
(b3) a heavy chain variable region comprising the complementarity determining region (CDR) 1 of SEQ ID NO: 29, the CDR 2 of SEQ ID NO: 33, and the CDR 3 of SEQ ID NO: 37 (the third antibody variable region), and a light chain variable region comprising the CDR 1 of SEQ ID NO: 7, the CDR 2 of SEQ ID NO: 13, and the CDR 3 of SEQ ID NO: 19 (the fourth antibody variable region).

In specific embodiments, the CLDN6 antigen-binding moiety ("second antigen-binding moiety") comprises the antibody variable regions that comprise human antibody frameworks or humanized antibody frameworks.

In specific embodiments, the CLDN6 antigen-binding moiety ("second antigen-binding moiety") comprises (d1) or (d2) below:

(d1) a heavy chain variable region comprising the amino acid sequence of SEQ ID NO: 2 (the third antibody variable region), and a light chain variable region comprising the amino acid sequence of SEQ ID NO: 26 (the fourth antibody variable region);
(d2) a heavy chain variable region comprising the amino acid sequence of SEQ ID NO: 1 (the third antibody variable region), and a light chain variable region comprising the amino acid sequence of SEQ ID NO: 25 (the fourth antibody variable region); and
(d3) a heavy chain variable region comprising the amino acid sequence of SEQ ID NO: 25 (the third antibody variable region), and a light chain variable region comprising the amino acid sequence of SEQ ID NO: 1 (the fourth antibody variable region).

In one embodiment, the CLDN6 antigen-binding moiety ("second antigen-binding moiety") comprises a heavy chain variable region sequence that is at least about 95%, 96%, 97%, 98%, 99% or 100% identical to SEQ ID NO: 2 (the third antibody variable region) and a light chain variable region sequence that is at least about 95%, 96%, 97%, 98%, 99% or 100% identical to SEQ ID NO: 26 (the fourth antibody variable region).

In one embodiment, the CLDN6 antigen-binding moiety ("second antigen-binding moiety") comprises a heavy chain variable region sequence that is at least about 95%, 96%, 97%, 98%, 99% or 100% identical to SEQ ID NO: 1 (the third antibody variable region) and a light chain variable region sequence that is at least about 95%, 96%, 97%, 98%, 99% or 100% identical to SEQ ID NO: 25 (the fourth antibody variable region).

In one embodiment, the CLDN6 antigen-binding moiety ("second antigen-binding moiety") comprises a heavy chain variable region sequence that is at least about 95%, 96%, 97%, 98%, 99% or 100% identical to SEQ ID NO: 25 (the third antibody variable region) and a light chain variable region sequence that is at least about 95%, 96%, 97%, 98%, 99% or 100% identical to SEQ ID NO: 1 (the fourth antibody variable region).

In specific embodiments, the CLDN6 antigen-binding moiety ("second antigen-binding moiety") comprises any one of (k01) to (k09) below:

(k01) a heavy chain comprising the amino acid sequence of SEQ ID NO: 41 (chain 1) and a light chain comprising the amino acid sequence of SEQ ID NO: 50 (chain 2);
(k02) a heavy chain comprising the amino acid sequence of SEQ ID NO: 42 (chain 1) and a light chain comprising the amino acid sequence of SEQ ID NO: 51 (chain 2);
(k03) a heavy chain comprising the amino acid sequence of SEQ ID NO: 44 (chain 1) and a light chain comprising the amino acid sequence of SEQ ID NO: 52 (chain 2);
(k04) a heavy chain comprising the amino acid sequence of SEQ ID NO: 45 (chain 1) and a light chain comprising the amino acid sequence of SEQ ID NO: 50 (chain 2);
(k05) a heavy chain comprising the amino acid sequence of SEQ ID NO: 46 (chain 1) and a light chain comprising the amino acid sequence of SEQ ID NO: 51 (chain 2);
(k06) a heavy chain comprising the amino acid sequence of SEQ ID NO: 47 (chain 1) and a light chain comprising the amino acid sequence of SEQ ID NO: 52 (chain 2);
(k07) a heavy chain comprising the amino acid sequence of SEQ ID NO: 48 (chain 1) and a light chain comprising the amino acid sequence of SEQ ID NO: 53 (chain 2);
(k08) a heavy chain comprising the amino acid sequence of SEQ ID NO: 49 (chain 1) and a light chain comprising the amino acid sequence of SEQ ID NO: 53 (chain 2);
(k09) a heavy chain comprising the amino acid sequence of SEQ ID NO: 43 (chain 1) and a light chain comprising the amino acid sequence of SEQ ID NO: 52 (chain 2).

In certain embodiments, the CLDN6 antigen-binding moiety ("second antigen-binding moiety") comprises antibody variable regions. In certain embodiments, the CLDN6 antigen-binding moiety ("second antigen-binding moiety") comprises the third antibody variable region and the fourth antibody variable region described above.

In the multispecific antigen-binding molecule described above, as long as it has binding activity to CD3, CD137, a T-cell receptor complex, or CLDN6, one or more amino acids may be substituted, deleted, added, and/or inserted in the amino acid sequences of the heavy chain and light chain CDR1, CDR2, and CDR3, and the heavy chain variable region, the light chain variable region, the whole heavy chain, and the whole light chain. Methods well known to those skilled in the art for preparing amino acid sequences in which one or more amino acids have been substituted, deleted, added, and/or inserted include a method of introducing mutations into proteins. For example, those skilled in the art can prepare mutant multispecific antigen-binding molecules comprising a combination of antibody variable regions that are functionally equivalent to the combination of antibody variable regions in the original multispecific antigen-binding molecule, by appropriately introducing mutations into the amino acid sequence of an antibody having binding activity to CD3, CD137, a T cell receptor complex, or CLDN6 using site-directed mutagenesis (Hashimoto-Gotoh, T., Mizuno, T., Ogasahara, Y., and Nakagawa, M. (1995) An oligodeoxyribonucleotide-directed dual amber method for site-directed mutagenesis. Gene 152, 271-275; Zoller, MJ, and Smith, M.(1983) Oligonucleotide-directed mutagenesis of DNA fragments cloned into M13 vectors. Methods Enzymol. 100, 468-500; Kramer, W., Drutsa, V., Jansen, HW., Kramer, B., Pflugfelder, M., and Fritz, HJ. (1984) The gapped duplex DNA approach to oligonucleotide-directed mutation construction. Nucleic Acids Res. 12, 9441-9456; Kramer, W., and Fritz, HJ. (1987) Oligonucleotide-directed construction of mutations via gapped duplex DNA Methods. Enzymol. 154, 350-367; and Kunkel, TA. (1985) Rapid and efficient site-specific mutagenesis without phenotypic selection. Proc Natl Acad Sci USA. 82, 488-492) and such. In the present invention, "functionally equivalent" means that the binding affinities for an antigen are equivalent, or alternatively, it means that the cytotoxic activities against claudin 6-expressing cells or tissues containing these cells are equivalent when it is used as a multispecific antigen-binding molecule. The binding affinity and cytotoxic activity can be measured based on the description herein. The details are described below.

The number of amino acids to be altered is not limited, and can be, for example, 40 or less, 30 or less, 20 or less, preferably 18 or less, 16 or less, 15 or less, 12 or less, 10 or less, 9 or less, 8 or less, 7 or less, 6 or less, 5 or less, 4 or less, 3 or less, or 2 or less.

When an amino acid residue is altered, the amino acid is preferably mutated into a different amino acid that conserves the properties of the amino acid side chain. Examples of amino acid side chain properties are hydrophobic amino acids (A, I, L, M, F, P, W, Y, and V), hydrophilic amino acids (R, D, N, C, E, Q, G, H, K, S, and T), amino acids having an aliphatic side chain (G, A, V, L, I, and P), amino acids having a hydroxyl group-containing side chain (S, T, and Y), amino acids having a sulfur atom-containing side chain (C and M), amino acids having a carboxylic acid- and amide-containing side chain (D, N, E, and Q), amino acids having a basic side chain (R, K, and H), and amino acids having an aromatic-containing side chain (H, F, Y, and W) (amino acids are represented by one-letter codes in parentheses). Amino acid substitutions within each of these groups are referred to as conservative substitutions. It is already known that a polypeptide having a modified amino acid sequence in which one or more amino acid residues in a given amino acid sequence are deleted, added, and/or substituted with other amino acids retains its biological activity (Mark, D. F. et al., Proc. Natl. Acad. Sci. USA (1984)81:5662-6; Zoller, M. J. and Smith, M., Nucleic Acids Res.(1982)10:6487-500; Wang, A. et al., Science (1984) 224:1431-3; and Dalbadie-McFarland, G. et al., Proc. Natl. Acad. Sci. USA (1982)79:6409-13).

Antigen

As used herein, the term "antigen" refers to a site (e.g. a contiguous stretch of amino acids or a conformational configuration made up of different regions of non-contiguous amino acids) on a polypeptide macromolecule to which an antigen binding moiety binds, forming an antigen binding moiety-antigen complex. Useful antigenic determinants can be found, for example, on the surfaces of tumor cells, on the surfaces of virus-infected cells, on the surfaces of other diseased cells, on the surface of immune cells, free in blood serum, and/or in the extracellular matrix (ECM). The proteins referred to as antigens herein (e.g. CD3, CD137, CLDN6) can be any native form the proteins from any vertebrate source, including mammals such as primates (e.g. humans) and rodents (e.g. mice and rats), unless otherwise indicated. In a particular embodiment the antigen is a human CD3, human CD137 or human CLDN6. Where reference is made to a specific protein herein, the term encompasses the "full-length", unprocessed protein as well as any form of the protein that results from processing in the cell. The term also encompasses naturally occurring variants of the protein, e.g. splice variants or allelic variants.

In certain embodiments, the multispecific antigen-binding molecule described herein binds to an epitope of CD3, CD137 or CLDN6 that is conserved among the CD3, CD137 or CLDN6 from different species. In certain embodiments, the multi specific antigen-binding molecule of the present application is a trispecific antigen-binding molecule, i.e. it is capable of specifically binding to three different antigens - capable of binding to CD3 and CD137 but does not bind to both antigens simultaneously, and is capable of specifically binding to CLDN6.

Claudin-6 (CLDN6) and other Claudin family proteins

The term "CLDN6", as used herein, refers to any native Claudin-6 from any vertebrate source, including mammals such as primates (e.g. humans) and rodents (e.g., mice and rats), unless otherwise indicated. The amino acid sequence of human CLDN6 (hCLDN6) is shown in SEQ ID NO: 196 or 197, and the amino acid sequence of mouse CLDN6 (mCLDN6) is shown in SEQ ID NO: 201.

There are many other proteins within Claudin family other than CLDN6, such as CLDN3, CLDN4, and CLDN9. The amino acid sequences of human CLDN3 (hCLDN3), human CLDN4 (hCLDN4) and human CLDN9 (hCLDN9) are shown in SEQ ID Nos: 199, 200 and 198, respectively. The amino acid sequences of mouse CLDN3 (mCLDN3), mouse CLDN4 (mCLDN4) and mouse CLDN9 (mCLDN9) are shown in SEQ ID Nos: 203, 204 and 202, respectively.

CD3

In certain embodiments, the multispecific antigen-binding molecule specifically binds to the whole or a portion of a partial peptide of CD3. In a particular embodiment, CD3 is human CD3 or cynomolgus CD3, most particularly human CD3. In a particular embodiment the multispecific antigen-binding molecule is cross-reactive for (i.e. specifically binds to) human and cynomolgus CD3. In some embodiments, the multispecific antigen-binding molecule is capable of specific binding to the epsilon subunit of CD3, in particular the human CD3 epsilon subunit of CD3 which is shown in SEQ ID NO: 170 (NP_000724.1) (RefSeq registration numbers are shown within the parentheses). In some embodiments, the multispecific antigen-binding molecule is capable of specific binding to the CD3 epsilon chain expressed on the surface of eukaryotic cells. In some embodiments, the multispecific antigen-binding molecule binds to the CD3 epsilon chain expressed on the surface of T cells.

CD137

In certain embodiments, the CD137 is human CD137. In some embodiments, favorable examples of an antigen-binding molecule of the present disclosure include antigen-binding molecules that bind to the same epitope as the human CD137 epitope bound by the antibody selected from the group consisting of:

antibody that recognize a region comprising the SPCPPNSFSSAGGQRTCD ICRQCKGVFRTRKECSSTSNAECDCTPGFHCLGAGCSMCEQDCKQGQELTKKG C
sequence (SEQ ID NO: 182),
antibody that recognize a region comprising the DCTPGFHCLGAGCSMCEQDC KQGQELTKKGC sequence (SEQ ID NO: 181),
antibody that recognize a region comprising the LQDPCSNC PAGTFCDNNRNQIC SPCPPNSF S SAGGQRTCDICRQCKGVFRTRKECS STSNAEC sequence (SEQ ID NO: 183), and
antibody that recognize a region comprising the LQDPCSNCPAGTFCDNNRN QIC sequence (SEQ ID NO: 180) in the human CD137 protein.

Antigen-binding domain

The term "antigen-binding domain" refers to the part of an antibody that comprises the area which specifically binds to and is complementary to part or all of an antigen. An antigen-binding domain may be provided by, for example, one or more antibody variable domains (also called antibody variable regions). Preferably, the antigen-binding domains contain both the antibody light chain variable region (VL) and antibody heavy chain variable region (VH). Such preferable antigen-binding domains include, for example, "single-chain Fv (scFv)", "single-chain antibody", "Fv", "single-chain Fv2 (scFv2)", "Fab", and "F (ab')2". An antigen-binding domain may also be provided by single-domain antibodies.

Single-domain antibody

In the present specification, the term "single-domain antibody" is not limited by its structure as long as the domain can exert antigen binding activity by itself. It is known that a general antibody, for example, an IgG antibody, exhibits antigen binding activity in a state where a variable region is formed by the pairing of VH and VL, whereas the own domain structure of the single-domain antibody can exert antigen binding activity by itself without pairing with another domain. Usually, the single-domain antibody has a relatively low molecular weight and exists in the form of a monomer.

Examples of the single-domain antibody include, but are not limited to, antigen-binding molecules congenitally lacking a light chain, such as VHH of an animal of the family Camelidae and shark VNAR, and antibody fragments containing the whole or a portion of an antibody VH domain or the whole or a portion of an antibody VL domain. Examples of the single-domain antibody which is an antibody fragment containing the whole or a portion of an antibody VH or VL domain include, but are not limited to, artificially prepared single-domain antibodies originating from human antibody VH or human antibody VL as described in U.S. Patent No. 6,248,516 B1, etc. In some embodiments of the present invention, one single-domain antibody has three CDRs (CDR1, CDR2 and CDR3).

The single-domain antibody can be obtained from an animal capable of producing the single-domain antibody or by the immunization of the animal capable of producing the single-domain antibody. Examples of the animal capable of producing the single-domain antibody include, but are not limited to, animals of the family Camelidae, and transgenic animals harboring a gene capable of raising the single-domain antibody. The animals of the family Camelidae include camels, lamas, alpacas, one-hump camels and guanacos, etc. Examples of the transgenic animals harboring a gene capable of raising the single-domain antibody include, but are not limited to, transgenic animals described in International Publication No. WO2015/143414 and U.S. Patent Publication No. US2011/0123527 A1. The framework sequences of the single-domain antibody obtained from the animal may be converted to human germline sequences or sequences similar thereto to obtain a humanized single-domain antibody. The humanized single-domain antibody (e.g., humanized VHH) is also one embodiment of the single-domain antibody of the present invention.

Alternatively, the single-domain antibody can be obtained by ELISA, panning, or the like from a polypeptide library containing single-domain antibodies. Examples of the polypeptide library containing single-domain antibodies include, but are not limited to, naive antibody libraries obtained from various animals or humans (e.g., Methods in Molecular Biology 2012 911 (65-78); and Biochimica et Biophysica Acta - Proteins and Proteomics 2006 1764: 8 (1307-1319)), antibody libraries obtained by the immunization of various animals (e.g., Journal of Applied Microbiology 2014 117: 2 (528-536)), and synthetic antibody libraries prepared from antibody genes of various animals or humans (e.g., Journal of Biomolecular Screening 2016 21: 1 (35-43); Journal of Biological Chemistry 2016 291:24 (12641-12657); and AIDS 2016 30: 11 (1691-1701)).

Variable region

The term "variable region" or "variable domain" refers to the domain of an antibody heavy or light chain that is involved in binding the antibody to antigen. The variable domains of the heavy chain and light chain (VH and VL, respectively) of a native antibody generally have similar structures, with each domain comprising four conserved framework regions (FRs) and three hypervariable regions (HVRs). (See, e.g., Kindt et al. Kuby Immunology, 6th ed., W.H. Freeman and Co., page 91 (2007).) A single VH or VL domain may be sufficient to confer antigen-binding specificity. Furthermore, antibodies that bind a particular antigen may be isolated using a VH or VL domain from an antibody that binds the antigen to screen a library of complementary VL or VH domains, respectively. See, e.g., Portolano et al., J. Immunol. 150:880-887 (1993); Clarkson et al., Nature 352:624-628 (1991).

HVR or CDR

The term "hypervariable region" or "HVR" as used herein refers to each of the regions of an antibody variable domain which are hypervariable in sequence ("complementarity determining regions" or "CDRs") and/or form structurally defined loops ("hypervariable loops") and/or contain the antigen-contacting residues ("antigen contacts"). Hypervariable regions (HVRs) are also referred to as "complementarity determining regions" (CDRs), and these terms are used herein interchangeably in reference to portions of the variable region that form the antigen binding regions. Generally, antibodies comprise six HVRs: three in the VH (H1, H2, H3), and three in the VL (L1, L2, L3). Exemplary HVRs herein include:

(a) hypervariable loops occurring at amino acid residues 26-32 (L1), 50-52 (L2), 91-96 (L3), 26-32 (H1), 53-55 (H2), and 96-101 (H3) (Chothia and Lesk, J. Mol. Biol. 196:901-917 (1987));
(b) CDRs occurring at amino acid residues 24-34 (L1), 50-56 (L2), 89-97 (L3), 31-35b (H1), 50-65 (H2), and 95-102 (H3) (Kabat et al., Sequences of Proteins of Immunological Interest, 5th Ed. Public Health Service, National Institutes of Health, Bethesda, MD (1991));
(c) antigen contacts occurring at amino acid residues 27c-36 (L1), 46-55 (L2), 89-96 (L3), 30-35b (H1), 47-58 (H2), and 93-101 (H3) (MacCallum et al. J. Mol. Biol. 262: 732-745 (1996)); and
(d) combinations of (a), (b), and/or (c), including HVR amino acid residues 46-56 (L2), 47-56 (L2), 48-56 (L2), 49-56 (L2), 26-35 (H1), 26-35b (H1), 49-65 (H2), 93-102 (H3), and 94-102 (H3).

Unless otherwise indicated, HVR residues and other residues in the variable domain (e.g., FR residues) are numbered herein according to Kabat et al., supra.
HVR-H1, HVR-H2, HVR-H3, HVR-L1, HVR-L2, and HVR-L3 are also mentioned as "H-CDR1 ", "H-CDR2", "H-CDR3", "L-CDR1 ", "L-CDR2", and "L-CDR3", respectively.

Capable of binding to CD3 and CD137

Whether the antibody variable region of the present disclosure is "capable of binding to CD3 and CD137" can be determined by a method known in the art.
This can be determined by, for example, an electrochemiluminescence method (ECL method) (BMC Research Notes 2011, 4: 281).
Specifically, for example, a low-molecular antibody composed of a region capable of binding to CD3 and CD137, for example, a Fab region, of a biotin-labeled antigen-binding molecule to be tested, or a monovalent antibody (antibody lacking one of the two Fab regions carried by a usual antibody) thereof is mixed with CD3 or CD137 labeled with sulfo-tag (Ru complex), and the mixture is added onto a streptavidin-immobilized plate. In this operation, the biotin-labeled antigen-binding molecule to be tested binds to streptavidin on the plate. Light is developed from the sulfo-tag, and the luminescence signal can be detected using Sector Imager 600 or 2400 (MSD K.K.) or the like to thereby confirm the binding of the aforementioned region of the antigen-binding molecule to be tested to CD3 or CD137.
Alternatively, this assay may be conducted by ELISA, FACS (fluorescence activated cell sorting), ALPHAScreen (amplified luminescent proximity homogeneous assay screen), the BIACORE method based on a surface plasmon resonance (SPR) phenomenon, etc. (Proc. Natl. Acad. Sci. USA (2006) 103 (11), 4005-4010).
Specifically, the assay can be conducted using, for example, an interaction analyzer Biacore (GE Healthcare Japan Corp.) based on a surface plasmon resonance (SPR) phenomenon. The Biacore analyzer includes any model such as Biacore T100, T200, X100, A100, 4000, 3000, 2000, 1000, or C. Any sensor chip for Biacore, such as a CM7, CM5, CM4, CM3, C1, SA, NTA, L1, HPA, or Au chip, can be used as a sensor chip. Proteins for capturing the antigen-binding molecule of the present disclosure, such as protein A, protein G, protein L, anti-human IgG antibodies, anti-human IgG-Fab, anti-human L chain antibodies, anti-human Fc antibodies, antigenic proteins, or antigenic peptides, are immobilized onto the sensor chip by a coupling method such as amine coupling, disulfide coupling, or aldehyde coupling. CD3 or CD137 is injected thereon as an analyte, and the interaction is measured to obtain a sensorgram. In this operation, the concentration of CD3 or CD137 can be selected within the range of a few micro M to a few pM according to the interaction strength (e.g., KD) of the assay sample.
Alternatively, CD3 or CD137 may be immobilized instead of the antigen-binding molecule onto the sensor chip, with which the antibody sample to be evaluated is in turn allowed to interact. Whether the antibody variable region of the antigen-binding molecule of the present disclosure has binding activity against CD3 or CD137 can be confirmed on the basis of a dissociation constant (KD) value calculated from the sensorgram of the interaction or on the basis of the degree of increase in the sensorgram after the action of the antigen-binding molecule sample over the level before the action. In some embodiments, binding activity or affinity of the antibody variable region of the present disclosure to the antigen of interest (i.e. CD3 or CD137) are assessed at 37 degrees C (for CD137) or 25 degrees C (for CD3) using e.g., Biacore T200 instrument (GE Healthcare) or Biacore 8K instrument (GE Healthcare). Anti-human Fc (e.g., GE Healthcare) is immobilized onto all flow cells of a CM4 sensor chip using amine coupling kit (e.g, GE Healthcare). The antigen binding molecules or antibody variable regions are captured onto the anti-Fc sensor surfaces, then the antigen (CD3 or CD137) is injected over the flow cell. The capture levels of the antigen binding molecules or antibody variable regions may be aimed at 200 resonance unit (RU). Recombinant human CD3 or CD137 may be injected at 400 to 25 nM prepared by two-fold serial dilution, followed by dissociation. All antigen binding molecules or antibody variable regions and analytes are prepared in ACES pH 7.4 containing 20 mM ACES, 150 mM NaCl, 0.05% Tween 20, 0.005% NaN3. Sensor surface is regenerated each cycle with 3M MgCl2. Binding affinity are determined by processing and fitting the data to 1: 1 binding model using e.g., Biacore T200 Evaluation software, version 2.0 (GE Healthcare) or Biacore 8K Evaluation software (GE Healthcare). The KD values are calculated for assessing the specific binding activity or affinity of the antigen binding domains of the present disclosure.

The ALPHAScreen is carried out by the ALPHA technology using two types of beads (donor and acceptor) on the basis of the following principle: luminescence signals are detected only when these two beads are located in proximity through the biological interaction between a molecule bound with the donor bead and a molecule bound with the acceptor bead. A laser-excited photosensitizer in the donor bead converts ambient oxygen to singlet oxygen having an excited state. The singlet oxygen diffuses around the donor bead and reaches the acceptor bead located in proximity thereto to thereby cause chemiluminescent reaction in the bead, which finally emits light. In the absence of the interaction between the molecule bound with the donor bead and the molecule bound with the acceptor bead, singlet oxygen produced by the donor bead does not reach the acceptor bead. Thus, no chemiluminescent reaction occurs.

One (ligand) of the substances between which the interaction is to be observed is immobilized onto a thin gold film of a sensor chip. The sensor chip is irradiated with light from the back such that total reflection occurs at the interface between the thin gold film and glass. As a result, a site having a drop in reflection intensity (SPR signal) is formed in a portion of reflected light. The other (analyte) of the substances between which the interaction is to be observed is injected on the surface of the sensor chip. Upon binding of the analyte to the ligand, the mass of the immobilized ligand molecule is increased to change the refractive index of the solvent on the sensor chip surface. This change in the refractive index shifts the position of the SPR signal (on the contrary, the dissociation of the bound molecules gets the signal back to the original position). The Biacore system plots on the ordinate the amount of the shift, i.e., change in mass on the sensor chip surface, and displays time-dependent change in mass as assay data (sensorgram). The amount of the analyte bound to the ligand captured on the sensor chip surface (amount of change in response on the sensorgram between before and after the interaction of the analyte) can be determined from the sensorgram. However, since the amount bound also depends on the amount of the ligand, the comparison must be performed under conditions where substantially the same amounts of the ligand are used. Kinetics, i.e., an association rate constant (ka) and a dissociation rate constant (kd), can be determined from the curve of the sensorgram, while affinity (KD) can be determined from the ratio between these constants. Inhibition assay is also preferably used in the BIACORE method. Examples of the inhibition assay are described in Proc. Natl. Acad. Sci. USA (2006) 103 (11), 4005-4010.

Does not bind to CD3 and CD137 (4-1BB) at the same time

As described above, binding either one of CD3 or CD137 comprises not binding to CD3 and CD 137 (4-1BB) at the same time. The term "does not bind to CD3 and CD137 (4-1BB) at the same time" or "does not bind to CD3 and CD137 (4-1BB) simultaneously" means that the antigen-binding moiety or antibody variable region of the present disclosure cannot bind to CD137 in a state bound with CD3 whereas the antigen-binding moiety or antibody variable region cannot bind to CD3 in a state bound with CD137. In this context, the phrase "not bind to CD3 and CD137 at the same time" also includes not cross-linking a cell expressing CD3 to a cell expressing CD137, or not binding to CD3 and CD 137 each expressed on a different cell, at the same time. Such an antibody variable region is not particularly limited as long as the antibody variable region has these functions. Examples thereof can include variable regions derived from an IgG-type antibody variable region by the alteration of a portion of its amino acids so as to bind to the desired antigen. The amino acid to be altered is selected from, for example, amino acids whose alteration does not cancel the binding to the antigen, in an antibody variable region binding to CD3 or CD137.

In this context, the phrase "expressed on different cells" merely means that the antigens are expressed on separate cells. The combination of such cells may be, for example, the same types of cells such as a T cell and another T cell, or may be different types of cells such as a T cell and an NK cell.

Whether the multispecific antigen-binding molecule contained in the anticancer agent, the pharmaceutical composition, the combination, or the kit of the present disclosure, or used in the method or use of the present disclosure "bind to either one of CD3 or CD137" when binding to an antigen can be confirmed by: confirming the antigen-binding molecule to have binding activity against both CD3 and CD137; then allowing either CD3 or CD137 to bind in advance to the antigen-binding molecule comprising the variable region having this binding activity; and then determining the presence or absence of its binding activity against the other one by the method mentioned above. Alternatively, this can also be confirmed by determining whether the binding of the antigen-binding molecule to either CD3 or CD137 immobilized on an ELISA plate or a sensor chip is inhibited by the addition of the other one into the solution. In some embodiments, the binding of the antigen-binding molecule of the present disclosure to either CD3 or CD137 is inhibited by binding of the antigen-binding molecule to the other by at least 50%, preferably 60% or more, more preferably 70% or more, more preferably 80% or more, further preferably 90% or more, or even more preferably 95% or more.

In one aspect, while one antigen (e.g. CD3) is immobilized, the inhibition of the binding of the antigen-binding molecule to CD3 can be determined in the presence of the other antigen (e.g. CD137) by methods known in prior art (i.e. ELISA, BIACORE, and so on). In another aspect, while CD137 is immobilized, the inhibition of the binding of the antigen-binding molecule to CD137 also can be determined in the presence of CD3. When either one of two aspects mentioned above is conducted, the antigen-binding molecule of the present disclosure is determined not to bind to CD3 and CD137 at the same time if the binding is inhibited by at least 50%, preferably 60% or more, preferably 70% or more, further preferably 80% or more, further preferably 90% or more, or even more preferably 95% or more.

In some embodiments, the concentration of the antigen injected as an analyte is at least 1-fold, 2-fold, 5-fold, 10-fold, 30-fold, 50-fold, or 100-fold higher than the concentration of the other antigen to be immobilized.

In preferable manner, the concentration of the antigen injected as an analyte is 100-fold higher than the concentration of the other antigen to be immobilized and the binding is inhibited by at least 80%.

In one embodiment, the ratio of the KD value for the CD3 (analyte)-binding activity of the antigen-binding molecule to the CD137 (immobilized)-binding activity of the antigen-binding molecule (KD (CD3)/ KD (CD137)) is calculated and the CD3 (analyte) concentration which is 10-fold, 50-fold, 100-fold, or 200-fold of the ratio of the KD value (KD(CD3)/KD(CD137) higher than the CD137 (immobilized) concentration can be used for the competition measurement above, (e.g. 1-fold, 5-fold, 10-fold, or 20-fold higher concentration can be selected when the ratio of the KD value is 0.1. Furthermore, 100-fold, 500-fold, 1000-fold, or 2000-fold higher concentration can be selected when the ratio of the KD value is 10.)

In one aspect, while one antigen (e.g. CD3) is immobilized, the attenuation of the binding signal of the antigen-binding molecule to CD3 can be determined in the presence of the other antigen (e.g. CD137) by methods known in prior art (i.e. ELISA, ECL and so on). In another aspect, while CD137 is immobilized, the attenuation of the binding signal of the antigen-binding molecule to CD137 also can be determined in the presence of CD3. When either one of two aspects mentioned above is conducted, the antigen-binding molecule of the present disclosure is determined not to bind to CD3 and CD137 at the same time if the binding signal is attenuated by at least 50%, preferably 60% or more, preferably 70% or more, further preferably 80% or more, further preferably 90% or more, or even more preferably 95% or more.

In some embodiments, the concentration of the antigen injected as an analyte is at least 1-fold, 2-fold, 5-fold, 10-fold, 30-fold, 50-fold, or 100-fold higher than the concentration of the other antigen to be immobilized.

In preferable manner, the concentration of the antigen injected as an analyte is 100-fold higher than the concentration of the other antigen to be immobilized and the binding is inhibited by at least 80%.

In one embodiment, the ratio of the KD value for the CD3 (analyte)-binding activity of the antigen-binding molecule to the CD137 (immobilized)-binding activity of the antigen-binding molecule (KD (CD3)/ KD (CD137)) is calculated and the CD3 (analyte) concentration which is 10-fold, 50-fold, 100-fold, or 200-fold of the ratio of the KD value (KD(CD3)/KD(CD137) higher than the CD137 (immobilized) concentration can be used for the measurement above, (e.g. 1-fold, 5-fold, 10-fold, or 20-fold higher concentration can be selected when the ratio of the KD value is 0.1. Furthermore, 100-fold, 500-fold, 1000-fold, or 2000-fold higher concentration can be selected when the ratio of the KD value is 10.)

Specifically, in the case of using, for example, the ECL method, a biotin-labeled antigen-binding molecule to be tested, CD3 labeled with sulfo-tag (Ru complex), and an unlabeled CD137 are prepared. When the antigen-binding molecule to be tested is capable of binding to CD3 and CD137, but binds to either CD3 or CD137, the luminescence signal of the sulfo-tag is detected in the absence of the unlabeled CD137 by adding the mixture of the antigen-binding molecule to be tested and labeled CD3 onto a streptavidin-immobilized plate, followed by light development. By contrast, the luminescence signal is decreased in the presence of unlabeled CD137. This decrease in luminescence signal can be quantified to determine relative binding activity. This analysis may be similarly conducted using the labeled CD137 and the unlabeled CD3.

In the case of the ALPHAScreen, the antigen-binding molecule to be tested interacts with CD3 in the absence of the competing CD137 to generate signals of 520 to 620 nm. The untagged CD137 competes with CD3 for the interaction with the antigen-binding molecule to be tested. Decrease in fluorescence caused as a result of the competition can be quantified to thereby determine relative binding activity. The polypeptide biotinylation using sulfo-NHS-biotin or the like is known in the art. CD3 can be tagged with GST by an appropriately adopted method which involves, for example: fusing a polynucleotide encoding CD3 in flame with a polynucleotide encoding GST; and allowing the resulting fusion gene to be expressed by cells or the like harboring vectors capable of expression thereof, followed by purification using a glutathione column. The obtained signals are preferably analyzed using, for example, software GRAPHPAD PRISM (GraphPad Software, Inc., San Diego) adapted to a one-site competition model based on nonlinear regression analysis. This analysis may be similarly conducted using the tagged CD137 and the untagged CD3.

Alternatively, a method using fluorescence resonance energy transfer (FRET) may be used. FRET is a phenomenon in which excitation energy is transferred directly between two fluorescent molecules located in proximity to each other by electron resonance. When FRET occurs, the excitation energy of a donor (fluorescent molecule having an excited state) is transferred to an acceptor (another fluorescent molecule located near the donor) so that the fluorescence emitted from the donor disappears (to be precise, the lifetime of the fluorescence is shortened) and instead, the fluorescence is emitted from the acceptor. By use of this phenomenon, whether or not bind to CD3 and CD137 at the same time can be analyzed. For example, when CD3 carrying a fluorescence donor and CD137 carrying a fluorescence acceptor bind to the antigen-binding molecule to be tested at the same time, the fluorescence of the donor disappears while the fluorescence is emitted from the acceptor. Therefore, change in fluorescence wavelength is observed. Such an antibody is confirmed to bind to CD3 and CD137 at the same time. On the other hand, if the mixing of CD3, CD137, and the antigen-binding molecule to be tested does not change the fluorescence wavelength of the fluorescence donor bound with CD3, this antigen-binding molecule to be tested can be regarded as antigen binding domain that is capable of binding to CD3 and CD137, but binds to either CD3 or CD137.

For example, a biotin-labeled antigen-binding molecule to be tested is allowed to bind to streptavidin on the donor bead, while CD3 tagged with glutathione S transferase (GST) is allowed to bind to the acceptor bead. The antigen-binding molecule to be tested interacts with CD3 in the absence of the competing second antigen to generate signals of 520 to 620 nm. The untagged second antigen competes with CD3 for the interaction with the antigen-binding molecule to be tested. Decrease in fluorescence caused as a result of the competition can be quantified to thereby determine relative binding activity. The polypeptide biotinylation using sulfo-NHS-biotin or the like is known in the art. CD3 can be tagged with GST by an appropriately adopted method which involves, for example: fusing a polynucleotide encoding CD3 in flame with a polynucleotide encoding GST; and allowing the resulting fusion gene to be expressed by cells or the like harboring vectors capable of expression thereof, followed by purification using a glutathione column. The obtained signals are preferably analyzed using, for example, software GRAPHPAD PRISM (GraphPad Software, Inc., San Diego) adapted to a one-site competition model based on nonlinear regression analysis.

The tagging is not limited to the GST tagging and may be carried out with any tag such as, but not limited to, a histidine tag, MBP, CBP, a Flag tag, an HA tag, a V5 tag, or a c-myc tag. The binding of the antigen-binding molecule to be tested to the donor bead is not limited to the binding using biotin-streptavidin reaction. Particularly, when the antigen-binding molecule to be tested comprises Fc, a possible method involves allowing the antigen-binding molecule to be tested to bind via an Fc-recognizing protein such as protein A or protein G on the donor bead.

Also, the case where the variable region cannot bind to CD3 and CD137 each expressed on a different cell, at the same time, can also be assayed by a method known in the art.

Specifically, the antigen-binding molecule to be tested has been confirmed to be positive in ECL-ELISA for detecting binding to CD3 and CD137 at the same time is also mixed with a cell expressing CD3 and a cell expressing CD137. The antigen-binding molecule to be tested can be shown to be incapable of binding to CD3 and CD137 expressed on different cells, at the same time unless the antigen-binding molecule and these cells bind to each other at the same time. This assay can be conducted by, for example, cell-based ECL-ELISA. The cell expressing CD3 is immobilized onto a plate in advance. After binding of the antigen-binding molecule to be tested thereto, the cell expressing CD137 is added to the plate. A different antigen expressed only on the cell expressing CD137 is detected using a sulfo-tag-labeled antibody against this antigen. A signal is observed when the antigen-binding molecule binds to the two antigens respectively expressed on the two cells, at the same time. No signal is observed when the antigen-binding molecule does not bind to these antigens at the same time.

Alternatively, this assay may be conducted by the ALPHAScreen method. The antigen-binding molecule to be tested is mixed with a cell expressing CD3 bound with the donor bead and a cell expressing CD137 bound with the acceptor bead. A signal is observed when the antigen-binding molecule binds to the two antigens expressed on the two cells respectively, at the same time. No signal is observed when the antigen-binding molecule does not bind to these antigens at the same time.

Alternatively, this assay may also be conducted by an Octet interaction analysis method. First, a cell expressing CD3 tagged with a peptide tag is allowed to bind to a biosensor that recognizes the peptide tag. A cell expressing CD137 and the antigen-binding molecule to be tested are placed in wells and analyzed for interaction. A large wavelength shift caused by the binding of the antigen-binding molecule to be tested and the cell expressing CD137 to the biosensor is observed when the antigen-binding molecule binds to the two antigens expressed on the two cells respectively, at the same time. A small wavelength shift caused by the binding of only the antigen-binding molecule to be tested to the biosensor is observed when the antigen-binding molecule does not bind to these antigens at the same time.

Instead of these methods based on the binding activity, assay based on biological activity may be conducted. For example, a cell expressing CD3 and a cell expressing CD137 are mixed with the antigen-binding molecule to be tested, and cultured. The two antigens expressed on the two cells respectively are mutually activated via the antigen-binding molecule to be tested when the antigen-binding molecule binds to these two antigens at the same time. Therefore, change in activation signal, such as increase in the respective downstream phosphorylation levels of the antigens, can be detected. Alternatively, cytokine production is induced as a result of the activation. Therefore, the amount of cytokines produced can be measured to thereby confirm whether or not to bind to the two cells at the same time. Alternatively, cytotoxicity against a cell expressing CD137 is induced as a result of the activation. Alternatively, the expression of a reporter gene is induced by a promoter which is activated at the downstream of the signal transduction pathway of CD137 or CD3 as a result of the activation. Therefore, the cytotoxicity or the amount of reporter proteins produced can be measured to thereby confirm whether or not to bind to the two cells at the same time.

The multispecific antigen-binding molecule contained in the anticancer agent, the pharmaceutical composition, the combination, or the kit of the present disclosure, or used in the method or use of the present disclosure, is an antigen-binding molecule whose binding to CD3 and CD137 is not simultaneous (i.e., does not bind to CD3 and CD137 simultaneously); therefore, simultaneous binding of CD3 and/or CD137 expressed on different immune cells (for example, T cells) by the same antigen-binding molecule will not occur, thereby circumventing toxicity due to undesirable cross-linking between different immune cells which is considered to be responsible for adverse reactions when a conventional multispecific antigen-binding molecule capable of simultaneously binding to CD3 and a second molecule (for example, CD137) expressed on T cells is administered in vivo. The toxicity of the multispecific antigen-binding molecule when administered in vivo can be determined from cytokine production and such. Low toxicity refers to absence of induced immune activation such as CLDN6-independent cytokine production when compared to a multispecific antibody used as a control.

Fab molecule

A "Fab molecule" refers to a protein consisting of the VH and CH1 domain of the heavy chain (the "Fab heavy chain") and the VL and CL domain of the light chain (the "Fab light chain") of an immunoglobulin.

Fused

By "fused" is meant that the components (e.g. a Fab molecule and an Fc domain subunit) are linked by peptide bonds, either directly or via one or more peptide linkers.

"Crossover" Fab

By a "crossover" Fab molecule (also termed "Crossfab") is meant a Fab molecule wherein either the variable regions or the constant regions of the Fab heavy and light chain are exchanged, i.e. the crossover Fab molecule comprises a peptide chain composed of the light chain variable region and the heavy chain constant region, and a peptide chain composed of the heavy chain variable region and the light chain constant region. For clarity, in a crossover Fab molecule wherein the variable regions of the Fab light chain and the Fab heavy chain are exchanged, the peptide chain comprising the heavy chain constant region is referred to herein as the "heavy chain" of the crossover Fab molecule. Conversely, in a crossover Fab molecule wherein the constant regions of the Fab light chain and the Fab heavy chain are exchanged, the peptide chain comprising the heavy chain variable region is referred to herein as the "heavy chain" of the crossover Fab molecule.

"Conventional" Fab

In contrast thereto, by a "conventional" Fab molecule is meant a Fab molecule in its natural format, i.e. comprising a heavy chain composed of the heavy chain variable and constant regions (VH-CH1), and a light chain composed of the light chain variable and constant regions (VL-CL). The term "immunoglobulin molecule" refers to a protein having the structure of a naturally occurring antibody. For example, immunoglobulins of the IgG class are heterotetrameric glycoproteins of about 150,000 daltons, composed of two light chains and two heavy chains that are disulfide-bonded. From N- to C-terminus, each heavy chain has a variable region (VH), also called a variable heavy domain or a heavy chain variable domain, followed by three constant domains (CH1, CH2, and CH3), also called a heavy chain constant region. Similarly, from N- to C-terminus, each light chain has a variable region (VL), also called a variable light domain or a light chain variable domain, followed by a constant light (CL) domain, also called a light chain constant region. The heavy chain of an immunoglobulin may be assigned to one of five types, called alpha (IgA), delta (IgD), epsilon (IgE), gamma (IgG), or mu (IgM), some of which may be further divided into subtypes, e.g. gamma 1 (IgG1), gamma 2 (IgG2), gamma 3 (IgG3), gamma 4 (IgG4), alpha 1 (IgA1) and alpha 2 (IgA2). The light chain of an immunoglobulin may be assigned to one of two types, called kappa and lambda, based on the amino acid sequence of its constant domain. An immunoglobulin essentially consists of two Fab molecules and an Fc domain, linked via the immunoglobulin hinge region.

Affinity

"Affinity" refers to the strength of the sum total of noncovalent interactions between a single binding site of a molecule (e.g., an antigen-binding molecule or antibody) and its binding partner (e.g., an antigen). Unless indicated otherwise, as used herein, "binding affinity" refers to intrinsic binding affinity which reflects a 1:1 interaction between members of a binding pair (e.g., antigen-binding molecule and antigen, or antibody and antigen). The affinity of a molecule X for its partner Y can generally be represented by the dissociation constant (KD), which is the ratio of dissociation and association rate constants (koff and kon, respectively). Thus, equivalent affinities may comprise different rate constants, as long as the ratio of the rate constants remains the same. Affinity can be measured by well-established methods known in the art, including those described herein. A particular method for measuring affinity is Surface Plasmon Resonance (SPR).

Methods to determine affinity

In certain embodiments, the antigen-binding molecule or antibody provided herein has a dissociation constant (KD) of 1 micro M or less, 120 nM or less, 100 nM or less, 80 nM or less, 70 nM or less, 50 nM or less, 40 nM or less, 30 nM or less, 20 nM or less, 10 nM or less, 2 nM or less, 1 nM or less, 0.1 nM or less, 0.01 nM or less, or 0.001 nM or less (e.g., $10^{-8}$ M or less, $10^{-8}$ M to $10^{-13}$ M, $10^{-9}$ M to $10^{-13}$ M) for its antigen. In certain embodiments, the KD value of the antibody/antigen-binding molecule for CD3, CD137 or CLDN6 falls within the range of 1-40, 1-50, 1-70, 1-80, 30-50, 30-70, 30-80, 40-70, 40-80, or 60-80 nM.

In one embodiment, KD is measured by a radiolabeled antigen-binding assay (RIA). In one embodiment, an RIA is performed with the Fab version of an antibody of interest and its antigen. For example, solution binding affinity of Fabs for antigen is measured by equilibrating Fab with a minimal concentration of ($^{125}$I)-labeled antigen in the presence of a titration series of unlabeled antigen, then capturing bound antigen with an anti-Fab antibody-coated plate (see, e.g., Chen et al., J. Mol. Biol. 293:865-881(1999)). To establish conditions for the assay, MICROTITER (registered trademark) multi-well plates (Thermo Scientific) are coated overnight with 5 micro g/ml of a capturing anti-Fab antibody (Cappel Labs) in 50 mM sodium carbonate (pH 9.6), and subsequently blocked with 2% (w/v) bovine serum albumin in PBS for two to five hours at room temperature (approximately 23 degrees C). In a non-adsorbent plate (Nunc #269620), 100 pM or 26 pM [$^{125}$I]-antigen are mixed with serial dilutions of a Fab of interest (e.g., consistent with assessment of the anti-VEGF antibody, Fab-12, in Presta et al., Cancer Res. 57:4593-4599 (1997)). The Fab of interest is then incubated overnight; however, the incubation may continue for a longer period (e.g., about 65 hours) to ensure that equilibrium is reached. Thereafter, the mixtures are transferred to the capture plate for incubation at room temperature (e.g., for one hour). The solution is then removed and the plate washed eight times with 0.1% polysorbate 20 (TWEEN-20 (registered trademark)) in PBS. When the plates have dried, 150 micro l/well of scintillant (MICROSCINT-20 ™; Packard) is added, and the plates are counted on a TOPCOUNT™ gamma counter (Packard) for ten minutes. Concentrations of each Fab that give less than or equal to 20% of maximal binding are chosen for use in competitive binding assays.

According to another embodiment, Kd is measured using a BIACORE (registered trademark) surface plasmon resonance assay. For example, an assay using a BIACORE (registered trademark)-2000 or a BIACORE(registered trademark)-3000 (BIAcore, Inc., Piscataway, NJ) is performed at 25 degrees C with immobilized antigen CM5 chips at ~10 response units (RU). In one embodiment, carboxymethylated dextran biosensor chips (CM5, BIACORE, Inc.) are activated with N-ethyl-N'- (3-dimethylaminopropyl)-carbodiimide hydrochloride (EDC) and N-hydroxysuccinimide (NHS) according to the supplier's instructions. Antigen is diluted with 10 mM sodium acetate, pH 4.8, to 5 micro g/ml (~0.2 micro M) before injection at a flow rate of 5 micro l/minute to achieve approximately 10 response units (RU) of coupled protein. Following the injection of antigen, 1 M ethanolamine is injected to block unreacted groups. For kinetics measurements, two-fold serial dilutions of Fab (0.78 nM to 500 nM) are injected in PBS with 0.05% polysorbate 20 (TWEEN-20™) surfactant (PBST) at 25 degrees C at a flow rate of approximately 25 micro l/min. Association rates ($k_{on}$) and dissociation rates ($k_{off}$) are calculated using a simple one-to-one Langmuir binding model (BIACORE (registered trademark) Evaluation Software version 3.2) by simultaneously fitting the association and dissociation sensorgrams. The equilibrium dissociation constant (Kd) is calculated as the ratio $k_{off}/k_{on}$. See, e.g., Chen et al., J. Mol. Biol. 293:865-881 (1999). If the on-rate exceeds $10^6$ $M^{-1}$ $s^{-1}$ by the surface plasmon resonance assay above, then the on-rate can be determined by using a fluorescent quenching technique that measures the increase or decrease in fluorescence emission intensity (excitation = 295 nm; emission = 340 nm, 16 nm band-pass) at 25 degrees C of a 20 nM anti-antigen antibody (Fab form) in PBS, pH 7.2, in the presence of increasing concentrations of antigen as measured in a spectrometer, such as a stop-flow equipped spectrophotometer (Aviv Instruments) or a 8000-series SLM-AMINCO™ spectrophotometer (ThermoSpectronic) with a stirred cuvette.

According to the methods for measuring the affinity of the antigen-binding molecule or the antibody described above, persons skilled in art can carry out affinity measurement for other antigen-binding molecules or antibodies, towards various kind of antigens.

Antibody

The term "antibody" herein is used in the broadest sense and encompasses various antibody structures, including but not limited to monoclonal antibodies, polyclonal antibodies, multispecific antibodies (e.g., bispecific antibodies, trispecific antibodies), and antibody fragments so long as they exhibit the desired antigen-binding activity.

In certain embodiments, the multispecific antibody described herein binds to an epitope of CD3, CD137 or CLDN6 that is conserved among the CD3, CD137 or CLDN6 from different species. In certain embodiments, the multispecific antibody of the present disclosure is a tri-specific antibody, which is an antibody capable of specifically binding to three different types of antigens. That is, in certain embodiments, the multispecific antibody of the present disclosure is a tri-specific antibody capable of binding to CD3 and CD137, and binds to either CD3 or CD137, i.e., it does not bind to both antigens CD3 and CD137 simultaneously, and is also capable of specifically binding to CLDN6.

Class of antibody

The "class" of an antibody refers to the type of constant domain or constant region possessed by its heavy chain. There are five major classes of antibodies: IgA, IgD, IgE, IgG, and IgM, and several of these may be further divided into subclasses (isotypes), e.g., IgG1, IgG2, IgG3, IgG4, IgA1, and IgA2. The heavy chain constant domains that correspond to the different classes of immunoglobulins are called alpha, delta, epsilon, gamma, and mu, respectively.

Unless otherwise indicated, amino acid residues in the light chain constant region are numbered herein according to Kabat et al., and numbering of amino acid residues in the heavy chain constant region is according to the EU numbering system, also called the EU index, as described in Kabat et al., Sequences of Proteins of Immunological Interest, 5th Ed. Public Health Service, National Institutes of Health, Bethesda, MD, 1991.

Framework

"Framework" or "FR" refers to variable domain residues other than hypervariable region (HVR) residues. The FR of a variable domain generally consists of four FR domains: FR1, FR2, FR3, and FR4. Accordingly, the HVR and FR sequences generally appear in the following sequence in VH (or VL): FR1-H1(L1)-FR2-H2(L2)-FR3-H3(L3)-FR4.

Human consensus framework

A "human consensus framework" is a framework which represents the most commonly occurring amino acid residues in a selection of human immunoglobulin VL or VH framework sequences. Generally, the selection of human immunoglobulin VL or VH sequences is from a subgroup of variable domain sequences. Generally, the subgroup of sequences is a subgroup as in Kabat et al., Sequences of Proteins of Immunological Interest, Fifth Edition, NIH Publication 91-3242, Bethesda MD (1991), vols. 1-3. In one embodiment, for the VL, the subgroup is subgroup kappa I as in Kabat et al., supra. In one embodiment, for the VH, the subgroup is subgroup III as in Kabat et al., supra.

Chimeric antibody

The term "chimeric" antibody refers to an antibody in which a portion of the heavy and/or light chain is derived from a particular source or species, while the remainder of the heavy and/or light chain is derived from a different source or species. Similarly, the term "chimeric antibody variable domain" refers to an antibody variable region in which a portion of the heavy and/or light chain variable region is derived from a particular source or species, while the remainder of the heavy and/or light chain variable region is derived from a different source or species.

Humanized antibody

A "humanized" antibody refers to a chimeric antibody comprising amino acid residues from non-human HVRs and amino acid residues from human FRs. In certain embodiments, a humanized antibody will comprise substantially all of at least one, and typically two, variable domains, in which all or substantially all of the HVRs (e.g., CDRs) correspond to those of a non-human antibody, and all or substantially all of the FRs correspond to those of a human antibody. A humanized antibody optionally may comprise at least a portion of an antibody constant region derived from a human antibody. A "humanized form" of an antibody, e.g., a non-human antibody, refers to an antibody that has undergone humanization. A "humanized antibody variable region" refers to the variable region of a humanized antibody.

Human antibody

A "human antibody" is one which possesses an amino acid sequence which corresponds to that of an antibody produced by a human or a human cell or derived from a non-human source that utilizes human antibody repertoires or other human antibody-encoding sequences. This definition of a human antibody specifically excludes a humanized antibody comprising non-human antigen-binding residues. A "human antibody variable region" refers to the variable region of a human antibody.

Polynucleotide (nucleic acid)

"Polynucleotide" or "nucleic acid" as used interchangeably herein, refers to polymers of nucleotides of any length, and include DNA and RNA. The nucleotides can be deoxyribonucleotides, ribonucleotides, modified nucleotides or bases, and/or their analogs, or any substrate that can be incorporated into a polymer by DNA or RNA polymerase or by a synthetic reaction. A polynucleotide may comprise modified nucleotides, such as methylated nucleotides and their analogs. A sequence of nucleotides may be interrupted by non-nucleotide components. A polynucleotide may comprise modification(s) made after synthesis, such as conjugation to a label. Other types of modifications include, for example, "caps," substitution of one or more of the naturally occurring nucleotides with an analog, internucleotide modifications such as, for example, those with uncharged linkages (e.g., methyl phosphonates, phosphotriesters, phosphoramidates, carbamates, etc.) and with charged linkages (e.g., phosphorothioates, phosphorodithioates, etc.), those containing pendant moieties, such as, for example, proteins (e.g., nucleases, toxins, antibodies, signal peptides, poly-L-lysine, etc.), those with intercalators (e.g., acridine, psoralen, etc.), those containing chelators (e.g., metals, radioactive metals, boron, oxidative metals, etc.), those containing alkylators, those with modified linkages (e.g., alpha anomeric nucleic acids, etc.), as well as unmodified forms of the polynucleotides(s). Further, any of the hydroxyl groups ordinarily present in the sugars may be replaced, for example, by phosphonate groups, phosphate groups, protected by standard protecting groups, or activated to prepare additional linkages to additional nucleotides, or may be conjugated to solid or semi-solid supports. The 5' and 3' terminal OH can be phosphorylated or substituted with amines or organic capping group moieties of from 1 to 20 carbon atoms. Other hydroxyls may also be derivatized to standard protecting groups. Polynucleotides can also contain analogous forms of ribose or deoxyribose sugars that are generally known in the art, including, for example, 2'-O-methyl-, 2'-O-allyl-, 2'-fluoro- or 2'-azido-ribose, carbocyclic sugar analogs, alpha-anomeric sugars, epimeric sugars such as arabinose, xyloses or lyxoses, pyranose sugars, furanose sugars, sedoheptuloses, acyclic analogs, and basic nucleoside analogs such as methyl riboside. One or more phosphodiester linkages may be replaced by alternative linking groups. These alternative linking groups include, but are not limited to, embodiments wherein phosphate is replaced by P(O)S ("thioate"), P(S)S ("dithioate"), (O)$NR_2$ ("amidate"), P(O)R, P(O)OR', CO, or CH2 ("formacetal"), in which each R or R' is independently H or substituted or unsubstituted alkyl (1-20 C) optionally containing an ether (-O-) linkage, aryl, alkenyl, cycloalkyl, cycloalkenyl or araldyl. Not all linkages in a polynucleotide need be identical. The preceding description applies to all polynucleotides referred to herein, including RNA and DNA.

Isolated (nucleic acid)

An "isolated" nucleic acid molecule is one which has been separated from a component of its natural environment. An isolated nucleic acid molecule further includes a nucleic acid molecule contained in cells that ordinarily contain the nucleic acid molecule, but the nucleic acid molecule is present extrachromosomally or at a chromosomal location that is different from its natural chromosomal location.

Vector

The term "vector," as used herein, refers to a nucleic acid molecule capable of propagating another nucleic acid to which it is linked. The term includes the vector as a self-replicating nucleic acid structure as well as the vector incorporated into the genome of a host cell into which it has been introduced. Certain vectors are capable of directing the expression of nucleic acids to which they are operatively linked. Such vectors are referred to herein as "expression vectors." Vectors could be introduced into host cells using virus or electroporation. However, introduction of vectors is not limited to in vitro method. For example, vectors could also be introduced into a subject using in vivo method directly.

Host cell

The terms "host cell," "host cell line," and "host cell culture" are used interchangeably and refer to cells into which exogenous nucleic acid has been introduced, including the progeny of such cells. Host cells include "transformants" and "transformed cells," which include the primary transformed cell and progeny derived therefrom without regard to the number of passages. Progeny may not be completely identical in nucleic acid content to a parent cell, but may contain mutations. Mutant progeny that have the same function or biological activity as screened or selected for in the originally transformed cell are included herein.

Specificity

"Specific" means that a molecule that binds specifically to one or more binding partners does not show any significant binding to molecules other than the partners. Furthermore, "specific" is also used when an antigen-binding site is specific to a particular epitope of multiple epitopes contained in an antigen. If an antigen-binding molecule binds specifically to an antigen, it is also described as "the antigen-binding molecule has/shows specificity to/towards the antigen". When an epitope bound by an antigen-binding site is contained in multiple different antigens, an antigen-binding molecule containing the antigen-binding site can bind to various antigens that have the epitope.

Antibody fragment

An "antibody fragment" refers to a molecule other than an intact antibody that comprises a portion of an intact antibody that binds the antigen to which the intact antibody binds. Examples of antibody fragments include but are not limited to Fv, Fab, Fab', Fab'-SH, F(ab')2, diabodies, linear antibodies, single-chain antibody molecules (e.g. scFv), and single-domain antibodies. For a review of certain antibody fragments, see Hudson et al., Nat Med 9, 129-134 (2003). For a review of scFv fragments, see e.g. Pluckthun, in The Pharmacology of Monoclonal Antibodies, vol. 113, Rosenburg and Moore eds., Springer-Verlag, New York, pp. 269-315 (1994); see also WO 93/16185 ; and U.S. Patent Nos. 5,571,894 and 5,587,458 . For discussion of Fab and F(ab')2 fragments comprising salvage receptor binding epitope residues and having increased in vivo half-life, see U.S. Patent No. 5,869,046 . Diabodies are antibody fragments with two antigen-binding sites that may be bivalent or bispecific. See, for example, EP 404,097 ; WO 1993/01161 ; Hudson et al., Nat Med 9, 129-134 (2003); and Hollinger et al., Proc Natl Acad Sci USA 90, 6444-6448 (1993). Triabodies and tetrabodies are also described in Hudson et al., Nat Med 9, 129-134 (2003). Single-domain antibodies are antibody fragments comprising all or a portion of the heavy chain variable domain or all or a portion of the light chain variable domain of an antibody. In certain embodiments, a single-domain antibody is a human single-domain antibody (Domantis, Inc., Waltham, MA; see e.g. U.S. Patent No. 6,248,516 B1). Antibody fragments can be made by various techniques, including but not limited to proteolytic digestion of an intact antibody as well as production by recombinant host cells (e.g. E. coli or phage), as described herein.

Variable fragment (Fv)

Herein, the term "variable fragment (Fv)" refers to the minimum unit of an antibody-derived antigen-binding site that is composed of a pair of the antibody light chain variable region (VL) and antibody heavy chain variable region (VH). In 1988, Skerra and Pluckthun found that homogeneous and active antibodies can be prepared from the E. coli periplasm fraction by inserting an antibody gene downstream of a bacterial signal sequence and inducing expression of the gene in E. coli (Science (1988) 240(4855), 1038-1041). In the Fv prepared from the periplasm fraction, VH associates with VL in a manner so as to bind to an antigen.

scFv, single-chain antibody, and sc$(Fv)_2$

Herein, the terms "scFv", "single-chain antibody", and "sc$(Fv)_2$" all refer to an antibody fragment of a single polypeptide chain that contains variable regions derived from the heavy and light chains, but not the constant region. In general, a single-chain antibody also contains a polypeptide linker between the VH and VL domains, which enables formation of a desired structure that is thought to allow antigen-binding. The single-chain antibody is discussed in detail by Pluckthun in "The Pharmacology of Monoclonal Antibodies, Vol. 113, Rosenburg and Moore, eds., Springer-Verlag, New York, 269-315 (1994)". See also International Patent Publication WO 1988/001649; US Patent Nos. 4,946,778 and 5,260,203. In a particular embodiment, the single-chain antibody can be bispecific and/or humanized.

An scFv is a single chain low molecule weight antibody in which VH and VL forming Fv are linked together by a peptide linker (Proc. Natl. Acad. Sci. U.S.A. (1988) 85(16), 5879-5883). VH and VL can be retained in close proximity by the peptide linker.

sc$(Fv)_2$ is a single chain antibody in which four variable regions of two VL and two VH are linked by linkers such as peptide linkers to form a single chain (J Immunol. Methods (1999) 231(1-2), 177-189). The two VH and two VL may be derived from different monoclonal antibodies. Such sc$(Fv)_2$ preferably includes, for example, a bispecific sc$(Fv)_2$ that recognizes two epitopes present in a single antigen as disclosed in the Journal of Immunology (1994) 152(11), 5368-5374. sc$(Fv)_2$ can be produced by methods known to those skilled in the art. For example, sc$(Fv)_2$ can be produced

by linking scFv by a linker such as a peptide linker.

Herein, an sc(Fv)$_2$ includes two VH units and two VL units which are arranged in the order of VH, VL, VH, and VL ([VH]-linker-[VL]-linker-[VH]-linker-[VL]) beginning from the N terminus of a single-chain polypeptide. The order of the two VH units and two VL units is not limited to the above form, and they may be arranged in any order. Examples of the form are listed below.

[VL]-linker-[VH]-linker-[VH]-linker-[VL]
[VH]-linker-[VL]-linker-[VL]-linker-[VH]
[VH]-linker-[VH]-linker-[VL]-linker-[VL]
[VL] -linker- [VL] -linker- [VH] -linker- [VH]
[VL]-linker-[VH]-linker-[VL]-linker-[VH]

The molecular form of sc(Fv)$_2$ is also described in detail in WO 2006/132352. According to these descriptions, those skilled in the art can appropriately prepare desired sc(Fv)$_2$ to produce the polypeptide complexes disclosed herein.

Furthermore, the antigen-binding molecules or antibodies of the present disclosure may be conjugated with a carrier polymer such as PEG or an organic compound such as an anticancer agent. Alternatively, a sugar chain addition sequence is preferably inserted into the antigen-binding molecules or antibodies such that the sugar chain produces a desired effect.

The linkers to be used for linking the variable regions of an antibody comprise arbitrary peptide linkers that can be introduced by genetic engineering, synthetic linkers, and linkers disclosed in, for example, Protein Engineering, 9(3), 299-305, 1996. However, peptide linkers are preferred in the present disclosure. The length of the peptide linkers is not particularly limited, and can be suitably selected by those skilled in the art according to the purpose. The length is preferably five amino acids or more (without particular limitation, the upper limit is generally 30 amino acids or less, preferably 20 amino acids or less), and particularly preferably 15 amino acids. When sc(Fv)$_2$ contains three peptide linkers, their length may be all the same or different.

For example, such peptide linkers include:

Ser,
Gly-Ser,
Gly-Gly-Ser,
Ser-Gly-Gly,
Gly-Gly-Gly-Ser (SEQ ID NO: 171),
Ser-Gly-Gly-Gly (SEQ ID NO: 172),
Gly-Gly-Gly-Gly-Ser (SEQ ID NO: 173),
Ser-Gly-Gly-Gly-Gly (SEQ ID NO: 174),
Gly-Gly-Gly-Gly-Gly-Ser (SEQ ID NO: 175),
Ser-Gly-Gly-Gly-Gly-Gly (SEQ ID NO: 176),
Gly-Gly-Gly-Gly-Gly-Gly-Ser (SEQ ID NO: 177),
Ser-Gly-Gly-Gly-Gly-Gly-Gly (SEQ ID NO: 178),
(Gly-Gly-Gly-Gly-Ser (SEQ ID NO: 173))n, and
(Ser-Gly-Gly-Gly-Gly (SEQ ID NO: 174))n,

where n is an integer of 1 or larger. The length or sequences of peptide linkers can be selected accordingly by those skilled in the art depending on the purpose.

Synthetic linkers (chemical crosslinking agents) are routinely used to crosslink peptides, and examples include:

N-hydroxy succinimide (NHS),
disuccinimidyl suberate (DSS),
bis(sulfosuccinimidyl) suberate (BS3),
dithiobis(succinimidyl propionate) (DSP),
dithiobis(sulfosuccinimidyl propionate) (DTSSP),
ethylene glycol bis(succinimidyl succinate) (EGS),
ethylene glycol bis(sulfosuccinimidyl succinate) (sulfo-EGS),
disuccinimidyl tartrate (DST), disulfosuccinimidyl tartrate (sulfo-DST),
bis[2-(succinimidoxycarbonyloxy)ethyl] sulfone (BSOCOES), and
bis[2-(sulfosuccinimidoxycarbonyloxy)ethyl] sulfone (sulfo-BSOCOES). These crosslinking agents are commercially available.

In general, three linkers are required to link four antibody variable regions together. The linkers to be used may be of the same type or different types.

Fab, F(ab')$_2$, and Fab'

"Fab" consists of a single light chain, and a CH1 domain and variable region from a single heavy chain. The heavy chain of Fab molecule cannot form disulfide bonds with another heavy chain molecule.

"F(ab')$_2$" or "Fab" is produced by treating an immunoglobulin (monoclonal antibody) with a protease such as pepsin and papain, and refers to an antibody fragment generated by digesting an immunoglobulin (monoclonal antibody) near the disulfide bonds present between the hinge regions in each of the two H chains. For example, papain cleaves IgG upstream of the disulfide bonds present between the hinge regions in each of the two H chains to generate two homologous antibody fragments, in which an L chain comprising VL (L-chain variable region) and CL (L-chain constant region) is linked to an H-chain fragment comprising VH (H-chain variable region) and CH gamma 1 (gamma 1 region in an H-chain constant region) via a disulfide bond at their C-terminal regions. Each of these two homologous antibody fragments is called Fab'.

"F(ab')$_2$" consists of two light chains and two heavy chains comprising the constant region of a CH1 domain and a portion of CH2 domains so that disulfide bonds are formed between the two heavy chains. The F(ab')$_2$ disclosed herein can be preferably produced as follows. A whole monoclonal antibody or such comprising a desired antigen-binding site is partially digested with a protease such as pepsin; and Fc fragments are removed by adsorption onto a Protein A column. The protease is not particularly limited, as long as it can cleave the whole antibody in a selective manner to produce F(ab')$_2$ under an appropriate setup enzyme reaction condition such as pH. Such proteases include, for example, pepsin and ficin.

Fc region

The term "Fc region" or "Fc domain" refers to a region comprising a fragment consisting of a hinge or a portion thereof and CH2 and CH3 domains in an antibody molecule. The Fc region of IgG class means, but is not limited to, a region from, for example, cysteine 226 (EU numbering (also referred to as EU index herein)) to the C terminus or proline 230 (EU numbering) to the C terminus. The Fc region can be preferably obtained by the partial digestion of, for example, an IgG1, IgG2, IgG3, or IgG4 monoclonal antibody with a proteolytic enzyme such as pepsin followed by the reelution of a fraction adsorbed on a protein A column or a protein G column. Such a proteolytic enzyme is not particularly limited as long as the enzyme is capable of digesting a whole antibody to restrictively form Fab or F(ab')2 under appropriately set reaction conditions (e.g., pH) of the enzyme. Examples thereof can include pepsin and papain.

An Fc region derived from, for example, naturally occurring IgG can be used as the "Fc region" of the present disclosure. In this context, the naturally occurring IgG means a polypeptide that contains an amino acid sequence identical to that of IgG found in nature and belongs to a class of an antibody substantially encoded by an immunoglobulin gamma gene. The naturally occurring human IgG means, for example, naturally occurring human IgG1, naturally occurring human IgG2, naturally occurring human IgG3, or naturally occurring human IgG4. The naturally occurring IgG also includes variants or the like spontaneously derived therefrom. A plurality of allotype sequences based on gene polymorphism are described as the constant regions of human IgG1, human IgG2, human IgG3, and human IgG4 antibodies in Sequences of proteins of immunological interest, NIH Publication No. 91-3242, any of which can be used in the present disclosure. Particularly, the sequence of human IgG1 may have DEL or EEM as an amino acid sequence of EU numbering positions 356 to 358.

In some embodiments, the Fc domain of the multispecific antigen-binding molecule consists of a pair of polypeptide chains comprising heavy chain domains of an immunoglobulin molecule. For example, the Fc domain of an immunoglobulin G (IgG) molecule is a dimer, each subunit of which comprises the CH2 and CH3 IgG heavy chain constant domains. The two subunits of the Fc domain are capable of stable association with each other. In one embodiment the multispecific antigen-binding molecule described herein comprises not more than one Fc domain.

In one embodiment described herein, the Fc domain of the multispecificantigen binding molecule is an IgG Fc domain. In a particular embodiment, the Fc domain is an IgG1 Fc domain. In another embodiment, the Fc domain is an IgG1 Fc domain. In a further particular embodiment, the Fc domain is a human IgG1 Fc region.

In one aspect, the multispecific antigen-binding molecules contained in the anticancer agent, the pharmaceutical composition, the combination, or the kit of the present disclosure, or used in the method or use of the present disclosure further comprising

(iii) an Fc domain which exhibits reduced binding affinity to human Fc gamma receptor, as compared to a native human IgG1 Fc domain,

wherein the Fc domain is composed of a first Fc-region subunit and a second Fc-region subunit that are capable of stable association.

In one aspect, the multispecific antigen-binding molecules contained in the anticancer agent, the pharmaceutical composition, the combination, or the kit of the present disclosure, or used in the method or use of the present disclosure further comprising

(iii) an Fc domain which exhibits reduced binding affinity to human Fc gamma receptor, as compared to a native human IgG1 Fc domain,
wherein the Fc domain comprises (e1) or (e2) below:

(e1) the first Fc-region subunit comprising Cys at position 349, Ser at position 366, Ala at position 368 and Val at position 407, and the second Fc-region comprising Cys at position 354 and Trp at position 366;
(e2) the first Fc-region subunit comprising Glu at position 439, and the second Fc-region comprising Lys at position 356;

wherein the amino acid positions are numbered according to EU index.

In one aspect, the multispecific antigen-binding molecules contained in the anticancer agent, the pharmaceutical composition, the combination, or the kit of the present disclosure, or used in the method or use of the present disclosure further comprising

(iii) an Fc domain which exhibits reduced binding affinity to human Fc gamma receptor, as compared to a native human IgG1 Fc domain,
wherein the first and/or the second Fc-region subunit comprised in the Fc domain comprises (f1) or (f2) below:

(f1) Ala at position 234 and Ala at position 235;
(f2) Ala at position 234, Ala at position 235 and Ala at position 297;

wherein the amino acid positions are numbered according to EU index.

In one aspect, the multispecific antigen-binding molecules contained in the anticancer agent, the pharmaceutical composition, the combination, or the kit of the present disclosure, or used in the method or use of the present disclosure further comprising

(iii) an Fc domain which exhibits reduced binding affinity to human Fc gamma receptor, as compared to a native human IgG1 Fc domain,
wherein the Fc domain further exhibits stronger FcRn binding affinity to human FcRn, as compared to a native human IgG1 Fc domain.

In one aspect, the multispecific antigen-binding molecules contained in the anticancer agent, the pharmaceutical composition, the combination, or the kit of the present disclosure, or used in the method or use of the present disclosure further comprising

(iii) an Fc domain which exhibits reduced binding affinity to human Fc gamma receptor, as compared to a native human IgG1 Fc domain,
wherein the first and/or the second Fc region subunit comprised in the Fc domain comprises Leu at position 428, Ala at position 434, Arg at position 438, and Glu at position 440,
wherein the amino acid positions are numbered according to EU index.

In one aspect, a chemotherapeutic agent and the multispecific antigen-binding molecule described above are used in combination in the anticancer agent, the pharmaceutical composition, the combination, the kit, the method, or the use of the present disclosure. In an embodiment, a platinum preparation and the multispecific antigen-binding molecule described above are used in combination. In a certain embodiment, carboplatin or cisplatin and the multispecific antigen-binding molecule described above are used in combination. In certain embodiments, an alkaloid and the multispecific antigen-binding molecule described above are used in combination. In certain embodiments, a plant alkaloid and the multispecific antigen-binding molecule described above are used in combination. In certain embodiments, a topoisomerase inhibitor and the multispecific antigen-binding molecule described above are used in combination. In certain embodiments, irinotecan and the multispecific antigen-binding molecule described above are used in combination.

In certain embodiments, an antimetabolite and the multispecific antigen-binding molecule described above are used in combination. In certain embodiments, gemcitabine and the multispecific antigen-binding molecule described above are used in combination.

In one aspect, an immune checkpoint inhibitor and the multispecific antigen-binding molecule described above are used in combination in the anticancer agent, the pharmaceutical composition, the combination, the kit, the method, or the use of the present disclosure. In certain embodiments, an anti-PD-L1 antibody and the multispecific antigen-binding molecule described above are used in combination.

In one aspect, a PARP inhibitor and the multispecific antigen-binding molecule described above are used in combination in the anticancer agent, the pharmaceutical composition, the combination, the kit, the method, or the use of the present disclosure. In certain embodiments, olaparib and the multispecific antigen-binding molecule described above are used in combination.

Fc region with a reduced Fc receptor (Fc gamma receptor)-binding activity

In certain embodiments, the Fc domain of the multispecific antigen-binding molecules described herein exhibits reduced binding affinity to an Fc receptor, as compared to a native IgG1 Fc domain. In one such embodiment the Fc domain (or the multispecific antigen-binding molecule comprising said Fc domain) exhibits less than 50%, preferably less than 20%, more preferably less than 10% and most preferably less than 5% of the binding affinity to an Fc receptor, as compared to a native IgG1 Fc domain (or a multispecific antigen-binding molecule comprising a native IgG1 Fc domain). In one embodiment, the Fc domain (or the multispecific antigen-binding molecule comprising said Fc domain) does not substantially bind to an Fc receptor. In a particular embodiment, the Fc receptor is an Fc gamma receptor. In one embodiment the Fc receptor is a human Fc receptor. In one embodiment, the Fc receptor is an activating Fc receptor. In a specific embodiment the Fc receptor is an activating human Fc gamma receptor, more specifically human Fc gamma RIIIa, Fc gamma RI or Fc gamma RIIa, most specifically human Fc gamma RIIIa.

In certain embodiments, the Fc domain of the multispecific antigen-binding molecule comprises one or more amino acid mutation that reduces the binding affinity of the Fc domain to an Fc receptor. Typically, the same one or more amino acid mutation is present in each of the two subunits of the Fc domain. In one embodiment the amino acid mutation reduces the binding affinity of the Fc domain to an Fc receptor. In one embodiment, the amino acid mutation reduces the binding affinity of the Fc domain to an Fc receptor by at least 2-fold, at least 5-fold, or at least 10-fold. In embodiments where there is more than one amino acid mutation that reduces the binding affinity of the Fc domain to the Fc receptor, the combination of these amino acid mutations may reduce the binding affinity of the Fc domain to an Fc receptor by at least 10-fold, at least 20-fold, or even at least 50-fold. In one embodiment the multispecific antigen-binding molecule comprising an engineered Fc domain exhibits less than 20%, particularly less than 10%, more particularly less than 5% of the binding affinity to an Fc receptor as compared to a multispecific antigen-binding molecule comprising a non-engineered Fc domain. In a particular embodiment the Fc receptor is an Fc gamma receptor. In some embodiments, the Fc receptor is a human Fc receptor. In some embodiments the Fc receptor is an activating Fc receptor. In a specific embodiment, the Fc receptor is an activating human Fc gamma receptor, more specifically human Fc gamma RIIIa, Fc gamma RI or Fc gamma RIIa, most specifically human Fc gamma RIIIa. Preferably, binding to each of these receptors is reduced.

In one embodiment, the amino acid mutation that reduces the binding affinity of the Fc domain to an Fc receptor is an amino acid substitution. In one embodiment, the Fc domain comprises an amino acid substitution at a position selected from the group of E233, L234, L235, N297, P331 and P329. In a more specific embodiment, the Fc domain comprises an amino acid substitution at a position selected from the group of L234, L235 and P329. In some embodiments, the Fc domain comprises the amino acid substitutions L234A and L235A. In one such embodiment, the Fc domain is an IgG1 Fc domain, particularly a human IgG1 Fc domain. In one embodiment, the Fc domain comprises an amino acid substitution at position P329. In a more specific embodiment, the amino acid substitution is P329A or P329G, particularly P329G. In one embodiment the Fc domain comprises an amino acid substitution at position P329 and a further amino acid substitution at a position selected from E233, L234, L235, N297 and P331. In a more specific embodiment, the further amino acid substitution is E233P, L234A, L235A, L235E, N297A, N297D or P331S. In particular embodiments, the Fc domain comprises amino acid substitutions at positions P329, L234 and L235. In more particular embodiments the Fc domain comprises the amino acid mutations L234A, L235A and P329G ("P329G LALA"). In one such embodiment, the Fc domain is an IgG1 Fc domain, particularly a human IgG1 Fc domain. The "P329G LALA" combination of amino acid substitutions almost completely abolishes Fc gamma receptor (as well as complement) binding of a human IgG1 Fc domain, as described in PCT publication no. WO 2012/130831. WO 2012/130831 also describes methods of preparing such mutant Fc domains and methods for determining its properties such as Fc receptor binding or effector functions.

IgG4 antibodies exhibit reduced binding affinity to Fc receptors and reduced effector functions as compared to IgG1 antibodies. Hence, in some embodiments, the Fc domain of the T cell activating bispecific antigen binding molecules described herein is an IgG4 Fc domain, particularly a human IgG4 Fc domain. In one embodiment, the IgG4 Fc domain comprises amino acid substitutions at position S228, specifically the amino acid substitution S228P. To further reduce its binding affinity to an Fc receptor and/or its effector function, in one embodiment the IgG4 Fc domain comprises an amino acid substitution at position L235, specifically the amino acid substitution L235E. In another embodiment, the IgG4 Fc domain comprises an amino acid substitution at position P329, specifically the amino acid substitution P329G. In a particular embodiment, the IgG4 Fc domain comprises amino acid substitutions at positions S228, L235 and P329, specifically amino acid substitutions S228P, L235E and P329G. Such IgG4 Fc domain mutants and their Fc gamma receptor binding properties are described in PCT publication no. WO 2012/130831.

In certain embodiments, N-glycosylation of the Fc domain has been eliminated. In one such embodiment, the Fc domain comprises an amino acid mutation at position N297, particularly an amino acid substitution replacing asparagine by alanine (N297A) or aspartic acid (N297D).

In a particular preferred embodiment, the Fc domain exhibiting reduced binding affinity to an Fc receptor, as compared to a native IgG1 Fc domain, is a human IgG1 Fc domain comprising the amino acid substitutions L234A, L235A and N297A.

Mutant Fc domains can be prepared by amino acid deletion, substitution, insertion or modification using genetic or chemical methods well known in the art. Genetic methods may include site-specific mutagenesis of the encoding DNA sequence, PCR, gene synthesis, and the like. The correct nucleotide changes can be verified for example by sequencing.

Binding to Fc receptors can be easily determined e.g. by ELISA, or by Surface Plasmon Resonance (SPR) using standard instrumentation such as a BIAcore instrument (GE Healthcare), and Fc receptors such as may be obtained by recombinant expression. A suitable such binding assay is described herein. Alternatively, binding affinity of Fc domains or cell activating bispecific antigen binding molecules comprising an Fc domain for Fc receptors may be evaluated using cell lines known to express particular Fc receptors, such as human NK cells expressing Fc gamma IIIa receptor.

Fc receptor

The term "Fc receptor" or "FcR" refers to a receptor that binds to the Fc region of an antibody. In some embodiments, an FcR is a native human FcR. In some embodiments, an FcR is one which binds an IgG antibody (a gamma receptor) and includes receptors of the Fc gamma RI, Fc gamma RII, and Fc gamma RIII subclasses, including allelic variants and alternatively spliced forms of those receptors. Fc gamma RII receptors include Fc gamma RIIA (an "activating receptor") and Fc gamma RIIB (an "inhibiting receptor"), which have similar amino acid sequences that differ primarily in the cytoplasmic domains thereof. Activating receptor Fc gamma RIIA contains an immunoreceptor tyrosine-based activation motif (ITAM) in its cytoplasmic domain. Inhibiting receptor Fc gamma RIIB contains an immunoreceptor tyrosine-based inhibition motif (ITIM) in its cytoplasmic domain. (see, e.g., Daeron, Annu. Rev. Immunol. 15:203-234 (1997)). FcRs are reviewed, for example, in Ravetch and Kinet, Annu. Rev. Immunol 9:457-92 (1991); Capel et al., Immunomethods 4:25-34 (1994); and de Haas et al., J. Lab. Clin. Med. 126:330-41 (1995). Other FcRs, including those to be identified in the future, are encompassed by the term "FcR" herein.

The term "Fc receptor" or "FcR" also includes the neonatal receptor, FcRn, which is responsible for the transfer of maternal IgGs to the fetus (Guyer et al., J. Immunol. 117:587 (1976) and Kim et al., J. Immunol. 24:249 (1994)) and regulation of homeostasis of immunoglobulins. Methods of measuring binding to FcRn are known (see, e.g., Ghetie and Ward., Immunol. Today 18(12):592-598 (1997); Ghetie et al., Nature Biotechnology, 15(7):637-640 (1997); Hinton et al., J. Biol. Chem. 279(8):6213-6216 (2004); WO 2004/92219 (Hinton et al.).

Binding to human FcRn in vivo and plasma half life of human FcRn high affinity binding polypeptides can be assayed, e.g., in transgenic mice or transfected human cell lines expressing human FcRn, or in primates to which the polypeptides with a variant Fc region are administered. WO 2000/42072 (Presta) describes antibody variants with increased or decreased binding to FcRs. See also, e.g., Shields et al. J. Biol. Chem. 9(2):6591-6604 (2001).

Fc gamma receptor

Fc gamma receptor refers to a receptor capable of binding to the Fc domain of monoclonal IgG1, IgG2, IgG3, or IgG4 antibodies, and includes all members belonging to the family of proteins substantially encoded by an Fc gamma receptor gene. In human, the family includes Fc gamma RI (CD64) including isoforms Fc gamma RIa, Fc gamma RIb and Fc gamma RIc; Fc gamma RII (CD32) including isoforms Fc gamma RIIa (including allotype H131 and R131), Fc gamma RIIb (including Fc gamma RIIb-1 and Fc gamma RIIb-2), and Fc gamma RIIc; and Fc gamma RIII (CD16) including isoform Fc gamma RIIIa (including allotype V158 and F158) and Fc gamma RIIIb (including allotype Fc gamma RIIIb-NA1 and Fc gamma RIIIb-NA2); as well as all unidentified human Fc gamma receptors, Fc gamma receptor isoforms, and allotypes thereof. However, Fc gamma receptor is not limited to these examples. Without being limited thereto, Fc gamma receptor includes those derived from humans, mice, rats, rabbits, and monkeys. Fc gamma receptor may be derived from any organisms. Mouse Fc gamma receptor includes, without being limited to, Fc gamma RI (CD64), Fc gamma RII (CD32), Fc gamma RIII (CD16), and Fc gamma RIII-2 (CD16-2), as well as all unidentified mouse Fc gamma receptors, Fc gamma receptor isoforms, and allotypes thereof. Such preferred Fc gamma receptors include, for example, human Fc gamma RI (CD64), Fc gamma RIIA (CD32), Fc gamma RIIB (CD32), Fc gamma RIIIA (CD16), and/or Fc gamma RIIIB (CD16). The polynucleotide sequence and amino acid sequence of Fc gamma RI are shown in RefSeq accession number NM 000566.3 and RefSeq accession number NP_000557.1, respectively; the polynucleotide sequence and amino acid sequence of Fc gamma RIIA are shown in RefSeq accession number BC020823.1 and RefSeq accession number AAH20823.1, respectively; the polynucleotide sequence and amino acid sequence of Fc gamma RIIB are shown in RefSeq accession number BC146678.1 and RefSeq accession number AAI46679.1, respectively; the polynucleotide sequence and amino acid sequence of Fc gamma RIIIA are shown in RefSeq accession number BC033678.1 and RefSeq accession number AAH33678.1, respectively; and the polynucleotide sequence and amino acid sequence of Fc gamma RIIIB are shown in RefSeq accession number BC128562.1 and RefSeq accession number AAI28563.1, respectively. Whether an Fc gamma receptor has binding activity to the Fc domain of a monoclonal IgG1, IgG2, IgG3, or IgG4 antibody can be assessed by ALPHA screen (Amplified Luminescent Proximity Homogeneous Assay), surface plasmon resonance (SPR)-based BIACORE method, and others (Proc. Natl. Acad. Sci. USA (2006) 103(11), 4005-4010), in addition to the above-described FACS and ELISA formats.

Meanwhile, "Fc ligand" or "effector ligand" refers to a molecule and preferably a polypeptide that binds to an antibody Fc domain, forming an Fc/Fc ligand complex. The molecule may be derived from any organisms. The binding of an Fc ligand to Fc preferably induces one or more effector functions. Such Fc ligands include, but are not limited to, Fc receptors, Fc gamma receptor, Fc alpha receptor, Fc beta receptor, FcRn, C1q, and C3, mannan-binding lectin, mannose receptor, Staphylococcus Protein A, Staphylococcus Protein G, and viral Fc gamma receptors. The Fc ligands also include Fc receptor homologs (FcRH) (Davis et al., (2002) Immunological Reviews 190, 123-136), which are a family of Fc receptors homologous to Fc gamma receptor. The Fc ligands also include unidentified molecules that bind to Fc.

Fc gamma receptor-binding activity

The impaired binding activity of Fc domain to any of the Fc gamma receptors Fc gamma RI, Fc gamma RIIA, Fc gamma RIIB, Fc gamma RIIIA, and/or Fc gamma RIIIB can be assessed by using the above-described FACS and ELISA formats as well as ALPHA screen (Amplified Luminescent Proximity Homogeneous Assay) and surface plasmon resonance (SPR)-based BIACORE method (Proc. Natl. Acad. Sci. USA (2006) 103(11), 4005-4010).

ALPHA screen is performed by the ALPHA technology based on the principle described below using two types of beads: donor and acceptor beads. A luminescent signal is detected only when molecules linked to the donor beads interact biologically with molecules linked to the acceptor beads and when the two beads are located in close proximity. Excited by laser beam, the photosensitizer in a donor bead converts oxygen around the bead into excited singlet oxygen. When the singlet oxygen diffuses around the donor beads and reaches the acceptor beads located in close proximity, a chemiluminescent reaction within the acceptor beads is induced. This reaction ultimately results in light emission. If molecules linked to the donor beads do not interact with molecules linked to the acceptor beads, the singlet oxygen produced by donor beads do not reach the acceptor beads and chemiluminescent reaction does not occur.

For example, a biotin-labeled antigen-binding molecule or antibody is immobilized to the donor beads and glutathione S-transferase (GST)-tagged Fc gamma receptor is immobilized to the acceptor beads. In the absence of an antigen-binding molecule or antibody comprising a competitive mutant Fc domain, Fc gamma receptor interacts with an antigen-binding molecule or antibody comprising a wild-type Fc domain, inducing a signal of 520 to 620 nm as a result. The antigen-binding molecule or antibody having a non-tagged mutant Fc domain competes with the antigen-binding molecule or antibody comprising a wild-type Fc domain for the interaction with Fc gamma receptor. The relative binding affinity can be determined by quantifying the reduction of fluorescence as a result of competition. Methods for biotinylating the antigen-binding molecules or antibodies such as antibodies using Sulfo-NHS-biotin or the like are known. Appropriate methods for adding the GST tag to an Fc gamma receptor include methods that involve fusing polypeptides encoding Fc gamma receptor and GST in-frame, expressing the fused gene using cells introduced with a vector carrying the gene, and then purifying using a glutathione column. The induced signal can be preferably analyzed, for example, by fitting to a one-site competition model based on nonlinear regression analysis using software such as GRAPHPAD PRISM (GraphPad; San Diego).

One of the substances for observing their interaction is immobilized as a ligand onto the gold thin layer of a sensor chip. When light is shed on the rear surface of the sensor chip so that total reflection occurs at the interface between the gold thin layer and glass, the intensity of reflected light is partially reduced at a certain site (SPR signal). The other substance for observing their interaction is injected as an analyte onto the surface of the sensor chip. The mass of immobilized ligand molecule increases when the analyte binds to the ligand. This alters the refraction index of solvent on the surface of the sensor chip. The change in refraction index causes a positional shift of SPR signal (conversely, the dissociation shifts the signal back to the original position). In the Biacore system, the amount of shift described above (i.e., the change of mass on the sensor chip surface) is plotted on the vertical axis, and thus the change of mass over time is shown as measured data (sensorgram). Kinetic parameters (association rate constant (ka) and dissociation rate constant (kd)) are determined from the curve of sensorgram, and affinity (KD) is determined from the ratio between these two constants. Inhibition assay is preferably used in the BIACORE methods. Examples of such inhibition assay are described in Proc. Natl. Acad. Sci. USA (2006) 103(11), 4005-4010.

Production and purification of multispecific antigen-binding molecules

In some embodiments, multispecific antigen-binding molecules are isolated multispecific antigen-binding molecules.

Multispecific antigen-binding molecules described herein comprise two different antigen-binding moieties (e.g. the "first antigen-binding moiety" and the "second antigen-binding moiety"), fused to one or the other of the two subunits of the Fc domain, thus the two subunits of the Fc domain are typically comprised in two non-identical polypeptide chains. Recombinant co-expression of these polypeptides and subsequent dimerization leads to several possible combinations of the two polypeptides. To improve the yield and purity of multispecific antigen-binding molecules in recombinant production, it will thus be advantageous to introduce in the Fc domain of the multispecific antigen-binding molecule a modification promoting the association of the desired polypeptides.

Accordingly, in particular embodiments, the Fc domain of the multispecific antigen-binding molecule described herein comprises a modification promoting the association of the first and the second subunit of the Fc domain. The site of most extensive protein-protein interaction between the two subunits of a human IgG Fc domain is in the CH3 domain of the Fc domain. Thus, in one embodiment, said modification is in the CH3 domain of the Fc domain.

In a specific embodiment, said modification is a so-called "knob-into-hole" modification, comprising a "knob" modification in one of the two subunits of the Fc domain and a "hole" modification in the other one of the two subunits of the Fc domain. The knob-into-hole technology is described e.g. in US 5,731,168 ; US 7,695,936 ; Ridgway et al., Prot Eng 9, 617-621 (1996) and Carter, J Immunol Meth 248, 7-15 (2001). Generally, the method involves introducing a protuberance ("knob") at the interface of a first polypeptide and a corresponding cavity ("hole") in the interface of a second polypeptide, such that the protuberance can be positioned in the cavity so as to promote heterodimer formation and hinder homodimer formation. Protuberances are constructed by replacing small amino acid side chains from the interface of the first polypeptide with larger side chains (e.g. tyrosine or tryptophan). Compensatory cavities of identical or similar size to the protuberances are created in the interface of the second polypeptide by replacing large amino acid side chains with smaller ones (e.g. alanine or threonine).

Accordingly, in a particular embodiment, in the CH3 domain of the first subunit of the Fc domain of the multispecific antigen-binding molecule an amino acid residue is replaced with an amino acid residue having a larger side chain volume, thereby generating a protuberance within the CH3 domain of the first subunit which is positionable in a cavity within the CH3 domain of the second subunit, and in the CH3 domain of the second subunit of the Fc domain an amino acid residue is replaced with an amino acid residue having a smaller side chain volume, thereby generating a cavity within the CH3 domain of the second subunit within which the protuberance within the CH3 domain of the first subunit is positionable.

The protuberance and cavity can be made by altering the nucleic acid encoding the polypeptides, e.g. by site-specific mutagenesis, or by peptide synthesis.

In a specific embodiment, in the CH3 domain of the first subunit of the Fc domain the threonine residue at position 366 is replaced with a tryptophan residue (T366W), and in the CH3 domain of the second subunit of the Fc domain the tyrosine residue at position 407 is replaced with a valine residue (Y407V). In one embodiment, in the second subunit of the Fc domain additionally the threonine residue at position 366 is replaced with a serine residue (T366S) and the leucine residue at position 368 is replaced with an alanine residue (L368A).

In yet a further embodiment, in the first subunit of the Fc domain additionally the serine residue at position 354 is replaced with a cysteine residue (S354C), and in the second subunit of the Fc domain additionally the tyrosine residue at position 349 is replaced by a cysteine residue (Y349C). Introduction of these two cysteine residues results in formation of a disulfide bridge between the two subunits of the Fc domain, further stabilizing the dimer (Carter, J Immunol Methods 248, 7-15 (2001)).

In other embodiments, other techniques for promoting the association among H chains and between L and H chains having the desired combinations can be applied to the multispecific antigen-binding molecules of the present disclosure.

For example, techniques for suppressing undesired H-chain association by introducing electrostatic repulsion at the interface of the second constant region or the third constant region of the antibody H chain (CH2 or CH3) can be applied to multispecific antibody association (WO2006/106905).

In the technique of suppressing unintended H-chain association by introducing electrostatic repulsion at the interface of CH2 or CH3, examples of amino acid residues in contact at the interface of the other constant region of the H chain include regions corresponding to the residues at EU numbering positions 356, 439, 357, 370, 399, and 409 in the CH3 region.

More specifically, examples include an antibody comprising two types of H-chain CH3 regions, in which one to three pairs of amino acid residues in the first H-chain CH3 region, selected from the pairs of amino acid residues indicated in (1) to (3) below, carry the same type of charge: (1) amino acid residues comprised in the H chain CH3 region at EU numbering positions 356 and 439; (2) amino acid residues comprised in the H-chain CH3 region at EU numbering positions 357 and 370; and (3) amino acid residues comprised in the H-chain CH3 region at EU numbering positions 399 and 409.

Furthermore, the antibody may be an antibody in which pairs of the amino acid residues in the second H-chain CH3 region which is different from the first H-chain CH3 region mentioned above, are selected from the aforementioned pairs of amino acid residues of (1) to (3), wherein the one to three pairs of amino acid residues that correspond to the aforementioned pairs of amino acid residues of (1) to (3) carrying the same type of charges in the first H-chain CH3 region mentioned above carry opposite charges from the corresponding amino acid residues in the first H-chain CH3 region mentioned above.

Each of the amino acid residues indicated in (1) to (3) above come close to each other during association. Those skilled in the art can find out positions that correspond to the above-mentioned amino acid residues of (1) to (3) in a desired H-chain CH3 region or H-chain constant region by homology modeling and such using commercially available software, and amino acid residues of these positions can be appropriately subjected to modification.

In the antibodies mentioned above, "charged amino acid residues" are preferably selected, for example, from amino acid residues included in either one of the following groups:

(a) glutamic acid (E) and aspartic acid (D); and
(b) lysine (K), arginine (R), and histidine (H).

In the above-mentioned antibodies, the phrase "carrying the same charge" means, for example, that all of the two or more amino acid residues are selected from the amino acid residues included in either one of groups (a) and (b) mentioned above. The phrase "carrying opposite charges" means, for example, that when at least one of the amino acid residues among two or more amino acid residues is selected from the amino acid residues included in either one of groups (a) and (b) mentioned above, the remaining amino acid residues are selected from the amino acid residues included in the other group.

In a preferred embodiment, the antibodies mentioned above may have their first H-chain CH3 region and second H-chain CH3 region crosslinked by disulfide bonds.

In the present disclosure, amino acid residues subjected to modification are not limited to the above-mentioned amino acid residues of the antibody variable regions or the antibody constant regions. Those skilled in the art can identify the amino acid residues that form an interface in mutant polypeptides or heteromultimers by homology modeling and such using commercially available software; and amino acid residues of these positions can then be subjected to modification so as to regulate the association.

In addition, other known techniques can also be used for formation of the multispecific antigen-binding molecules contained in the anticancer agent, the pharmaceutical composition, the combination, or the kit of the present disclosure, or used in the method or use of the present disclosure. Association of polypeptides having different sequences can be induced efficiently by complementary association of CH3 using a strand-exchange engineered domain CH3 produced by changing part of one of the H-chain CH3s of an antibody to a corresponding IgA-derived sequence and introducing a corresponding IgA-derived sequence into the complementary portion of the other H-chain CH3 (Protein Engineering Design & Selection, 23; 195-202, 2010). This known technique can also be used to efficiently form multispecific antigen-binding molecules of interest.

In addition, technologies for antibody production using association of antibody CH1 and CL and association of VH and VL as described in WO 2011/028952, WO2014/018572, and Nat Biotechnol. 2014 Feb; 32(2):191-8; technologies for producing bispecific antibodies using separately prepared monoclonal antibodies in combination (Fab Arm Exchange) as described in WO2008/119353 and WO2011/131746; technologies for regulating association between antibody heavy-chain CH3s as described in WO2012/058768 and WO2013/063702; technologies for producing multispecific antibodies composed of two types of light chains and one type of heavy chain as described in WO2012/023053; technologies for producing multispecific antibodies using two bacterial cell strains that individually express one of the chains of an antibody comprising a single H chain and a single L chain as described by Christoph et al. (Nature Biotechnology Vol. 31, p 753-758 (2013)); and such may be used for the formation of multispecific antigen-binding molecules.

Alternatively, even when a multispecific antigen-binding molecule of interest cannot be formed efficiently, the multispecific antigen-binding molecule of the present disclosure can be obtained by separating and purifying the multispecific antigen-binding molecule of interest from the produced molecules. For example, a method for enabling purification of two types of homomeric forms and the heteromeric antibody of interest by ion-exchange chromatography by imparting a difference in isoelectric points by introducing amino acid substitutions into the variable regions of the two types of H chains has been reported (WO2007114325). To date, as a method for purifying heteromeric antibodies, methods using Protein A to purify a heterodimeric antibody comprising a mouse IgG2a H chain that binds to Protein A and a rat IgG2b H chain that does not bind to Protein A have been reported (WO98050431 and WO95033844). Furthermore, a heterodimeric antibody can be purified efficiently on its own by using H chains comprising substitution of amino acid residues at EU numbering positions 435 and 436, which is the IgG-Protein A binding site, with Tyr, His, or such which are amino acids that yield a different Protein A affinity, or using H chains with a different protein A affinity, to change the interaction of each of the H chains with Protein A, and then using a Protein A column.

Furthermore, an Fc region whose Fc region C-terminal heterogeneity has been improved can be appropriately used as an Fc region of the present disclosure. More specifically, the present disclosure provides Fc regions produced by deleting glycine at position 446 and lysine at position 447 as specified by EU numbering from the amino acid sequences of two polypeptides constituting an Fc region derived from IgG1, IgG2, IgG3, or IgG4.

Multispecific antigen-binding molecules prepared as described herein may be purified by art-known techniques such as high performance liquid chromatography, ion exchange chromatography, gel electrophoresis, affinity chromatography, size exclusion chromatography, and the like. The actual conditions used to purify a particular protein will depend, in part, on factors such as net charge, hydrophobicity, hydrophilicity etc., and will be apparent to those having skill in the art. For affinity chromatography purification an antibody, ligand, receptor or antigen can be used to which the multispecific antigen-binding molecule binds. For example, for affinity chromatography purification of multispecific antigen-binding molecules of the invention, a matrix with protein A or protein G may be used. Sequential Protein A or G affinity chromatography and size exclusion chromatography can be used to isolate a multispecific antigen-binding molecule. The purity of the multispecific antigen-binding molecule can be determined by any of a variety of well-known analytical methods including gel electrophoresis, high pressure liquid chromatography, and the like.

Antibody-dependent cell-mediated cytotoxicity

"Antibody-dependent cell-mediated cytotoxicity" or "ADCC" refers to a form of cytotoxicity in which secreted Ig bound onto Fc receptors (FcRs) present on certain cytotoxic cells (e.g. NK cells, neutrophils, and macrophages) enable these cytotoxic effector cells to bind specifically to an antigen-bearing target cell and subsequently kill the target cell with cytotoxins. The primary cells for mediating ADCC, NK cells, express Fc gamma RIII only, whereas monocytes express Fc gamma RI, Fc gamma RII, and Fc gamma RIII. FcR expression on hematopoietic cells is summarized in Table 3 on page 464 of Ravetch and Kinet, Annu. Rev. Immunol 9:457-92 (1991). To assess ADCC activity of a molecule of interest, an in vitro ADCC assay, such as that described in US Patent No. 5,500,362 or 5,821,337 or U.S. Patent No. 6,737,056 (Presta), may be performed. Useful effector cells for such assays include PBMC and NK cells. Alternatively, or additionally, ADCC activity of the molecule of interest may be assessed in vivo, e.g., in an animal model such as that disclosed in Clynes et al. PNAS (USA) 95:652-656 (1998).

Complement dependent cytotoxicity

"Complement dependent cytotoxicity" or "CDC" refers to the lysis of a target cell in the presence of complement. Activation of the classical complement pathway is initiated by the binding of the first component of the complement system (C1q) to antibodies (of the appropriate subclass), which are bound to their cognate antigen. To assess complement activation, a CDC assay, e.g., as described in Gazzano-Santoro et al., J. Immunol. Methods 202:163 (1996), may be performed. Polypeptide variants with altered Fc region amino acid sequences (polypeptides with a variant Fc region) and increased or decreased C1q binding capability are described, e.g., in US Patent No. 6,194,551 B1 and WO 1999/51642. See also, e.g., Idusogie et al. J. Immunol. 164: 4178-4184 (2000).

T cell dependent cellular cytotoxicity

"T cell dependent cellular cytotoxicity" or "TDCC" refers to a form of cytotoxicity in which an antigen-binding molecule binds to both an antigen expressed on the target cell, and another antigen expressed on T cell, that redirect T cell near to the target cell, as cytotoxicity against the target cell is induced due to the T cell. The method to assess T cell dependent cellular cytotoxicity, an in vitro TDCC assay, is also described in the "Measurement of T cell dependent cellular cytotoxicity" section of this description.

Measurement of T cell dependent cellular cytotoxicity

In the embodiment that the antigen-binding molecule binds to both CLDN6 and CD3/CD137, the methods described below are preferably used as a method for assessing or determining T cell dependent cellular cytotoxicity (TDCC) caused by contacting an antigen-binding molecule of the present disclosure with CLDN6-expressing cells to which the antigen-binding site in the antigen-binding molecules of the present disclosure binds. The methods for assessing or determining the cytotoxic activity in vitro include methods for determining the activity of cytotoxic T-cells or the like. Whether an antigen-binding molecule of the present disclosure has the activity of inducing T-cell mediated cellular cytotoxicity can be determined by known methods (see, for example, Current protocols in Immunology, Chapter 7. Immunologic studies in humans, Editor, John E, Coligan et al., John Wiley & Sons, Inc., (1993)). In the cytotoxicity assay, an antigen-binding molecule which is able to bind to an antigen different from CLDN6 and which is not expressed in the cells, and CD3/CD137, is used as a control antigen-binding molecule. The control antigen-binding molecule is assayed in the same manner. Then, the activity is assessed by testing whether an antigen-binding molecule of the present disclosure exhibits a stronger cytotoxic activity than that of a control antigen-binding molecule.

Meanwhile, the in vivo anti-tumor efficacy is assessed or determined, for example, by the following procedure. Cells expressing the antigen to which the antigen-binding site in an antigen-binding molecule of the present disclosure binds are transplanted intracutaneously or subcutaneously to a nonhuman animal subject. Then, from the day of transplantation or thereafter, a test antigen-binding molecule is administered into vein or peritoneal cavity every day or at intervals of several days. The tumor size is measured over time. Difference in the change of tumor size can be defined as the cytotoxic activity. As in an in vitro assay, a control antigen-binding molecule is administered. The antigen-binding molecule of the present disclosure can be judged to have cytotoxic activity when the tumor size is smaller in the group administered with the antigen-binding molecule of the present disclosure than in the group administered with the control antigen-binding molecule.

An MTT method and measurement of isotope-labeled thymidine uptake into cells are preferably used to assess or determine the effect of contact with an antigen-binding molecule of the present disclosure to suppress the growth of cells expressing an antigen to which the antigen-binding site in the antigen-binding molecule binds.

Meanwhile, the same methods described above for assessing or determining the in vivo cytotoxic activity can be used preferably to assess or determine the activity of suppressing cell growth in vivo.

The TDCC of an antibody or antigen-binding molecule of the disclosure can be evaluated by any suitable method known in the art. For example, TDCC can be measured by lactate dehydrogenase (LDH) release assay. In this assay, target cells (e.g. CLDN6-expressing cells) are incubated with T cells (e.g. PBMCs) in the presence of a test antibody or antigen-binding molecule, and the activity of LDH that has been released from target cells killed by T cells is measured using a suitable reagent. Typically, the cytotoxic activity is calculated as a percentage of the LDH activity resulting from the incubation with the antibody or antigen-binding molecule relative to the LDH activity resulting from 100% killing of target cells (e.g. lysed by treatment with Triton-X). If the cytotoxic activity calculated as mentioned above is higher, the test antibody or antigen-binding molecule is determined to have higher TDCC.

Additionally or alternatively, for example, TDCC can also be measured by real-time cell growth inhibition assay. In this assay, target cells (e.g. CLDN6-expressing cells) are incubated with T cells (e.g. PBMCs) in the presence of a test antibody or antigen-binding molecule on a 96-well plate, and the growth of the target cells is monitored by methods known in the art, for example, by using a suitable analyzing instrument (e.g. xCELLigence Real-Time Cell Analyzer). The rate of cell growth inhibition (CGI: %) is determined from the cell index value according to the formulation given as CGI (%) = 100-(CIAb x 100 / CINoAb). "CIAb" represents the cell index value of wells with the antibody or antigen-binding molecule on a specific experimental time and "CINoAb" represents the average cell index value of wells without the antibody or antigen-binding molecule. If the CGI rate of the antibody or antigen-binding molecule is high, i.e., has a significantly positive value, it can be said that the antibody or antigen-binding molecule has TDCC activity..

In one aspect, an antibody or antigen-binding molecule of the disclosure has T cell activation activity. T cell activation can be assayed by methods known in the art, such as a method using an engineered T cell line that expresses a reporter gene (e.g. luciferase) in response to its activation (e.g. Jurkat / NFAT-RE Reporter Cell Line (T Cell Activation Bioassay, Promega)). In this method, target cells (e.g.CLDN6-expressing cells) are cultured with T cells in the presence of a test antibody or antigen-binding molecule, and then the level or activity of the expression product of the reporter gene is measured by appropriate methods as an index of T cell activation. When the reporter gene is a luciferase gene, luminescence arising from reaction between luciferase and its substrate may be measured as an index of T cell activation. If T cell activation measured as described above is higher, the test antibody or antigen-binding molecule is determined to have higher T cell activation activity.

In one aspect, the multispecific antigen-binding molecule or antibody of the present disclosure shows cytotoxic activity equivalent to or higher than (i.e., not less than) that of a multispecific antibody used as a control. Measurement and comparison of cytotoxic activity, and determination of whether the molecule or antibody shows cytotoxic activity equivalent to or higher than (i.e., not less than) that of a control can be carried out according to the description in the "Measurement of T cell-dependent cytotoxicity" section described above. In certain embodiments, the multispecific antibody used as a control is a multispecific antibody having the same structure as the multispecific antigen-binding molecule or the antibody of the present disclosure, except that the first antigen-binding moiety can only bind to CD3. In certain embodiments, the multispecific antibody used as a control is a multispecific antibody (CS3348) comprising an antigen-binding moiety capable of binding to a T cell receptor complex, which is a moiety containing a heavy chain comprising the amino acid sequence of SEQ ID NO: 194 and a light chain comprising the amino acid sequence of SEQ ID NO: 192, and an antigen-binding moiety capable of binding to CLDN6, which is a moiety containing a heavy chain comprising the amino acid sequence of SEQ ID NO: 193 and a light chain comprising the amino acid sequence of SEQ ID NO: 195.

Pharmaceutical composition

In one aspect, the present disclosure provides a pharmaceutical composition comprising the multispecific antigen-binding molecule or antibody of this disclosure. In certain embodiments, the pharmaceutical composition of the present disclosure induces T-cell-dependent cytotoxicity, in other words, the pharmaceutical composition of this disclosure is a therapeutic agent for inducing cytotoxicity. In certain embodiments, the pharmaceutical composition of the present disclosure is used for treating and/or preventing cancer. In certain embodiments, the pharmaceutical composition of the present disclosure is used for treating and/or preventing CLDN6-positive cancer or CLDN6-expressing cancer including ovary cancer, non-small cell lung cancer, gastric cancer, liver cancer, endometrial cancer, germ cell tumor, testis cancer, breast cancer, cervical cancer, esophageal cancer, pancreatic cancer, cholangiocarcinoma, kidney cancer, head and neck cancer, large bowel cancer, urinary bladder cancer, and atypical teratoid rhabdoid tumor (AT/RT); and other CLDN6-positive cancer or CLDN6-expressing cancer. In certain embodiments, the pharmaceutical composition of the present disclosure is a cell growth suppressor (cell growth inhibitor). In certain embodiments, the pharmaceutical composition of the present disclosure is an anticancer agent. In certain embodiments, the pharmaceutical composition of the present disclosure is a cytotoxicity-inducing agent, an immune response activator against cancer cells or cancer cell-comprising tumor tissues, a cancer therapeutic agent, or a cancer preventive agent.

If necessary, the pharmaceutical composition of the present disclosure, the therapeutic agent for inducing cytotoxicity, cell growth suppressor, cytotoxicity-inducing agent, immune response activator against cancer cells or cancer cell-comprising tumor tissues, cancer therapeutic agent, cancer preventive agent, or anticancer agent of this disclosure can be formulated with various types of antigen-binding molecules or antibodies. For example, the cytotoxic action against cells expressing an antigen can be enhanced by a cocktail of a plurality of the multispecific antigen-binding molecules or antibodies of the present disclosure.

Pharmaceutical compositions comprising a multispecific antigen-binding molecule or antibody as described herein are prepared by mixing such antigen-binding molecule or antibody having the desired degree of purity with one or more optional pharmaceutically acceptable carriers (Remington's Pharmaceutical Sciences 16th edition, Osol, A. Ed. (1980)), in the form of lyophilized formulations or aqueous solutions. Pharmaceutically acceptable carriers are generally nontoxic to recipients at the dosages and concentrations employed, and include, but are not limited to: buffers such as phosphate, citrate, and other organic acids; antioxidants including ascorbic acid and methionine; preservatives (such as octadecyldimethylbenzyl ammonium chloride; hexamethonium chloride; benzalkonium chloride; benzethonium chloride; phenol, butyl or benzyl alcohol; alkyl parabens such as methyl or propyl paraben; catechol; resorcinol; cyclohexanol; 3-pentanol; and m-cresol); low molecular weight (less than about 10 residues) polypeptides; proteins, such as serum albumin, gelatin, or immunoglobulins; hydrophilic polymers such as polyvinylpyrrolidone; amino acids such as glycine, glutamine, asparagine, histidine, arginine, or lysine; monosaccharides, disaccharides, and other carbohydrates including glucose, mannose, or dextrins; chelating agents such as EDTA; sugars such as sucrose, mannitol, trehalose or sorbitol; salt-forming counter-ions such as sodium; metal complexes (e.g. Zn-protein complexes); and/or non-ionic surfactants such as polyethylene glycol (PEG). Exemplary pharmaceutically acceptable carriers herein further include interstitial drug dispersion agents such as soluble neutral-active hyaluronidase glycoproteins (sHASEGP), for example, human soluble PH-20 hyaluronidase glycoproteins, such as rHuPH20 (HYLENEX (registered trademark), Baxter International, Inc.). Certain exemplary sHASEGPs and methods of use, including rHuPH20, are described in US Patent Publication Nos. 2005/0260186 and 2006/0104968. In one aspect, a sHASEGP is combined with one or more additional glycosaminoglycanases such as chondroitinases.

Exemplary lyophilized antibody formulations are described in US Patent No. 6,267,958. Aqueous antibody formulations include those described in US Patent No. 6,171,586 and WO2006/044908, the latter formulations including a histidine-acetate buffer.

The formulation herein may also contain more than one active ingredient as necessary for the particular indication being treated, preferably those with complementary activities that do not adversely affect each other. Such active ingredients are suitably present in combination in amounts that are effective for the purpose intended.

If necessary, the antigen-binding molecules or antibodies of the present disclosure may be encapsulated in microcapsules (microcapsules made from hydroxymethylcellulose, gelatin, poly[methylmethacrylate], and the like), and made into components of colloidal drug delivery systems (liposomes, albumin microspheres, microemulsions, nanoparticles, and nano-capsules) (for example, see "Remington's Pharmaceutical Science 16th edition", Oslo Ed. (1980)). Moreover, methods for preparing agents as sustained-release agents are known, and these can be applied to the antigen-binding molecules of the present disclosure (J. Biomed. Mater. Res. (1981) 15, 267-277; Chemtech. (1982) 12, 98-105; US Patent No. 3773719; European Patent Application (EP) Nos. EP58481 and EP133988; Biopolymers (1983) 22, 547-556).

If necessary, the vectors comprising nucleic acid molecule encodes the multispecific antigen-binding molecules or antibodies of the present disclosure may be introduced to subjects, to express the antigen-binding molecules or antibodies of the present disclosure directly within the subject. An example of vectors that is possible to be used is adenovirus, but not limited to. It is also possible to administer the nucleic acid molecule encodes the antigen-binding molecules or antibodies of the present disclosure directly into a subject, or transfer the nucleic acid molecule encodes the antigen-binding molecules or antibodies of the present disclosure via electroporation to a subject, or administer cells comprises nucleic acid molecule encodes the antigen-binding molecules or antibodies of the present disclosure to be expressed and secreted into a subject, to express and secrete the antigen-binding molecules or antibodies of the present disclosure in the subject continuously.

The pharmaceutical compositions, cell growth-suppressing agents, or anticancer agents of the present disclosure may be administered either orally or parenterally to patients. These are preferably administered by parental administration, and specifically, such administration methods include injection, nasal administration, transpulmonary administration, and percutaneous administration. Injections include, for example, intravenous injections, intramuscular injections, intraperitoneal injections, and subcutaneous injections. For example, pharmaceutical compositions, therapeutic agents for inducing cellular cytotoxicity, cell growth-suppressing agents, or anticancer agents of the present disclosure can be administered locally or systemically by injection. Furthermore, appropriate administration methods can be selected according to the patient's age and symptoms. The administered dose can be selected, for example, from the range of 0.0001 mg to 1,000 mg per kg of body weight for each administration. Alternatively, the dose can be selected, for example, from the range of 0.001 mg/body to 100,000 mg/body per patient. However, the dose of a pharmaceutical composition of the present disclosure is not limited to these doses.

Preferably, a pharmaceutical composition of the present disclosure comprises a multispecific antigen-binding molecule or antibody as described herein. In one aspect, the composition is a pharmaceutical composition for use in inducing cellular cytotoxicity. In another aspect, the composition is a pharmaceutical composition for use in treating or preventing cancer. Preferably, the cancer is CLDN6-expressing cancer. The pharmaceutical composition of the present disclosure can be used for treating or preventing cancer. Thus, the present disclosure provides a method for treating or preventing cancer, in which the multispecific antigen-binding molecule or antibody as described herein is administered to a patient in need thereof

The present disclosure also provides methods for damaging cells expressing CLDN6 or CLDN6-positive cancer, or for suppressing the cell growth by contacting the cells expressing CLDN6 with an antigen-binding molecule of the present disclosure that binds to CLDN6. Cells to which an antigen-binding molecule of the present disclosure binds are not particularly limited, as long as they express CLDN6. Specifically, in the present disclosure, the preferred CLDN6-expressing cancer or CLDN6-positive cancer including ovarian cancer, non-small cell lung cancer, gastric cancer, liver cancer, endometrial cancer, germ cell tumor, testis cancer, breast cancer, cervical cancer, esophageal cancer, pancreatic cancer, cholangiocarcinoma, kidney cancer, head and neck cancer, large bowel cancer, urinary bladder cancer, or atypical teratoid rhabdoid tumor (AT/RT).

In the present disclosure, "contact" can be carried out, for example, by adding an antigen-binding molecule of the present disclosure to culture media of cells expressing CLDN6 cultured in vitro. In this case, an antigen-binding molecule to be added can be used in an appropriate form, such as a solution or solid prepared by lyophilization or the like. When the antigen-binding molecule of the present disclosure is added as an aqueous solution, the solution may be a pure aqueous solution containing the antigen-binding molecule alone or a solution containing, for example, an above-described surfactant, excipient, coloring agent, flavoring agent, preservative, stabilizer, buffering agent, suspending agent, isotonizing agent, binder, disintegrator, lubricant, fluidity accelerator, and corrigent. The added concentration is not particularly limited; however, the final concentration in a culture medium is preferably in a range of 1 pg/ml to 1 g/ml, more preferably 1 ng/ml to 1 mg/ml, and still more preferably 1 micro g/ml to 1 mg/ml.

In another embodiment of the present disclosure, "contact" can also be carried out by administration to non-human animals transplanted with CLDN6-expressing cells in vivo or to animals having cancer cells expressing CLDN6 endogenously. The administration method may be oral or parenteral, and parenteral administration is particularly preferred. Specifically, the parenteral administration method includes injection, nasal administration, pulmonary administration, and percutaneous administration. Injections include, for example, intravenous injections, intramuscular injections, intraperitoneal injections, and subcutaneous injections. For example, pharmaceutical compositions, therapeutic agents for inducing cellular cytotoxicity, cell growth-suppressing agents, or anticancer agents of the present disclosure can be administered locally or systemically by injection. Furthermore, an appropriate administration method can be selected according to the age and symptoms of an animal subject. When the antigen-binding molecule is administered as an aqueous solution, the solution may be a pure aqueous solution containing the antigen-binding molecule alone or a solution containing, for example, an above-described surfactant, excipient, coloring agent, flavoring agent, preservative, stabilizer, buffering agent, suspending agent, isotonizing agent, binder, disintegrator, lubricant, fluidity accelerator, and corrigent. The administered dose can be selected, for example, from the range of 0.0001 to 1,000 mg per kg of body weight for each administration. Alternatively, the dose can be selected, for example, from the range of 0.001 to 100,000 mg/body for each patient. However, the dose of an antigen-binding molecule of the present disclosure is not limited to these examples.

Pharmaceutical formulation and pharmaceutical composition

The term "pharmaceutical formulation" or "pharmaceutical composition" refers to a preparation which is in such form as to permit the biological activity of an active ingredient contained therein to be effective, and which contains no additional components which are unacceptably toxic to a subject to which the formulation would be administered.

Pharmaceutically acceptable carrier

A "pharmaceutically acceptable carrier" refers to an ingredient in a pharmaceutical formulation, other than an active ingredient, which is nontoxic to a subject. A pharmaceutically acceptable carrier includes, but is not limited to, a buffer, excipient, stabilizer, or preservative.

Treatment

As used herein, "treatment" (and grammatical variations thereof such as "treat" or "treating") refers to clinical intervention in an attempt to alter the natural course of the individual being treated, and can be performed either for prophylaxis or during the course of clinical pathology. Desirable effects of treatment include, but are not limited to, preventing occurrence or recurrence of disease, alleviation of symptoms, diminishment of any direct or indirect pathological consequences of the disease, preventing metastasis, decreasing the rate of disease progression, amelioration or palliation of the disease state, and remission or improved prognosis. In some embodiments, antigen-binding molecules or antibodies of the present disclosure are used to delay development of a disease or to slow the progression of a disease.

In some embodiments, the present invention relates to methods for preventing resistance to cancer chemotherapy, or preventing recurrence or metastasis of cancer, particularly during or after cancer therapy including treating the cancer by the methods of the present invention. In some embodiments, "treating" in the present invention means that single-agent administration or combination therapy using the anticancer agent or the pharmaceutical composition of the present invention decreases the number of cancer cells in individuals, suppresses cancer cell proliferation, decreases tumor size, suppresses infiltration of cancer cells into peripheral organs, suppresses cancer cell metastasis, or ameliorates various symptoms caused by cancer. Furthermore, in some embodiments, "preventing" in the present invention means inhibiting increase in the number of cancer cells due to repopulation of cancer cells that have been decreased, inhibiting repopulation of cancer cells whose proliferation has been suppressed, and inhibiting re-increase of tumor size which has been decreased.

Cancer

The terms "cancer" and "cancerous" refer to or describe the physiological condition in mammals that is typically characterized by uncontrolled cell growth/proliferation.

As used herein, "cancer" refers not only to epithelial malignancies such as ovary cancer or gastric cancer, but also to non-epithelial malignancies including hematopoietic cancers such as chronic lymphocytic leukemia or Hodgkin's lymphoma. Herein, the terms "cancer," "carcinoma," "tumor," "neoplasm," and such are not differentiated from each other and are mutually interchangeable. Furthermore, in an embodiment herein, the terms include primary cancer, advanced cancer, metastatic cancer, recurrent cancer, or a combination thereof.

In certain embodiments, the cancer is a CLDN6-expressing or CLDN6-positive cancer which include ovary cancer, non-small cell lung cancer, gastric cancer, liver cancer, endometrial cancer, germ cell tumor, testis cancer, breast cancer, cervical cancer, esophageal cancer, pancreatic cancer, cholangiocarcinoma, kidney cancer, head and neck cancer, large bowel cancer, urinary bladder cancer, and atypical teratoid rhabdoid tumor (AT/RT); and other CLDN6-positive cancer or CLDN6-expressing cancer.

Tumor

The term "tumor" refers to all neoplastic cell growth and proliferation, whether malignant or benign, and all precancerous and cancerous cells and tissues. The terms "cancer," "cancerous," "cell proliferative disorder," "proliferative disorder" and "tumor" used herein are not mutually exclusive.

Metastasis

"Metastasis" refers to migration of cancer cells from where they first occurred to other organs, by entering into the blood vessels or lymphatic vessels, and flowing with the blood and lymph stream, and then proliferating at the organs where they migrated. "Disseminate" refers to detachment of cancer cells from the organ where the cancer developed, and then spreading of those cells in a proximal space within the body, such as the thoracic cavity or the peritoneal cavity. In certain embodiments, "metastasized to the peritoneum" refers to peritoneal dissemination and spread of cancer cells at the peritoneum covering the peritoneal cavity. For example, "ovary cancer metastasized to the peritoneum" means that ovary cancer cells advance from the ovary to the peritoneum by extra-pelvic peritoneal dissemination metastasis, and spread as if they were strewn about the peritoneum. Cancer cells of cancer that has metastasized to the peritoneum are present, for example, in the peritoneal fluid of the patient.

In certain embodiments, the cancer is cancer metastasized to the peritoneum. Furthermore, in certain embodiments, the cancer is peritoneally disseminated cancer.

Combination therapies

The multispecific antigen-binding molecule described above may be administered in combination with one or a plurality of other agents in a therapy. For example, the multispecific antigen-binding molecule described herein may be administered simultaneously with at least an additional therapeutic agent. Furthermore, the multispecific antigen-binding molecule described herein may be administered before or after the administration of at least one additional therapeutic agent.

When used herein, the term "use in combination" in relation to administration of a therapeutic method refers to use of more than one therapeutic methods or therapeutic agents. The use of the term "combining" does not limit the order in which the therapeutic methods or the therapeutic agents are administered to a subject. A therapeutic method or agent may be administered before, simultaneously to, or after administering the second therapeutic method or agent. Preferably, the therapeutic methods or agents are administered to a subject in an order, at an amount, and/or within a time interval that enables the therapeutic methods or agents to act together. In specific embodiments, the therapeutic methods or agents are administered to a subject in an order, at an amount, and/or within a time interval that offers higher benefit than when they are administered respectively by separate methods, or especially when they are administered individually. Preferably, the higher benefit is a synergistic effect.

The term "therapeutic agent" encompasses any agent administered to treat a symptom or disease in an individual in need of such treatment. Such additional therapeutic agent may comprise any active ingredients suitable for the particular indication being treated, preferably those with complementary activities that do not adversely affect each other. In certain embodiments, an additional therapeutic agent is an immunomodulatory agent, a cytostatic agent, an inhibitor of cell adhesion, a cytotoxic agent, an activator of cell apoptosis, or an agent that increases the sensitivity of cells to apoptotic inducers. In a particular embodiment, the additional therapeutic agent is an anti-cancer agent, for example a microtubule disruptor, an antimetabolite, a topoisomerase inhibitor, a DNA intercalator, an alkylating agent, a hormonal therapy, a kinase inhibitor, a receptor antagonist, an activator of tumor cell apoptosis, or an antiangiogenic agent.

Such other agents are suitably present in combination in amounts that are effective for the purpose intended. The effective amount of such other agents depends on the amount of multispecific antigen-binding molecules used, the type of disorder or treatment, and other factors discussed above. The multispecific antigen-binding molecules are generally used in the same dosages and with administration routes as described herein, or about from 1 to 99% of the dosages described herein, or in any dosage and by any route that is empirically/clinically determined to be appropriate.

In a non-limiting embodiment of the present invention, the combination therapy of the present invention provides a method for injuring cells, suppressing cell proliferation, activating immunity against cancer cells or cancer cell-comprising tumor tissues, treating cancer, or preventing cancer, wherein the method comprises administering the above-described multispecific antigen molecule with an effective amount of at least one other anticancer agent. In some embodiments, the combination therapy of the present invention has a higher effect of injuring cells, suppressing cell proliferation, activating immunity against cancer cells or cancer cell-comprising tumor tissues, treating cancer, or preventing cancer, as compared to monotherapy using the above-described multispecific antigen-binding molecule or other anticancer agents. In another embodiment, the combination therapy of the present invention has a synergistic effect or an additive effect of injuring cells, suppressing cell proliferation, activating immunity against cancer cells or cancer cell-comprising tumor tissues, treating cancer, or preventing cancer.

In some embodiments, "effective amount" in the present invention refers to a dose of the above-described multispecific antigen-binding molecule and/or other therapeutic agents that are effective for treating or preventing a disease in an individual. The disease is not particularly limited, but is preferably cancer. The type of cancer is not particularly limited, but is preferably a tumor containing CLDN6-expressing cancer cells.

In addition, "administering an effective amount of at least one TGFβ inducing agent" refers to administering an amount of the TGFβ inducing agent sufficient to induce TGFβ in a cell of interest.

In addition, "administering an effective amount of at least one CLDN6 expression inducing agent" refers to administering an amount of the CLDN6 expression inducing agent sufficient to induce CLDN6 expression in a cell of interest.

Such combination therapies described above encompass combined administration (where two or more types of therapeutic agents are contained in the same or separate compositions), and separate administration. In the case of separate administration, administration of the multispecific antigen-binding molecule described herein may occur simultaneously with or at different times from administration of the additional therapeutic agent and/or adjuvant. More specifically, administration of the multispecific antigen molecule may occur prior to, simultaneously with, and/or following, administration of the additional therapeutic agent and/or adjuvant. Multispecific antigen-binding molecules described herein can also be used in combination with radiation therapy.

In some embodiments, in cancer therapy or cancer prevention with at least one other anticancer agent, the combination therapy of the present invention provides methods of enhancing therapeutic effect or preventive effect of the at least one other anticancer agent by using the multispecific antigen-binding molecule described above. In another embodiment, in cancer therapy or cancer prevention with the multispecific antigen-binding molecule described above, the combination therapy of the present invention provides methods of enhancing therapeutic effect or preventive effect of the multispecific antigen-binding molecule by using at least one agent selected from a group consisting of another anticancer agent, a TGFβ inducing agent, and CLDN6 expression inducing agent. Here, enhancement of therapeutic effect or preventive effect refers to, for example, increase in efficacy rate of the treatment, decrease in the amount of the anticancer agent that is administered for the therapy, and/or shortening of the period of therapy with an anticancer agent, but is not limited thereto. In another embodiment, the combination therapy of the present invention provides methods of extending progression-free survival period in individuals, the method comprising administering effective amounts of the above-described multispecific antigen-binding molecule and at least one agent selected from a group consisting of another anticancer agent, a TGFβ inducing agent, and CLDN6 expression inducing agent.

In some embodiments, the combination therapy of the present invention comprises administering the above-described multispecific antigen-binding molecule and at least one other anticancer agent. The multispecific antigen-binding molecule and the at least one other anticancer agent can be administered by any appropriate method known in the art. For example, the multispecific antigen-binding molecule and the at least one other anticancer agent can be administered in parallel (i.e., simultaneously) and/or successively (i.e., at different time points). For example, when the multispecific antigen-binding molecule and two or more types of other anticancer agents (for example, a first other anticancer agent and a second other anticancer agent) are used in combination, the multispecific antigen-binding molecule and the two or more types of other anticancer agents are administered in any order. For example, each of the multispecific antigen-binding molecule and the two or more types of other anticancer agents can be administered successively (i.e., all at different time points); the multispecific antigen-binding molecule and the first other anticancer agent can be administered simultaneously and the second other anticancer agent can be administered before or after the simultaneous administration; or the first and second other anticancer agents can be administered simultaneously and the multispecific antigen-binding molecule can be administered before or after the simultaneous administration. In some embodiments, when the multispecific antigen-binding molecule and the at least one other anticancer agent are administered successively (i.e., at different time points), the administration interval of the multispecific antigen-binding molecule and the at least one other anticancer agent is not particularly limited, and the interval can be set by taking into account factors such as the administration route and dosage form. The administration interval is, for example, 0 to 168 hours, preferably 0 to 72 hours, more preferably 0 to 24 hours, and even more preferably 0 to 12 hours, but is not limited thereto.

Furthermore, in some embodiments, the combination therapy of the present invention comprises administering the above-described multispecific antigen-binding molecule and at least one TGFβ inducing agent. The multispecific antigen-binding molecule and the at least one TGFβ inducing agent can be administered by any appropriate method known in the art. Furthermore, in some embodiments, the combination therapy of the present invention comprises administering the above-described multispecific antigen-binding molecule and at least one CLDN6 expression inducing agent. The multispecific antigen-binding molecule and the at least one CLDN6 expression inducing agent can be administered by any appropriate method known in the art.

In some embodiments, the above-described multispecific antigen-binding molecule and the at least one other anticancer agent are administered simultaneously. In some embodiments, the multispecific antigen-binding molecule is administered periodically (i.e., intermittently). In some embodiments, the multispecific antigen-binding molecule is administered before the administration of the at least one other anticancer agent. In some embodiments, the multispecific antigen-binding molecule is administered after the administration of the at least one other anticancer agent.

In some embodiments, the at least one other anticancer agent is administered periodically (i.e., intermittently). In some embodiments, the at least one other anticancer agent is administered before the administration of the multispecific antigen-binding molecule. In some embodiments, the at least one other anticancer agent is administered after the administration of the multispecific antigen-binding molecule.

In some embodiments, the multispecific antigen-binding molecules described herein and anticancer agents which are known or described herein may be used in the above-described combination therapies using a multispecific antigen-binding molecule and at least one other anticancer agent.

Furthermore, in some embodiments, the multispecific antigen-binding molecules described herein and TGFβ inducing agents which are known or described herein may be used in the above-described combination therapies using a multispecific antigen-binding molecule and at least one TGFβ inducing agent.

Furthermore, in some embodiments, the multispecific antigen-binding molecules described herein and CLDN6 expression inducing agents which are known or described herein may be used in the above-described combination therapies using a multispecific antigen-binding molecule and at least one CLDN6 expression inducing agent.

In some embodiments, an additional therapeutic method can be performed in addition to the above-described combination therapies using a multispecific antigen-binding molecule and at least one other anticancer agent. In some embodiments, a therapeutic method to be added to the combination therapy of the present invention may comprise additional administration of the multispecific antigen-binding molecule and/or the at least one other anticancer agent.

A non-limiting embodiment of the present invention provides pharmaceutical compositions and such comprising the above-described multispecific antigen-binding molecule, at least one other anticancer agent, or a combination of the multispecific antigen-binding molecule and the at least one other anticancer agent. The pharmaceutical compositions are cytotoxicity-inducing agents, cell growth suppressors (cell growth inhibitors), immune response activators against cancer cells or cancer cell-comprising tumor tissues, cancer therapeutic agents, or cancer preventive agents. In some embodiments, the pharmaceutical compositions and such of the present invention can be used in the combination therapy of the present invention. In some embodiments, the pharmaceutical compositions and such of the present invention are highly effective for damaging cells, suppressing cell proliferation, activating immunity against cancer cells or cancer cell-comprising tumor tissues, treating or preventing cancer, due to combined use of the above-described multispecific antigen-binding molecule with the at least one other anticancer agent, as compared to monotherapy using the multispecific antigen-binding molecule or the at least one other anticancer agent. In another embodiment, the pharmaceutical compositions of the present invention have synergistic effects or additive effects on damaging cells, suppressing cell proliferation, activating immunity against cancer cells or cancer cell-comprising tumor tissues, treating or preventing cancer, due to combined use of the above-described multispecific antigen-binding molecule and the at least one other anticancer agent. In certain embodiments, administration of at least one other anticancer agent enhances the antitumor effects of the above-described multispecific antigen-binding molecules. Furthermore, in certain embodiments, administration of the above-described multispecific antigen-binding molecule enhances the antitumor effects of the at least one other anticancer agent.

In some embodiments, the pharmaceutical compositions and such according to the present invention "comprising a combination of a multispecific antigen-binding molecule and at least one other anticancer agent" refers to pharmaceutical compositions and such in which the above-described multispecific antigen-binding molecule and the at least one other anticancer agent are combined for use in simultaneous, separate, and/or sequential administration in treating or preventing a disease. For example, the pharmaceutical compositions and such of the present invention can be provided in the form of a combination preparation containing both a multispecific antigen-binding molecule and at least one other anticancer agent. Alternatively, for example, as the pharmaceutical compositions and such of the present invention, a pharmaceutical agent containing a multispecific antigen-binding molecule and a pharmaceutical agent containing at least one other anticancer agent can be provided separately, and these pharmaceutical agents may be used simultaneously or sequentially. The disease is not particularly limited, but is preferably cancer.

In some embodiments, the present invention provides pharmaceutical compositions and such for use in combination with at least one agent selected from a group consisting of another anticancer agent, a TGFβ inducing agent, and CLDN6 expression inducing agent, the compositions comprising the above-described multispecific antigen-binding molecule as an active ingredient. In some embodiments, the present invention provides pharmaceutical compositions and such for use in combination with the above-described multispecific antigen-binding molecule, the compositions comprising at least one other anticancer agent as an active ingredient.

In some embodiments, the present invention provides pharmaceutical compositions and such for enhancing therapeutic effects of at least one other anticancer agent in cancer therapy with the at least one other anticancer agent, by combining the above-described multispecific antigen-binding molecule with the other anticancer agent.

In some embodiments, the present invention provides pharmaceutical compositions and such for enhancing therapeutic effects of the above-described multispecific antigen-binding molecule in cancer therapy with the multispecific antibody, by combining other anticancer agents with the multispecific antigen-binding molecule.

In some embodiments, the present invention provides use of the above-described multispecific antigen-binding molecule and/or other anticancer agents for the production of pharmaceutical compositions and such comprising as active ingredients the multispecific antigen-binding molecule and/or the at least one other anticancer agent.

In the present invention, comprising as active ingredients the above-described multispecific antigen-binding molecule and/or the at least one other anticancer agent means comprising the multispecific antigen-binding molecule and/or at least one other anticancer agent as major active ingredients, and does not limit the proportional content of the multispecific antigen-binding molecule and/or the at least one other anticancer agent.

In some embodiments, the multispecific antigen-binding molecule described herein and at least one other anticancer agent which is known or described herein can be used in the above-described pharmaceutical compositions and the like.

Herein, "at least one other anticancer agent" means one, two, three, four, five, or more types of anticancer agents.

Herein, "at least one other anticancer agent" or "other anticancer agents" mean that the anticancer agent comprises as an active ingredient a substance different from the multispecific antigen-binding molecule described herein. More specifically, when the expression "other anticancer agents" is used, it only specifies that the anticancer agent comprises as an active ingredient or active ingredients a substance or substances different from the multispecific antigen-binding molecule, and is not limited to an agent in which the multispecific antigen-binding molecule used in combination with at least one other anticancer agent is used as the anticancer agent. For example, the anticancer agents, pharmaceutical compositions, combinations, kits, methods, or uses characterized by concurrent use (combined use) of a multispecific antigen-binding molecule with at least one other anticancer agent encompass embodiments in which an anticancer agent other than the at least one other anticancer agent is not used, in which case, embodiments in which the multispecific antigen-binding molecule is used as, for example, a potentiator, concomitant drug, or additive of the at least one other anticancer agent are encompassed.

In a non-limiting embodiment of the present invention, the above-described at least one other anticancer agent includes, but are not limited to, nitrogen mustard analogues, alkyl sulfonates, ethylene imines, nitrosoureas, epoxides, other alkylating agents, folic acid analogues, purine analogues, pyrimidine analogues, other antimetabolites, vinca alkaloids or analogues, podophyllotoxin derivatives, camptothecan analogues, colchicine derivatives, taxanes, other alkaloids or plant alkaloids or natural substances, topoisomerase inhibitors, actinomycines, anthracyclines or related substances, other cytotoxic antibiotics, platinum preparations (platinum compounds), methylhydrazines, kinase inhibitors, angiogenic inhibitors, hormonal agents, inhibitors of DNA modification enzymes, immunostimulants, proteasome inhibitors, enzymes, histone deacetylase inhibitors, inhibitors of DNA modification enzymes, cytokine preparations, retinoids, immune checkpoint inhibitors, indoleamine 2,3-Dioxygenase (IDO) inhibitors, co-stimulatory molecule activators, natural killer cell activators, poly ADP-ribose polymerase (PARP) inhibitors, monoclonal antibodies, other molecular-targeted agents, and other anticancer agents. In a non-limiting embodiment, at least one other anticancer agent in the present invention includes, but is not limited to, for example, antibodies described in WO2015/174439 and WO2015/156268.

In some embodiments, an "immune checkpoint molecule" of the present invention refers to a molecule that is expressed on immunocompetent cells (including T cells) or cancer cells and binds to a ligand to transduce to the immunocompetent cells signals for inhibiting immune response. Examples of immune checkpoint molecules and ligands thereof include, but are not limited to, molecules such as PD-1, CTLA-4, TIM3, LAG3, PD-L1, PD-L2, BTNL2, B7-H3, B7-H4, CD48, CD80, 2B4, BTLA, CD160, CD60, CD86i, and VISTA. In some embodiments, an "immune checkpoint inhibitor" of the present invention refers to a pharmaceutical agent that inhibits binding between an immune checkpoint molecule and its ligand, and thereby inhibits signal transduction mediated by the immune checkpoint molecule.

In some Examples, "PARP inhibition" in the present invention refers to interference of repair of single-strand break by inhibiting poly(ADP-ribose) polymerase (PARP), especially PARP-1 and PARP-2. PARP inhibitor is a pharmaceutical agent having a function of interference of repair of single-strand break by inhibiting PARP. Some cancers such as breast cancer and ovary cancer are known to show abnormalities in repair of double-strand break due to a BRCA gene mutation, and PARP inhibitor refers to a pharmaceutical agent having antitumor effects due to synthetic lethality against these cancers.

A non-limiting embodiment of the present invention provides pharmaceutical compositions and such in which the at least one other anticancer agent is a chemotherapeutic agent, an immune checkpoint inhibitor, a PARP inhibitor, a T cell-activating agonist agent, and/or an angiogenic inhibitor, or more specifically, the at least one other anticancer agent is one or a plurality of anticancer agents selected from the group consisting of chemotherapeutic agents, immune checkpoint inhibitors, PARP inhibitors, T cell-activating agonist agents, and angiogenic inhibitors. In the case where a plurality of other anticancer agents are used, they can be selected from the same type of anticancer agents, or from different types of anticancer agents. For example, two or more agents can be selected from chemotherapeutic agents, or one or more agents can be selected from chemotherapeutic agents and immune checkpoint inhibitors respectively. The selection is made similarly when a plurality of agents are selected from other types of anticancer agents.

In a non-limiting embodiment of the present invention, the at least one other anticancer agent includes an agent that enhances CLDN6 expression in cells. Examples of the cells whose expression of CLDN6 is enhanced include cancer cells, cells in tumor tissues, and/or cells near tumor tissues. That is, the at least one other anticancer agent includes agents whose administration leads to induction or enhancement of CLDN6 expression in cancer cells, cells in tumor tissues, and/or cells near tumor tissues.

An agent that enhances CLDN6 expression in cells can be identified by analyzing the change in CLDN6 expression before and after the administration of the agent to a cell line of interest. For example, when change in the amount of CLDN6 expression before and after the administration of the agent to a cell line of interest is analyzed by common techniques such as qPCR, FACS, and Western Blotting analysis and the increase in the amount of CLDN6 expression is observed, the agent is identified as an agent that enhances CLDN6 expression in cells.

Specifically, for example, the agent is added to a cancer cell line, RNA is purified from the recovered cells, and then cDNA synthesis is performed, real-time PCR is performed using the cDNAs as templates and using CLDN6-specific primers, and CLDN6 expression is compared and analyzed relative to that of the cells to which the agent was not added. Alternatively, for example, the agent is added to a cancer cell line, the cells are stained with an anti-CLDN6 antibody, and CLDN6 expressed on the cell membrane is compared and analyzed by using a flow cytometer relative to that of the cells to which the agent was not added. Alternatively, for example, the agent is added to a cancer cell line, and a lysate of the cells is used to compare and analyze CLDN6 expression by Western blotting using the anti-CLDN6 antibody, relative to that of the cells to which the agent was not administered. Each of these techniques can be carried out by commonly known protocols (https://www.cellsignal.jp/learn-and-support/protocols/protocol-western (CST), https://www.takara-bio.co.jp/research/prt/pdfs/prt2.pdf (TaKaRa Bio), https://www.thermofisher.com/jp/ja/home/life-science/cell-analysis/cell-analysis-learning-center/molecular-probes-school-of-fluorescence/flow-cytometry-basics/flow-cytometry-fundamentals/how-flow-cytometer-works.html (ThermoFisher)).

Examples of an agent that enhances CLDN6 expression in cancer cells include carboplatin, cisplatin, irinotecan, gemcitabine, and other chemotherapeutic agents.

In a non-limiting embodiment of the present invention, the at least one other anticancer agent includes an agent that induces TGF-β expression in cells. Examples of the cells whose expression of TGF-β is induced include cancer cells, cells in tumor tissues, and/or cells near tumor tissues. That is, the at least one other anticancer agent includes agents whose administration leads to induction or enhancement of TGF-β expression in cancer cells, cells in tumor tissues, and/or cells near tumor tissues. For example, the at least one other anticancer agent induces expression of TGF-β1 in cancer cells. Furthermore, for example, administration of the at least one other anticancer agent induces expression of TGF-β1 in tumor tissues. Moreover, for example, administration of the at least one other anticancer agent induces expression of TGF-β1 near tumor tissues.

An agent that enhances TGF-β expression in cancer cells can be identified, for example, by adding the agent to a cancer cell line, and analyzing the change in TGF-β expression. For example, when change in the amount of TGF-β expression before and after the administration of the agent to a cell line of interest is analyzed by a common technique such as qPCR and ELISA measurement of TGF-β concentration secreted into the cell supernatant, and the increase in the amount of TGF-β expression is observed, the agent is identified as an agent that enhances TGF-β expression in cells.

Specifically, an agent is added to a cancer cell line, RNA is purified from the recovered cells, and then cDNA synthesis is performed, real-time PCR is performed using the synthesized cDNAs as templates and using TGF-β-specific primers, and TGF-β expression is compared and analyzed relative to that of the cells to which the agent was not added. Alternatively, the agent is added to a cancer cell line, the concentration of TGF-β secreted into the cancer cell culture supernatant is measured by ELISA, and the concentration is compared and analyzed relative to that in cells to which the agent was not added. Each of these techniques are publicly known methods and can be carried out by commonly known protocols (for example, https://www.cellsignal.jp/learn-and-support/protocols/protocol-western (CST), Human/Mouse/Rat/Porcine/Canine TGF-beta 1 Quantikine ELISA (https://www.rndsystems.com/products/human-mouse-rat-porcine-canine-tgf-beta-1-quantikine-elisa_db100b(R & D system)).

Examples of an agent that induces TGF-β1 expression in cancer cells include doxorubicin, paclitaxel, carboplatin, cisplatin, irinotecan, gemcitabine, and other chemotherapeutic agents, and radiation.

In a non-limiting embodiment of the present invention, chemotherapeutic agents include, but are not limited to, antimetabolites, alkaloids, anthracyclines, and platinum preparations.

Preferred examples of an antimetabolite include, but are not limited to, enocitabine, capecitabine, carmofur, gemcitabine, cytarabine, tegafur, tegafur/uracil, nelarabine, fluorouracil, fludarabine, pemetrexed, pentostatin, and methotrexate. Examples of particularly preferred antimetabolites include gemcitabine.

Examples of alkaloids include plant alkaloids. Preferred examples of a plant alkaloid include, but are not limited to, irinotecan, etoposide, sobuzoxane, docetaxel, nogitecan, paclitaxel, vinorelbine, vincristine, vindesine, and vinblastine. Examples of particularly preferred plant alkaloids include topoisomerase inhibitors. Examples of particularly preferred plant alkaloids include paclitaxel and irinotecan.

Preferred examples of a platinum preparation include, but are not limited to, oxaliplatin, carboplatin, cisplatin, and nedaplatin. Examples of particularly preferred platinum preparations include carboplatin and cisplatin.

In a non-limiting embodiment of the present invention, preferred examples of immune checkpoint inhibitors include, but are not limited to, anti-PD-1 antibodies, anti-PD-L1 antibodies, anti-CTLA-4 antibodies, anti-TIM3 antibodies, and anti-LAG3 antibodies. Examples of anti-PD-1 antibodies include Pembrolizumab (CAS Registry Number:1374853-91-4), Nivolumab (CAS Registry Number:946414-94-4), MEDI0680, PDR001, BGB-A317, REGN2810, SHR-1210, PF-0680159I, and various known anti-PD-1 antibodies. Examples of anti-PD-L1 antibodies

include Atezolizumab (CAS Registry Number: 1380723-44-3), Avelumab (CAS Registry Number: 1537032-82-8), Durvalumab (CAS Registry Number: 1428935-60-7), MDX-11 05, and various known anti-PD-L1 antibodies. Examples of anti-CTLA-4 antibodies include Ipilimumab (CAS Registry Number: 477202-00-9), Tremelimumab (CAS Registry Number: 745013-59-6), and various known anti-CTLA-4 antibodies. Examples of anti-TIM3 antibodies include MBG452 and various known anti-TIM3 antibodies. Examples of anti-LAG3 antibodies include BMS-986016s LAG525 and various known anti-LAG3 antibodies. Examples of particularly preferable immune checkpoint inhibitors include anti-PD-L1 antibodies.

In a non-limiting embodiment of the present invention, preferred examples of a PARP inhibitor include olaparib, rucaparib, niraparib, veliparib, pamiparib, and talazoparib. Examples of particularly preferred PARP inhibitors include olaparib.

As a non-limiting embodiment of the present invention, examples of a T cell-activating agonist agent include, but are not limited to, agonistic antibodies to TNF receptor superfamily (TNFRSF) and agonistic antibodies to co-stimulatory molecules. Target molecules of the "agonistic antibodies to TNF receptor superfamily" are not particularly limited as long as they are factors that activate cells that express the TNF receptor superfamily (for example, T cells and NK cells), but are preferably factors belonging to the "TNF superfamily" or "TNF receptor superfamily". As factors belonging to the "TNF superfamily" or "TNF receptor superfamily," ligands having a trimeric structure and receptors with a trimeric structure to which the ligands bind, which contribute to activation of various immune cells are known (Nat. Rev. Immunol., 2012, 12, 339-51). Examples of factors belonging to the TNF superfamily or the TNF receptor superfamily include CD137, CD137L, CD40, CD40L, OX40, OX40L, CD27, CD70, HVEM, LIGHT, RANK, RANKL, CD30, CD153, GITR, GITRL, TNFRSF25, and TL1A. Preferred factors include, for example, CD 137. Examples of CD137 agonist antibodies include Urelumab (CAS Registry Number: 934823-49-1), PF-05082566, and various known CD137 agonist antibodies.

Examples of factors belonging to co-stimulatory molecules include TMIGD2, HHLA2, ICOS, ICOS Ligand, CD28, CD80, and CD86. Examples of OX40 agonist antibodies include MOXR0916, MEDI6469, MEDI0562, MEDI6383, PF-04518600s GSK-3174998, and various known OX40 agonist antibodies. Examples of CD40 agonist antibodies include RG-7876, ADC-1013, SEA-CD40, APX005M, Dacetuzumab, and various known CD40 agonist antibodies. Examples of GITR agonist antibodies include AMG228, AMK-1248, MK-4166, BMS-986156s TRX518, and various known GITR agonist antibodies. Examples of CD27 agonist antibodies include Varlilumab (CAS Registry Number: 1393344-72-3) and various known CD27 agonist antibodies.

In a non-limiting embodiment of the present invention, suitable examples of angiogenic inhibitors include, but are not limited to, VEGFR2 antibodies. In some Examples, angiogenic inhibitors in the present invention prevent extensive growth of blood vessels (angiogenesis) necessary for survival of tumors. For example, angiogenesis promoted by tumor cells to meet their increasing nutrition and oxygen demands, can be blocked by targeting various molecules. Examples of angiogenic inhibitors include bevacizumab, sorafenib, everolimus, temsirolimus, and various known angiogenic inhibitors.

Herein, "method for inducing TGF-β" means a method for inducing TGF-β in cells. Specifically, it is a method for inducing TGF-β1, TGF-β2, and/or TGF-β3 in cells by dose administration. The "method for inducing TGF-β" includes administering a "TGF-β-inducing agent". Performing the "method for inducing TGF-β" on a cell line enhances the expression of TGF-β.

"TGF-β-inducing agent" means an agent that induces TGF-β in cells. Specifically, it is an agent that induces TGF-β1, TGF-β2, and/or TGF-β3 in cells when it is administered. Adding the TGF-β-inducing agent to a cell line enhances the expression of TGF-β.

A TGF-β-inducing agent can be identified, for example, by adding an agent to a cell line, and analyzing the change in TGF-β expression. For example, when change in the amount of TGF-β expression before and after the administration of the agent to a cell line of interest is analyzed by a common technique such as qPCR and ELISA measurement of the concentration of TGF-β secreted into the cell supernatant and the increase in the amount of TGF-β expression is observed, the agent is identified as an agent that enhances TGF-β expression in cells.

Specifically, the agent is added to a cell line, RNA is purified from the recovered cells, and then cDNA synthesis is performed, real-time PCR is performed using the synthesized cDNAs as templates and using TGF-β-specific primers, and TGF-β expression is compared and analyzed relative to that of the cells to which the agent was not added. Alternatively, the agent is added to a cell line, the concentration of TGF-β secreted into the cell culture supernatant is measured by ELISA, and the concentration is compared and analyzed relative to that in cells to which the agent was not added. Each of these techniques are publicly known methods and can be carried out by commonly known protocols (for example, https://www.cellsignal.jp/learn-and-support/protocols/protocol-western (CST), Human/Mouse/Rat/Porcine/Canine TGF-beta 1 Quantikine ELISA (https://www.mdsystems.com/products/human-mouse-rat-porcine-canine-tgf-beta-1-quantikine-elisa_db100b)(R & D system)).

Herein, "CLDN6 expression-inducing agent" means an agent that induces CLDN6 expression in cells. Specifically, it is a method for enhancing CLDN6 in cells by dose administration.

Specifically, for example, the agent is added to a cell line, RNA is purified from the recovered cells, and then cDNA synthesis is performed, real-time PCR is performed using the synthesized cDNAs as templates and using CLDN6-specific primers, and CLDN6 expression is compared and analyzed relative to that of the cells to which the agent was not added. Alternatively, for example, the agent is added to a cancer cell line, the cells are stained with an anti-CLDN6 antibody, and by using a Flow cytometer, CLDN6 expressed on the cell membrane is compared and analyzed relative to that of the cells to which the agent was not added. Alternatively, for example, the agent is added to a cell line, and a lysate of the cells is used to compare and analyze CLDN6 expression by Western blotting using an anti-CLDN6 antibody, relative to that of the cells to which the agent was not administered. Each of these techniques can be carried out by commonly known protocols (for example, https://www.cellsignal.jp/learn-and-support/protocols/protocol-western (CST), https://www.takara-bio.co.jp/research/prt/pdfs/prt2.pdf (TaKaRa Bio), https://www.thermofisher.com/jp/ja/home/life-science/cell-analysis/cell-analysis-learning-center/molecular-probes-school-of-fluorescence/flow-cytometry-basics/flow-cytometry-fundamentals/how-flow-cytometer-works.html (ThermoFisher)).

Examples of a CLDN6 expression-inducing agent include, but are not limited to, carboplatin, cisplatin, irinotecan, gemcitabine, and other chemotherapeutic agents.

In some embodiments, any of at least one other anticancer agent of the present invention can be used and is not particularly limited as long as the therapeutic or preventive effects of the other anticancer agent becomes enhanced, or the therapeutic or preventive effects of the multispecific antigen-binding molecule becomes enhanced, when it is used in combination with the multispecific antigen-binding molecule of the present invention. In a specific embodiment, the therapeutic or preventive effect is antitumor effect.

In a non-limiting embodiment of the present invention, combination therapies of the invention may include, but is not limited to, the above-described multispecific antigen-binding molecule, at least one other therapeutic agent, immunomodulatory agent, cancer therapy vaccine, adoptive T cell therapy, and Treg elimination. Examples of a preferred cancer therapy vaccine include, but are not limited to, whole tumor cell vaccines, tumor antigen vaccines, vector-based vaccines, oncolytic virus vaccines, and dendritic cell vaccines. In addition to the above-descried therapeutic methods, multimodal therapy that involves combined use of surgery, radiation therapy, and such may be performed.

In a non-limiting embodiment of the present invention, combination therapies of the invention may be performed by combining the above-described multispecific antigen-binding molecule with cytokine therapy that uses a cytokine as an antitumor immune response potentiator, and examples of such therapeutic methods include, but are not limited to, cytokines such as IL-2, IL-7, IL-12, IL-15, IL-17, IL-18, IL-21, IL-21, IL-23, IL-27, GM-CSF, IFN$\alpha$ (interferon-$\alpha$), IFN$\alpha$-2b, IFN$\beta$, and IFN$\gamma$.

A non-limiting embodiment of the present invention provides a cytotoxicity-inducing agent, a cell growth suppressor, a cell growth inhibitor, an immune response activator, a cancer therapeutic agent, or a cancer preventive agent, comprising the above-described pharmaceutical composition.

In some embodiments, "individual" to which the above-described multi specific antigen-binding molecule and/or other anticancer agents are administered means a human or a non-human mammal, for example, a mammal such as horse, cattle, dog, sheep, or cat. Preferably, the individual is a human. The individual includes a patient (including human or non-human mammal). In some embodiments, the individual is a patient carrying cancer cells or cancer cell-comprising tumor tissues. Cancer cells or cancer cell-comprising tumor tissues, which are targets of anticancer agents or combination therapies in the present invention, are not particularly limited as long as they express CLDN6. Preferred CLDN6-expressing cells in the present invention, or more specifically CLDN6-positive cells, are cancer cells. More preferable examples of the type of cancer include, but are not limited to, ovary cancer, non-small cell lung cancer (NSCLC), gastric cancer, esophageal cancer, liver cancer, breast cancer, large bowel cancer (including colon cancer and rectum cancer), germ cell tumor (germinoma), testis cancer, uterine cancer, cervical cancer, cholangiocarcinoma, kidney cancer, head and neck cancer, pancreatic cancer (pancreatic ductal adenocarcinoma (PDAC)), urinary bladder cancer, and atypical teratoid rhabdoid tumor (AT/RT). More preferable examples of the type of cancer include, but are not limited to, ovary cancer, non-small cell lung cancer, gastric cancer, liver cancer, endometrial cancer, germ cell tumor, large bowel cancer, urinary bladder cancer, and atypical teratoid rhabdoid tumor.

In some embodiments, patients are those who have received therapy with the multispecific antibody and/or some kind of anticancer agent prior to the above-described combination therapy using the multispecific antigen-binding molecule and other anticancer agents. In some embodiments, the patients are those who cannot receive standard therapeutic methods, or those in which standard therapeutic methods are not effective. In some embodiments, the patients have early-stage cancer or end-stage cancer.

Furthermore, in some embodiments, the cancer types targeted for therapy by the anticancer agents or combination therapies using pharmaceutical compositions of the present invention are preferably cancer types in which the number of CLDN6 antigens on cell surface per cell is 100 or more; more preferably, cancer types in which the number of CLDN6 antigens on cell surface per cell is 200 or more, 300 or more, 400 or more, 500 or more, 600 or more, 700 or more, 800 or more, 900 or more, 1000 or more, 1200 or more, 1400 or more, 1600 or more, 1800 or more, or 2000 or more; or even more preferably, cancer types in which the number of CLDN6 antigens on cell surface per cell is 3000 or more, 4000 or more, 5000 or more, 6000 or more, 7000 or more, 8000 or more, 9000 or more, 10000 or more, 20000 or more, 30000 or more, 40000 or more, or 50000 or more.

The number of CLDN6 antigens on cell surface per cell can be appropriately determined using methods described herein or known to those skilled in the art, for example, by calculating the antibody binding capacity (ABC) of CLDN6 on the cell surface with flow cytometry using QIFIKIT (DAKO). The number of CLDN6 antigens on cell surface per cell in a tissue sample isolated from a target candidate can be determined in order to assess whether the candidate can be a target to which the anticancer agent or pharmaceutical composition of the present invention is administered. In the sample, when the number of CLDN6 antigens on cell surface per cell meets the criterion described above, the target from which the sample is derived can be the target to which the anticancer agent or pharmaceutical composition (combination therapy) of the present invention is administered.

In a non-limiting embodiment of the present invention, the anticancer agents of the present invention can be used to treat patients who have cancer unresponsive to treatment with other anticancer agents.

For example, the anticancer agents of the present invention can be used to treat patients who have cancer unresponsive to treatment with chemotherapeutic agents. Therapy using the anticancer agents of the present invention can be performed on patients with CLDN6-positive cancer, in whom administration of a chemotherapeutic agent failed to achieve a desired drug efficacy or resulted in recurrence of cancer, or whose cancer was found to be resistant to a chemotherapeutic agent. In other words, therapy using the anticancer agents of the present invention can be performed on CLDN6-positive cancer that has been already treated with a therapeutic method using a chemotherapeutic agent.

For example, the anticancer agents of the present invention can be used to treat patients who have cancer unresponsive to treatment with immune checkpoint inhibitors. Therapy using the anticancer agents of the present invention can be performed, for example, on patients with CLDN6-positive cancer, in whom administration of an immune checkpoint inhibitor failed to achieve a desired drug efficacy or resulted in recurrence of cancer, or whose cancer was found to be resistant to an immune checkpoint inhibitor. In other words, therapy using the anticancer agents of the present invention can be performed on CLDN6-positive cancer that has been already treated with a therapeutic method using an immune checkpoint inhibitor.

In a non-limiting embodiment of the present invention, the pharmaceutical compositions (combination therapy) of the present invention can be used to treat patients who have cancer unresponsive to treatment with other anticancer agents.

For example, the pharmaceutical compositions of the present invention can be used to treat patients who have cancer unresponsive to treatment with chemotherapeutic agents. Therapy using the pharmaceutical compositions of the present invention can be performed on patients with CLDN6-positive cancer, in whom administration of a chemotherapeutic agent failed to achieve a desired drug efficacy or resulted in recurrence of cancer, or whose cancer was found to be resistant to a chemotherapeutic agent. In other words, therapy using the pharmaceutical compositions of the present invention can be performed on CLDN6-positive cancer that has been already treated with a therapeutic method using a chemotherapeutic agent. Preferred examples of other anticancer agents contained in the pharmaceutical compositions include chemotherapeutic agents, but are not limited thereto.

Furthermore, for example, the pharmaceutical compositions of the present invention can be used to treat patients who have cancer unresponsive to treatment with immune checkpoint inhibitors. Therapy using the pharmaceutical compositions (combination therapy) of the present invention can be performed on patients with CLDN6-positive cancer, in whom administration of an immune checkpoint inhibitor failed to achieve a desired drug efficacy or resulted in recurrence of cancer, or whose cancer was found to be resistant to an immune checkpoint inhibitor. In other words, therapy using the pharmaceutical compositions (combination therapy) of the present invention can be performed on CLDN6-positive cancer that has been already treated with a therapeutic method using an immune checkpoint inhibitor. Preferred examples of other anticancer agents contained in the pharmaceutical compositions include immune checkpoint inhibitors, but are not limited thereto.

In a non-limiting embodiment of the present invention, the pharmaceutical compositions (combination therapy) of the present invention can be used to treat patients who have cancer unresponsive to treatment with the anticancer agent of the present invention. For example, therapy using the pharmaceutical compositions (combination therapy) of the present invention can be performed on patients with CLDN6-positive cancer, whose cancer has become resistant to the anticancer agent of the present invention after the administration of the anticancer agent or in whom administration of the anticancer agent of the present invention has failed to achieve a desired drug efficacy, or whose cancer was found to be resistant to the anticancer agent of the present invention. In other words, therapy using the pharmaceutical compositions (combination therapy) of the present invention can be performed on CLDN6-positive cancer that has been already treated with a therapeutic method using the anticancer agent of the present invention. Preferred examples of other anticancer agents contained in the pharmaceutical compositions include immune checkpoint inhibitors and chemotherapeutic agents, but are not limited thereto.

Regarding CLDN6-positive cancer (cancer confirmed to express CLDN6), those skilled in the art can appropriately examine and determine positivity for CLDN6 using methods known to those skilled in the art such as immunohistochemical staining, flow cytometry, or in situ hybridization.

In this embodiment, CLDN6-positive cancers previously treated with anticancer agents other than the anticancer agents of the present invention are presented as examples of CLDN6-positive cancers having resistance to other anticancer agents. For example, CLDN6-positive cancers previously treated with a platinum preparation are presented as examples of platinum preparation-resistant CLDN6-positive cancers, and CLDN6-positive cancers previously treated with an immune checkpoint inhibitor are presented as examples of immune checkpoint inhibitor-resistant CLDN6-positive cancers. "Resistant/resistance" may be replaced with "refractory."

For example, CLDN6-positive cancer which recurred after performing therapy comprising a platinum preparation is included in the CLDN6-positive cancer previously treated with a platinum preparation. CLDN6-positive cancer which recurred after performing therapy comprising an immune checkpoint inhibitor is included in the CLDN6-positive cancer previously treated with an immune checkpoint inhibitor.

Here, "resistant/resistance" as used herein is not limited as long as the cells or individuals do not respond (or are insensitive) to disease treatment or therapy and/or are in a state where their ability to generate a significant response (for example, partial and/or complete response) is decreased. For example, cancers having resistance to other anticancer agents may mean that the resistance was generated after treatment with an anticancer agent other than the anticancer agent of the present invention. For example, while a therapy may be effective in the early stages, the cancer may eventually acquire resistance to the therapy as the therapy is repeated, and in some cases, the cancer no longer regresses or even advances in the presence of other anticancer agents.

Furthermore, other examples of CLDN6-positive cancers having resistance to other anticancer agents include cancers that have recurred after therapy by administration of an anticancer agent other than the anticancer agent of the present invention.

The present disclosure also provides kits for use in the method of the present disclosure, which contain the antigen-binding molecule of the present disclosure or an antigen-binding molecule produced by the method of the present disclosure. The kits may be packaged with an additional pharmaceutically acceptable carrier or medium, or instruction manual describing how to use the kits, and such.

In another aspect of the invention, an article of manufacture containing materials useful for treating, preventing and/or diagnosing the disorders described above is provided. The article of manufacture comprises a container, and a label on the container or a package insert document accompanying the container. Suitable containers include, for example, bottles, vials, syringes, and IV solution bags. The containers may be formed from a variety of materials such as glass or plastic. The container may hold a composition by itself, or in combination with another composition effective for treating, preventing and/or diagnosing the symptoms, and may have a sterile access port (for example, the container may be a solution bag or a vial for intravenous administration, which has a stopper pierceable by a hypodermic injection needle). At least one active ingredient in the composition is a multispecific antigen-binding molecule of the present disclosure. The label or package insert document indicates that the composition is used for treating the symptoms of choice. Moreover, the article of manufacture may comprise (a) a first container with a composition contained therein, wherein the composition comprises a multispecific antigen-binding molecule of the present invention; and (b) a second container with a composition contained therein, wherein the composition comprises a further cytotoxic agent (at least one other anticancer agent, or a first other anticancer agent when at least one other multiple types of other anticancer agents are used in combination) or an otherwise therapeutic agent. The article of manufacture may further comprise (c) a third container with a composition contained therein, wherein the composition comprises a further cytotoxic agent (a second other anticancer agent when multiple types of other anticancer agents are used in combination) or an otherwise therapeutic agent. The article of manufacture in this embodiment of the present invention may further comprise a package insert document indicating that the composition may be used to treat specific symptoms. Alternatively, or in addition, an article of manufacture may further comprise a second (or third or fourth) container containing a pharmaceutically-acceptable buffer, such as bacteriostatic water for injection (BWFI), phosphate-buffered saline, Ringer's solution, and dextrose solution. It may further include other materials desirable from a commercial viewpoint and user standpoint, including other buffers, diluents, filters, needles, and syringes.

Package insert document

The term "package insert document" is used to refer to a written explanation customarily included in commercial packages of therapeutic products, and contain information about the indications, usage, dosage, administration method, combination therapy, contraindications, and/or warnings concerning the use of such therapeutic products.

In some embodiments, the present invention provides a kit comprising (1) the multispecific antigen-binding molecule described above, (2) a container, and (3) an instruction or a label indicating that the multispecific antigen-binding molecule and at least one type of anticancer agent are administered in combination to an individual for treating cancer in the individual.

In other embodiments, the present invention provides a kit comprising (1) at least one other anticancer agent, (2) a container, and (3) an instruction or a label indicating that the at least one other anticancer agent and at least one type of the multispecific antigen-binding molecule described above are administered in combination to an individual for treating cancer in the individual.

In other embodiments, the present invention provides a kit comprising (1) the multispecific antigen-binding molecule described above, (2) at least one other anticancer agent, (3) a container, and (4) an instruction or a label indicating that the multispecific antigen-binding molecule and the at least one other anticancer agent are administered in combination to an individual for treating cancer in the individual.

In some embodiments, the kit further comprises a pharmaceutically acceptable carrier. Preferably, the kit may further comprise a sterile diluent stored in a separate additional container. More specifically, the kit may further comprise a second (or third) container containing a pharmaceutically-acceptable buffer, such as bacteriostatic water for injection (BWFI), phosphate-buffered saline, Ringer’s solution, and dextrose solution.

The kit of the present disclosure may further comprise other materials desirable from a commercial viewpoint and user standpoint, including other buffers, diluents, filters, needles, and syringes. The kit may further comprise an instruction on combination therapy for treating or preventing cancer.

In some embodiments, in the kit of the present disclosure, a multispecific antigen-binding molecule and/or an anticancer agent may be loaded into a container.

In some embodiments, an "instruction" refers to a written instruction usually included in commercial packages carrying a pharmaceutical, and can contain information about the indications, usage, dosage, administration, contraindications, and/or warnings regarding the use of the pharmaceutical.

In some embodiments, the kit of the present disclosure is provided, for example, as a commercial package for a therapeutic product.

The kits may be those which are used exclusively for the purpose of combined use of the multispecific antigen-binding molecule of the present invention and at least one other anticancer agent; however, the kits may be those which are used for other purposes as long as they are used for the purpose of combined use of the multispecific antigen-binding molecule of the present invention and at least one other anticancer agent. For example, as long as the instruction or label of the kit of the present invention indicates the combined administration to an individual, the instruction or label may indicate other embodiments, for example, use of the multispecific antigen-binding molecule or the at least one other anticancer agent by itself.

In the present specification, when the term "and/or" is used, it indicates any combination of each of the terms of interest indicated before and after "and/or." Specifically, for example, "A, B, and/or C" includes the following seven variations: (i) A, (ii) B, (iii) C, (iv) A and B, (v) A and C, (vi) B and C, and (vii) A, B, and C.

In the present invention, the indefinite article "a" or "an" refers to one, or two or more (that is, at least one) grammatical object referred to by the indefinite article. For example, "a component" refers to one component or two or more components.

Those skilled in the art will naturally appreciate that any combinations of one or more of the embodiments described herein are also included in the present invention as long as they are not technically inconsistent based on common technical knowledge of those skilled in the art.

All documents cited herein are incorporated herein by reference.

The following are the Examples of methods and compositions of the present disclosure. It is understood that various other embodiments may be practiced, given the general description provided above.

[Example]

[Example 1] Expression of CLDN6 in various cancer tissues

CLDN6 expression was compared in various cancer tissues by referring to the Cancer Genome Atlas (TCGA) data.

As shown in Fig. 1, CLDN6 expression was found to be enhanced in ovary cancer, lung adenocarcinoma (non-small cell lung cancer, NSCLC), germ cell tumor (germinoma), and uterine (uterus) cancer. Furthermore, although the frequency was not high, CLDN6 expression was observed in cholangiocarcinoma, cervical (cervix uteri) cancer, breast cancer, large bowel cancer (colon cancer and rectum cancer), liver cancer, kidney cancer, esophageal (esophagus) cancer, pancreatic (pancreas) cancer, gastric (stomach) cancer, urinary bladder cancer, head and neck cancer (upper aerodigestive tract cancer), and such.

[Example 2] Screening of affinity matured variants derived from parental Dual-Fab H183L072 for improvement in in vitro cytotoxicity on tumor cells

2.1 Sequence of affinity matured variants

To increase the binding affinity of parental Dual-Fab H183L072 (Heavy chain: SEQ ID NO: 90; Light chain: SEQ ID NO: 142), more than 1,000 Dual-Fab variants were generated using H183L072 as a template by introducing single or multiple mutations on variable region. Antibodies were expressed using Expi293 (Invitrogen) and purified by Protein A purification followed by gel filtration, when gel filtration was necessary. The sequences of 15 represented variants with multiple mutations are listed in Table 1 and binding kinetics were evaluated at 25 degrees C and/or 37 degrees C using Biacore T200 instrument (GE Healthcare) as described below in the Example 2.2.2.

(Table 1)

| variant name | VH name | VL name | SEQ ID NOs | | | | | | | |
|---|---|---|---|---|---|---|---|---|---|---|
| | | | VH | H-CDR1 | H-CDR2 | H-CDR3 | VL | L-CDR1 | L-CDR2 | L-CDR3 |
| H183/L072 | dBBDu183H | dBBDu072L | 90 | 103 | 116 | 129 | 142 | 147 | 152 | 157 |
| H0868L0581 | dBBDu183H0868 | dBBDu072L0581 | 91 | 104 | 117 | 130 | 143 | 148 | 153 | 158 |
| H1550L0918 | dBBDu183H1550 | dBBDu072L0918 | 92 | 105 | 118 | 131 | 144 | 149 | 154 | 159 |
| H1571L0581 | dBBDu183H1571 | dBBDu072L0581 | 93 | 106 | 119 | 132 | 143 | 148 | 153 | 158 |
| H1610L0581 | dBBDu183H1610 | dBBDu072L0581 | 94 | 107 | 120 | 133 | 143 | 148 | 153 | 158 |
| H1610L0939 | dBBDu183H1610 | dBBDu072L0939 | 94 | 107 | 120 | 133 | 145 | 150 | 155 | 160 |
| H1643L0581 | dBBDu183H1643 | dBBDu072L0581 | 95 | 108 | 121 | 134 | 143 | 148 | 153 | 158 |
| H1647L0581 | dBBDu183H1647 | dBBDu072L0581 | 96 | 109 | 122 | 135 | 143 | 148 | 153 | 158 |
| H1649L0581 | dBBDu183H1649 | dBBDu072L0581 | 97 | 110 | 123 | 136 | 143 | 148 | 153 | 158 |
| H1649L0943 | dBBDu183H1649 | dBBDu072L0943 | 97 | 110 | 123 | 136 | 146 | 151 | 156 | 161 |
| H1651L0581 | dBBDu183H1651 | dBBDu072L0581 | 98 | 111 | 124 | 137 | 143 | 148 | 153 | 158 |
| H1652L0943 | dBBDu183H1652 | dBBDu072L0943 | 99 | 112 | 125 | 138 | 146 | 151 | 156 | 161 |
| H1673L0943 | dBBDu183H1673 | dBBDu072L0943 | 100 | 113 | 126 | 139 | 146 | 151 | 156 | 161 |
| H1673L0581 | dBBDu183H1673 | dBBDu072L0581 | 100 | 113 | 126 | 139 | 143 | 148 | 153 | 158 |
| H2591L0581 | dBBDu183H2591 | dBBDu072L0581 | 101 | 114 | 127 | 140 | 143 | 148 | 153 | 158 |
| H2594L0581 | dBBDu183H2594 | dBBDu072L0581 | 102 | 115 | 128 | 141 | 143 | 148 | 153 | 158 |
| CD3ε | CD3εVH | CD3εVL | 162 | | | | 163 | | | |
| CD137 | CD137VH | CD137VL | 164 | | | | 165 | | | |

2.2. Binding kinetics information of affinity matured variants

2.2.1 Expression and purification of human CD3 and CD137

The gamma and epsilon subunits of the human CD3 complex (human CD3eg linker) were linked by a 29-mer linker and a Flag-tag was fused to the C-terminal end of the gamma subunit (SEQ ID NO: 169). This construct was expressed transiently using FreeStyle293F cell line (Thermo Fisher). Conditioned media expressing human CD3eg linker was concentrated using a column packed with Q HP resins (GE healthcare) and then applied to FLAG-tag affinity chromatography. Fractions containing human CD3eg linker were collected and subsequently subjected to a Superdex 200 gel filtration column (GE healthcare) equilibrated with 1x D-PBS. Fractions containing human CD3eg linker were then pooled and stored at -80 degrees C.

Human CD137 extracellular domain (ECD) (SEQ ID NO: 179) with hexahistidine (His-tag) and biotin acceptor peptide (BAP) on its C-terminus was expressed transiently using FreeStyle293F cell line (Thermo Fisher). Conditioned media expressing human CD137 ECD was applied to a HisTrap HP column (GE healthcare) and eluted with buffer containing imidazole (Nacalai). Fractions containing human CD137 ECD were collected and subsequently subjected to a Superdex 200 gel filtration column (GE healthcare) equilibrated with 1x D-PBS. Fractions containing human CD137 ECD were then pooled and stored at -80 degrees C.

2.2.2 Affinity measurement towards human CD3 and CD137

Binding affinity of Dual-Fab antibodies (Dual-Ig) to human CD3 were assessed at 25 degrees C using Biacore T200 instrument (GE Healthcare). Anti-human Fc (GE Healthcare) was immobilized onto all flow cells of a CM4 sensor chip using amine coupling kit (GE Healthcare). Antibodies were captured onto the anti-Fc sensor surfaces, then recombinant human CD3 or CD137 was injected over the flow cell. All antibodies and analytes were prepared in ACES pH 7.4 containing 20 mM ACES, 150 mM NaCl, 0.05% Tween 20, and 0.005% NaN3. Sensor surface was regenerated each cycle with 3M $MgCl_2$. Binding affinity were determined by processing and fitting the data to 1: 1 binding model using Biacore T200 Evaluation software, version 2.0 (GE Healthcare). CD137 binding affinity assay was conducted in same condition except assay temperature was set at 37 degrees C. Binding affinity of Dual-Fab antibodies to recombinant human CD3 and CD137 are shown in Table 2 (the expression E used to express the $K_{on}$, $K_{off}$, and KD values in the table means " 10 to the power of" and, for instance, 3.54E+04 = $3.54*10^4$).

(Table 2)

| | CD3 | | | CD137 | | |
|---|---|---|---|---|---|---|
| | Kon (M-1·s-1) | Koff (s-1) | KD (M) | Kon (M-1·s-1) | Koff (s-1) | KD (M) |
| H183L072 | 3.54E+04 | 1.20E-02 | 3.40E-07 | 3.47E+03 | 1.96E-02 | 5.66E-06 |
| H0868L0581 | 1.23E+05 | 1.94E-02 | 1.57E-07 | 1.22E+04 | 1.36E-03 | 1.11E-07 |
| H1550L0918 | 7.20E+04 | 3.16E-03 | 4.38E-08 | 1.09E+04 | 5.79E-03 | 5.30E-07 |
| H1571L0581 | 1.42E+05 | 1.56E-02 | 1.10E-07 | 1.21E+04 | 1.05E-03 | 8.68E-08 |
| H1610L0581 | 6.80E+04 | 1.42E-03 | 2.09E-08 | 1.07E+04 | 1.10E-03 | 1.03E-07 |
| H1610L0939 | 5.00E+04 | 2.53E-03 | 5.07E-08 | 1.30E+04 | 8.01E-04 | 6.18E-08 |
| H1643L0581 | 9.46E+04 | 2.51E-02 | 2.65E-07 | 1.23E+04 | 6.06E-04 | 4.94E-08 |
| H1647L0581 | 4.43E+04 | 1.01E-01 | 2.28E-06 | 9.98E+03 | 6.47E-04 | 6.48E-08 |
| H1649L0581 | 7.50E+04 | 3.36E-02 | 4.49E-07 | 1.29E+04 | 5.53E-04 | 4.28E-08 |
| H1649L0943 | 6.10E+04 | 4.81E-02 | 7.89E-07 | 1.43E+04 | 4.68E-04 | 3.28E-08 |
| H1651L0581 | 7.18E+04 | 3.71E-02 | 5.17E-07 | 1.40E+04 | 6.03E-04 | 4.32E-08 |
| H1652L0943 | 6.23E+04 | 6.36E-02 | 1.02E-06 | 1.29E+04 | 4.70E-04 | 3.64E-08 |
| H1673L0581 | 7.96E+04 | 1.06E-03 | 1.33E-08 | 1.19E+04 | 9.60E-04 | 8.04E-08 |
| H1673L0943 | 5.50E+04 | 1.16E-03 | 2.10E-08 | 1.22E+04 | 7.22E-04 | 5.91E-08 |
| H2591L0581 | 1.02E+05 | 5.35E-02 | 5.25E-07 | 2.04E+04 | 7.42E-04 | 3.63E-08 |
| H2594L0581 | 9.83E+04 | 5.84E-02 | 5.93E-07 | 2.09E+04 | 1.63E-03 | 7.81E-08 |

[Example 3] X-ray crystal structure analysis of H0868L0581/hCD137 complex

3.1. Preparation of antibody for co-crystal analysis

H0868L581 was selected for co-crystal analysis with hCD137 protein. The bivalent antibody was transiently transfected and expressed using an Expi293 Expression system (Thermo Fisher Scientific). Culture supernatants were harvested and antibodies were purified from the supernatants using MabSelect SuRe affinity chromatography (GE Healthcare) followed by gel filtration chromatography using Superdex200 (GE Healthcare).

3.2. Expression and purification of extracellular domain (24-186) of human CD137

Extracellular domain of human CD137 fused to Fc via Factor Xa cleavable linker (CD137-FFc, SEQ ID NO: 166) was expressed in the HEK293 Cell in the presence of kifunensine. The CD137-FFc from culture medium was purified by affinity chromatography (HiTrap MabSelect SuRe column, GE Healthcare) and size exclusion chromatography (HiLoad 16/600 Superdex 200 pg column, GE healthcare). Fc was cleaved with Factor Xa and the resultant CD137 extracellular domain was further purified with tandemly connected gel filtration column (HiLoad 16/600 Superdex 200 pg, GE healthcare) and Protein A column (HiTrap MabSelect SuRe 1ml, GE Healthcare) and subsequently purified using Benzamidine Sepharose resin (GE Healthcare). Fractions containing CD137 extracellular domain were pooled and stored at -80 degrees C.

3.3. Preparation of Fab fragment of H0868L0581 and anti-CD137 control antibody

Antibodies for crystal structure analysis were transiently transfected and expressed using an Expi293 Expression system (Thermo Fisher Scientific). Culture supernatants were harvested and antibodies were purified from the supernatants using MabSelect SuRe affinity chromatography (GE Healthcare) followed by gel filtration chromatography using Superdex200 (GE Healthcare). Fab fragments of H0868L0581 and known anti-CD137 control antibody (called as 137Ctrl hereafter, Heavy chain SEQ ID NO: 167, Light chain SEQ ID NO: 168) were prepared by the conventional method using limited digestion with Lys-C (Roche), followed by loading onto a protein A column (MabSlect SuRe, GE Healthcare) to remove Fc fragments, a cation exchange column (HiTrap SP HP, GE Healthcare), and a gel filtration column (Superdex200 16/60, GE Healthcare). Fractions containing Fab fragment were pooled and stored at - 80 degrees C.

3.4. Preparation of H0868L0581 Fab, 137Ctrl and human CD137 complex

Purified CD137 was mixed with GST-tag fused Endoglycosidase F1(in-house) for deglycosylation, followed by purification of CD137 using gel filtration column (HiLoad 16/600 Superdex 200 pg, GE healthcare) and Protein A column (HiTrap MabSelect SuRe 1ml, GE Healthcare). Purified CD137 was mixed with H0868L0581 Fab. The complex was purified by gel filtration column (Superdex 200 Increase 10/300 GL, GE healthcare) and subsequently purified H0868L0581 Fab and CD137 complex was mixed with 137Ctrl. The ternary complex was purified by gel filtration chromatography (Superdex200 10/300 increase, GE Healthcare) using a column equilibrated with 25 mM HEPES pH 7.3, 100 mM NaCl.

3.5. Crystallization

The purified complexes were concentrated to about 10 mg/mL, and crystallization was carried out by the sitting drop vapor diffusion method at 21 degrees C. The reservoir solution consisted of 0.1M Tris hydrochloride pH8.5, 25.0% v/v Polyethylene glycol monomethyl ether 550.

3.6. Data collection and structure determination

X-ray diffraction data were measured by X06SA at SLS. During the measurement, the crystal was constantly placed in a nitrogen stream at -178 degrees C to maintain it in a frozen state, and a total of 1440 X-ray diffraction images were collected using an Eiger X16M (DECTRIS) attached to a beam line, while rotating the crystal 0.25 degrees at a time. Determining the cell parameters, indexing the diffraction spots, and processing the diffraction data obtained from the diffraction images were performed using the autoPROC program (Acta. Cryst. 2011, D67: 293-302), XDS Package (Acta. Cryst. 2010, D66: 125-132), and AIMLESS (Acta. Cryst. 2013, D69: 1204-1214), and finally the diffraction intensity data up to 3.705 angstrom resolution was obtained. The crystallography data statistics are shown in Table 3.

The structure was determined by molecular replacement with the program Phaser (J. Appl. Cryst. 2007, 40: 658-674). The search model was derived from the published crystal structure (PDB code: 4NKI and 6MI2). A model was built with the Coot program (Acta Cryst. 2010, D66: 486-501) and refined with the program Refmac5 (Acta Cryst. 2011, D67: 355-367) and PHENIX (Acta Cryst. 2010, D66: 213-221). The crystallographic reliability factor (R) for the diffraction intensity data from 77.585-3.705 angstrom was 22.33 %, with a Free R value of 27.50%. The structure refinement statistics are shown in Table 3.

(Table 3)

X-ray data collection and refinement statistics

| **Data collection** | | |
|---|---|---|
| Space group | | *C*2 |
| Unit Cell | | |
| | *a,b,c* (Å) | 233.795, 74.019, 81.986 |
| | α,β,γ (°) | 90.000, 108.858, 90.000 |
| Resolution (Å) | | 77.585-3.705 |
| Total reflections | | 99,488 |
| Unique reflections | | 14,221 |
| Completeness (highest resolution shell) (%) | | 99.2 (99.7) |
| $R_{merge}$ [a] (highest resolution shell) | | 0.161 (1.052) |
| **Refinement** | | |
| Resolution (Å) | | 48.822-3.705 |
| Reflections | | 14,195 |
| $R$ factor [b] ($R_{free}$ [c]) (%) | | 22.33 (27.50) |
| rms deviation from ideal | | |
| | Bond lengths (Å) | 0.003 |
| | Bond angles (°) | 0.618 |

a; $R_{merge} = \sum hkl \sum j|Ij\ (hkl) - \langle I(hkl)\rangle\ |/\sum hkl \sum j|Ij\ (hkl)|$, where $Ij\ (hkl)$ and $\langle I\ (hkl)\rangle$ are the intensity of measurement j and the mean intensity for the reflection with indices *hkl,* respectively.

b; $R$ factor $= \sum hkl|F_{calc}(hkl)| - |F_{obs}\ (hkl)|/\sum hkl|F_{obs}\ (hkl)|$, where $F_{obs}$ and $F_{calc}$ are the observed and calculated structure factor amplitudes, respectively.

c; $R_{free}$ is calculated with 5% of the reflection randomly set aside.

3.7. Identification of the interaction sites of H0868L0581 Fab and CD137

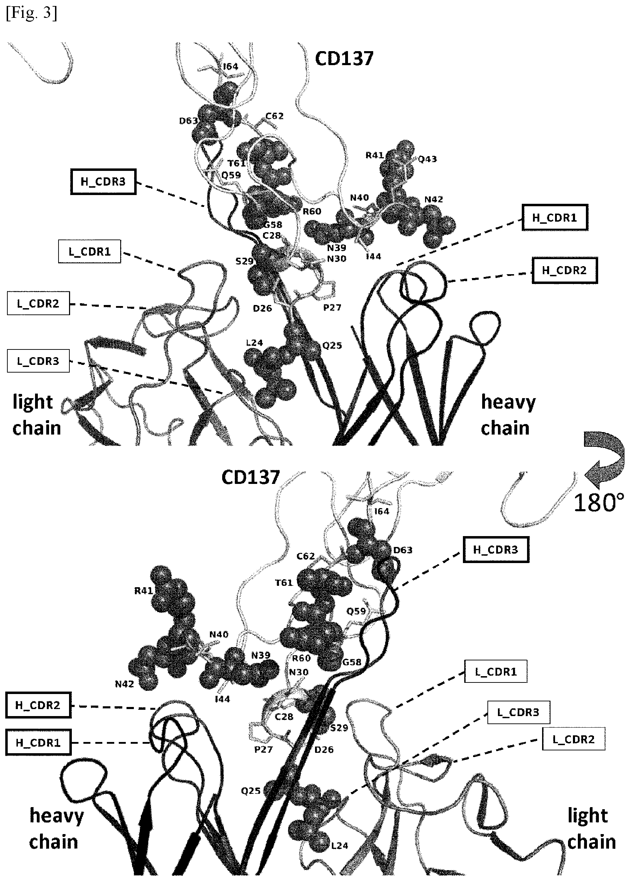

The crystal structure of the ternary complex of H0868L0581 Fab, 137Ctrl and CD137 was determined at 3.705 angstrom resolution. In Figures 2 and 3, the epitope of the H0868L0581 Fab contact region is mapped in the CD137 amino acid sequence and in the crystal structure, respectively. The epitope includes the amino acid residues of CD137 that contain one or more atoms located within 4.5 angstrom distance from any part of the H0868L0581 Fab in the crystal structure. In addition, the epitope within 3.0 angstrom is highlighted in Figures 2 and 3.

As shown in Figures 2 and 3, the crystal structure showed that the L24-N30 in CRD1 of CD137 bound in a pocket formed between Heavy chain and Light chain of H0868L0581 Fab, particularly L24-S29 were deeply buried in a manner that the N-terminus of CD137 was oriented toward the depth of the pocket. In addition, N39-I44 in CRD1 and G58-164 in CRD2 in CD137 were recognized by Heavy chain CDRs of H0868L0581 Fab. CRD is the name of domains divided by the structure formed by Cys-Cys called CRD reference as described in WO2015/156268.

We identified an anti-human CD137 antibody which recognizes the N-terminus region, especially L24-N30, of human CD137, and also identified that the antibody against this region can activate CD137 on cells.

[Example 3] Generation of anti-CLDN6/Dual-Fab tri-specific antibody

Tri-specific antibodies with one arm targeting claudin-6 and the other arm with dual targeting function to CD3 and CD137 were generated by utilizing FAST-Ig (WO2013065708) or CrossMab technology (Figure 4). The target antigen of each Fv region in the tri-specific antibodies was shown in Table 4. The naming rule of each of chain was shown in Figure 4, and the SEQ ID NOs are shown in Table 5. Sequence of each variable region is shown in Table 6.

Fc region was Fc gamma R silent and deglycosylated. FcRn enhanced mutations Act5 (M428L, N434A, Q438R, S440E) were applied to improve PK of antibodies. The engineered components applied to each antibody were shown in Tables 7-1 and 7-2, with the details of FAST06, FAST22 and FAST30 shown in Table 8. All antibodies were expressed as tri-specific form by transient expression in Expi293 cells (Invitrogen) and purified according to Reference Example 1.

(Table 4)

| Antibody names and Fv names | | | | |
|---|---|---|---|---|
| Antibody ID | Antibody name | Antibody format | Fv A (anti-CLDN6) | Fv B (Dual Fab) |
| Ab01 | CLDN6AE25EK/DualAE05KE-SG13251326 | Fast-Ig | CLDN6AE25EK | DualAE05KE |
| Ab02 | CLDN6AE25EK/DualAE15KE-SG13251326 | Fast-Ig | CLDN6AE25EK | DualAE15KE |
| Ab03 | CLDN6AE25/DualAE05-SG14001326 | Fast-Ig | CLDN6AE25 | DualAE05 |
| Ab04 | CLDN6AE25/DualAE15-SG14001326 | Fast-Ig | CLDN6AE25 | DualAE15 |
| Ab05 | xCLDN6AE25/DualAE05-xSG1386k1385hV11 | CrossMab | xCLDN6AE25 | DualAE05 |
| Ab06 | xCLDN6AE25/DualAE15-xSG1386kl385hV11 | CrossMab | xCLDN6AE25 | DualAE15 |
| Ab07 | CLDN6AE25EK/DualAE05KE-SG 14051406 | Fast-Ig | CLDN6AE25EK | DualAE05KE |
| Ab08 | CLDN6AE25EK/DualAE15KE-SG14051406 | Fast-Ig | CLDN6AE25EK | DualAE15KE |
| Ab09 | CLDN6AE25/DualAE05-SG14071406 | Fast-Ig | CLDN6AE25 | DualAE05 |
| Ab10 | CLDN6AE25/DualAE15-SG14071406 | Fast-Ig | CLDN6AE25 | DualAE15 |
| Ab11 | xCLDN6AE25/DualAE05-xSG1384k1383hV11 | CrossMab | xCLDN6AE25 | DualAE05 |
| Ab12 | xCLDN6AE25/DualAE15-xSG1384k1383hV11 | CrossMab | xCLDN6AE25 | DualAE15 |
| Ab13 | CLDN6AE25/DualAE05-SG13251326 | Fast-Ig | CLDN6AE25 | DualAE05 |
| Ab14 | CLDN6AE25/DualAE 5-SG13251326 | Fast-Ig | CLDN6AE25 | DualAE15 |
| Ab15 | CLDN6AE25/DualAE05-SG14051406 | Fast-Ig | CLDN6AE25 | DualAE05 |
| Ab16 | CLDN6AE25/DualAE15-SG14051406 | Fast-Ig | CLDN6AE25 | DualAE15 |
| Ab17 | xCLDN6AE25/DualAE05-xSG1350k1349hV11 | CrossMab | xCLDN6AE25 | DualAE05 |
| Ab18 | xCLDN6AE25/DualAE15-xSG1350k1349hV11 | CrossMab | xCLDN6AE25 | DualAE15 |

(Table 5)

| Antibody names and SEQ ID Nos | | | | | |
|---|---|---|---|---|---|
| | | SEQ ID NOs | | | |
| Antibody ID | Antibody name | Chain 1 | Chain 2 | Chain 3 | Chain 4 |
| Ab01 | CLDN6AE25EK/DualAE05KE-SG 13251326 | 41 | 50 | 54 | 68 |
| Ab02 | CLDN6AE25EK/DualAE15KE-SG13251326 | 41 | 50 | 55 | 68 |
| Ab03 | CLDN6AE25/DualAE05-SG14001326 | 42 | 51 | 56 | 69 |
| Ab04 | CLDN6AE25/DualAE15-SG14001326 | 42 | 51 | 57 | 69 |
| Ab05 | xCLDN6AE25/DualAE05-xSG1386k1385hV11 | 44 | 52 | 60 | 70 |
| Ab06 | xCLDN6AE25/DualAE15-xSG1386k1385hV11 | 44 | 52 | 61 | 70 |
| Ab07 | CLDN6AE25EK/DualAE05KE-SG14051406 | 45 | 50 | 62 | 68 |
| Ab08 | CLDN6AE25EK/DualAE15KE-SG14051406 | 45 | 50 | 63 | 68 |
| Ab09 | CLDN6AE25/DualAE05-SG14071406 | 46 | 51 | 64 | 69 |

(continued)

| Antibody names and SEQ ID Nos | | | | | | |
|---|---|---|---|---|---|---|
| | | SEQ ID NOs | | | |
| Antibody ID | Antibody name | Chain 1 | Chain 2 | Chain 3 | Chain 4 |
| Ab10 | CLDN6AE25/DualAE15-SG14071406 | 46 | 51 | 65 | 69 |
| Ab11 | xCLDN6AE25/DualAE05-xSG1384k1383hV11 | 47 | 52 | 66 | 70 |
| Ab12 | xCLDN6AE25/DualAE15-xSG1384k1383hV11 | 47 | 52 | 67 | 70 |
| Ab13 | CLDN6AE25/DualAE05-SG13251326 | 48 | 53 | 56 | 71 |
| Ab14 | CLDN6AE25/DualAE15-SG13251326 | 48 | 53 | 57 | 71 |
| Ab15 | CLDN6AE25/DualAE05-SG14051406 | 49 | 53 | 64 | 71 |
| Ab16 | CLDN6AE25/DualAE15-SG14051406 | 49 | 53 | 65 | 71 |
| Ab17 | xCLDN6AE25/DualAE05-xSG1350k1349hV11 | 43 | 52 | 58 | 70 |
| Ab18 | xCLDN6AE25/DualAE15-xSG1350k1349hV11 | 43 | 52 | 59 | 70 |

(Table 6)

| Fv names, VH, VL and their CDR1-3 SEQ ID NOs | | | | | | | | | | |
|---|---|---|---|---|---|---|---|---|---|---|
| | | | SEQ ID NOs | | | | | | | |
| | | | Variable region in the same polypeptide chain with heavy chain constant region | | | | Variable region in the same polypeptide chain with light chain constant region | | | |
| Fv name | Variable region in the same polypeptide chain with heavy chain constant region | Variable region in the same polypeptide chain with light chain constant region | Variable region | CDR1 | CDR2 | CDR3 | Variable region | CDR1 | CDR2 | CDR3 |
| CLDN6AE25EK | 55HQ39E | 54L0532Q38K | 2 | 8 | 14 | 20 | 26 | 30 | 34 | 38 |
| CLDN6AE25 | 65H | 54L0532 | 1 | 7 | 13 | 19 | 25 | 29 | 33 | 37 |
| xCLDN6AE25 | 54L0532 | 65H | 25 | 29 | 33 | 37 | 1 | 7 | 13 | 19 |
| DualAE05KE | dBBDu183H1643.Q39K | dBBDu072L0581.Q38E | 3 | 9 | 15 | 21 | 27 | 31 | 35 | 39 |
| DualAE15KE | dBBDu183H2594.Q39K | dBBDu072L0581.Q38E | 4 | 10 | 16 | 22 | 27 | 31 | 35 | 39 |
| DualAE05 | dBBDu183H1643 | dBBDu072L0581 | 5 | 11 | 17 | 23 | 28 | 32 | 36 | 40 |
| DualAE15 | dBBDu183H2594 | dBBDu072L0581 | 5 | 12 | 18 | 24 | 28 | 32 | 36 | 40 |

(Table 7-1)

| Engineered components applied to each antibody | | | | |
|---|---|---|---|---|
| Antibody ID | Antibody name | FcRn binding enhancement | H/L pairing | Heavy Chain Paring |
| Ab01 | CLDN6AE25EK/DualAE05KE-SG13251326 | | FAST22 | S3 |
| Ab02 | CLDN6AE25EK/DualAE15KE-SG13251326 | | FAST22 | S3 |
| Ab03 | CLDN6AE25/DualAE05-SG14001326 | | FAST6 | S3 |
| Ab04 | CLDN6AE25/DualAE15-SG14001326 | | FAST6 | S3 |
| Ab05 | xCLDN6AE25/DualAE05-xSG1386k1385hV11 | | Cross-mab | Knob into Hole |
| Ab06 | xCLDN6AE25/DualAE15-xSG1386k1385hV11 | | Cross-mab | Knob into Hole |
| Ab07 | CLDN6AE25EK/DualAE05KE-SG14051406 | ActS (+) | FAST22 | S3 |
| Ab08 | CLDN6AE25EK/DualAE15KE-SG14051406 | ActS (+) | FAST22 | S3 |
| Ab09 | CLDN6AE25/DualAE05-SG14071406 | ActS (+) | FAST6 | S3 |
| Ab10 | CLDN6AE25/DualAE15-SG14071406 | Act5 (+) | FAST6 | S3 |
| Ab11 | xCLDN6AE25/DualAE05-xSG1384k1383hV11 | Act5 (+) | Cross-mab | Knob into Hole |
| Ab12 | xCLDN6AE25/DualAE15-xSG1384k1383hV11 | Act5 (+) | Cross-mab | Knob into Hole |
| Ab13 | CLDN6AE25/DualAE05-SG13251326 | | FAST30 | S3 |
| Ab14 | CLDN6AE25/DualAE15-SG13251326 | | FAST30 | S3 |
| Ab15 | CLDN6AE25/DualAE05-SG14051406 | Act5 (+) | FAST30 | S3 |

(Table 7-2)

| Continuation of Table 7-1 | | | | |
|---|---|---|---|---|
| Antibody ID | Antibody name | FcRn binding enhancement | H/L pairing | Heavy Chain Paring |
| Ab16 | CLDN6AE25/DualAE15-SG14051406 | Act5 (+) | FAST30 | S3 |
| Ab17 | xCLDN6AE25/DualAE05-xSG1350k1349hV11 | | Cross-mab | Knob into Hole |
| Ab18 | xCLDN6AE25/DualAE15-xSG1350k1349hV11 | | Cross-mab | Knob into Hole |

(Table 8)

| Details of FAST-Ig engineering in variable regions | | | | | | | | | | | | | | | | | | |
|---|---|---|---|---|---|---|---|---|---|---|---|---|---|---|---|---|---|---|
| No. | Dual Fab side (chain 3 and chain 4) | | | | | | | | | anti-CLDN6 side (chain 1 and chain 2) | | | | | | | | |
| | Heavy chain (chain 3) | | | | Light chain (chain 4) | | | | | Heavy chain (chain 1) | | | | Light chain (chain 2) | | | | |
| | Q39 | K147 | Q175 | K213 | Q38 | E123 | S131 | Q160 | T180 | Q39 | K147 | Q175 | K213 | Q38 | E123 | S131 | Q160 | T180 |
| FAST06 | - | - | K | - | - | - | E | E | | | E | E | | | | K | K | |
| FAST22 | K | - | K | - | E | - | E | E | E | E | E | E | E | K | K | K | K | |
| FAST30 | | - | K | - | | - | E | E | E | | E | E | E | | K | K | K | |

[Example 5] FACS analysis and specificity against CLDN family

Amino acid sequences are highly conserved among CLDN3, CLDN4, CLDN6 and CLDN9. Thus we examined CLDN6 binding specificity of CLDN6 binding Fv comprising 65HQ39E as VH and 54L0532Q38K as VL, by FACS analysis. hCLDN6BaF, hCLDN3/BaF, hCLDN4/BaF, and hCLDN9BaF were incubated with an anti-CLDN6/CD3 bispecific antibody (CS2961) comprising the CLDN6 binding Fv CLDN6AE25EK and a CD3 binding Fv (heavy chain variable region SEQ ID NO: 184, light chain variable region SEQ ID NO: 185) at 15 micro g/ml. Another anti-CLDN6/CD3 bispecific antibody (6PHU3/TR01) and an antibody without binding capability to CLDN6 (KLH/TR01) were used as a staining control. 6PHU3/TR01 and KLH/TR01 comprise the same CD3 binding Fv (heavy chain variable region SEQ ID No: 188, light chain variable region SEQ ID NO: 189). The CLDN6 binding Fv of 6PHU3/TR01 comprises a heavy chain variable region shown in SEQ ID NO: 190 and a light chain variable region shown in SEQ ID NO: 191. KLH/TR01 comprises a KLH binding Fv (heavy chain variable region SEQ ID NO: 186, light chain variable region SEQ ID NO: 187).

Binding of each antibody was detected with Alexa Fluor 488-conjugated anti human IgG (Invitrogen). Dead cells were separated by eFlour 780 (Invitrogen) staining.

As shown in Figure 5, CS2961 showed better specificity towards CLDN6 compared with 6PHU3/TR01.

[Example 6] Measurement of T-cell-dependent cell cytotoxicity of anti-CLDN6/CD3 bispecific antibodies and anti-CLDN6/Dual-Fab tri-specific antibodies

Figure 6 shows the T-cell-dependent cell cytotoxicity of an anti-CLDN6/CD3 bispecific antibody (CS3348) and five anti-CLDN6/Dual-Fab tri-specific antibodies (PPU4134, PPU4135, PPU4136, PPU4137, and PPU4138) against NIH:OVCAR-3 (high CLDN6 expression ovarian cancer cell line), and A2780 and COV413A (low CLDN6 expression ovarian cancer cell lines). Sequences of the antibodies are shown in Table 9.

(Table 9)

| Antibody name | Other name | SEQ ID NOs: | | | |
|---|---|---|---|---|---|
| | | Chain 1 | Chain 2 | Chain 3 | Chain 4 |
| CS3348 | - | 193 | 195 | 194 | 192 |
| PPU4134 | Ab01 | 41 | 50 | 54 | 68 |
| PPU4135 | Ab03 | 42 | 51 | 56 | 69 |
| PPU4136 | Ab04 | 42 | 51 | 57 | 69 |
| PPU4137 | Ab17 | 43 | 52 | 58 | 70 |
| PPU4138 | Ab18 | 43 | 52 | 59 | 70 |

Cell cytotoxicity was evaluated by LDH assay using human PBMCs. 15,000 target cells and 150,000 human PBMCs (E/T = 10) were seeded into each well of a 96-well U-bottom plate and incubated with various concentrations of antibody for overnight at 37 degree C and 5% $CO_2$. Target cell killing was measured by LDH cytotoxicity detection kit (Takara Bio). The cytotoxic activity (%) of each antibody was calculated using the following formula.

$$\text{Cytotoxic activity (\%)} = (A - B - C) \times 100 / (D - C)$$

"A" represents the average absorbance value of wells treated with antibody and PBMCs, "B" represents the average absorbance value of wells with effector cell PBMCs only, "C" represents the average absorbance value of wells with untreated target cells only, and "D" represents the average absorbance value of wells with target cells lysed with Triton-X. Further, the cytotoxicity calculated in a well containing PBMCs and target cells without antibody was set to 0%. All the anti-CLDN6/Dual-Fab tri-specific antibodies showed T-cell-dependent cell cytotoxicity against CLDN6 expressing cells.

[Example 7] Generation of CD137/CD3 double humanized mouse

Human CD137 knock-in (KI) mouse strain was generated by replacing mouse endogenous Cd137 genomic region with human CD137 genomic sequence using mouse embryonic stem cells. Human CD3 EDG-replaced mouse was established as a strain in which all three components of the CD3 complex -- CD3e, CD3d, and CD3g -- are replaced with their human counterparts, CD3E, CD3D, and CD3G (Scientific Rep. 2018; 8: 46960). CD137/CD3 double humanized mouse strain was established by crossbreeding the human CD137 KI mice with the human CD3 EDG-replaced mice.

[Example 8] Assessment of in vivo efficacy of Anti-CLDN6/Dual-Fab Tri-specific antibodies with hCD3/hCD137 mice

Antibodies prepared in Example 4 are evaluated for their in vivo efficacy using tumor-bearing models.

For in vivo efficacy evaluation, CD3/CD137 double humanized mice established in Example 6, which is called as "hCD3/hCD137 mice" hereafter, are used. Cells which have stable expression of human CLDN6 are transplanted into the hCD3/hCD137 mice, and the hCD3/hCD137 mice with confirmed tumor formation are treated by administration of the antibodies.

More specifically, in drug efficacy tests of the antibodies using tumor-bearing models, the tests below are performed. CLDN6 expressing cells ($1x10^6$ cells) are transplanted into the inguinal subcutaneous region of hCD3/hCD137 mice. The day of transplantation is defined as day 0. On day 9 after transplantation, the mice are randomized into groups according to their body weight and tumor size. On the day of randomization, the antibodies are administered intravenously through the caudate vein at 6 mg/kg. The antibodies are administered only once. Tumor volume and body weight are measured with anti-tumor testing system (ANTES version 7.0.0.0) every 3-4 days.

In another in vivo efficacy evaluation, CLDN6 expressing cells are transplanted into the right flank of hCD3/hCD137 mice. On day 9, the mice are randomized into groups on the basis of their tumor volume and body weight, and injected i.v. with vehicle or antibodies prepared in Example 3. Tumor volume is measured twice per week. For IL-6 analysis, mice are bled at 2h after treatment. Plasma samples are analyzed with Bio-Plex Pro Mouse Cytokine Th1 Panel according to the manufacture's protocol.

[Example 9] In vitro assay of cytotoxic activity using lactate dehydrogenase release assay.

Cytotoxic activity of anti-CLDN6/Dual-Fab tri-specific antibody PPU4135 was evaluated by lactate dehydrogenase (LDH) release assay.
Human gastric cancer cell line NUGC-3 (JCRB), human teratocarcinoma cell line PA-1 (ATCC), human uterus cancer cell line SNG-M (JCRB), human testicular germ cell tumor cell line NEC8 (JCRB), human yolk-sac tumor cell line NEC14 (JCRB), human atypical teratoid rhabdoid tumor cell line CHLA-02-ATRT (ATCC), human urinary bladder cancer cell line HT-1197 (ATCC), and human large bowel cancer cell line OUMS-23 (JCRB), which express human CLDN6, were used as target cells.

After frozen PBMCs (CTL) were washed by CTL anti-aggregate wash and RPMI-1640 Medium (SIGMA) containing 10% FBS (called as 10%FBS/RPMI), the PBMCs were adjusted to $3 \times 10^6$ cells / mL. These PBMCs were used as effector cells. Target cells were detached from culture flask and seeded at 100 micro L/well containing $1.5 \times 10^4$ cells on u-bottom clear 96-wells plate (Corning). 50 micro L of human PMBC solution ($1.5 \times 10^5$ cells) and 50 micro L of the prepared antibody at a concentration selected from 0.004, 0.04, 0.4, 4, or 40 nM were added into wells respectively. After overnight incubation at 37 degree C, plate was centrifuged and 100 micro L culture supernatant from each well was transferred to a new flat bottom clear 96-wells plate. Then 100 micro L of LDH detection reagent (Dye solution containing catalyst, TaKaRa) was added to each well, followed by 30 minutes incubation at room temperature. Absorbance at 490 nm and 620 nm was measured by EnVision (PerkinElmer Japan).

The rate of cytotoxic activity (%) was calculated from the difference between 490 nm and 620 nm absorbance according to following formula.

$$\text{Cytotoxic activity (\%)} = (A - B - C) \times 100 / (D - C)$$

"A" represents the average absorbance value of wells treated with antibody and PBMCs, "B" represents the average absorbance value of wells with effector cell PBMCs only, "C" represents the average absorbance value of wells with untreated target cells only, and "D" represents the average absorbance value of wells with target cells lysed with Triton-X. The average absorbance value of culture medium wells was subtracted from all the absorbance values. Further, the cytotoxicity calculated in a well containing PBMCs and target cells without antibody was set to 0%. The anti-CLDN6/Dual-Fab tri-specific antibodies showed T-cell-dependent cell cytotoxicity against all the cell lines used.

The results are shown in Figure 8.

[Example 10] Real-time cell growth inhibition assay (xCELLigence assay)

The T-cell dependent growth inhibition mediated by the anti-CLDN6/Dual-Fab tri-specific antibodies was assessed by cell proliferation assay using an xCELLigence RTCA MP instrument (ACEA Biosciences).

Human ovarian cancer cell line NIH:OVCAR-3 (ATCC) and human lung cancer cell line NCI-H1435 (ATCC), which express human CLDN6, were used as target cells.

50 mL of peripheral blood was collected from healthy adult volunteers by a syringe that had been previously injected with 500 micro L of 1,000 units / mL heparin solution (NovoNordisk). Peripheral blood equally divided into four equal parts by dilution with PBS (-) was injected with 15 mL of Ficoll-Paque PLUS and centrifuged in a Leucosep lymphocyte separation tube (Greiner Bio-One). After centrifuging the separation tube (2150 rpm for 10 minutes at room temperature), the peripheral blood mononuclear cell (hereinafter referred as PBMC) fraction layer was separated. After washing the PBMCs once with RPMI-1640 Medium (SIGMA) containing 10% FBS (called as 10%FBS/RPMI), the PBMCs were adjusted to 4 x $10^5$ cells / mL. These PBMCs were used as effector cells.

1 $\times$ $10^4$ target cells were plated on E-Plate 96 plate (Roche Diagnostics) at 100 micro L/well. After overnight culture, 2 x $10^4$ T cells together with the antibody at a concentration selected from 0.004, 0.04, 0.4, 4 or 40 nM were added at 50 micro L/well, respectively. Cell growth was monitored every 15 min, using xCELLigence, for 72 hours during the incubation of the plates. The rate of cell growth inhibition (CGI: %) was determined from the cell index value according to the formulation given as CGI (%) = 100 - ($CI_{Ab}$ $\times$ 100 / $CI_{NoAb}$). "$CI_{Ab}$" represents the cell index value of wells with antibody on a specific experimental time and "$CI_{NoAb}$" represents the average cell index value of the wells without antibody at the same experimental time.

The results show that all the anti-CLDN6/Dual-Fab tri-specific antibodies inhibited the cell growth of CLDN6 expressing cancer cell lines (OVCAR-3 and NCI-H1435) in a dose dependent manner.
The results are shown in Figure 9.

[Example 11] T cell Activation in NFAT-luc2 Jurkat Cell Lines Co-Cultured with CLDN6 Expressing Tumor Cells

T cell activation through CD3 binding by the anti-CLDN6/Dual-Fab tri-specific antibodies was measured by luciferase assay system using GloResponse NFAT-luc2 Jurkat cells (Promega, J1601) as effector cells. Human ovarian cancer cell line OVCAR-3(ATCC) and lung adenocarcinoma cell lines NCI-H1435 (ATCC) were used as a claudin-6 endogenously expressing cells. Human bladder cancer cell line 5637 (ATCC) was used as CLDN6 negative cells.

Assay was performed as below. First, the above-described cancer cell lines were detached from culture flask and plated at 25micro L/well (2 x $10^4$ cells) into white flat bottom 96-wells plate (Coster #3917). Next, 1 x $10^5$ Jurkat / NFAT-RE Reporter Cell Line together with the antibody at a concentration selected from 0.003, 0.03, 0.3, 3 or 30 nM were added at 25micro L/well, respectively. After overnight culture at 37 degree C, Bio-Glo reagent (Promega #G7941) was added at 75 micro L/well followed by further incubation at room temperature for 10 minutes. Then, luminescence arising from activating Jurkat cells was measured by EnSpire (PerkinElmer Japan). The luminescence fold of each well was calculated by making comparison between the wells with and without antibody.

The results of NFAT signal activation properties of anti-CLDN6/Dual-Fab tri-specific antibodies and CS3348 using CLDN6 expressing human cell lines (OVCAR3 and NCI-H1435) and CLDN6 negative cell line (5637) as target cells are shown in Figure 10.

In the presence of the claudin-6 positive cell lines, NFAT activation by all antibodies was observed in a dose dependent manner. On the other hand, almost no activation was observed even at the high concentration of antibody in the presence of the claudin-6 negative cell line 5637.

[Example 12] NF kappa B Activation in Jurkat Expressing Human 4-1BB and Luciferase Reporter Cell Lines Co-Cultured with CLDN6 Expressing Tumor Cells

NF kappa B activation through CD137 binding by the anti-CLDN6/Dual-Fab tri-specific antibodies was evaluated using the GloResponse™ NF kappa B luc2/4-1BB Jurkat (Promega, CS196004). Human ovarian cancer cell line OVCAR-3(ATCC) and lung adenocarcinoma cell line NCI-H1435 (ATCC) were used as a claudin-6 endogenously expressing cells. Human bladder cancer cell line 5637 (ATCC) was used as CLDN6 negative cells.

Assay was performed as below. First, the above- described cancer cell lines were detached from culture flask and plated at 25micro L/well (2.5 x $10^4$ cells) into white flat bottom 96-wells plate (Coster #3917). Next, 5 x $10^4$ cells of NF kappa B luc2/4-1BB Jurkat Reporter Cell Line were transferred, and 25ul medium containing titrated antibodies were mixed. Assay plates were incubated at 37 degree C for 6hrs, then Bio-Glo reagent (Promega #G7941) was added at 75micro L/well followed by further incubation at room temperature for 10 minutes. Then, luminescence arising from activating Jurkat cells was measured by EnVision (PerkinElmer Japan). The luminescence fold of each well was calculated by making comparison between the wells with each antibody (0.003, 0.03, 0.3, 3 and 30 nM) and without antibody.

The results of NF kappa B signal activation properties of anti-CLDN6/Dual-Fab tri-specific antibodies and CS3348 using CLDN6 expressing human cell lines (OVCAR3 and NCI-H1435) and CLDN6 negative cell line (5637) as target cells are shown in Figure 11.

In the presence of the claudin-6 positive cell lines, NF kappa B activation by all antibodies was observed in a dose dependent manner. Especially, much greater activation was observed in the presence of anti-CLDN6/Dual-Fab tri-specific antibodies. On the other hand, no activation was observed even at the high concentration of antibody in the presence of the claudin-6 negative cell line 5637.

[Example 13] In vivo anti-tumor efficacy study

The in vivo anti-tumor efficacy of the anti-CLDN6/Dual-Fab tri-specific antibodies was evaluated using tumor bearing mice model. The human cancer cell line expressing human CLDN6 (NCI-H1435 or OV-90) was transplanted subcutaneously into humanized NOG mice transplanted with human stem cell derived from cord blood (HuNOG mice model). Tumor bearing mice were randomized to treatment groups to receive an administration of the antibody, or vehicle as a control (Table 10).

After randomization and grouping the mice according to tumor size and body weight from day 8 (NCI-H1435) or day 16 (OV90) after transplantation, anti-CLDN6/Dual-Fab tri-specific antibodies were administered intravenously. The anti-CLDN6/Dual-Fab tri-specific antibodies were administered only once. The length (L) and width (W) of the tumor mass were measured, and tumor volume (TV) was calculated as: TV = (L x W x W) / 2.

Anti-tumor efficacy was observed in the anti-CLDN6/Dual-Fab tri-specific antibodies-administered groups compared with the vehicle-administered control group (Figures 12 and 13).

(Table 10)

| Details of study groups for in vivo anti-tumor efficacy evaluation a. Study groups using NCI-H1435/HuNOG mice model | | |
|---|---|---|
| Group | Antibody | Dose |
| 1 | vehicle | - |
| 2 | CS3348 | 1 mg/kg |
| 3 | PPU4134 | 1 mg/kg |
| 4 | PPU4135 | 1 mg/kg |
| 5 | PPU4136 | 1 mg/kg |
| 6 | PPU4137 | 1 mg/kg |
| 7 | PPU4138 | 1 mg/kg |
| b. Study groups using OV-90/HuNOG mice model | | |
| Group | Antibody | Dose |
| 1 | vehicle | - |
| 2 | CS3348 | 0.05 mg/kg |
| 3 | CS3348 | 0.2 mg/kg |
| 4 | PPU4135 | 0.05 mg/kg |
| 5 | PPU4135 | 0.2 mg/kg |

[Example 14] Toxicology study of the anti-CLDN6/Dual-Fab tri-specific antibody

Potential toxicity of PPU4135 antibody (anti-CLDN6/Dual-Fab tri-specific antibody) was evaluated in toxicity study using cynomolgus monkeys compared with CS3348 antibody (anti-CLDN6/CD3 bispecific antibody). Because both PPU4135 and CS3348 antibodies cross-reacted with their antigens of cynomolgus monkey, cynomolgus monkey was selected as the animal species for evaluations in the in vivo toxicology studies. Summary of single dose toxicology studies is shown in Table 11. Since toxicological findings seemed to be more sensitive in male than female animals in the toxicity study with CS3348 (Figure 14), 2 males were used for evaluation of the PPU4135-mediated toxicity. In these studies, dose levels were set at 100 (for CS3348) or 90 (for PPU4135) micro g/kg, which were approximately 2.57-fold efficacious concentration producing 80% of the maximal response.

(Table 11)

| Summary of Toxicology Studies | | | | |
|---|---|---|---|---|
| **Study Type** | **Species** | **Treatment/ Observation period** | **Animals** | **Dose (micro g/kg)** |
| Single-dose toxicity: | | | | |
| [CS3348] Single dose | Cynomolgus monkey (3 to 4 years of age) | Single IV dose, 8 days | 1 male and 1 female | 100 |
| [PPU4135] Single dose | Cynomolgus monkey (4 years of age) | Single IV dose, 29 days | 2 males | 90 |
| IV = intravenous | | | | |

In the male animals treated with CS3348 or PPU4135, plasma exposure levels were comparable between PPU4135 treated group and CS3348 treated group until Day 8. Increased AST (aspartate aminotransferase), ALT (alanine aminotransferase) and GLDH (glutamate dehydrogenase) (hepatic enzymes); ALP (alkaline phosphatase), TBIL (total bilirubin), GGT (gamma glytamyltranspeptidase) and TBA (total bile acid) (hepatobiliary damage parameters); and CRP (C-reactive protein) (inflammatory marker) were noted after single administration of these antibodies (Figure 14). Although the differences of the hepatic enzyme levels between these antibodies treated male animals were slight (Figure 14), the hepatobiliary damage parameters and inflammatory marker elevations were dramatically mitigated by PPU4135 administration rather than CS3348 administration throughout the studies (Figure 14). These results suggest that test article-mediated hepatotoxicity, mainly hepatobiliary damage, is attenuated by using the anti-CLDN6/Dual-Fab tri-specific antibody rather than using the anti-CLDN6/CD3 bispecific antibody.

[Example 15] Characterization of anti-CLDN6/Dual-Fab tri-specific antibody

The binding affinity of the anti-CLDN6/Dual-Fab tri-specific antibody against human and cynomolgus (cyno) CLDN6 VLP (virus-like particle) at pH 7.4 was determined at 25 degree C using Biacore T200 instrument (GE Healthcare). Anti-human CD81 (BD Pharmingen) antibody was immobilized onto all flow cells of a C1 sensor chip using amine coupling kit (GE Healthcare). Human and cyno CLDN6 VLP were captured onto the sensor surface by the anti-human CD81 antibody. Each VLP was 5-fold dilution by buffer (20 mM NaPhosphate, 150 mM NaCl, 0.1 mg/mL BSA, 0.005% $NaN_3$, pH 7.4). Tested antibody was prepared in buffer (20 mM NaPhosphate, 150 mM NaCl, 0.1 mg/mL BSA, 0.005% $NaN_3$, pH 7.4). Anti-CLDN6/Dual-Fab tri-specific antibody was injected at 50 and 200 nM, followed by dissociation. Sensor surface was regenerated each cycle with 0.1% SDS and 100 mM $H_3PO_4$. As shown in Table 12, binding affinity of PPU4135 towards cyno CLDN6 is comparable with that towards human CLDN6. Binding affinities were determined by processing and fitting the data to 1: 1 binding model using Biacore T200 Evaluation software, version 2.0 (GE Healthcare).

The binding affinity of the anti-CLDN6/Dual-Fab tri-specific antibody against recombinant human and cyno CD3eg (gamma and epsilon subunits of CD3) at pH 7.4 was determined at 25 degree C using Biacore 8K instrument (GE Healthcare). The binding affinity of the anti-CLDN6/Dual-Fab tri-specific antibody against recombinant human and cyno CD137 at pH 7.4 was determined at 37 degree C using Biacore 8K instrument (GE Healthcare). Anti-human Fc (GE Healthcare) antibody was immobilized onto all flow cells of a CM4 sensor chip using amine coupling kit (GE Healthcare). Tested antibody and analytes were prepared in ACES pH 7.4 containing 20 mM ACES, 150 mM NaCl, 0.05% Tween 20, 0.005% $NaN_3$. Anti-CLDN6/Dual-Fab tri-specific antibody was captured onto the sensor surface by anti-human Fc. Antibody capture levels were aimed at 300 resonance unit (RU). Recombinant CD3eg and CD137 were injected at both 500 and 2000 nM, followed by dissociation. Sensor surface was regenerated each cycle with 3M $MgCl_2$. As shown in Table 12, binding affinities of PPU4135 towards cyno CD3eg and cyno CD137 are comparable with those towards human CD3eg and CD137, respectively. Binding affinities were determined by processing and fitting the data to 1: 1 binding model using Biacore Insight Evaluation software (GE Healthcare).

Table 12)

| Binding affinity of PPU4135 towards human and cyno antigens | | | |
|---|---|---|---|
| Antigen | affinity | | |
| | ka (1/Ms) | kd (1/s) | KD (M) |
| human CLDN6 | 2.17E+05 | 7.94E-04 | 3.65E-09 |

(continued)

| Binding affinity of PPU4135 towards human and cyno antigens | | | |
|---|---|---|---|
| Antigen | affinity | | |
| | ka (1/Ms) | kd (1/s) | KD (M) |
| cyno CLDN6 | 2.18E+05 | 8.21E-04 | 3.77E-09 |
| human CD3eg | 7.80E+04 | 2.66E-02 | 3.41E-07 |
| cyno CD3eg | 1.01E+05 | 3.32E-02 | 3.29E-07 |
| human CD137 | 1.26E+04 | 5.91E-04 | 4.68E-08 |
| cyno CD137 | 8.35E+03 | 1.46E-03 | 1.74E-07 |
| (The expression E used to express the ka (1/Ms), kd (1/s), and KD values in the table means "10 to the power of" and, for instance, 2.17E+05 = $2.17*10^5$) | | | |

[Example 16] In vivo drug efficacy evaluation test in a peritoneal dissemination model

To evaluate the effect of anti-CLDN6/Dual-Fab tri-specific antibody CS4135 (the same antibody as PPU4135 described in Table 9 of Example 6 described above) against peritoneal metastasis of ovary cancer, the drug efficacy was evaluated using a mouse peritoneal dissemination model. Human ovarian cancer cell line OV-90 (ATCC) was used as target cells. OV-90 cells were transplanted intraperitoneally to NOD/ShiJic-scid Jcl mice at 5 x $10^6$ cells/500 μL/animal. Three days after transplantation, human T cells separated from human PBMC and expansively cultured using Dynabeads Human T-Activator CD3/CD28 (Gibco) were administered intravenously at 3 x $10^7$ cells/400 μL. Furthermore, the vehicle (PBS containing 0.05% Tween-20) or a CS4135 antibody solution at 5 mg/kg was administered intravenously.

After tumor transplantation, the mice were observed every 2 to 7 days, and the conditions of the mice were assessed according to the humane endpoint guideline. The survival rate of each group is shown in Fig. 15.

As a result, in the vehicle-administered group, mice showing aggravated conditions were observed from 48 days after transplantation, and aggravated conditions were observed in all cases by 55 days after transplantation. On the other hand, in the CS4135 antibody-administered group, mice showing aggravated conditions were observed from 57 days after transplantation, and the survival rate was 78% at 66 days after transplantation and 22% at 80 days after transplantation. The 50% survival rate was found 71 days after transplantation. This result indicated the therapeutic effects of the CS4135 antibody on peritoneal metastasis of ovary cancer.

[Example 17] Drug efficacy evaluation test for combined use of anti-CLDN6/Dual-Fab tri-specific antibody CS4135 and a chemotherapeutic agent

17.1 Change in CLDN6 expression in various cancer cells by treatment with a platinum preparation

Human ovarian cancer cell line (NIH-OVCAR3, ATCC), human lung cancer cell line (NCI-H1435, ATCC), and human endometrial cancer cell line (SNG-M, Health Science Research Resources Bank) were treated with cisplatin (CDDP, Nichiiko) or carboplatin (CBDCA, Sandoz) to analyze the effect of the treatment on CLDN6 expression.

Each cell was subjected to the addition of cisplatin at 0.1 μg/mL (OVCAR3) or 0.6 μg/mL (H1435, SNG-M), or carboplatin at 0.5 μg/mL (OVCAR3) or 5 μg/mL (H1435, SNG-M), and then cultured for five days. After culturing, the cells were recovered, and CLDN6 expression was analyzed by FACS.

In FACS analysis, cells were detached from the culturing flask and then reacted with an anti-CLDN6 antibody (AE3-20 mIgG2a, Japanese Patent No. 5848863) or an isotype antibody (mIgG2a, BioLegend) at 2 μg/mL, at 4°C for one hour. Next, reaction with Alexa Flour 488-labeled anti-mouse IgG (ThermoFisher) at 10 μg/mL was performed at 4°C for one hour, and the amount of CLDN6 expression was analyzed using a flow cytometer (FACSVerse, BD Biosciences).

Fig. 16 presents the results of analyzing CLDN6 expression by FACS in each cell type treated with cisplatin or carboplatin for 5 days and in each cell type that were untreated. In all cells, cisplatin or carboplatin was found to cause increase in CLDN6 expression. Cisplatin and carboplatin were therefore confirmed to be agents that enhance the expression of CLDN6 in cancer cells.

Next, measurement was carried out based on a luciferase assay system using GloResponse NFAT-luc2 Jurkat cells (Promega, J1601) to see if T-cell-activation ability of CS4135 antibody via CD3 binding will change depending on the presence or absence of treatment by a chemotherapeutic agent.

The assay was performed as follows. First, as described above, OVCAR3 and H1435 cells treated with a chemotherapeutic agent for 5 days were detached from a culture flask and plated at 25 μL/well (2 x $10^4$ cells) onto a white flat-bottom 96-well plate (Coster #3917). Next, 1 x $10^5$ Jurkat / NFAT-RE reporter cell line together with the CS4135 antibody at a concentration selected from 0.003, 0.03, 0.3, 3, or 30 nM were added at 25 μL/well. After culturing overnight at 37°C, Bio-Glo reagent (Promega #G7941) was added at 75 μL/well followed by further incubation at room temperature for 10 minutes. Then, luminescence arose from activated Jurkat cells was measured using an EnSpire (PerkinElmer Japan) device. The luminescence magnification of each well was calculated by comparing the wells with the antibody to the wells without the antibody.

Fig. 17 presents the results of analyzing T cell activation ability of CS4135 via CD3, for the cells that were treated with cisplatin or carboplatin for 5 days or for the cells that were untreated, using Jurkat luciferase assay. T cell activation ability of CS4135 was confirmed to be enhanced with respect to cells treated with cisplatin or carboplatin for 5 days, suggesting that the chemotherapeutic agent may enhance the cytotoxicity of CS4135.

17.2 Induction of CLDN6 expression in a vivo tumor transplant by carboplatin administration

SNG-M (1 x $10^7$ cells) was transplanted subcutaneously to NOD/scid mice (CLEA Japan). After tumor engraftment, the vehicle (physiological saline) or carboplatin at 40 mg/kg or 80 mg/kg was administered intraperitoneally on the day of transplantation (day 0) and 3 days after transplantation (day 3), and the tumor was sampled 6 days after transplantation (day 6).

RNA was purified from the tumor (RNeasy Mini Kit, QIAGEN), and then cDNA synthesis was performed (Suprescript IV VILO Master Mix, ThermoFisher). Using the cDNAs as templates, real-time PCR was performed using a CLDN6-specific primer (Power SYBR (registered trademark) Green Master Mix, ThermoFisher) to analyze CLDN6 expression (QuantStudio 12K Flex Real-Time PCR System, ThermoFisher).

Sequences of the primers for human CLDN6 and primers for human GAPDH used as an internal control are as follows:

hCLDN6-1: GGG TGG ACG TCT TAT CAG GA (SEQ ID NO: 206)
hCLDN6-2: GAG CTC TCT TCA CCC CT (SEQ ID NO: 207)
hGAPDH-F: GAG TCC ACT GGC GTC TTC AC (SEQ ID NO: 208)
hGAPDH-R: ATC TTG AGG CTG TTG TCA TAC TT (SEQ ID NO: 209)

Fig. 18 presents the results of analyzing CLDN6 expression in the tumor by qPCR, for the tumor sampled after administering the vehicle or carboplatin at 40 mg/kg or 80 mg/kg twice to SNG-M tumor-engrafted mice. CLDN6 expression in the tumor was confirmed to be increased in the carboplatin-administered groups as compared to the vehicle-administered group.

17.3 Evaluation of antitumor activity of the CS4135 antibody by a xenograft transplantation model using huNOG mice

17.3.1 Preparation of cell lines and a xenograft transplantation model, and method for evaluating antitumor activity

In this test, NOG (huNOG) mice (described in the aforementioned Example 13) transplanted with human uterus cancer cell line SNG-M and human CD34-positive cells were used. The SNG-M cell line was transplanted subcutaneously to the right flank of the mice, and the mice were divided into groups 13 days after transplantation. Individuals whose tumor volume reached 130 to 244 $mm^3$ were divided into groups. Vehicle 1 and CS4135 were administered into the mouse tail vein 13 days after transplantation. PBS containing 0.05% Tween-20 was used as Vehicle 1. Vehicle 2 and carboplatin (Bristol Myers Squibb Company) were administered intraperitoneally to the mice 13, 16, and 20 days after transplantation. Physiological saline was used as Vehicle 2.

[Table 13]

| Group | n | Pharmaceutical agent | Dose | Day of administration | Administration route |
|---|---|---|---|---|---|
| 1 | 8 | Vehicle 1 (0.05% Tween 20 PBS) | - | 13 | Into the tail vein |
| | | Vehicle 2 (Physiological saline) | - | 13, 16, 20 | Intraperitoneal |
| 2 | 8 | CS4135 | 0.5 mg/kg | 13 | Into the tail vein |
| | | Vehicle 2 | - | 13, 16, 20 | Intraperitoneal |

(continued)

| Group | n | Pharmaceutical agent | Dose | Day of administration | Administration route |
|---|---|---|---|---|---|
| 3 | 8 | Vehicle 1 | - | 13 | Into the tail vein |
| | | Carboplatin | 40 mg/kg | 13, 16, 20 | Intraperitoneal |
| 4 | 8 | CS4135 | 0.5 mg/kg | 13 | Into the tail vein |
| | | Carboplatin | 40 mg/kg | 13, 16, 20 | Intraperitoneal |

Tumor volume was measured 13, 16, 20, 23, 26, and 30 days after transplantation, and the average tumor volume was indicated for each group.

The tumor volume was calculated by the following equation:

Tumor volume ($mm^3$) = major axis (mm) x minor axis (mm) x minor axis (mm)/2

Tumor Growth Inhibition (TGI) was calculated by the following equation:

TV change ($mm^3$) = tumor volume at 30 days after transplantation - tumor volume when divided into groups

-TG1 (%) = (1 - average TV change in each group / average TV change in the vehicle control group)) x 100

As a result, at 30 days after transplantation, the combined administration group that received the CS4135 antibody and carboplatin showed TGI of 65%, while the single-agent administration group that received the CS4135 antibody and the single-agent administration group that received carboplatin showed TGI of 35% and 44%, respectively. Therefore, the combined administration of the CS4135 antibody and carboplatin was shown to have stronger antitumor effects as compared to the single-agent administration of the CS4135 antibody and the single-agent administration of carboplatin (Fig. 19, Table 14).

[Table 14]

| Tumor growth inhibition rate at 30 days after transplantation (Tumor growth inhibition: TG I (%)) | | | | |
|---|---|---|---|---|
| | Vehicle control | CS4135 | Carboplatin | CS4135 + Carboplatin |
| TGI | - | 35 | 44 | 65 |

17.3.2 Preparation of cell lines and a xenograft transplantation model, and method for evaluating antitumor activity

In this test, NOG (huNOG) mice (described in the aforementioned Example 13) transplanted with ovarian cancer cell line OVCAR3 and human CD34-positive cells were used. The OVCAR3 cell line was transplanted subcutaneously to the right flank of the mice, and the mice were divided into groups 38 days after transplantation. Individuals whose tumor volume reached 119 to 210 $mm^3$ were divided into groups. Vehicle 1 and CS4135 were administered into the mouse tail vein 38 and 45 days after transplantation. PBS containing 0.05% Tween-20 was used as Vehicle 1. Vehicle 2 and carboplatin (Bristol Myers Squibb Company) were administered intraperitoneally to the mice 38 and 45 days after transplantation. Physiological saline was used as Vehicle 2.

[Table 15]

| Group | n | Pharmaceutical agent | Dose | Day of administration | Administration route |
|---|---|---|---|---|---|
| 1 | 5 | Vehicle 1 (0. 05% Tween 20 PBS) | - | 38, 45 | Into the tail vein |
| | | Vehicle 2 (Physiological saline) | - | 38, 45 | Intraperitoneal |

(continued)

| Group | n | Pharmaceutical agent | Dose | Day of administration | Administration route |
|---|---|---|---|---|---|
| 2 | 5 | CS4135 | 0.1mg/kg | 38, 45 | Into the tail vein |
| | | Vehicle 2 | - | 38, 45 | Intraperitoneal |
| 3 | 5 | Vehicle 1 | - | 38, 45 | Into the tail vein |
| | | Carboplatin | 60mg/kg | 38, 45 | Intraperitoneal |
| 4 | 5 | CS4135 | 0.1mg/kg | 38, 45 | Into the tail vein |
| | | Carboplatin | 60mg/kg | 38, 45 | Intraperitoneal |
| 5 | 5 | CS4135 | 0.1mg/kg | 48, 55 | Into the tail vein |
| | | Carboplatin | 60mg/kg | 38, 45 | Intraperitoneal |

Tumor volume was measured 38, 42, 45, 48, 52, and 56 days after transplantation, and the average tumor volume was indicated for each group. The tumor volume was calculated by the following equation:

Tumor volume ($mm^3$) = major axis (mm) x minor axis (mm) x minor axis (mm)/2

Tumor Growth Inhibition (TGI) was calculated by the following equation:

TV change ($mm^3$) = tumor volume at 56 days after transplantation - tumor volume when divided into groups

TG1 (%) = (1 - average TV change in each group / average TV change in the vehicle control group)) x 100

As a result, at 56 days after transplantation, the combined (simultaneous) administration group that received the CS4135 antibody and carboplatin showed TGI of 180%, while the single-agent administration group that received the CS4135 and the single-agent administration group that received carboplatin showed TGI of 18% and - 9%, respectively. Therefore, the combined administration of the CS4135 antibody and carboplatin was shown to have stronger synergistic antitumor effects as compared to the single-agent administration of the CS4135 and the single-agent administration of carboplatin (Fig. 20, Table 16).

Furthermore, when CS4135 was successively administered following carboplatin administration, TGI at 56 days after transplantation was 180%, the same as the group that received simultaneous administration of carboplatin and CS4135. Therefore, CS4135 was shown to be similarly effective, not only when administered simultaneously with carboplatin, also when administered successively after carboplatin administration (Fig. 21, Table 16).

[Table 16]

| Tumor growth inhibition rate at 56 days after transplantation (Tumor growth inhibition: TGI (%)) | | | | | |
|---|---|---|---|---|---|
| | Vehicle control | CS4135 | Carboplatin | CS4135 + Carboplatin | Carboplatin → CS4135 |
| TGI | - | 18 | -9 | 180 | 180 |

17.4 Evaluation of antitumor activity of the CS4135 antibody by a xenograft transplantation model using huNOG mice

17.4.1 Preparation of cell lines and a xenograft transplantation model, and method for evaluating antitumor activity

In this test, NOG(huNOG) mice (described in the aforementioned Example 13) transplanted with ovarian cancer cell line NIH:OVCAR-3 (OVCAR3, ATCC) and human CD34-positive cells were used. The OVCAR3 cell line was transplanted subcutaneously to the right flank of the mice, and the mice were divided into groups 39 days after transplantation. Individuals whose tumor volume reached 160 to 263 $mm^3$ were divided into groups. Vehicle 1 and CS4135 were administered into the mouse tail vein 39 and 46 days after transplantation. PBS containing 0.05% Tween-20 was used as Vehicle 1. Vehicle 2 and irinotecan hydrochloride (Sawai Pharmaceutical Co., Ltd.) were administered into the mouse tail vein 39 and 46 days after transplantation. Physiological saline was used as Vehicle 2.

[Table 17]

| Group | n | Pharmaceutical agent | Dose | Day of administration | Administration route |
|---|---|---|---|---|---|
| 1 | 5 | Vehicle 1 (0.05% Tween 20 PBS) | - | 39, 46 | Into the tail vein |
| | | Vehicle 2 (Physiological saline) | - | 39, 46 | Into the tail vein |
| 2 | 5 | CS4135 | 0.1 mg/kg | 39, 46 | Into the tail vein |
| | | Vehicle 2 | - | 39, 46 | Into the tail vein |
| 3 | 5 | Vehicle 1 | - | 39, 46 | Into the tail vein |
| | | Irinotecan hydrochloride | 25 mg/kg | 39, 46 | Into the tail vein |
| 4 | 5 | CS4135 | 0.1 mg/kg | 39, 46 | Into the tail vein |
| | | Irinotecan hydrochloride | 25 mg/kg | 39, 46 | Into the tail vein |

Tumor volume was measured 39, 42, 46, 49, 53, 57, and 60 days after transplantation, and the average tumor volume is indicated for each group. The tumor volume was calculated by the following equation:

Tumor volume ($mm^3$) = major axis (mm) x minor axis (mm) x minor axis (mm)/2

Tumor Growth Inhibition (TGI) was calculated by the following equation:

TV change ($mm^3$) = tumor volume at 60 days after transplantation - tumor volume when divided into groups

TG1 (%) = (1 - average TV change in each group / average TV change in the vehicle control group)) x 100

As a result, at 60 days after transplantation, the combined administration group that received the CS4135 antibody and irinotecan hydrochloride showed TGI of 106% , while the single-agent administration group that received the CS4135 antibody and the single-agent administration group that received irinotecan hydrochloride showed TGI of -2% and 65%, respectively. Furthermore, statistically significant difference in their tumor volumes was observed at 60 days after transplantation, between the combined administration group that received the CS4135 antibody and irinotecan hydrochloride and the single-agent administration group that received irinotecan hydrochloride. Therefore, the combined administration of the CS4135 antibody and irinotecan hydrochloride was shown to have stronger and synergistic antitumor effects as compared to the single-agent administration of the CS4135 antibody and the single-agent administration of irinotecan hydrochloride (Fig. 22, Table 18).

[Table 18]

| Tumor growth inhibition rate at 60 days after transplantation (Tumor growth inhibition: TGI (%)) | | | | |
|---|---|---|---|---|
| | Vehicle control | CS4135 | Irinotecan hydrochrolide | CS4135 + Irinotecan hydrochloride |
| TGI | - | -2 | 65 | 106 |

[Example 18] Drug efficacy evaluation test for combined use of anti-CLDN6/Dual-Fab tri-specific antibody CS4135 and an immune checkpoint inhibitor

18.1.1 Preparation of cell lines and a syngenic transplantation model, and method for evaluating antitumor activity

In this test, lung cancer cell line LLC1 forced to express claudin 6 (CLDN6) (CLDN6-LLC1) and hCD137 KI/hCD3Tg mice (described in the above-mentioned Example 7) were used. The CLDN6-LLC1 cell line was transplanted subcutaneously to the right flank of the mice, and the mice were divided into groups 7 days after transplantation. Individuals whose tumor volume reached 262 to 353 $mm^3$ were divided into groups. The vehicle and CS4135 were administered into the mouse tail vein 8 days after transplantation. PBS containing 0.05% Tween-20 was used as the vehicle.

[Table 19]

| Group | n | Pharmaceutical agent | Dose | Day of administration | Administration route |
|---|---|---|---|---|---|
| 1 | 5 | Vehicle (0.05% Tween 20 PBS) | - | 8 | Into the tail vein |
| 2 | 5 | CS4135 | 0.25 mg/kg | 8 | Into the tail vein |
| 3 | 5 | Vehicle | - | 8 | Into the tail vein |
| 4 | 5 | CS4135 | 0.25 mg/kg | 8 | Into the tail vein |

Tumor volume was measured 7, 10, and 14 days after transplantation, and the average tumor volume was indicated for each group. The tumor volume was calculated by the following equation:

Tumor volume ($mm^3$) = major axis (mm) x minor axis (mm) x minor axis (mm)/2

Tumor Growth Inhibition (TGI) was calculated by the following equation:

TV change ($mm^3$) = tumor volume at 14 days after transplantation - tumor volume when divided into groups

-TG1 (%) = (1 - average TV change in each group / average TV change in the vehicle control group)) x 100

The results are shown in Fig. 23. At 14 days after transplantation, the CS4135-administered group showed TGI of 71%. The tumor was resected at 10 and 14 days after transplantation (2 and 6 days after administration, respectively (Day 2 and Day 6)), and the number of CD8-positive T cells in the tumor tissue was detected as follows.

[Table 20]

| Tumor growth inhibition rate at 14 days after transplantation (Tumor growth inhibition: TGI (%)) | | |
|---|---|---|
| | Vehicle control | CS4135 |
| TGI | - | 71 |

18.1.2. Resection of tumor tissues from CLDN6-LLC1 cell line-transplanted mice and preparation of a lymphocyte fraction

The resected tumor tissue was weighed, and then a lymphocyte fraction was isolated. The lymphocyte fraction obtained by tissue disruption using a Tumor Dissociation Kit, mouse (Miltenyi Biotec) and then by use of a cell strainer was used. The obtained tumor-derived lymphocyte fraction was subjected to flow cytometry (FCM) analysis.

18.1.3. Calculation of the number of CD8-positive T cells per tumor weight according to flow cytometry (FCM) analysis

FCM was used to analyze the number of tumor-derived T cells. The number of CD8-positive T cells per tumor weight was calculated using the tumor weight value and the number of CD8-positive T cells according to FCM. In the FCM analysis, an anti-CD45 antibody (BD Biosciences), an anti-CD3 antibody (BioLegend), an anti-CD8 antibody (BioLegend), and an anti-CD4 antibody (BioLegend) were used. BD LSRFortessa X-20 (BDBiosciences) was used for the measurements.

As a result, at 14 days after tumor transplantation (6 days after administration (Day 6)), significant increase in the number of CD8-positive T cells per tumor weight was observed in the CS4135-administered group (Fig. 24).

18.2.1 Preparation of cell lines and a syngenic transplantation model, and method for evaluating antitumor activity

In this test, lung cancer cell line LLC1 forced to express claudin 6 (CLDN6) (CLDN6-LLC1) and hCD137 KI/hCD3Tg mice (described in the aforementioned Example 7) were used. The CLDN6-LLC1 cell line was transplanted subcutaneously to the right flank of the mice, and the mice were divided into groups 6 days after transplantation. Individuals whose tumor volume reached 104 to 140 $mm^3$ were divided into groups. The vehicle and the CS4135 antibody were administered into the mouse tail vein 6 days after transplantation. PBS containing 0.05% Tween-20 was used as the vehicle. The vehicle and anti-mouse PD-L1 antibody (BioX cell) were administered intraperitoneally to the mice 6, 8, 10, 12, 14, and 17 days after transplantation.

[Table 21]

| Group | n | Pharmaceutical agent | Dose | Day of administration | Administration route |
|---|---|---|---|---|---|
| 1 | 5 | Vehicle (0.05% Tween 20 PBS) | - | 6 | Into the tail vein |
| | | Vehicle | - | 6, 8, 10, 12, 14, 17 | Intraperitoneal |
| 2 | 5 | CS4135 | 0.2 mg/kg | 6 | Into the tail vein |
| | | Vehicle | - | 6, 8, 10, 12, 14, 17 | Intraperitoneal |
| 3 | 5 | Vehicle | - | 6 | Into the tail vein |
| | | Anti-mouse PD-L1 antibody | 10 mg/kg | 6, 8, 10, 12, 14, 17 | Intraperitoneal |
| 4 | 5 | CS4135 | 0.2 mg/kg | 6 | Into the tail vein |
| | | Anti-mouse PD-L1 antibody | 10 mg/kg | 6, 8, 10, 12, 14, 17 | Intraperitoneal |

Tumor volume was measured 6, 10, 12, 14, and 17 days after transplantation, and the average tumor volume was indicated for each group. The tumor volume was calculated by the following equation:

Tumor volume ($mm^3$) = major axis (mm) x minor axis (mm) x minor axis (mm)/2

Tumor Growth Inhibition (TGI) was calculated by the following equation:

Tumor volume ($mm^3$) = tumor volume 17 at days after transplantaiton - tumor volue when divided into groups-TGI(%) = (1 - (average TV change in each group / average TV change in the vehicle control group)) x 100

As a result, at 17 days after transplantation, the combined administration group that received the CS4135 antibody and the anti-mouse PD-L1 antibody showed TGI of 108%, while the single-agent administration group that received the CS4135 antibody and the single-agent administration group that received the anti-mouse PD-L1 antibody showed TGI of 71% and -1%, respectively. Therefore, the combined administration of the CS4135 antibody and the anti-mouse PD-L1 antibody was shown to have stronger synergistic antitumor effects as compared to the single-agent administration of the CS4135 antibody and the single-agent administration of the anti-mouse PD-L1 antibody (Fig. 25, Table 22).

[Table 22]

| Tumor growth inhibition rate at 17 days after transplantation (Tumor growth inhibition: TGI (%)) | | | | |
|---|---|---|---|---|
| | Vehicle control | CS4135 | Anti-mouse PD-L1 antibody | CS4135 + Anti-mouse PD-L1 antibody |
| Tumor growth inhibition (TGI) | - | 71 | -1 | 108 |

[Example 19] Drug efficacy evaluation test for combined use of anti-CLDN6/Dual-Fab tri-specific antibody CS4135 and a PARP inhibitor

19. 1. Assessment of induction of CLDN6 expression by administration of a PARP inhibitor

Induction of CLDN6 expression by a PARP inhibitor (olaparib) was assessed. BRCA1-deficient ovarian cancer cell line UWB1.289 (ATCC) or BRAC1 wildtype ovarian cancer cell line OV-90 (ATCC) which express human CLDN6 were used.

Target cells ($3 \times 10^5$ cells) were plated onto a 6-well plate at 3 mL/well. After culturing overnight, olaparib was added at a final concentration of 0.03, 0.1, 0.3, 1, or 3 μM. DMSO was added to the control well. After incubating the plate for 3 days, the cells were recovered and the amount of CLDN6 expression on the cell surface was determined by FACS analysis using an anti-CLDN6 antibody. The level of fluorescence in the target cells recovered from the wells, to which various concentrations of olaparib were added, are shown in Fig. 27, wherein the level of fluorescence in the target cells recovered from the control well was defined as 1.

In BRCA1-deficient ovarian cancer cell line UWB1.289, olaparib concentration-dependent enhancement of CLDN6 expression induction was observed. On the other hand, in BRAC1 wildtype ovarian cancer cell line OV-90, enhancement of CLDN6 expression induction was not observed at any concentration of olaparib.

19.2. Cytotoxic activity of anti-CLDN6/Dual-Fab tri-specific antibody CS4135 on PARP-inhibitor-administered cells

The effect of the enhanced induction of CLDN6 expression in BRCA1-deficient ovarian cancer cell line UWB1.289, caused by the PARP inhibitor (olaparib), on the cytotoxic activity of anti-CLDN6/Dual-Fab tri-specific antibody CS4135 was assessed.

BRCA1-deficient ovarian cancer cell line UWB1.289 (ATCC) or BRAC1 wildtype ovarian cancer cell line OV-90 (ATCC) which express human CLDN6 were used as target cells.

Each target cell ($3 \times 10^5$ cells) were seeded at 5 mL into a $25cm^2$-flask. Two flasks were prepared for each target cell. After culturing overnight, olaparib was added at a final concentration of 3 μM to one of the two flasks. DMSO was added to the other flask. After incubating the flasks for three days, the target cells were recovered, and the cytotoxic activity of the CS4135 antibody was evaluated by lactase dehydrogenase (LDH) release assay using human PBMC as the effector cell. The results are shown in Fig. 28.

In BRCA1-deficient ovarian cancer cell line UWB1.289, enhancement of cytotoxicity of PPU4135 by the addition of olaparib was observed. On the other hand, in BRAC1 wildtype ovarian cancer cell line OV-90, addition of olaparib had no effect on cytotoxic activity.

[Example 20] Analysis of the mechanism of the CS4135 antibody using real-time cell growth inhibition assay (xCELLigence assay)

The mechanism of T-cell dependent growth inhibition mediated by the anti-CLDN6/Dual-Fab tri-specific antibodies was assessed by cell proliferation assay using an xCELLigence RTCA MP instrument (ACEA Biosciences).

Mouse colon cancer cell line MC38/CLDN6 expressing human CLDN6 was used as target cells. Spleens were collected aseptically from hCD3 transgenic mice and hCD3/hCD137 knock-in mice. The spleen was ground in RPMI-1640 medium containing 10% FBS, passed through a 70-μm cell strainer, and then centrifuged (at 1200 rpm for 10 minutes at room temperature) to isolate spleen cells. The isolated spleen cells were hemolyzed, then T cells alone were isolated using CD3 MicroBeads (Miltenyi Biotec), and used as effector cells.

The background was measured by adding the culture medium to an E-Plate 96 plate (Roche Diagnostics) at 50 μL/well, and then $5 \times 10^3$ target cells were plated at 50 μL/well. After culturing overnight, 1 nM (final concentration) of CS4135 together with the KLH/CD137 bispecific antibody at a concentration selected from 100 nM or 500 nM (final concentration) were added at 25 μL/well, respectively. Then, $2.5 \times 10^4$ effector cells were added at 50 μL/well. Cell growth was monitored every 10 min using xCELLigence over 48 hours during the incubation of the plates. The rate of cell growth inhibition (CGI: %) was determined from the cell index value according to the following formula:

$$-GI (\%) = 100 - (CI_{Ab} \text{ x } 100 / CI_{NoAb}),$$

wherein "$CI_{Ab}$" represents the cell index value of wells with antibody on a specific experimental time, and "$CI_{NoAb}$" represents the average cell index value of the wells without antibody at the same experimental time. Each cell index value used was a value corrected by defining the cell index value of each well before adding the antibody and effector cells as 1.

When T cells derived from hCD3/hCD137 knock-in mice were used, the cell growth inhibition rate shown by the CS4135 antibody when the KLH/CD137 bispecific antibody was not added was significantly higher than when T cells derived from hCD3 transgenic mice were used; however, the cell growth inhibition rate decreases dependent on the dose of KLH/CD137 bispecific antibody, and when 500 nM (final concentration) of the KLH/CD137 bispecific antibody was added, the cell growth inhibition rate decreased to the same level as when using hCD3 transgenic mice-derived T cells. This result showed that the CD137 signal produced by the CS4135 antibody enhances the cytotoxic activity. The results are shown in Fig. 28.

[Example 21]

21.1 Induction of CLDN6 expression by chemotherapeutic agents

Various chemotherapeutic agents were added to human ovarian cancer cell line (NIH:OVCAR-3, ATCC) to analyze the change in CLDN6 expression. Regarding the NIH:OVCAR-3 cells seeded on culture plates, RNAs were purified (RNeasy Mini Kit, QIAGEN) from cells to which no pharmaceutical agent was added (untreated cells), or from cells collected 6 days after addition of carboplatin (0.5 μg/mL), cisplatin (0.1 μg/mL), irinotecan (0.25 μg/mL), or gemcitabine (2 ng/mL), and then cDNA synthesis (Superscript IV VILO Master Mix, ThermoFisher) was performed. By using the cDNAs as templates, real-time PCR was performed using CLDN6-specific primers (Power SYBR (registered trademark) Green Master Mix, ThermoFisher) to analyze CLDN6 expression (QuantStudio 12K Flex Real-Time PCR System, ThermoFisher).

Sequences of the primers for human CLDN6 and the primers for human GAPDH that were used as the internal control are as follows:

hCLDN6-1: GGG TGG ACG TCT TAT CAG GA (SEQ ID NO: 206)
hCLDN6-2: GAG CTC TCT TCA CCC CT (SEQ ID NO: 207)
hGAPDH-F: GAG TCC ACT GGC GTC TTC AC (SEQ ID NO: 208)
hGAPDH-R: ATC TTG AGG CTG TTG TCA TAC TT (SEQ ID NO: 209)

Fig. 29 shows the result of analyzing CLDN6 expression by qPCR for NIH:OVCAR-3 cells to which no pharmaceutical agent was added (untreated), or to which carboplatin, cisplatin, irinotecan, or gemcitabine was added. Increase in CLDN6 expression was observed in the carboplatin-, cisplatin-, irinotecan-, and gemcitabine-treated cells as compared to the cells without addition of a pharmaceutical agent.

21.2 Induction of TGF-β1 expression by chemotherapeutic agents

Next, the cDNAs prepared from the human ovarian cancer cell line (NIH:OVCAR-3, ATCC) treated with carboplatin, cisplatin, irinotecan, or gemcitabine, which were used in Example 21.1, were used to perform real-time PCR with TGF-β1-specific primers (Power SYBR (registered trademark) Green Master Mix, ThermoFisher), and TGF-β1 expression was analyzed (QuantStudio 12K Flex Real-Time PCR System, ThermoFisher).

Sequences of the primers for human TGF-β1 are as follows:

hTGF-β1-F: AGTGGTTGAGCCGTGGAG (SEQ ID NO: 214)
hTGF-β1-R: CGGTAGTGAACCCGTTGAT (SEQ ID NO: 215)

The same primers as those in the above-mentioned Example 21.1 were used as the primers for human GAPDH used as the internal control.

Fig. 30 shows the result of analyzing TGF-β1 expression by qPCR in NIH:OVCAR-3 cells to which no pharmaceutical agent was added (untreated), or to which carboplatin, cisplatin, irinotecan, or gemcitabine was added. Increase in TGF-β1 expression was observed in the carboplatin-, cisplatin-, irinotecan-, and gemcitabine-treated cells as compared to the cells without addition of a pharmaceutical agent.

21.3 Induction of CLDN6 expression by TGF-β

Example 21.1 confirmed that treatment of cells with a chemotherapeutic agent increases the expression of CLDN6.

Furthermore, Example 21.2 confirmed the induction of TGF-β1 expression in cancer cells by chemotherapeutic agents. Anticancer agents other than the chemotherapeutic agents confirmed in Example 21.2, are also known to induce TGF-β1 expression. For example, chemotherapeutic agents such as doxorubicin and paclitaxel, and radiation have been reported to induce expression of TGF-β1 in cancer cells (Barcellos-Hoff et al., J Clin Invest. 1994 Feb;93(2):892-9; and Bhola et al., J Clin Invest. 2013 Mar;123(3):1348-58).

Therefore, it was speculated that TGF-β1 induced by treatment with an anticancer agent may affect CLDN6 expression. Accordingly, analyzes were performed to determine whether TGF-β1 induces CLDN6 expression in the human ovarian cancer cell line (NIH:OVCAR-3, ATCC). Regarding the NIH:OVCAR-3 cells seeded on culture plates, RNAs were purified (RNeasy Mini Kit, QIAGEN) from untreated cells or from cells collected 5 days after addition of TGF-β1 (R & D Systems, 10 ng/ml), and then cDNA synthesis (Superscript IV VILO Master Mix, ThermoFisher) was performed. By using the cDNAs as templates, real-time PCR was performed using CLDN6-specific primers (Power SYBR (registered trademark) Green Master Mix, ThermoFisher) to analyze CLDN6 expression (QuantStudio 12K Flex Real-Time PCR System, ThermoFisher).

The same primers as those in the above-mentioned Example 21.1 were used as the primers for human CLDN6 and for the primers for human GAPDH used as the internal control.

Fig. 31 shows the result of analyzing CLDN6 expression by qPCR in NIH:OVCAR-3 cells that were untreated, or to which TGF-β1 was added. Increase in CLDN6 expression was observed in the TGF-β1-added cells as compared to the untreated cells.

Next, analyses were performed to determine whether TGF-β1 induces CLDN6 expression in other ovarian cancer cell lines. In addition to NIH:OVCAR-3 cells, COV413A cells (ECACC), COV413B (ECACC), and COV362 (ECACC) were seeded on culture plates, and TGF-β1 was added at 10 ng/mL. Four days later, cells were recovered, and CLDN6 expression was analyzed by FACS analysis.

In the FACS analysis, cells were detached from the culturing flask to produce a cell suspension, these cells were stained using an anti-CLDN6 antibody (CS4135 mIgG1: the heavy-chain and light-chain amino acid sequences are shown in SEQ ID NOs: 210 and 211, respectively, and the heavy-chain and light-chain nucleotide sequences are shown in SEQ ID NOs: 212 and 213, respectively) labeled with Alexa Flour 488 (Alexa Fluor 488 antibody labeling kit, ThermoFisher Scientific), and the amount of CLDN6 expression was analyzed using a flow cytometer (FACSlyric, BD Biosciences). The CS4135 mIgG1 antibody is a chimeric antibody produced by linking the Fc region of a mouse IgG1 to the Fab of the CS4135 antibody.

Fig. 32 shows the results of analyzing CLDN6 expression by FACS in each cell type stimulated with TGF-β1 and each type of untreated cell. In all cells, it was observed that TGF-β1 stimulation increased CLDN6 expression. That is, TGF-β1 was confirmed to be an agent that induces CLDN6 expression in various cancer cells. Therefore, while not wishing to be bound to any particular theory, an agent that induces TGF-β1 expression is considered to enhance CLDN6 expression, and combined use of an agent that induces TGF-β1 expression and a CLDN6-binding antibody is considered to provide additive or synergistic anticancer effects.

[Reference Example 1] Purification of anti-CLDN6/Dual-Fab tri-specific antibodies

The heavy and light chain variable regions were cloned into expression vectors containing the heavy chain and light chain constant regions with respective mutations for hetero-dimerization.

For large scale preparation of anti-CLDN6/Dual-Fab tri-specific antibodies for in vitro and in vivo studies, the antibodies were transiently expressed using Expi293F cells (Life technologies), according to the manufacturer's instructions. Culture medium containing recombinant antibodies was first purified with MabSelect Sure (GE healthcare) column and eluted with 50mM Acetic acid. Eluted antibodies were neutralized with 1.5M Tris HCl/1M Arginine HCl buffer. ProA eluates were then loaded onto the cation exchange HiTrap SP-HP (GE healthcare) column in 20 mM Sodium Phosphate, pH6 buffer and eluted with 20 mM Sodium Phosphate, 1M NaCl, pH6 buffer. Fractions containing the bispecific antibody were pooled and concentrated. To remove high molecular weight and/or low molecular weight components, size exclusion chromatography was performed in P1 buffer (20mM Histidine, 150mM Arginine, 162.1mM Asp, pH6.0) using Superdex 200 column (GE healthcare). Purified bispecific antibodies were concentrated and stored in -80 degrees C freezer.

[Reference Example 2] Generation of Claudin expressing cells

Ba/F3 cells expressing human CLDN6 (hCLDN6/BaF), Ba/F3 cells expressing human CLDN9 (hCLDN9/BaF), Ba/F3 cells expressing human CLDN3 (hCLDN3/BaF), Ba/F3 cells expressing human CLDN4 (hCLDN4/BaF), Ba/F3 cells expressing mouse CLDN6 (mCLDN6/BaF), Ba/F3 cells expressing mouse CLDN9 (mCLDN9/BaF), Ba/F3 cells expressing mouse CLDN3 (mCLDN3/BaF), and Ba/F3 cells expressing mouse CLDN4 (mCLDN4/BaF), were established by transfecting human CLDN6, human CLDN9 (SEQ ID NO: 198), human CLDN3 (SEQ ID NO: 199), human CLDN4 (SEQ ID NO: 200), mouse CLDN6 (SEQ ID NO: 201), mouse CLDN9 (SEQ ID NO: 202), mouse CLDN3 (SEQ ID NO: 203), and mouse CLDN4 (SEQ ID NO:204) expression vectors into mouse pro B cell line Ba/F3 respectively.

Claudin family proteins have two extracellular domains which are accessible to antibody. With regard to amino acid sequence similarity between the extracellular domains of human CLDN6 and human CLDN9, the first extracellular domain is almost the same, and there are only two different amino acids in the second extracellular domain (Figure 18). The glutamine at position 156 of human Claudin 6 (position 156 in the sequence shown in SEQ ID NO: 196 or 197) was substituted to leucine to make a human CLDN6 mutant comprising the same amino acid as human Claudin 9 at position 156. This human CLDN6 mutant was named as hCLDN6(Q156L) (SEQ ID NO: 205). Ba/F3 transfectant stably expressing hCLDN6(Q156L) was generated using similar method described above. The established Ba/F3 transfectant was named as hCLDN6(Q156L)BaF.

FreeStyle™ 293-F transfectant cells transiently expressing human and mouse CLDN3, 4, 6, and 9 were generated by introducing expressing vector of human and mouse CLDNs (including CLDN6, CLDN9, CLDN3, and CLDN4) into FreeStyle™ 293-F cells (Invitrogen) using 293fectin (Invitrogen). The generated FreeStyle™ 293-F transfectant cells were named as hCLDN3/FS293, hCLDN4/FS293, hCLDN6/FS293, hCLDN9/FS293, mCLDN3/FS293, mCLDN4/FS293, mCLDN6/FS293, and mCLDN9/FS293, respectively.

[Industrial Applicability]

The present disclosure provides anticancer agents comprising a multispecific antigen-binding molecule that is capable of binding to CD3 and CD137 (4-1BB), that binds to either CD3 or CD137 and that is capable of binding to CLDN6; combination therapies using the anticancer agents with at least one other anticancer agent; and pharmaceutical compositions and such for use in the combination therapies. The multispecific antigen-binding molecules included in the anticancer agents, pharmaceutical compositions, combinations, or kits of the present disclosure, or used in the methods or uses of the present disclosure, can be used to target CLDN6-expressing cells, for use in immunotherapy for treating various cancers, particularly CLDN6-related cancer, such as CLDN6-positive cancer.

[Sequence Listing]

## Claims

**1.** An anticancer agent comprising as an active ingredient a multispecific antigen-binding molecule, the multispecific antigen-binding molecule comprising (i) a first antigen-binding moiety that is capable of binding to CD3 and CD137 and that binds to either CD3 or CD137, and (ii) a second antigen-binding moiety that is capable of binding to claudin 6 (CLDN6).

**2.** An anticancer agent comprising as an active ingredient a multispecific antigen-binding molecule of any one of (1) to (6) below:

(1) a multispecific antigen-binding molecule that comprises a first antibody variable region comprising the complementarity determining region (CDR) 1 of SEQ ID NO: 11, the CDR2 of SEQ ID NO: 17, and the CDR3 of SEQ ID NO: 23; a second antibody variable region comprising the CDR1 of SEQ ID NO: 32, the CDR2 of SEQ ID NO: 36, and the CDR3 of SEQ ID NO: 40; a third antibody variable region comprising the complementarity determining region (CDR) 1 of SEQ ID NO: 7, the CDR2 of SEQ ID NO: 13, and the CDR3 of SEQ ID NO: 19; and a fourth antibody variable region comprising the CDR1 of SEQ ID NO: 29, the CDR2 of SEQ ID NO: 33, and the CDR3 of SEQ ID NO: 37;
(2) a multispecific antigen-binding molecule that comprises a first antibody variable region comprising the complementarity determining region (CDR) 1 of SEQ ID NO: 9, the CDR2 of SEQ ID NO: 15, and the CDR3 of SEQ ID NO: 21; a second antibody variable region comprising the CDR1 of SEQ ID NO: 31, the CDR2 of SEQ ID NO: 35, and the CDR3 of SEQ ID NO: 39; a third antibody variable region comprising the complementarity determining region (CDR) 1 of SEQ ID NO: 8, the CDR2 of SEQ ID NO: 14, and the CDR3 of SEQ ID NO: 20; and a fourth antibody variable region comprising the CDR1 of SEQ ID NO: 30, the CDR2 of SEQ ID NO: 34, and the CDR3 of SEQ ID NO: 38;
(3) a multispecific antigen-binding molecule that comprises a first antibody variable region comprising the complementarity determining region (CDR) 1 of SEQ ID NO: 10, the CDR2 of SEQ ID NO: 16, and the CDR3 of SEQ ID NO: 22; a second antibody variable region comprising the CDR1 of SEQ ID NO: 31, the CDR2 of SEQ ID NO: 35, and the CDR3 of SEQ ID NO: 39; a third antibody variable region comprising the complementarity determining region (CDR) 1 of SEQ ID NO: 8, the CDR2 of SEQ ID NO: 14, and the CDR3 of SEQ ID NO: 20; and a fourth antibody variable region comprising the CDR1 of SEQ ID NO: 30, the CDR2 of SEQ ID NO: 34, and the CDR3 of SEQ ID NO: 38;
(4) a multispecific antigen-binding molecule that comprises a first antibody variable region comprising the complementarity determining region (CDR) 1 of SEQ ID NO: 12, the CDR2 of SEQ ID NO: 18, and the CDR3 of SEQ ID NO: 24; a second antibody variable region comprising the CDR1 of SEQ ID NO: 32, the CDR2 of SEQ ID NO: 36, and the CDR3 of SEQ ID NO: 40; a third antibody variable region comprising the complementarity determining region (CDR) 1 of SEQ ID NO: 7, the CDR2 of SEQ ID NO: 13, and the CDR3 of SEQ ID NO: 19; and a fourth antibody variable region comprising the CDR1 of SEQ ID NO: 29, the CDR2 of SEQ ID NO: 33, and the CDR3 of SEQ ID NO: 37;

(5) a multispecific antigen-binding molecule that comprises a first antibody variable region comprising the complementarity determining region (CDR) 1 of SEQ ID NO: 11, the CDR2 of SEQ ID NO: 17, and the CDR3 of SEQ ID NO: 23; a second antibody variable region comprising the CDR1 of SEQ ID NO: 32, the CDR2 of SEQ ID NO: 36, and the CDR3 of SEQ ID NO: 40; a third antibody variable region comprising the complementarity determining region (CDR) 1 of SEQ ID NO: 29, the CDR2 of SEQ ID NO: 33, and the CDR3 of SEQ ID NO: 37; and a fourth antibody variable region comprising the CDR1 of SEQ ID NO: 7, the CDR2 of SEQ ID NO: 13, and the CDR3 of SEQ ID NO: 19; and

(6) a multispecific antigen-binding molecule that comprises a first antibody variable region comprising the complementarity determining region (CDR) 1 of SEQ ID NO: 12, the CDR2 of SEQ ID NO: 18, and the CDR3 of SEQ ID NO: 24; a second antibody variable region comprising the CDR1 of SEQ ID NO: 32, the CDR2 of SEQ ID NO: 36, and the CDR3 of SEQ ID NO: 40; a third antibody variable region comprising the complementarity determining region (CDR) 1 of SEQ ID NO: 29, the CDR2 of SEQ ID NO: 33, and the CDR3 of SEQ ID NO: 37; and a fourth antibody variable region comprising the CDR1 of SEQ ID NO: 7, the CDR2 of SEQ ID NO: 13, and the CDR3 of SEQ ID NO: 19.

**3.** The anticancer agent of claim 1 or 2, wherein the cancer of interest is CLDN6-positive cancer.

**4.** The anticancer agent of any one of claims 1 to 3, wherein the cancer of interest is at least one cancer selected from the group consisting of ovary cancer, non-small cell lung cancer, gastric cancer, liver cancer, endometrial cancer, germ cell tumor, large bowel cancer, urinary bladder cancer, and atypical teratoid rhabdoid tumor.

**5.** The anticancer agent of any one of claims 1 to 4, wherein the cancer of interest is cancer metastasized to the peritoneum.

**6.** A pharmaceutical composition for use in combination with at least one other anticancer agent, wherein the pharmaceutical composition comprises as an active ingredient a multispecific antigen-binding molecule, the multispecific antigen-binding molecule comprising (i) a first antigen-binding moiety that is capable of binding to CD3 and CD137 and that binds to either CD3 or CD137, and (ii) a second antigen-binding moiety that is capable of binding to claudin 6 (CLDN6).

**7.** A pharmaceutical composition for use in combination with at least one other anticancer agent, wherein the pharmaceutical composition comprises as an active ingredient the multispecific antigen-binding molecule of any one of (1) to (6) below:

(1) a multispecific antigen-binding molecule that comprises a first antibody variable region comprising the complementarity determining region (CDR) 1 of SEQ ID NO: 11, the CDR2 of SEQ ID NO: 17, and the CDR3 of SEQ ID NO: 23; a second antibody variable region comprising the CDR1 of SEQ ID NO: 32, the CDR2 of SEQ ID NO: 36, and the CDR3 of SEQ ID NO: 40; a third antibody variable region comprising the complementarity determining region (CDR) 1 of SEQ ID NO: 7, the CDR2 of SEQ ID NO: 13, and the CDR3 of SEQ ID NO: 19; and a fourth antibody variable region comprising the CDR1 of SEQ ID NO: 29, the CDR2 of SEQ ID NO: 33, and the CDR3 of SEQ ID NO: 37;

(2) a multispecific antigen-binding molecule that comprises a first antibody variable region comprising the complementarity determining region (CDR) 1 of SEQ ID NO: 9, the CDR2 of SEQ ID NO: 15, and the CDR3 of SEQ ID NO: 21; a second antibody variable region comprising the CDR1 of SEQ ID NO: 31, the CDR2 of SEQ ID NO: 35, and the CDR3 of SEQ ID NO: 39; a third antibody variable region comprising the complementarity determining region (CDR) 1 of SEQ ID NO: 8, the CDR2 of SEQ ID NO: 14, and the CDR3 of SEQ ID NO: 20; and a fourth antibody variable region comprising the CDR1 of SEQ ID NO: 30, the CDR2 of SEQ ID NO: 34, and the CDR3 of SEQ ID NO: 38;

(3) a multispecific antigen-binding molecule that comprises a first antibody variable region comprising the complementarity determining region (CDR) 1 of SEQ ID NO: 10, the CDR2 of SEQ ID NO: 16, and the CDR3 of SEQ ID NO: 22; a second antibody variable region comprising the CDR1 of SEQ ID NO: 31, the CDR2 of SEQ ID NO: 35, and the CDR3 of SEQ ID NO: 39; a third antibody variable region comprising the complementarity determining region (CDR) 1 of SEQ ID NO: 8, the CDR2 of SEQ ID NO: 14, and the CDR3 of SEQ ID NO: 20;

and a fourth antibody variable region comprising the CDR1 of SEQ ID NO: 30, the CDR2 of SEQ ID NO: 34, and the CDR3 of SEQ ID NO: 38;
(4) a multispecific antigen-binding molecule that comprises a first antibody variable region comprising the complementarity determining region (CDR) 1 of SEQ ID NO: 12, the CDR2 of SEQ ID NO: 18, and the CDR3 of SEQ ID NO: 24; a second antibody variable region comprising the CDR1 of SEQ ID NO: 32, the CDR2 of SEQ ID NO: 36, and the CDR3 of SEQ ID NO: 40; a third antibody variable region comprising the complementarity determining region (CDR) 1 of SEQ ID NO: 7, the CDR2 of SEQ ID NO: 13, and the CDR3 of SEQ ID NO: 19; and a fourth antibody variable region comprising the CDR1 of SEQ ID NO: 29, the CDR2 of SEQ ID NO: 33, and the CDR3 of SEQ ID NO: 37;
(5) a multispecific antigen-binding molecule that comprises a first antibody variable region comprising the complementarity determining region (CDR) 1 of SEQ ID NO: 11, the CDR2 of SEQ ID NO: 17, and the CDR3 of SEQ ID NO: 23; a second antibody variable region comprising the CDR1 of SEQ ID NO: 32, the CDR2 of SEQ ID NO: 36, and the CDR3 of SEQ ID NO: 40; a third antibody variable region comprising the complementarity determining region (CDR) 1 of SEQ ID NO: 29, the CDR2 of SEQ ID NO: 33, and the CDR3 of SEQ ID NO: 37; and a fourth antibody variable region comprising the CDR1 of SEQ ID NO: 7, the CDR2 of SEQ ID NO: 13, and the CDR3 of SEQ ID NO: 19; and
(6) a multispecific antigen-binding molecule that comprises a first antibody variable region comprising the complementarity determining region (CDR) 1 of SEQ ID NO: 12, the CDR2 of SEQ ID NO: 18, and the CDR3 of SEQ ID NO: 24; a second antibody variable region comprising the CDR1 of SEQ ID NO: 32, the CDR2 of SEQ ID NO: 36, and the CDR3 of SEQ ID NO: 40; a third antibody variable region comprising the complementarity determining region (CDR) 1 of SEQ ID NO: 29, the CDR2 of SEQ ID NO: 33, and the CDR3 of SEQ ID NO: 37; and a fourth antibody variable region comprising the CDR1 of SEQ ID NO: 7, the CDR2 of SEQ ID NO: 13, and the CDR3 of SEQ ID NO: 19.

**8.** The pharmaceutical composition of claim 6 or 7, wherein the cancer of interest is CLDN6-positive cancer.

**9.** The pharmaceutical composition of any one of claims 6 to 8, wherein the cancer of interest is any cancer selected from the group consisting of ovary cancer, non-small cell lung cancer, gastric cancer, liver cancer, endometrial cancer, germ cell tumor, large bowel cancer, urinary bladder cancer, and atypical teratoid rhabdoid tumor.

**10.** The pharmaceutical composition of any one of claims 6 to 9, wherein the cancer of interest is cancer metastasized to the peritoneum.

**11.** The pharmaceutical composition of any one of claims 6 to 10, wherein the multispecific antigen-binding molecule is administered before, simultaneously with, and/or after the administration of the at least one other anticancer agent.

**12.** The pharmaceutical composition of any one of claims 6 to 11, wherein the multispecific antigen-binding molecule is administered to cancer in which CLDN6 expression has been increased by the administration of the at least one other anticancer agent.

**13.** The pharmaceutical composition of any one of claims 6 to 12, wherein the at least one other anticancer agent is at least one selected from the group consisting of a chemotherapeutic agent, an immune checkpoint inhibitor, and a PARP inhibitor.

**14.** A cytotoxicity-inducing agent, a cell growth suppressor, a cell growth inhibitor, an immune response activator, a cancer therapeutic agent, or a cancer preventive agent, comprising the pharmaceutical composition of any one of claims 6 to 13.

**15.** A method for inducing cytotoxicity, suppressing cell proliferation, inhibiting cell proliferation, activating immune response, treating cancer, or preventing cancer in an individual, comprising administering an effective amount of a multispecific antigen-binding molecule and an effective amount of at least one other anticancer agent, wherein the multispecific antigen-binding molecule comprises (i) a first antigen-binding moiety that is capable of binding to CD3 and CD137 and that binds to either CD3 or CD137, and (ii) a second antigen-binding moiety that is capable of binding claudin 6 (CLDN6).

[Fig.1]

CLDN6 expression
TPM (log10)
10000
1000
100
10
1
0.1
Cholangiocarcinoma
Adrenocortical carcinoma
Acute myeloid leukemia
Cervical cancer
Breast cancer
Glioma
Glioblastoma
Sarcoma
Colon cancer
Liver cancer
Kidney cancer
Esophageal cancer
Mesothelioma
Pancreas cancer
Ovary cancer
DLBCL
NSCLC (Ad)
NSCLC (Sq)
Paraganglioma
Prostate cancer
Rectum cancer
Skin cancer
Germ cell tumor
Gastric cancer
Thyroid cancer
Thymic carcinoma
Upper aerodigestive tract cancer
Urinary bladder cancer
Uterine cancer
Uveal melanoma

[Fig.2]

CD137(24-186)
LQDPCSNCPAGTFCDNNRNQICSPCPPNSFSSAGGQRTCDICRQCK
GVFRTRKECSSTSNAECDCTPGFHCLGAGCSMCEQDCKQGQELTKKGCKD
CCFGTFNDQKRGICRPWTNCSLDGKSVLVNGTKERDVVCGPSPADLSPGA
SSVTPPAPAREPGHSPQ
< 3.0 Å
< 4.5 Å

[Fig. 3]

CD137
I64
D63
C62
T61
Q59
R41
Q43
H_CDR3
N40
N42
R60
G58
C28
H_CDR1
L_CDR1
N39
N30
S29
I44
H_CDR2
L_CDR2
D26
P27
L24
Q25
L_CDR3
light chain
heavy chain
CD137
180°
I64
D63
H_CDR3
C62
T61
R41
Q59
N40
N39
R60
G58
N30
L_CDR1
N42
I44
H_CDR2
C28
L_CDR3
S29
H_CDR1
P27
D26
L_CDR2
Q25
heavy chain
L24
light chain

[Fig. 4]

(a)
Tri-specific Ab (1+1)Ig
FAST-Ig
Chain 1
Chain 2
Chain 3
Chain 4
Fv A
Fv B
Fc region
(b)
Tri-specific Ab (1+1)
CrossMab
VH/VL CrossMab
Chain 1
Chain 2
Chain 3
Chain 4
Fv A
Fv B
Fc region
Variable heavy chain domain (VH)
Variable light chain domain (VL)
Constant heavy chain domain 1 (CH1)
Constant light chain domain (CL)

[Fig. 5]

KLH/TR01
CLDN6/CD3 Bispecific antibodies
CS2961
6PHU3/TR01
hCLDN6/BaF3
hCLDN3/BaF3
hCLDN4/BaF3
hCLDN9/BaF3
CLDN6_2961 - P1
CLDN6_6PHU3 - P1
CLDN3_2961 - P1
CLDN3_6PHU3 - P1
CLDN4_2961 - P1
CLDN4_6PHU3 - P1
CLDN9_2961 - P1
CLDN9_6PHU3 - P1
Count
FITC-A
17798, 130
48021, 200
68132, 189
4153, 202
7932, 153
65, 206
33815, 117
2170, 240

[Fig. 6]

LDH assay
NIH:OVCAR-3
A2780
COV413A
Cytotoxicity (%)
Ab conc. (nM)
CS3348
PPU4134 PPU4134: CLDN6AE25EK/DualAE05KE-SG13251326 : Ab01
PPU4135 PPU4135: CLDN6AE25/DualAE05-SG14001326: Ab03
PPU4136 PPU4136: CLDN6AE25/DualAE15-SG14001326: Ab04
PPU4137 PPU4137: xCLDN6AE25/DualAE05-xSG1350k1349hV11: Ab17
PPU4138 PPU4138: xCLDN6AE25/DualAE15-xSG1350k1349hV11: Ab18

[Fig. 7]


Extracellular domain 1

```
hCLDN6    1 MASAGMQILGVVLTLLGWVNGLVSCALPMWKVTAFIGNSIVVAQVVWEGLWMSCVVQSTGQMQCKVYDSLLALPQDLQAARALCVIALLV  90
hCLDN9    1 MASTGLELLGMTLAVLGWLGTLVSCALPLWKVTAFIGNSIVVAQVVWEGLWMSCVVQSTGQMQCKVYDSLLALPQDLQAARALCVVALLL  90

hCLDN6   91 ALFGLLVYLAGAKCTTCVEEKDSKARLVLTSGIVFVISGVLTLIPVCWTAHAIIRDFYNPLVAEAQKRELGASLYLGWAASGLLLLGGGL 180
hCLDN9   91 ALLGLLVAITGAQCTTCVEDEGAKARIVLTAGVLLLLSGILVLIPVCWTAHAIIQDFYNPLVAEALKRELGASLYLGWAAAALLMLGGGL 180

hCLDN6  181 LCCTCPSGGSQGPSHYMARYSTSAPAISRGPSEYPTKNYV*                                                  221
hCLDN9  181 LCCTCPPSHFERPRGPRLGYSIPSRSGASGLDKRDYV*                                                     218
```

Extracellular domain 2

[Fig. 8]

NUGC-3
PA-1
SNG-M
NEC8
NEC14
CHLA-02-ATRT
HT-1197
OUMS-23
Cytotoxicity (%)
Antibody concentration (nM)
0.001 0.01 0.1 1 10

[Fig. 9]

NIH:OVCAR-3
Cell growth inhibition (%)
120
80
40
0
-40
0.001
0.01
0.1
1
10
Ab conc. (nM)
NCI-H1435
Cell growth inhibition (%)
120
80
40
0
-40
0.001
0.01
0.1
1
10
Ab conc. (nM)
CS3348
PPU4135
PPU4136

[Fig. 10]

OVCAR3
Luminescence fold (RLU)
250
200
150
100
50
0
CS3348
PPU4135
PPU4136
PPU4137
PPU4138
KLH/TR01
0.003
0.03
0.3
3
30
Ab conc. (nM)
NCI-H1435
Luminescence fold (RLU)
160
140
120
100
80
60
40
20
0
CS3348
PPU4135
PPU4136
PPU4137
PPU4138
KLH/TR01
0.003
0.03
0.3
3
30
Ab conc. (nM)
5637
Luminescence fold (RLU)
50
45
40
35
30
25
20
15
10
5
0
CS3348
PPU4135
PPU4136
PPU4137
PPU4138
KLH/TR01
0.003
0.03
0.3
3
30
Ab conc. (nM)

[Fig. 11]

NCI-H1435
OVCAR3
5637
luminescence fold
Ab (nM)
CS3348
PPU4134
PPU4135
PPU4136
PPU4137
PPU4138
KLH/TR01

[Fig. 12]

Vehicle
CS3348 1mg/kg
PPU4134 1mg/kg
PPU4135 1mg/kg
PPU4136 1mg/kg
PPU4137 1mg/kg
PPU4138 1mg/kg
Tumor volume (mm³)
Days after tumor transplantation
Body weight (g)
Days after tumor transplantation

[Fig. 13]

0.05mg/kg
0.2mg/kg
Tumor volume (mm3)
2000
1500
1000
500
0
10
15
20
25
30
35
Days after tumor transplantation
Vehicle
CS3348
PPU4135

[Fig. 14]

ALT
ALT (U/L)
600
400
200
0
-15
1
8
15
Day
AST
AST (U/L)
PPU4135_90 µg/kg_Male (1)
PPU4135_90 µg/kg_Male (2)
CS3348_100 µg/kg_Male
CS3348_100 µg/kg_Female
GLDH
GLDH (U/L)
1500
1000
500
CRP
CRP (mg/dL)
10
8
6
4
2
ALP
ALP (U/L)
15000
10000
5000
GGT
GGT (U/L)
800
TBA
TBA (µmol/L)
1000
TBIL
TBIL (mg/dL)
5
3
1

[Fig. 15]

120
100
80
60
40
20
0
Survival rate (%)
Vehicle
CS4135
0
10
20
30
40
50
60
70
80
Days after tumor inoculation

[Fig. 16]

SNG-M
Fluorescence
1200
1000
800
600
400
200
0
NT
CDDP(0.6μg/ml)
CBDCA(5μg/ml)
Isotype
IgG
AE3-20

NCI-H1435
Fluorescence
1200
1000
800
600
400
200
0
NT
CDDP(0.6μg/ml)
CBDCA(5μg/ml)
Isotype
IgG
AE3-20

NIH-OVCAR3
Fluorescence
1200
000
800
600
400
200
0
NT
CDDP(0.1μg/ml)
CBDCA(0.5μg/ml)
Isotype
IgG
AE3-20

[Fig. 17]

NIH-OVCAR3
NFAT activation
400
300
200
100
0
-100
0.01
0.1
1
10
100
nM
NT
CDDP(0.1μg/ml)
CBDCA(0.5μg/ml)
NCI-H1435
NFAT activation
500
400
300
200
100
0
-100
0.01
0.1
1
10
100
nM
NT
CDDP(0.5μg/ml)
CBDCA(6μg/ml)

[Fig. 18]

CLDN6 expression
Relative expression of CLDN6 to GAPDH
0.006
0.004
0.002
0.000
Vehicle x2
40mg/kg x2
80mg/kg x2

[Fig. 19]

700
600
500
400
300
200
100
0
Tumor volume (mm3)
10
15
20
25
30
35
Days after tumor transplantation
Vehicle control
CS4135
Carboplatin
CS4135+Carboplatin

[Fig. 20]

Tumor volume(mm3)
300
200
100
0
35
40
45
50
55
60
Days after tumor transplantation
Vehicle control
CS4135
Carboplatin
CS4135+Carboplatin

[Fig. 21]

Tumor volume(mm3)
300
200
100
0
35
40
45
50
55
60
Days after tumor transplantation
Vehicle control
CS4135
Carboplatin
Carboplatin ⇒CS4135

[Fig. 22]

600
500
400
300
200
100
0
Tumor volume (mm³ +SD)
35
40
45
50
55
60
65
Days after tumor transplantation
Vehicle control
CS4135
Irinotecan hydrochloride
CS4135 +
Irinotecan hydrochloride
*
*: Student's t-test

[Fig. 23]

Tumor volume(mm3, +SD)
1200
1000
800
600
400
200
0
5 6 7 8 9 10 11 12 13 14 15
Days after tumor transplantation
Vehicle
CS4135
Vehicle
CS4135

[Fig. 24]

CD8+ T cells per tumor volume
(cells /mg)
60000
40000
20000
0
Day2
Day6
Vehicle
CS4135

[Fig. 25]

1000
800
600
400
200
0
5
10
15
20
Tumor volume(mm3)
Days after tumor transplantation
Vehicle control
CS4135
Anti-mouse PD-L1 antibody
CS4135+
Anti-mouse PD-L1 antibody

[Fig. 26]

2.0
1.5
1.0
0.5
Ratio of fluoroscence intensity
UWB1.289
OV90
0
0.03
0.1
0.3
1
3
Olaparib concentration (μM)

[Fig. 27]

UWB1.289
Cytotoxic activity (%)
15
10
5
0
-5
0.001
0.01
0.1
1
10
Antibody concentration (nM)
Olaparib
DMSO
OV90
Cytotoxic activity (%)
25
20
15
10
5
0
0.001
0.01
0.1
1
10
Antibody concentration (nM)
Olaparib
DMSO

[Fig. 28]

Cell growth inhibition rate (%)
60
50
40
30
20
10
0
KLH/CD137 Ab
0nM
0nM
100nM
500nM
Effector cell
hCD3 tgm
hCD3/hCD137 KI

[Fig. 29]

Human CLDN6 expression
Relative CLDN6 expression to GAPDH
0.04
0.03
0.02
0.01
0
Untreated
Carboplatin
Cisplatin
Irinotecan
Gemcitabine
Pharmaceutical agent

[Fig. 30]

Human TGFβ1 expression
Relative TGFβ1 expression to GAPDH
0.005
0.004
0.003
0.002
0.001
0
Untreated
Carboplatin
Cisplatin
Irinotecan
Gemcitabine
Pharmaceutical agent

[Fig. 31]

CLDN6
Relative expression to GAPDH
0.020
0.015
0.010
0.005
0.000
Untreated
TGFβ 1

[Fig. 32]

NIH:OVCAR-3
Cell count
100
50
0
0
10^2
10^3
10^4
10^5
CLDN6

COV362
Cell count
250
200
150
100
50
0
0
10^2
10^3
10^4
10^5
CLDN6

No stimulation

TGFβ1 stimulation

COV413A
Cell count
200
150
100
50
0
0
10^2
10^3
10^4
10^5
CLDN6

COV413B
Cell count
200
150
100
50
0
0
10^2
10^3
10^4
10^5
CLDN6

**INTERNATIONAL SEARCH REPORT**

International application No.
**PCT/JP2022/036060**

**A. CLASSIFICATION OF SUBJECT MATTER**

***A61K 39/395***(2006.01)i; ***A61P 35/00***(2006.01)i; ***A61P 43/00***(2006.01)i
FI: A61K39/395 T ZNA; A61P35/00; A61P43/00 121

According to International Patent Classification (IPC) or to both national classification and IPC

**B. FIELDS SEARCHED**

Minimum documentation searched (classification system followed by classification symbols)

A61K39/395; A61P35/00; A61P43/00

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

JSTPlus/JMEDPlus/JST7580 (JDreamIII); CAplus/MEDLINE/EMBASE/BIOSIS (STN)

**C. DOCUMENTS CONSIDERED TO BE RELEVANT**

| Category* | Citation of document, with indication, where appropriate, of the relevant passages | Relevant to claim No. |
|---|---|---|
| Y | WO 2019/111871 A1 (CHUGAI SEIYAKU KABUSHIKI KAISHA) 13 June 2019 (2019-06-13)<br>claims 1, 2, paragraphs [0033], [0134] | 1-15 |
| Y | JP 2016-500059 A (BIONTECH AG) 07 January 2016 (2016-01-07)<br>claims 1, 2, 7, 28, 33, paragraphs [0278]-[0294] | 1-15 |
| Y | WO 2019/135404 A1 (CHUGAI SEIYAKU KABUSHIKI KAISHA) 11 July 2019 (2019-07-11)<br>claims 1, 14, 15, paragraphs [0011], [0175], [0178], [0215], table 2 | 1-15 |
| Y | FABER, S. M. et al. Bispecific claudin-6 x CD3 antibodies in a 2 + 1 format demonstrate selectivity and activity on human ovarian cancer cells. Cancer Res. 01 July 2021, vol. 81, no. 13 Suppl. Abs no.1860<br>entire text | 1-15 |
| Y | WO 2020/168059 A1 (INTEGRAL MOLECULAR, INC.) 20 August 2020 (2020-08-20)<br>claim 34, p. 96 | 1-15 |

☑ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **22 November 2022** | **13 December 2022** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | Telephone No. |

Form PCT/ISA/210 (second sheet) (January 2015)

**INTERNATIONAL SEARCH REPORT**

International application No.
**PCT/JP2022/036060**

**C. DOCUMENTS CONSIDERED TO BE RELEVANT**

| Category* | Citation of document, with indication, where appropriate, of the relevant passages | Relevant to claim No. |
|---|---|---|
| Y | WO 2020/191344 A1 (THE REGENTS OF THE UNIVERSITY OF CALIFORNIA) 24 September 2020 (2020-09-24)<br>claims 1, 59, 60, 98-105 | 1-15 |
| Y | WO 2020/067399 A1 (CHUGAI SEIYAKU KABUSHIKI KAISHA) 02 April 2020 (2020-04-02)<br>table 1.1 | 2, 7 |
| P, X | JP 2021-168648 A (CHUGAI SEIYAKU KABUSHIKI KAISHA) 28 October 2021 (2021-10-28)<br>claims 1-15 | 1-15 |
| A | WO 2020/067419 A1 (CHUGAI SEIYAKU KABUSHIKI KAISHA) 02 April 2020 (2020-04-02)<br>entire text | 1-15 |

Form PCT/ISA/210 (second sheet) (January 2015)

**INTERNATIONAL SEARCH REPORT**

International application No.
**PCT/JP2022/036060**

**Box No. I Nucleotide and/or amino acid sequence(s) (Continuation of item 1.c of the first sheet)**

1. With regard to any nucleotide and/or amino acid sequence disclosed in the international application, the international search was carried out on the basis of a sequence listing:
   - a. ☑ forming part of the international application as filed:
     - ☑ in the form of an Annex C/ST.25 text file.
     - ☐ on paper or in the form of an image file.
   - b. ☐ furnished together with the international application under PCT Rule 13*ter*.1(a) for the purposes of international search only in the form of an Annex C/ST.25 text file.
   - c. ☐ furnished subsequent to the international filing date for the purposes of international search only:
     - ☐ in the form of an Annex C/ST.25 text file (Rule 13*ter*.1(a)).
     - ☐ on paper or in the form of an image file (Rule 13*ter*.1(b) and Administrative Instructions, Section 713).

2. ☐ In addition, in the case that more than one version or copy of a sequence listing has been filed or furnished, the required statements that the information in the subsequent or additional copies is identical to that forming part of the application as filed or does not go beyond the application as filed, as appropriate, were furnished.

3. Additional comments:

   "Annex C/ST.25 text file format" should be read as "ST.26 format".

Form PCT/ISA/210 (continuation of first sheet) (January 2015)

**INTERNATIONAL SEARCH REPORT**
**Information on patent family members**

International application No.
**PCT/JP2022/036060**

| Patent document cited in search report | Publication date (day/month/year) | Patent family member(s) | Publication date (day/month/year) |
|---|---|---|---|
| WO 2019/111871 A1 | 13 June 2019 | JP 2021-508441 A | |
| | | US 2020/0377595 A1 | |
| | | EP 3720963 A1 | |
| JP 2016-500059 A | 07 January 2016 | US 2016/0272711 A1 | |
| | | WO 2014/075697 A1 claims 1, 2, 7, 28, 33, pp. 85-93 | |
| | | EP 2920209 A1 | |
| | | CN 105073776 A | |
| | | KR 10-2015-0125642 A | |
| WO 2019/135404 A1 | 11 July 2019 | JP 2021-510064 A | |
| | | US 2021/0054076 A1 | |
| | | EP 3735463 A1 | |
| WO 2020/168059 A1 | 20 August 2020 | JP 2022-521723 A | |
| | | US 2020/0262915 A1 | |
| | | EP 3924389 A1 | |
| | | KR 10-2021-0128443 A | |
| | | CN 113767113 A | |
| WO 2020/191344 A1 | 24 September 2020 | JP 2022-525478 A | |
| | | US 2022/0177583 A1 | |
| | | EP 3941944 A1 | |
| | | CN 114206934 A | |
| WO 2020/067399 A1 | 02 April 2020 | JP 2022-501325 A | |
| | | US 2022/0112296 A1 | |
| | | EP 3856789 A1 | |
| | | KR 10-2021-0068061 A | |
| | | CN 113260634 A | |
| JP 2021-168648 A | 28 October 2021 | WO 2021/200939 A1 claims 1-15 | |
| | | KR 10-2021-0143923 A | |
| WO 2020/067419 A1 | 02 April 2020 | JP 2022-502010 A | |
| | | US 2021/0388087 A1 | |
| | | EP 3856785 A1 | |
| | | KR 10-2021-0068079 A | |
| | | CN 113166247 A | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2009087978 A
- WO 2011057788 A
- WO 2012003956 A
- WO 2012156018 A
- WO 2015069794 A
- WO 2014075697 A
- WO 2014075788 A
- US 4816567 A
- US 5648237 A
- US 5789199 A
- US 5840523 A
- US 5959177 A
- US 6040498 A
- US 6420548 B
- US 7125978 B
- US 6417429 B
- US 6248516 B1
- WO 2015143414 A
- US 20110123527 A1
- WO 9316185 A
- US 5571894 A
- US 5587458 A
- US 5869046 A
- EP 404097 A
- WO 199301161 A
- WO 1988001649 A
- US 4946778 A
- US 5260203 A
- WO 2006132352 A
- WO 2012130831 A
- WO 200492219 A, Hinton
- WO 200042072 A, Presta
- US 5731168 A
- US 7695936 B
- WO 2006106905 A
- WO 2011028952 A
- WO 2014018572 A
- WO 2008119353 A
- WO 2011131746 A
- WO 2012058768 A
- WO 2013063702 A
- WO 2012023053 A
- WO 2007114325 A
- WO 98050431 A
- WO 95033844 A
- US 5500362 A
- US 5821337 A
- US 6737056 B, Presta
- US 6194551 B1
- WO 199951642 A
- US 20050260186 A
- US 20060104968 A
- US 6267958 B
- US 6171586 B
- WO 2006044908 A
- US 3773719 A
- EP 58481 A
- EP 133988 A
- WO 2015174439 A
- WO 2015156268 A
- WO 2013065708 A
- JP 5848863 B


### Non-patent literature cited in the description


- **FURUSE ; TSUKITA.** *TRENDS in Cell Biology,* 2006, vol. 16, 181
- **WILCOX et al.** *Cell,* 2001, vol. 104, 165
- **RAHNER et al.** *GASTROENTEROLOGY,* 2001, vol. 120, 411
- **MORITA et al.** *Proc. Natl. Acad. Sci. USA,* 1999, vol. 96, 511
- *Dev Dyn.,* October 2004, vol. 231 (2), 425-31
- *Am J Physiol Renal Physiol.,* December 2006, vol. 291 (6), F1132-41
- *Int J Cancer.,* 01 November 2014, vol. 135 (9), 2206-14
- *Histopathology,* December 2012, vol. 61 (6), 1043-56
- *J Gastrointest Cancer.,* March 2010, vol. 41 (1), 52-9
- *Oncoimmunology.,* 29 October 2015, vol. 5 (3), e1091555
- **SAMBROOK et al.** Molecular Cloning: A Laboratory Manual. Cold Spring Harbor Laboratory Press, 2001
- Current Protocols in Molecular Biology. 2003
- Methods in Enzymology. Academic Press, Inc,
- PCR 2: A Practical Approach. 1995
- Antibodies, A Laboratory Manual. 1988
- Animal Cell Culture. 1987
- Oligonucleotide Synthesis. 1984

- Methods in Molecular Biology. Humana Press
- Cell Biology: A Laboratory Notebook. Academic Press, 1998
- **J. P. MATHER ; P.E. ROBERTS.** Introduction to Cell and Tissue Culture. Plenum Press, 1998
- Cell and Tissue Culture: Laboratory Procedures. J. Wiley and Sons, 1993
- Handbook of Experimental Immunology
- Gene Transfer Vectors for Mammalian Cells. 1987
- PCR: The Polymerase Chain Reaction. 1994
- Current Protocols in Immunology. 1991
- Short Protocols in Molecular Biology. Wiley and Sons, 1999
- **C.A. JANEWAY ; P. TRAVERS.** Immunobiology. 1997
- **P. FINCH.** *Antibodies,* 1997
- Antibodies: A Practical Approach. IRL Press, 1988
- Monoclonal Antibodies: A Practical Approach. Oxford University Press, 2000
- **E. HARLOW ; D. LANE.** Using Antibodies: A Laboratory Manual. Cold Spring Harbor Laboratory Press, 1999
- The Antibodies. Harwood Academic Publishers, 1995
- Cancer: Principles and Practice of Oncology. J.B. Lippincott Company, 1993
- **KUNKEL et al.** *Proc. Natl. Acad. Sci. USA,* 1985, vol. 82, 488-492
- *Annu Rev. Biophys. Biomol. Struct.,* 2006, vol. 35, 225-249
- *Proc. Natl. Acad. Sci. U.S.A.,* 2003, vol. 100 (11), 6353-6357
- **CHARLTON.** Methods in Molecular Biology. Humana Press, 2003, vol. 248, 245-254
- **GERNGROSS.** *Nat. Biotech.,* 2004, vol. 22, 1409-1414
- **LI et al.** *Nat. Biotech.,* 2006, vol. 24, 210-215
- **GRAHAM et al.** *J. Gen Virol.,* 1977, vol. 36, 59
- **MATHER.** *Biol. Reprod.,* 1980, vol. 23, 243-251
- **MATHER et al.** *Annals N.Y. Acad. Sci.,* 1982, vol. 383, 44-68
- **URLAUB et al.** *Proc. Natl. Acad. Sci. USA,* 1980, vol. 77, 4216
- **YAZAKI ; WU.** Methods in Molecular Biology. Humana Press, 2003, vol. 248, 255-268
- *Journal of Pharmaceutical Sciences,* 2008, vol. 97, 2426-2447
- *Analytical Biochemistry,* 2006, vol. 348, 24-39
- **HASHIMOTO-GOTOH, T. ; MIZUNO, T. ; OGASAHARA, Y. ; NAKAGAWA, M.** An oligodeoxyribonucleotide-directed dual amber method for site-directed mutagenesis. *Gene,* 1995, vol. 152, 271-275
- **ZOLLER, MJ ; SMITH, M.** Oligonucleotide-directed mutagenesis of DNA fragments cloned into M13 vectors. *Methods Enzymol.,* 1983, vol. 100, 468-500
- **KRAMER, W. ; DRUTSA, V. ; JANSEN, HW. ; KRAMER, B. ; PFLUGFELDER, M. ; FRITZ, HJ.** The gapped duplex DNA approach to oligonucleotide-directed mutation construction. *Nucleic Acids Res.,* 1984, vol. 12, 9441-9456
- **KRAMER, W. ; FRITZ, HJ.** Oligonucleotide-directed construction of mutations via gapped duplex DNA Methods. *Enzymol.,* 1987, vol. 154, 350-367
- **KUNKEL, TA.** Rapid and efficient site-specific mutagenesis without phenotypic selection. *Proc Natl Acad Sci USA.,* 1985, vol. 82, 488-492
- **MARK, D. F. et al.** *Proc. Natl. Acad. Sci. USA,* 1984, vol. 81, 5662-6
- **ZOLLER, M. J. ; SMITH, M.** *Nucleic Acids Res.,* 1982, vol. 10, 6487-500
- **WANG, A. et al.** *Science,* 1984, vol. 224, 1431-3
- **DALBADIE-MCFARLAND, G. et al.** *Proc. Natl. Acad. Sci. USA,* 1982, vol. 79, 6409-13
- *Methods in Molecular Biology,* 2012, vol. 911, 65-78
- *Biochimica et Biophysica Acta - Proteins and Proteomics,* 2006, vol. 1764 (8), 1307-1319
- *Journal of Applied Microbiology,* 2014, vol. 117 (2), 528-536
- *Journal of Biomolecular Screening,* 2016, vol. 21 (1), 35-43
- *Journal of Biological Chemistry,* 2016, vol. 291 (24), 12641-12657
- *AIDS,* 2016, vol. 30 (11), 1691-1701
- **KINDT et al.** Kuby Immunology. W.H. Freeman and Co, 2007, 91
- **PORTOLANO et al.** *J. Immunol.,* 1993, vol. 150, 880-887
- **CLARKSON et al.** *Nature,* 1991, vol. 352, 624-628
- **CHOTHIA ; LESK.** *J. Mol. Biol.,* 1987, vol. 196, 901-917
- **KABAT et al.** Sequences of Proteins of Immunological Interest. Public Health Service, National Institutes of Health, 1991
- **MACCALLUM et al.** *J. Mol. Biol.,* 1996, vol. 262, 732-745
- *BMC Research Notes,* 2011, vol. 4, 281
- *Proc. Natl. Acad. Sci. USA,* 2006, vol. 103 (11), 4005-4010
- **CHEN et al.** *J. Mol. Biol.,* 1999, vol. 293, 865-881
- **PRESTA et al.** *Cancer Res.,* 1997, vol. 57, 4593-4599
- **KABAT et al.** Sequences of Proteins of Immunological Interest. 1991
- **HUDSON et al.** *Nat Med,* 2003, vol. 9, 129-134

- **PLUCKTHUN.** The Pharmacology of Monoclonal Antibodies. Springer-Verlag, 1994, vol. 113, 269-315
- **HOLLINGER et al.** *Proc Natl Acad Sci USA,* 1993, vol. 90, 6444-6448
- *Science,* 1988, vol. 240 (4855), 1038-1041
- *Proc. Natl. Acad. Sci. U.S.A.,* 1988, vol. 85 (16), 5879-5883
- *J Immunol. Methods,* 1999, vol. 231 (1-2), 177-189
- *Journal of Immunology,* 1994, vol. 152 (11), 5368-5374
- *Protein Engineering,* 1996, vol. 9 (3), 299-305
- Sequences of proteins of immunological interest
- **DAERON.** *Annu. Rev. Immunol.,* 1997, vol. 15, 203-234
- **RAVETCH ; KINET.** *Annu. Rev. Immunol,* 1991, vol. 9, 457-92
- **CAPEL et al.** *Immunomethods,* 1994, vol. 4, 25-34
- **DE HAAS et al.** *J. Lab. Clin. Med.,* 1995, vol. 126, 330-41
- **GUYER et al.** *J. Immunol.,* 1976, vol. 117, 587
- **KIM et al.** *J. Immunol.,* 1994, vol. 24, 249
- **GHETIE ; WARD.** *Immunol. Today,* 1997, vol. 18 (12), 592-598
- **GHETIE et al.** *Nature Biotechnology,* 1997, vol. 15 (7), 637-640
- **HINTON et al.** *J. Biol. Chem.,* 2004, vol. 279 (8), 6213-6216
- **SHIELDS et al.** *J. Biol. Chem.,* 2001, vol. 9 (2), 6591-6604
- **DAVIS et al.** *Immunological Reviews,* 2002, vol. 190, 123-136
- **RIDGWAY et al.** *Prot Eng,* 1996, vol. 9, 617-621
- **CARTER.** *J Immunol Meth,* 2001, vol. 248, 7-15
- **CARTER.** *J Immunol Methods,* 2001, vol. 248, 7-15
- *Protein Engineering Design & Selection,* 2010, vol. 23, 195-202
- *Nat Biotechnol.,* February 2014, vol. 32 (2), 191-8
- **CHRISTOPH et al.** *Nature Biotechnology,* 2013, vol. 31, 753-758
- **CLYNES et al.** *PNAS (USA),* 1998, vol. 95, 652-656
- **GAZZANO-SANTORO et al.** *J. Immunol. Methods,* 1996, vol. 202, 163
- **IDUSOGIE et al.** *J. Immunol.,* 2000, vol. 164, 4178-4184
- Immunologic studies in humans. Current protocols in Immunology. John Wiley & Sons, Inc, 1993
- Remington's Pharmaceutical Sciences. 1980
- Remington's Pharmaceutical Science. 1980
- *J. Biomed. Mater. Res.,* 1981, vol. 15, 267-277
- *Chemtech.,* 1982, vol. 12, 98-105
- *Biopolymers,* 1983, vol. 22, 547-556
- *CHEMICAL ABSTRACTS,* 1374853-91-4
- *CHEMICAL ABSTRACTS,* 946414-94-4
- *CHEMICAL ABSTRACTS,* 1380723-44-3
- *CHEMICAL ABSTRACTS,* 1537032-82-8
- *CHEMICAL ABSTRACTS,* 1428935-60-7
- *CHEMICAL ABSTRACTS,* 477202-00-9
- *CHEMICAL ABSTRACTS,* 745013-59-6
- *Nat. Rev. Immunol.,* 2012, vol. 12, 339-51
- *CHEMICAL ABSTRACTS,* 934823-49-1
- *CHEMICAL ABSTRACTS,* 1393344-72-3
- *Acta. Cryst.,* 2011, vol. D67, 293-302
- *Acta. Cryst.,* 2010, vol. D66, 125-132
- *Acta. Cryst.,* 2013, vol. D69, 1204-1214
- *J. Appl. Cryst.,* 2007, vol. 40, 658-674
- *Acta Cryst,* 2010, vol. D66, 486-501
- *Acta Cryst.,* 2011, vol. D67, 355-367
- *Acta Cryst.,* 2010, vol. D66, 213-221
- *Scientific Rep.,* 2018, vol. 8, 46960
- **BARCELLOS-HOFF et al.** *J Clin Invest,* February 1994, vol. 93 (2), 892-9
- **BHOLA et al.** *J Clin Invest.,* March 2013, vol. 123 (3), 1348-58